# EUROPEAN PATENT APPLICATION

(11) **EP 3 187 492 A1**
(43) Date of publication of application: **05.07.2017**
(21) Application number: 15836638.5
(22) Date of filing: 28.08.2015
(51) Int. Cl.: C07D 239/47, A61K 31/439, A61K 31/513, A61K 31/5377, A61K 31/541, A61K 31/551

(54) **PYRIMIDINONE DERIVATIVE HAVING AUTOTAXIN-INHIBITORY ACTIVITY**

(30) Priority: 29.08.2014 JP 2014176147; 16.12.2014 JP 2014254314; 28.04.2015 JP 2015091959
(71) Applicant: The University of Tokyo, Tokyo 113-8654 (JP); Tohoku University, Sendai-shi, Miyagi 980-8577 (JP); Shionogi & Co., Ltd, Osaka-shi, Osaka 541-0045 (JP)
(72) Inventor: NAGANO, Tetsuo, Tokyo 113-8654 (JP); OKABE, Takayoshi, Tokyo 113-8654 (JP); KOJIMA, Hirotatsu, Tokyo 113-8654 (JP); KAWAGUCHI, Mitsuyasu, Tokyo 113-8654 (JP); NUREKI, Osamu, Tokyo 113-8654 (JP); ISHITANI, Ryuichiro, Tokyo 113-8654 (JP); NISHIMASU, Hiroshi, Tokyo 113-8654 (JP); AOKI, Junken, Sendai-shi Miyagi 980-8577 (JP); TANAKA, Nobuyuki, Toyonaka-shi Osaka 561-0825 (JP); KANDA, Yasuhiko, Toyonaka-shi Osaka 561-0825 (JP); KIOI, Yoshiyuki, Toyonaka-shi Osaka 561-0825 (JP); TATENO, Yusuke, Toyonaka-shi Osaka 561-0825 (JP); KIDA, Shiro, Toyonaka-shi Osaka 561-0825 (JP); YAMANE, Junji, Toyonaka-shi Osaka 561-0825 (JP); WADA, Toshihiro, Toyonaka-shi Osaka 561-0825 (JP)
(74) Representative: Vossius & Partner Patentanwälte Rechtsanwälte mbB
(86) International application number: PCT/JP2015/074522
(87) International publication number: WO 2016/031987

(57) **Abstract**

A. compound according to any one of formula (Ia) to (Ic) or (II), or its pha rmaceutically acceptable salt: 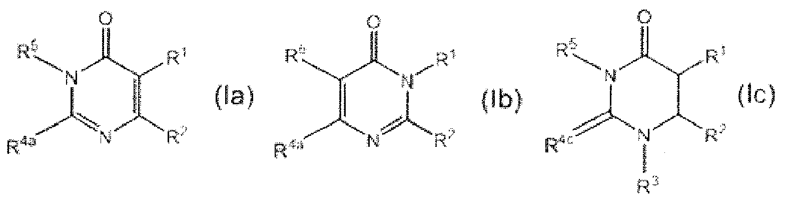 or 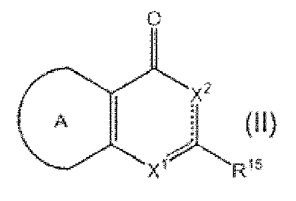 wherein R¹, R², R³, R^{4a}, R^{4c}, R⁵, R¹⁵, ring A, X¹ and X² are as defined in t he description.

## Description

### Technical Field

The present invention provides pyrimidinone derivatives having autotaxin inhibitory activity, and a pharmaceutical composition comprising said pyrimidinone derivatives as an active ingredient.

### Background Art

Lysophosphatidic acid (LPA) is a lipid mediator that exhibits a variety of effects, such as cell proliferation, intracellular calcium influx, cytoskeletal changes, cell migration, via signal transduction through G protein-coupled receptor expressed on cell surface (LPA1-6). It has been reported that the lipid is involved in abnormalities of living body, such as fibrosis, pain, cancer, inflammation, arteriosclerosis (Non-Patent Document 1).

LPA can be biosynthesized by several metabolic pathways, primarily via hydrolysisof lysophosphatidylcholine by autotaxin (ENPP2, ATX). ATX is a secreted protein of ENPP (Ectonucleotide pyrophosphatase and phosphodiesterase) family (ENPP1-7) and referred to as ENPP2. ATX is the only one of this family that has a lysophospholipase D activity and thus is involved in LPA production. It has been reported that inhibiting the enzyme activity of ATX to inhibit LPA production is effective in the treatment of fibrotic diseases (Non-patent Document 1).

Fibrosis can occur in any organ, and the mechanism of its progression is common regardless of the trigger involved.

Animal tissues and organs maintain its structure with fibers such as collagen, and injured tissues and orgens are restored to the original condition through the process of wound healing with collagen production. However, in case where the tissue receives immunological, chemical, mechanical, metabolic or other injuries repeatedly or experiences a greater degree of injury, excessive accumulation of fibrous connective tissue may occur. Accumulation of such connective tissue is irreversible, and fibers abnormally increased cause fibrosis that is associated with dysfunction of tissues and organs.

Pathological feature of chronic kidney disease includes renal glomerular fibrosis and tubulointerstitial fibrosis. Dropout and fibrosis of parenchymal cells prevail in the pathology of end-stage renal failure. In chronic kidney disease patients having tubulointerstitial fibrosis, the progress to renal failure is faster as compared to chronic kidney disease patients without such fibrosis.

For preventing and treating chronic kidney disease, treatments with an antihypertensive drug, such as angiotensin receptor antagonists and calcium antagonists, have been practiced, as well as advice on daily living and dietary. However, the effect from such conventional treatments is not enough to be satisfied, and there still exists an ongoing need for new drugs to make prevention and treatment more effective.

Patent Documents 1 and 2 disclose pyrimidinone derivaties that are glucocortinoid modulators.

Patent Document 3 discloses pyrimidinone derivatives that are anti-HIV agents.

Patent Document 4 discloses pyrimidinone derivatives that are a calcium receptor inhibitor.

Patent Documents 5 and 6 disclose pyrimidinone derivatives that are an endothelin receptor inhibitor.

Patent Document 7 discloses pyrimidinone derivatives that are an aldose reductase inhibitor.

Non-Patent Document 2 discloses pyrimidinone derivatives having immunosuppressive activity.

Patent Document 8 disclosed dihydroprinon derivatives that are phosphodiesterase.

Patent Documents 9-11 disclose 2- (benzimidazole-yl) purinone derivatives as immunosuppressive agents.

Non-Patent Documents 3-5 disclose dihydropurinone derivatives.

Non-Patent Document 6 disclose tetrahydropyridinopyrimidine derivatives.

However, it is not described or suggested that such compounds inhibit autotaxin or may be a therapeutic agent for chronic kidney disease.

On the other hand, Patent Documents 12 and 13 discloses imidazopyridinone derivatives having an inhibitory activity on autotaxin.

### Prior Art Documents

### Patent Documents

[Patent Document 1] WO2012/129074
[Patent Document 2] US Patent Application Publication 2006/025405
[Patent Document 3] WO2011/15641
[Patent Document 4] WO2007/62370
[Patent Document 5] WO2003/39539
[Patent Document 6] German Patent Application Publication 19612101.
[Patent Document 7] US Patent Publication 4786638
[Patent Document 8] European Patent Application Publication 1092718
[Patent Document 9] US Patent Application Publication 2009/0069289
[Patent Document 10] WO2010/0022358
[Patent Document 11] WO2008/043031
[Patent Document 12] WO2014/133112
[Patent Document 13] WO2015/064714

### Non-Patent Documents

[Non-Patent Document 1] Nature, vol.411, pp.494-498 (2001)
[Non-patent Document 2] Bioorganic&Medicinal Chemistry, vol.21, issue: 4, pp.1209-1218, 2013
[Non-patent Document 3] Journal de la Societe Chimique de Tunisie, Volume: 2, Issue: 8, Page: 13-17, Journal, 1988
[Non-patent Document 4] Journal de la Societe Chimique de Tunisie, Volume: 2, Issue: 2, Pages: 15-21, Journal, 1985
[Non-patent Document 5] Journal of the American Chemical Society, Volume: 135, Issue: 9, Pages: 3423-3438
[Non-patent Document 6] Journal of the Chemical Society, Pages: 399-402, Journal, 1945

### Summary of Invention

### Problem to be solved by the Invention

The object of the present invention is to provide fused pyrazole derivatives having an excellent inhibitory activity on autotaxin.

### Means for Solving the Problem

The present invention is based on the inventor's discovery of the fused pyrazole derivatives having an excellent inhibitory activity on autotaxin.

The present invention relates to the following.
(1) A compound according to any one of formula (Ia) to (Ic) or (II), or its pharmaceutically acceptable salt: 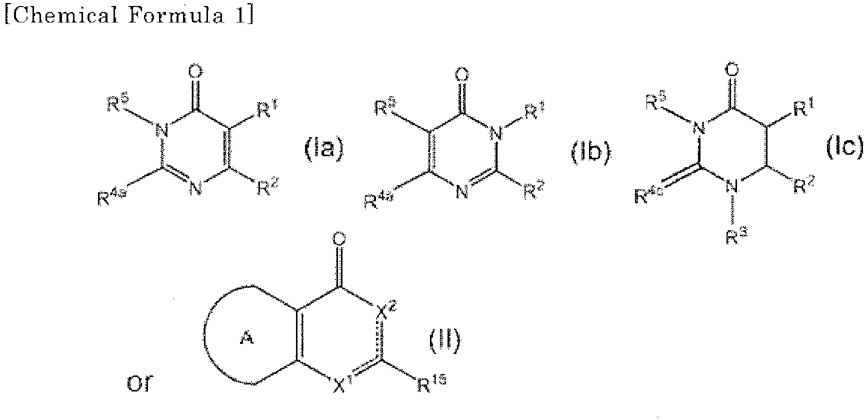 wherein
   R¹ is hydrogen, halogen, hydroxy, formyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl;
   R² is a group represented by formula^{:} 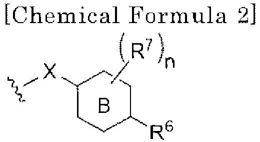
      X is a single bond or O,
      Ring B is a benzene ring, 6-membered aromatic heterocycle, non-aromatic carbocycle or 6-membered non-aromatic heterocycle,
      R⁶ is halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
      R⁷ are each independently, halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl , substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl , substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
      n is integer of 0 to 4,
   R³ is substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkylsulfonyl, substituted or unsubstituted alkenylsulfonyl, substituted or unsubstituted alkynylsulfonyl, substituted or unsubstituted aromatic carbocyclylsulfonyl, substituted or unsubstituted aromatic heterocyclylsulfonyl, substituted or unsubstituted non-aromatic carbocyclylsulfonyl or substituted or unsubstituted non-aromatic heterocyclylsulfonyl,
   R^{4a} is halogen, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted substituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclyl, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, a substituted or unsubstituted alkylsulfinyl, substituted or unsubstituted alkenylsulfinyl substituted, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, non-aromatic heterocyclylsulfonyl or substituted or unsubstituted aromatic heterocyclylsulfonyl,
   R^{4c} is O or N (R⁹),
   R⁹ is hydrogen, substituted or unsubstituted alkyl or substituted or unsubstituted alkyloxy,
   R⁵ is a group represented by formula: R¹¹-(C(R^{10a})(R^{10b}))ₘ-
      wherein
      R^{10a} are each independently hydrogen, halogen or substituted or unsubstituted alkyl,
      R^{10b} are each independently hydrogen, halogen or substituted or unsubstituted alkyl, or
      R^{10a} and R^{10b} attached to the same carbon may be taken together to form substituted or unsubstituted carbocycle or substituted or unsubstituted heterocycle,
      m is integer of 1 to 6,
      R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, substituted or unsubstituted sulfamoyl, or substituted or unsubstituted amino,
   the one of the two broken lines represent the existence and the other represents non-existence,
   R¹⁵ is substituted or unsubstituted aromatic carbocyclyl, substituted or unsubstituted aromatic heterocyclyl, substituted or unsubstituted non-aromatic carbocyclyl or substituted or unsubstituted non-aromatic heterocyclyl,
   the partial structure represented by formula: 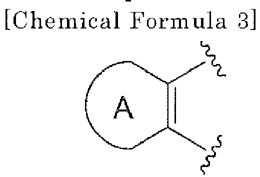 is 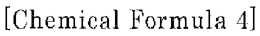 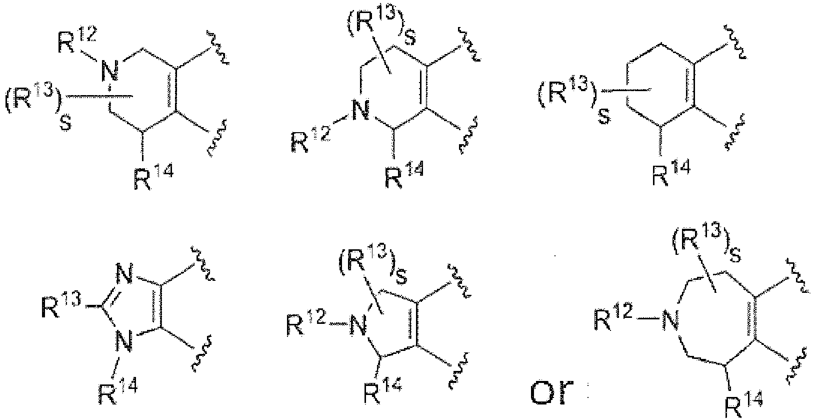
   R¹² is hydrogen, substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl or substituted or unsubstituted sulfamoyl,
   R¹³ is each independently hydroxy, halogen, cyano, substituted or unsubstituted alkyl, substituted or unsubstituted alkenyl or substituted or unsubstituted alkynyl,
   two R¹³ attached to the same carbon atom may be taken together to form oxo, non-aromatic carbocycle or substituted or unsubstituted aromatic heterocycle or substituted or unsubstituted non-aromatic heterocycle, or
   two R¹³ attached to the different carbon atom may be taken together to form non-aromatic carbocycle, or substituted or unsubstituted non-aromatic heterocycle,
   R¹⁴ is C1-C2 alkyl substituted with one or more substituents selected from Substituent group A consisting of substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted substituted alkynylthio, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio and substituted or unsubstituted amino, substituted or unsubstituted C3-C10 alkyl, substituted or unsubstituted C3-C10 alkenyl, substituted or unsubstituted C3-C10 alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkyloxy, substituted or unsubstituted alkenyloxy, substituted or unsubstituted alkynyloxy or a substituted or unsubstituted amino,
      s is integer of 0 to 4,

   (b-1) when the partial structure represented by formula: 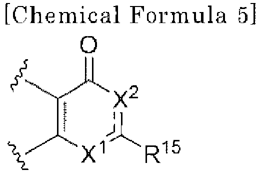 is a partial structure represented by formula: 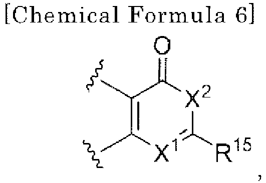
      X¹ is N or C (R¹⁶),
      R¹⁶ is hydrogen, halogen, substituted or unsubstituted alkyl, substituted or unsubstituted alkenyl or substituted or unsubstituted alkynyl,
      X² is N (R¹⁷) or C (R¹⁸) (R¹⁹),
      R¹⁷ is hydrogen, substituted or unsubstituted alkyl, substituted or unsubstituted alkenyl or substituted or unsubstituted alkynyl,
      R¹⁸ and R¹⁹ are each independently hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy or a substituted or unsubstituted amino,
   (b-2) when the partial structure represented by formula: 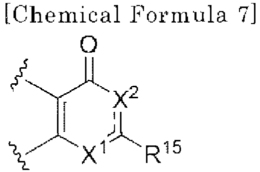 is a partial structure represented by formula: 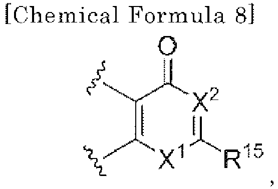
      X¹ is O,
      X² is N or C (R²⁰),
      R²⁰ is hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or substituted or unsubstituted alkynyloxy or a substituted or unsubstituted amino.
      provided that the following compounds (i) to (v) are excluded.
         (i) the compounds represented by formula(Ib), wherein (a) R¹ is hydrogen and R¹¹ is carboxy, unsubstituted methyloxycarbonyl or unsubstituted ethyloxycarbonyl, and the compounds wherein (b) R¹ is hydrogen, R^{4a} is unsubstituted methyloxymethyl or unsubstituted methyl, and R¹¹ is a substituted or unsubstituted phenyl,
         (ii) the compounds represented by formula(Ic), wherein (a) R² is unsubstituted methylphenyl, and R³ is unsubstituted methylthiophenyl, and the compounds wherein (b) R³ is unsubstituted methyl, and R¹¹ is piperazinyl substituted with unsubstituted naphthyl or unsubstituted isoquinolinyl,
         (iii) the compounds represented by (iii) (Ia), wherein R¹ is hydrogen or cyano, R² is p⁻ unsubstituted methyloxyphenyl, and R^{4a} is unsubstituted methyl,
         (iv) the compounds represented by formula(II), wherein the partial structure represented by formula: 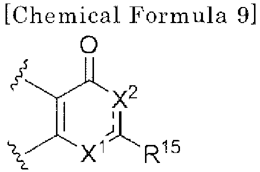 is a partial structure represented by formula: 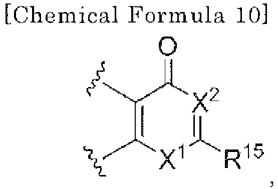
            X³ is N,
            X² is N(R¹⁷),
            R¹⁷ is hydrogen,
            R¹⁵ is substituted or unsubstituted phenyl or substituted or unsubstituted pyridyl, the partial structure represented by formula: 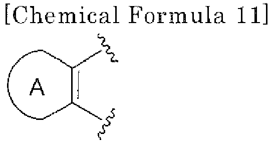 is a partial structure represented by formula: 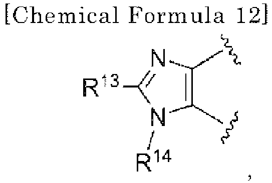
               R¹³ is unsubstituted alkyl or substituted or unsubstituted phenylmethyl, and
               R¹⁴ is unsubstituted propyl or unsubstituted phenylamino,
         (v) the compounds represented as follows: 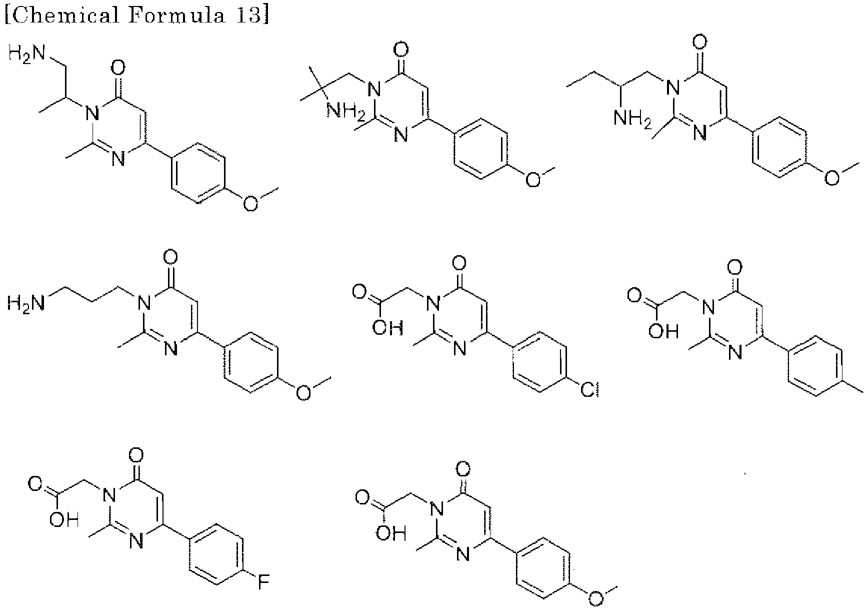 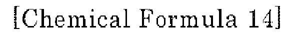 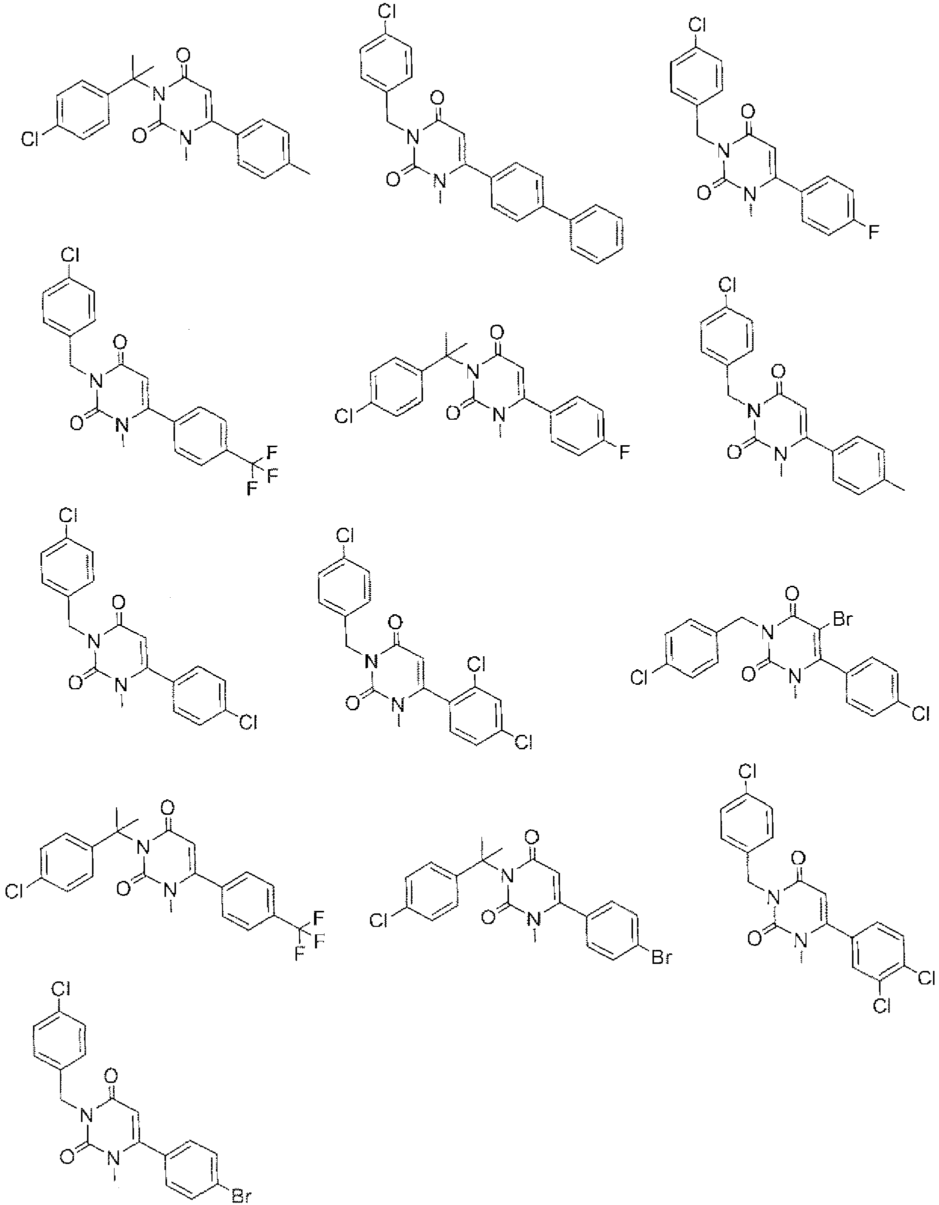 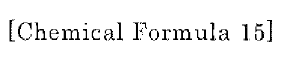 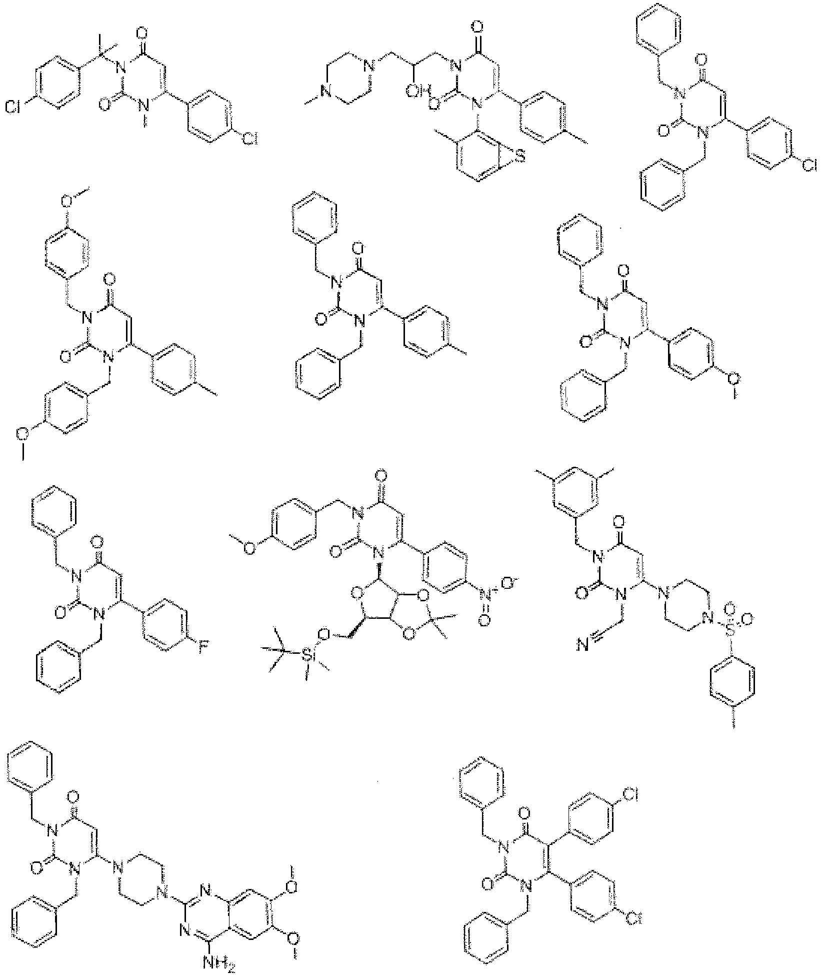 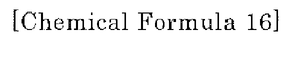 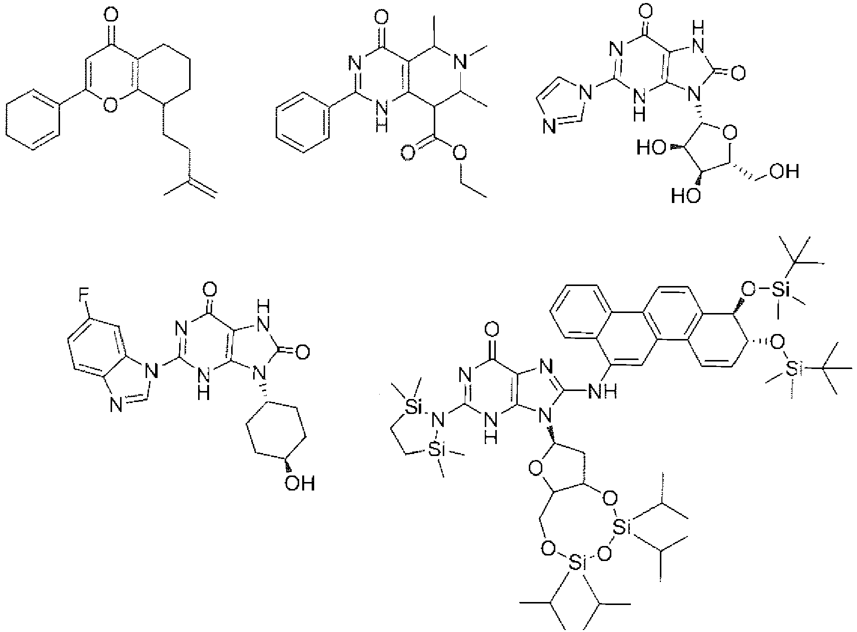
(2) The compound according to the above (I), represented by formula (Ia), or its pharmaceutically acceptable salt.
(3) The compound according to the above (1), represented by formula (Ib), or its pharmaceutically acceptable salt.
(4) The compound according to the above (1), represented by formula (Ic), or its pharmaceutically acceptable salt thereof.
(5) The compound according to the above (2), wherein R¹ is hydrogen or halogen, or its pharmaceutically acceptable salt.
(6) The compound according to the above (2) or (5), wherein R^{4a} is substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, or substituted or unsubstituted alkynylthio, its pharmaceutically acceptable salt.
(7) The compound according to the above (6), wherein R^{4a} is alkyl optionally substituted with one or more substitutent(s) selected from the Substituent group α (cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy, substituted or unsubstituted non-aromatic heterocyclylalkyloxy and substituted or unsubstituted aromatic heterocyclylalkyloxy), alkenyl optionally substituted with one or more substitutent(s) selected from the Substituent group α, alkynyl optionally substituted with one or more substitutent(s) selected from the Substituent group α, alkylamino optionally substituted with one or more substitutent(s) selected from the Substituent group α, alkenylamino optionally substituted with one or more substitutent(s) selected from the Substituent group α, alkenylamino optionally substituted with one or more substitutent(s) selected from the Substituent group α, alkyloxy optionally substituted with one or more substitutent(s) selected from the Substituent group α, alkenyloxy optionally substituted with one or more substitutent(s) selected from the Substituent group α, alkynyloxy optionally substituted with one or more substitutent(s) selected from the Substituent group α, alkylthio optionally substituted with one or more substitutent(s) selected from the Substituent group α, alkenylthio optionally substituted with one or more substitutent(s) selected from the Substituent group α, alkynylthio optionally substituted with one or more substitutent(s) selected from the Substituent group α, its pharmaceutically acceptable salt.
(8) The compound according to any one of the above (2) to (7), wherein R¹¹ is carboxy, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted non-aromatic heterocyclyloxycarbonyl, substituted or unsubstituted carbamoyl or substituted or unsubstituted sulfamoyl, its pharmaceutically acceptable salt.
(9) The compound according to the above (8), wherein R¹¹ is substituted or unsubstituted aromatic carbocyclyl, substituted or unsubstituted aromatic heterocyclyl, substituted or unsubstituted non-aromatic carbocyclyl, or substituted or unsubstituted non-aromatic heterocyclyl, or its pharmaceutically acceptable salt.
(10) The compound according to the above (9), wherein R¹¹ is aromatic carbocyclyl substituted with one or more substitutent(s) selected from the Substituent group β (carboxy, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclycarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, a substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, and substituted or unsubstituted aromatic carbocyclylsulfonyl), aromatic heterocyclyl substituted with one or more substitutent(s) selected from the Substituent group β, non-aromatic carbocyclyl substituted with one or more substitutent(s) selected from the Substituent group β, or non-aromatic heterocyclyl substituted with one or more substitutent(s) selected from the Substituent group β, or its pharmaceutically acceptable salt.
(11) The compound according to the above (9), wherein R¹¹ is substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted non-aromatic heterocyclyloxycarbonyl, substituted or unsubstituted carbamoyl or substituted or unsubstituted sulfamoyl, or its pharmaceutically acceptable salt.
(12) The compound according to the above (9), wherein R¹¹ is carbamoyl optionally substituted with one or more substitutent(s) selected from the Substituent group γ (substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl and substituted or unsubstituted non-aromatic heterocyclyl), or its pharmaceutically acceptable salt.
(13) The compound according to the above (3), wherein R¹ is hydrogen, R^{4a} is substituted or unsubstituted C2-C10 alkyl, substituted or unsubstituted alkenyl or substituted or unsubstituted alkynyl, or its pharmaceutically acceptable salt.
(14) The compound according to the above (5), wherein R¹ is hydrogen, R³ is substituted methyl, substituted or unsubstituted C2-C10 alkyl, substituted or unsubstituted alkenyl or substituted or unsubstituted alkynyl, or its pharmaceutically acceptable salt.
(15) The compound according to the above (1), represented by formula (II), or its pharmaceutically acceptable salt.
(16) The compound according to the above (15), wherein R¹⁵ is substituted aromatic carbocyclyl, substituted aromatic heterocyclyl, substituted non-aromatic carbocyclyl or substituted non-aromatic heterocyclyl,
   the partial structure represented by formula: 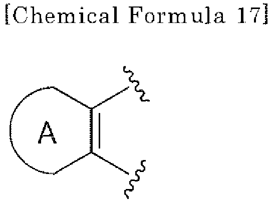 is a partial structure represented by formula: 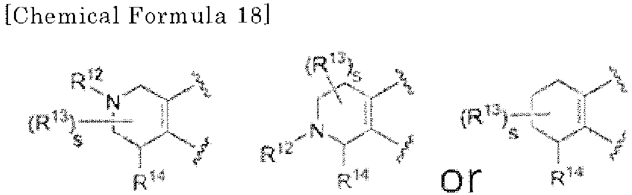 wherein each symbols is as defined above,
   or its pharmaceutically acceptable salt.
(17) The compound according to the above (16), wherein the partial structure represented by formula: 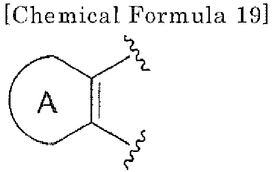 is a partial structure represented by formula: 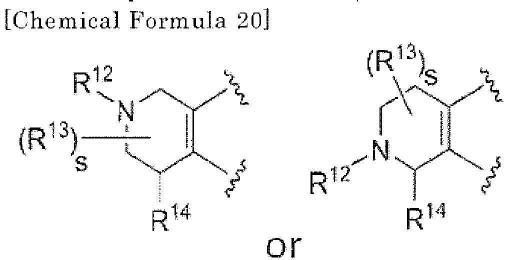 wherein each symbols is as defined above,
   or its pharmaceutically acceptable salt.
(18) The compound according to any one of the above (15) to (17), wherein R¹² is substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl or substituted or unsubstituted carbamoyl, or its pharmaceutically acceptable salt.
(19) The compound according to any one of the above (15) to (17), wherein R¹² is substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl or substituted or unsubstituted sulfamoyl, or its pharmaceutically acceptable salt.
(20) The compound according to any one of the above (15) to (19), wherein R¹³ is each independently, hydroxy, halogen, cyano, substituted or unsubstituted alkyl, substituted or unsubstituted alkenyl, substituted or unsubstituted carbamoyl or substituted or unsubstituted alkynyl, or its pharmaceutically acceptable salt.
(21) The compound according to the above (20), wherein s is integer of 1 to 4, or its pharmaceutically acceptable salt.
(22) The compound according to any one of the above (15) to (21), wherein R¹⁴ is C1-C2 alkyl substituted with one or more substituents selected from Substituent Group A, substituted or unsubstituted C3-C10 alkyl, substituted or unsubstituted C3-C10 alkenyl or substituted or unsubstituted C3-C10 alkynyl, or its pharmaceutically acceptable salt.
(23) The compound according to any one of the above (15) to (21), wherein R14 is substituted or unsubstituted C3-C10 alkyl, substituted or unsubstituted C3-C10 alkenyl or substituted or unsubstituted C3-C10 alkynyl, or its pharmaceutically acceptable salt.
(24) The compound according to any one of the above (15) to (23), wherein the partial structure represented by formula: 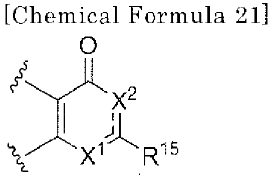 is a partial structure represented by formula: 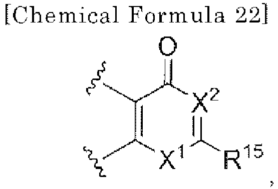 wherein each symbols is as defined above.
   or its pharmaceutically acceptable salt.
(25) The compound according to the above (24), wherein wherein X² is N (R¹⁶), R¹⁶ is hydrogen, substituted or unsubstituted alkyl, substituted or unsubstituted alkenyl or substituted or unsubstituted alkynyl, or its pharmaceutically acceptable salt.
(26) A pharmaceutical composition comprising the compound according to any one of the above (1) to (25) or its pharmaceutically acceptable salt as an active ingredient.
(27) The pharmaceutical composition according to the above (26), which is an autotaxin inhibitor.
(28) A method for the prevention or treatment of a disease related to autotaxin, which is characterized by administering the compound according to any one of (1) to (25) or its pharmaceutically acceptable salt.
(29) The compound according to any one of (1) to (25) or its pharmaceutically acceptable salt for the prevention or treatment of a disease relatred to autotaxin.
(29-1) Use of the compound according to any one of (1) to (25) or its pharmaceutically acceptable salt for the manufacture of a medicament for the prevention or treatment of a disease related to autotaxin.
(30) An autotaxin inhibitor comprising the compound of formula (II') or its pharmaceutically acceptable salt: 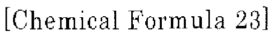 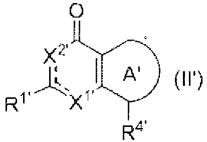
   R^{1'} is substituted or unsubstituted aromatic carbocyclyl, substituted or unsubstituted aromatic heterocyclyl, substituted or unsubstituted non-aromatic carbocyclyl or substituted or unsubstituted non-aromatic heterocyclyl,
   Ring A' is substituted or unsubstituted non-aromatic heterocycle, substituted or unsubstituted aromatic heterocycle substituted or unsubstituted non-aromatic carbocycle or substituted or unsubstituted aromatic carbocycle,
   R^{4'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkyloxy, substituted or unsubstituted alkenyloxy, substituted or unsubstituted alkynyloxy or a substituted or unsubstituted amino,
      (i) when the partial structure represented by formula: 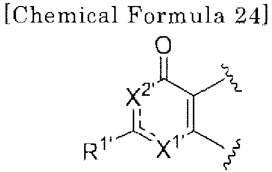 is a partial structure represented by formula: 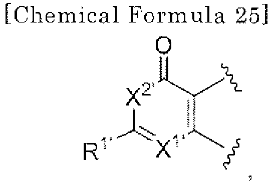
         X^{1'} is N or C (R^{5'}),
         R^{5'} is hydrogen, halogen, substituted or unsubstituted alkyl, substituted or unsubstituted alkenyl or substituted or unsubstituted alkynyl,
         X^{2'} is N (R^{6'}) or C (R^{7'})(R^{8'}),
         R^{6'} is hydrogen, substituted or unsubstituted alkyl, substituted or unsubstituted alkenyl or substituted or unsubstituted alkynyl, and
         R^{7'} and R^{8'} are each independently hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy or a substituted or unsubstituted amino,
      (ii) when the partial structure represented by formula: 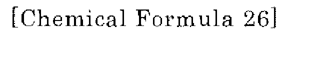 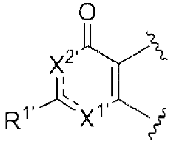 is a partial structure represented by formula: 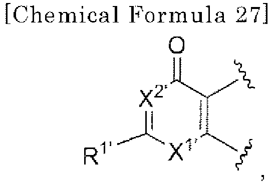
         X^{1'} is O,
         X^{2'} is N or C (R^{9'}), and
         R^{9'} is hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy or a substituted or unsubstituted amino. or its pharmaceutically acceptable salt.

(1') An autotaxin inhibitor comprising the compound according to any one of formula (Ia) to (Ic) or its pharmaceutically acceptable salt: 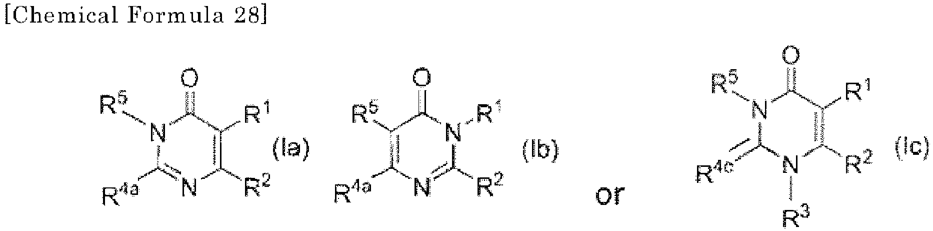
   R¹ is hydrogen, halogen, hydroxy, cyano, formyl, carboxy, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl;
   R², R³ and R^{4a} are each independently hydrogen, halogen, hydroxy, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfinyl (provided that at least one of R¹, R² and R^{4a} is not hydrogen);
   R^{4c} is O or N (R⁹),
   R⁹ is hydrogen, substituted or unsubstituted alkyl or substituted or unsubstituted alkyloxy,
   R⁵ is a group represented by formula: R¹¹-(C(R^{10a})(R^{10b}))ₘ-
   wherein R^{70a} and R^{10b} are each independently hydrogen, halogen or substituted or unsubstituted alkyl, or R^{10a} and R^{10b} attached to the same carbon may be taken together to form substituted or unsubstituted carbocycle or substituted or unsubstituted heterocycle,
   m is integer of 1 to 6, and
   R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, or substituted or unsubstituted sulfamoyl.
   (1") An autotaxin inhibitor comprising the compound according to any one of formula (Ia) to (Ic), or its pharmaceutically acceptable salt: 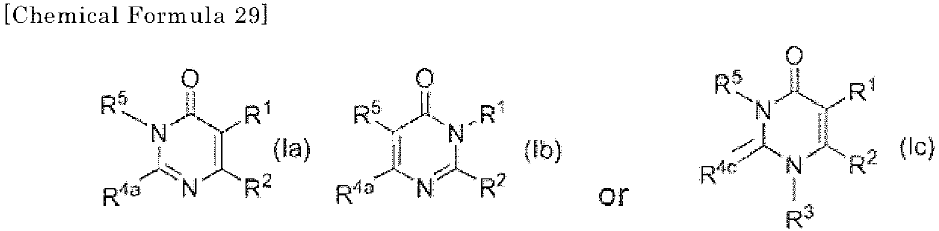
   R¹ is hydrogen, halogen, hydroxy, cyano, formyl, carboxy, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl;
   R², R³ and R^{4a} are each independently hydrogen, halogen, hydroxy, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfinyl (provided that at least one of R¹, R² and R^{4a} is not hydrogen);
   R^{4c} is O or N (R⁹),
   R⁹ is hydrogen, substituted or unsubstituted alkyl or substituted or unsubstituted alkyloxy,
   R⁵ is a group represented by formula: R¹¹ - (C(R^{10a}) (R^{10b})) ₘ-
   wherein R^{10a} are each independently hydrogen, halogen or substituted or unsubstituted alkyl,
   R^{10b} are each independently hydrogen, halogen or substituted or unsubstituted alkyl, or R^{10a} and R^{10b} attached to the same carbon may be taken together to form substituted or unsubstituted carbocycle or a substituted or unsubstituted heterocycle,
   m is integer of 1 to 6,
   R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, non-aromatic substituted or unsubstituted heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl, substituted or unsubstituted carbamoyl, substituted or unsubstituted sulfamoyl, or substituted or unsubstituted amino.
(2') The autotaxin inhibitor comprising the compound according to the above (1') to (1"), wherein R² is substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted aromatic carbocyclyloxy, substituted or unsubstituted aromatic heterocyclyloxy, substituted or unsubstituted non-aromatic carbocyclyloxy, non-aromatic substituted or unsubstituted heterocyclyloxy, substituted or unsubstituted aromatic carbocyclylamino, substituted or unsubstituted aromatic heterocyclylamino, substituted or unsubstituted non-aromatic carbocyclylamino or substituted or unsubstituted non-aromatic heterocyclylamino, its pharmaceutically acceptable salt.
(3') The compound according to any one of formula (Ia) to (Ic), or its pharmaceutically acceptable salt: 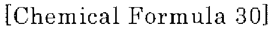 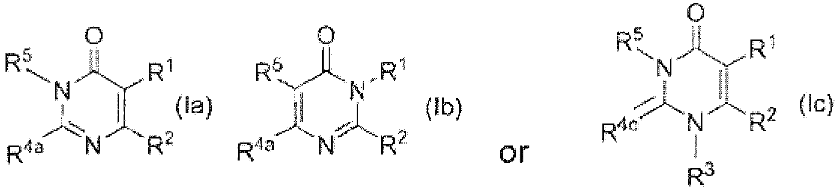
   wherein R¹ is hydrogen, halogen, hydroxy, formyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl;
   R² is a group represented by formula: 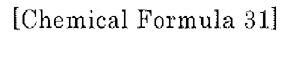 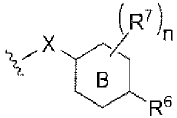
      X is a single bond or O,
      Ring B is a benzene ring, a 6-membered aromatic heterocycle, a non-aromatic carbocycle or 6-membered non-aromatic heterocycle,
      R⁶ is halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
      R⁷ are each independently, halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl , substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl , substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
      n is integer of 0 to 4,
   R³ is substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkylsulfonyl, substituted or unsubstituted alkenylsulfonyl, substituted or unsubstituted alkynylsulfonyl, substituted or unsubstituted aromatic carbocyclylsulfonyl, substituted or unsubstituted aromatic heterocyclylsulfonyl, substituted or unsubstituted non-aromatic carbocyclylsulfonyl or substituted or unsubstituted non-aromatic heterocyclylsulfonyl,
   R^{4a} is halogen, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted substituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclyl, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, a substituted or unsubstituted alkylsulfinyl, substituted or unsubstituted alkenylsulfinyl substituted, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, non-aromatic heterocyclylsulfonyl or substituted or unsubstituted aromatic heterocyclylsulfonyl,
   R^{4c} is O or N (R⁹),
   R⁹ is hydrogen, substituted or unsubstituted alkyl or substituted or unsubstituted alkyloxy,
   R⁵ is a group represented by formula: R¹¹-(C(R^{10a})(R^{10b}))m-
      wherein
      R^{10a} are each independently hydrogen, halogen or substituted or unsubstituted alkyl,
      R^{10b} are each independently hydrogen, halogen or substituted or unsubstituted alkyl, or
      R^{10a} and R^{10b} attached to the same carbon may be taken together to form substituted or unsubstituted carbocycle or a substituted or unsubstituted heterocycle,
      m is integer of 1 to 6,
      R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, non-aromatic substituted or unsubstituted heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, or substituted or unsubstituted sulfamoyl,
      provided that the following compounds (i) to (iii) are excluded.
         (i) the compounds represented by formula(Ib), wherein (a) R¹ is hydrogen and R¹¹ is carboxy, unsubstituted methyloxycarbonyl or unsubstituted ethyloxycarbonyl, and the compounds wherein (b) R¹ is hydrogen, R^{4a} is unsubstituted methyloxymethyl or unsubstituted methyl, and R¹¹ is substituted or unsubstituted phenyl,
         (ii) the compounds represented by formula(Ic), wherein (a) R² is methylphenyl, and R³ is methylthiophenyl, and the compounds wherein (b) R³ is methyl, and R¹¹ is piperazinyl substituted with naphthyl or isoquinolinyl,
         (iii) the compounds represented as follows: 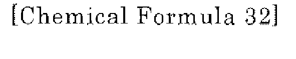 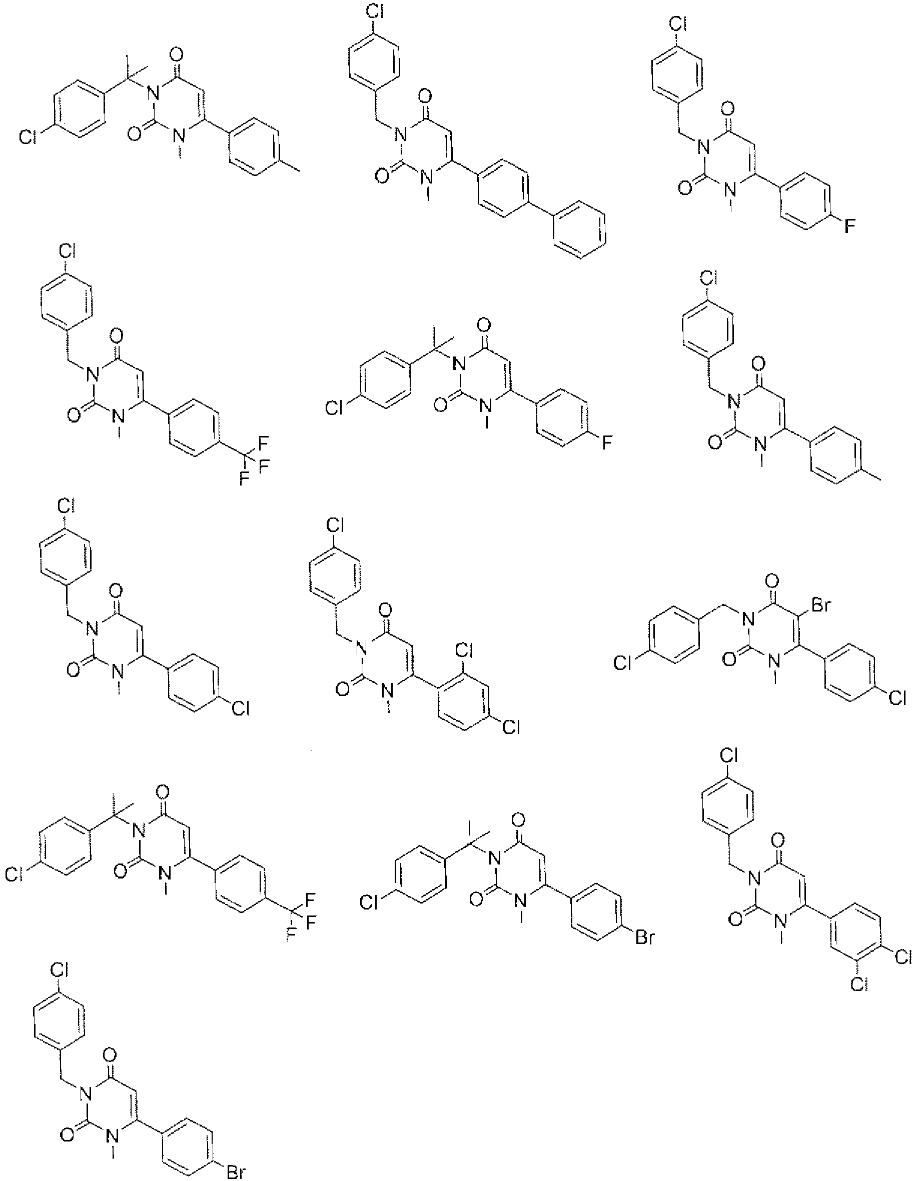 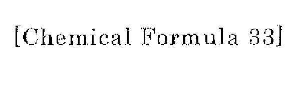 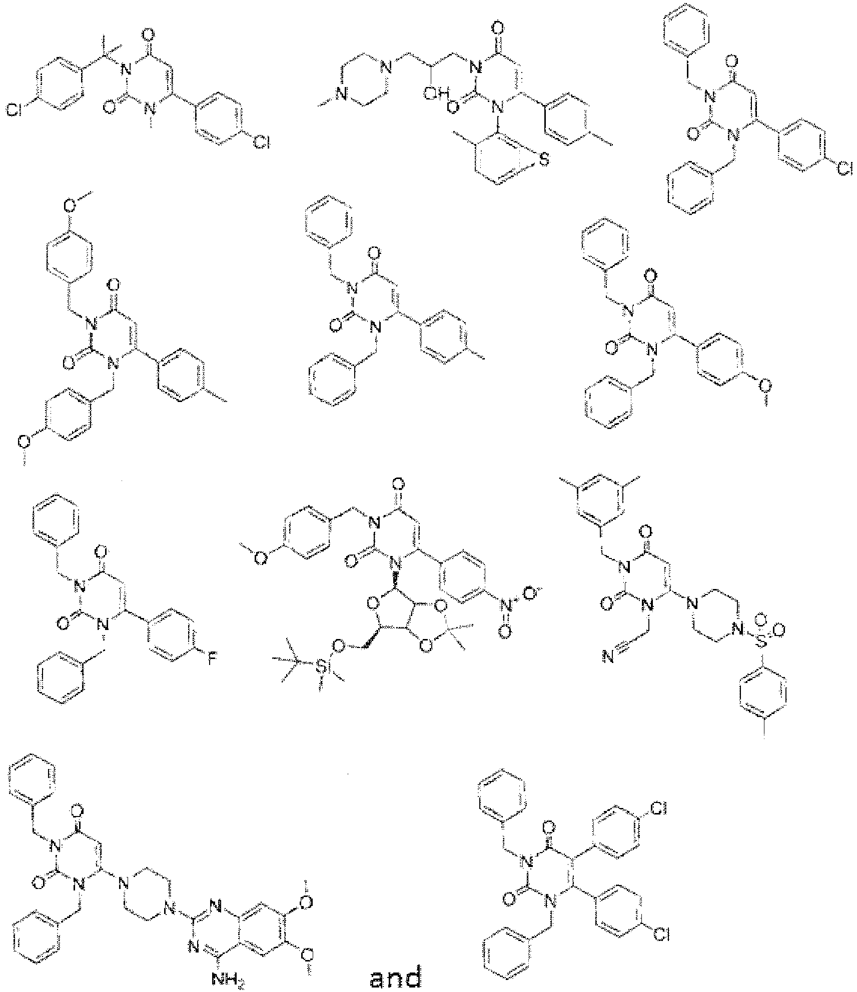 (3") A compound according to any one of formula (Ia) to (Ic), or its pharmaceutically acceptable salt: 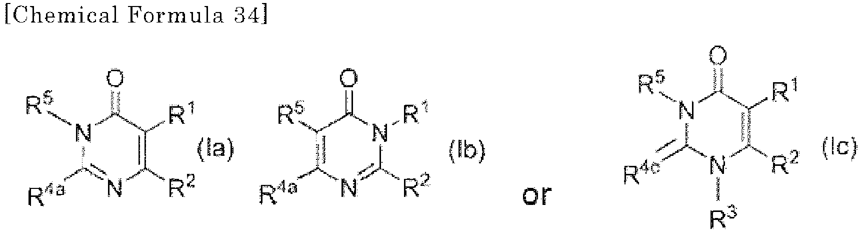
            wherein R¹ is hydrogen, halogen, hydroxy, formyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl;
            R² is a group represented by formula: 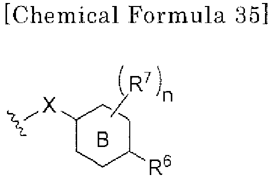
               X is a single bond or O,
               Ring B is a benzene ring, a 6-membered aromatic heterocycle, a non-aromatic carbocyclic or 6-membered non-aromatic heterocycle,
               R⁶ is halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
               R⁷ are each independently, halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl , substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl , substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
               n is integer of 0 to 4.,
            R³ is substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkylsulfonyl, substituted or unsubstituted alkenylsulfonyl, substituted or unsubstituted alkynylsulfonyl, substituted or unsubstituted aromatic carbocyclylsulfonyl, substituted or unsubstituted aromatic heterocyclylsulfonyl, substituted or unsubstituted non-aromatic carbocyclylsulfonyl or substituted or unsubstituted non-aromatic heterocyclylsulfonyl,
            R^{4a} is halogen, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted substituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclyl, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, a substituted or unsubstituted alkylsulfinyl, substituted or unsubstituted alkenylsulfinyl substituted, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, non-aromatic heterocyclylsulfonyl or substituted or unsubstituted aromatic heterocyclylsulfonyl,
            R^{4c} is O or N (R⁹),
            R⁹ is hydrogen, substituted or unsubstituted alkyl or substituted or unsubstituted alkyloxy,
            R⁵ is a group represented by formula: R¹¹-(C(R^{10a})(R^{10b}))m-
               wherein
               R^{10a} are each independently hydrogen, halogen or substituted or unsubstituted alkyl,
               R^{10b} are each independently hydrogen, halogen or substituted or unsubstituted alkyl, or
               R^{10a} and R^{10b} attached to the same carbon may be taken together to form substituted or unsubstituted carbocycle or a substituted or unsubstituted heterocycle,
               m is integer of 1 to 6,
               R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, non-aromatic substituted or unsubstituted heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, substituted or unsubstituted sulfamoyl or substituted or unsubstituted sulfamoyl,
            provided that the following compounds (i) to (iii) are excluded.
            (i) the compounds represented by formula(Ib), wherein (a) R¹ is hydrogen and R¹¹ is carboxy, unsubstituted methyloxycarbonyl or unsubstituted ethyloxycarbonyl, and the compounds wherein (b) R¹ is hydrogen, R^{4a} is unsubstituted methyloxymethyl or unsubstituted methyl, and R¹¹ is a substituted or unsubstituted phenyl,
            (ii) the compounds represented by formula(Ic), wherein (a) R² is unsubstituted methylphenyl, and R³ is methylthiophenyl, and the compounds wherein (b) R³ is unsubstituted methyl, and R¹¹ is piperazinyl substituted with unsubstituted naphthyl or unsubstituted isoquinolinyl,
            (iii) the compounds wherein represented by (iii) (Ia), wherein R¹ is hydrogen or cyano, R² is p⁻ unsubstituted methyloxyphenyl, and R^{4a} is unsubstituted methyl,
            (iv) the compounds represented as follows: 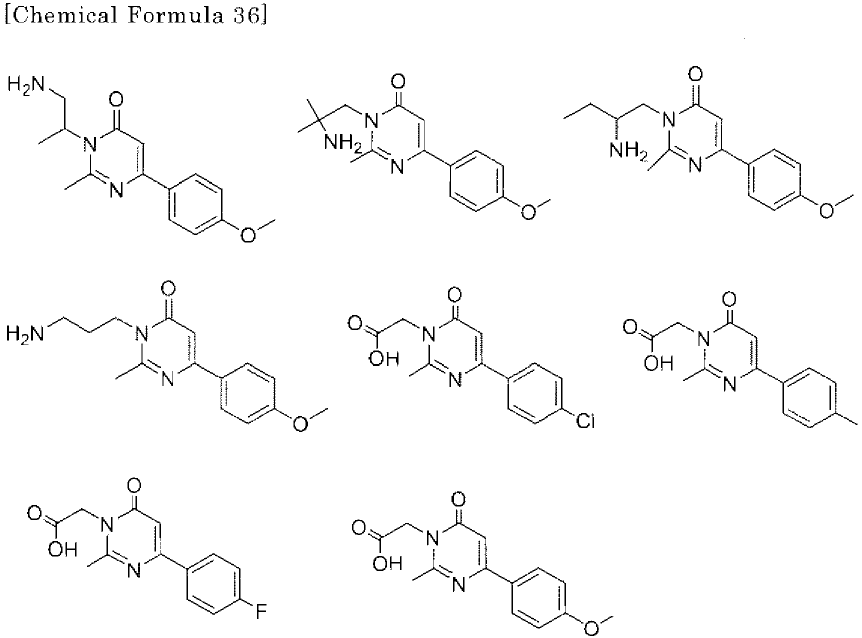 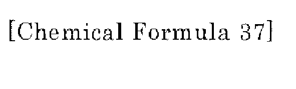 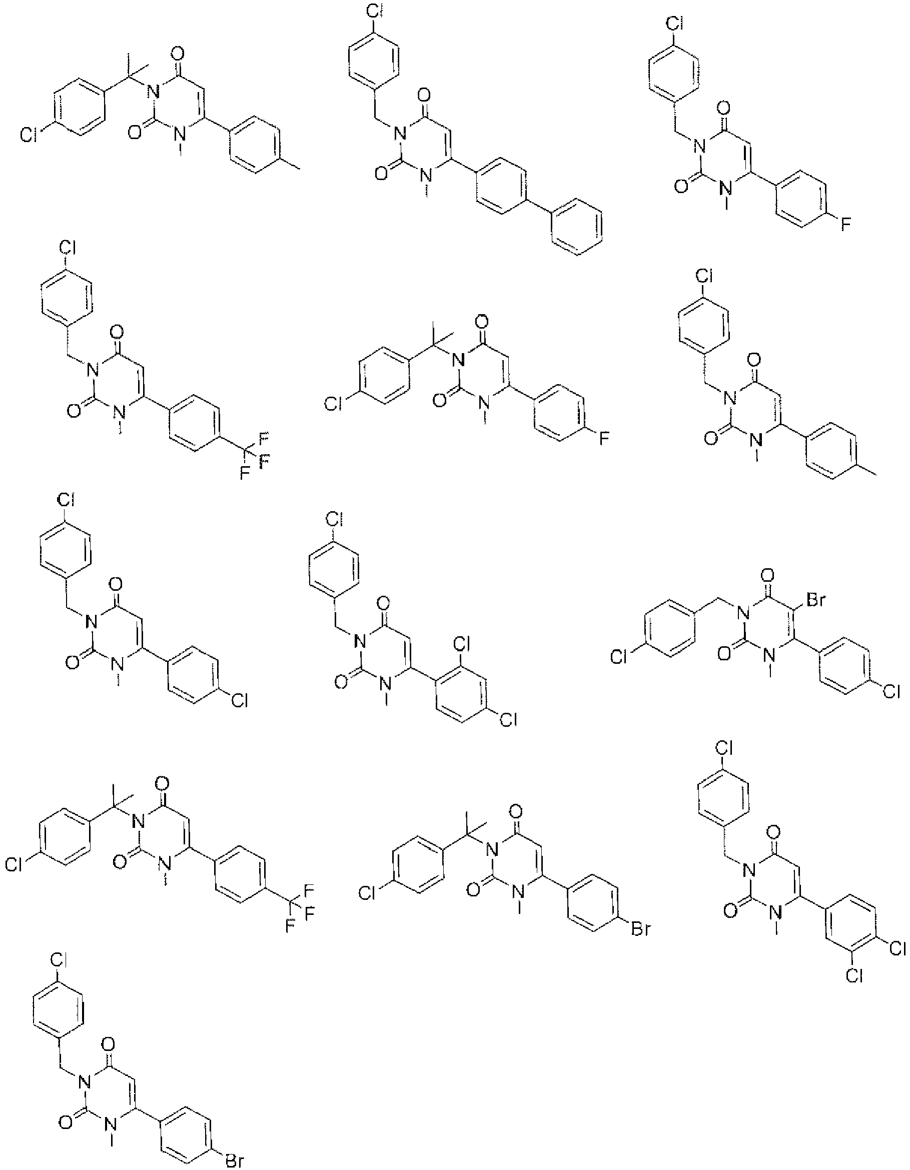 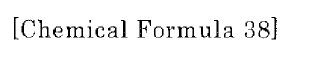 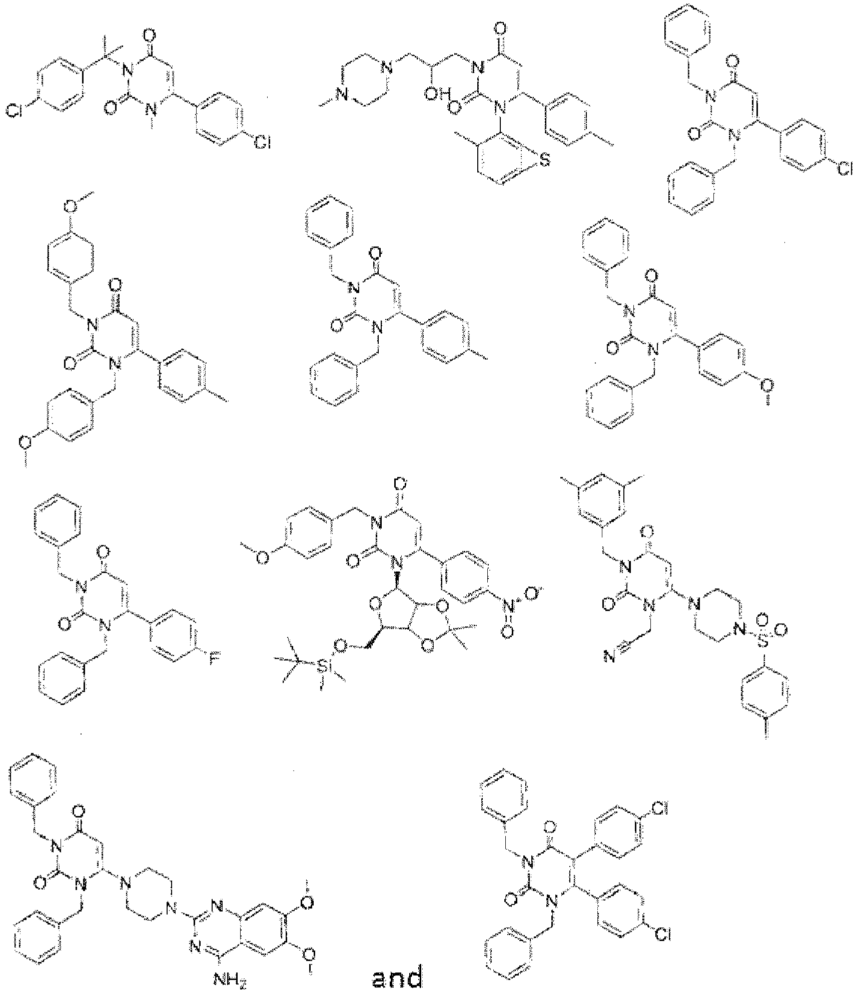
(4') The compound according to the above (3') or (3"), represented by formula (Ia), or its pharmaceutically acceptable salt.
(5') The compound according to the above (3') or (3"), represented by formula (Ib), or its pharmaceutically acceptable salt.
(6') The compound according to the above (3') or (3"), represented by formula (Ic), or its pharmaceutically acceptable salt thereof.
(7') The compound according to any one of the above (4') to (6'), wherein R¹ is hydrogen or halogen, or its pharmaceutically acceptable salt.
(8') The compound according to any one of the above (4') to (7'), wherein R^{4a} is substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, or substituted or unsubstituted alkynylthio, its pharmaceutically acceptable salt.
(9') The compound according to the above (8'), wherein R^{4a} is alkyl optionally substituted with one or more substitutent(s) selected from the Substituent group α (cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy, substituted or unsubstituted non-aromatic heterocyclylalkyloxy and substituted or unsubstituted aromatic heterocyclylalkyloxy), alkenyl optionally substituted with one or more substitutent(s) selected from the Substituent group α, alkynyl optionally substituted with one or more substitutent(s) selected from the Substituent group α, alkylamino optionally substituted with one or more substitutent(s) selected from the Substituent group α, alkenylamino optionally substituted with one or more substitutent(s) selected from the Substituent group α, alkynylamino optionally substituted with one or more substitutent(s) selected from the Substituent group α, alkyloxy optionally substituted with one or more substitutent(s) selected from the Substituent group α, alkenyloxy optionally substituted with one or more substitutent(s) selected from the Substituent group α, alkynyloxy optionally substituted with one or more substitutent(s) selected from the Substituent group α, alkylthio optionally substituted with one or more substitutent(s) selected from the Substituent group α, alkenylthio optionally substituted with one or more substitutent(s) selected from the Substituent group α, alkynylthio optionally substituted with one or more substitutent(s) selected from the Substituent group α, its pharmaceutically acceptable salt.
(10') The compound according to any one of the above (4') to (9'), wherein R¹¹ is carboxy, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted non-aromatic heterocyclyloxycarbonyl, substituted or unsubstituted carbamoyl or substituted or unsubstituted sulfamoyl, its pharmaceutically acceptable salt.
(11') The compound according to the above (10'), wherein R¹¹ is substituted or unsubstituted aromatic carbocyclyl, substituted or unsubstituted aromatic heterocyclyl, substituted or unsubstituted non-aromatic carbocyclyl, or substituted or unsubstituted non-aromatic heterocyclyl, or its pharmaceutically acceptable salt.
(12') The compound according to the above (11'), wherein R¹¹ is aromatic carbocyclyl substituted with one or more substitutent(s) selected from the Substituent group β(carboxy, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclycarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, a substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, and substituted or unsubstituted aromatic carbocyclylsulfonyl), aromatic heterocyclyl substituted with one or more substitutent(s) selected from the Substituent group 6, non-aromatic carbocyclyl substituted with one or more substitutent(s) selected from the Substituent group β, or non-aromatic heterocyclyl substituted with one or more substitutent(s) selected from the Substituent group β, or its pharmaceutically acceptable salt.
(13') The compound according to the above (10'), wherein R¹¹ is substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted non-aromatic heterocyclyloxycarbonyl, substituted or unsubstituted carbamoyl or substituted or unsubstituted sulfamoyl, or its pharmaceutically acceptable salt.
(14') The compound according to the above (10'), wherein R¹¹ is carbamoyl optionally substituted with one or more substitutent(s) selected from the Substituent group α (substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl and substituted or unsubstituted non-aromatic heterocyclyl), or its pharmaceutically acceptable salt.
(15') The compound according to the above (5'), wherein R¹ is hydrogen, R^{4a} is substituted or unsubstituted C2-C10 alkyl, substituted or unsubstituted alkenyl or substituted or unsubstituted alkynyl, or its pharmaceutically acceptable salt.
(16') The compound according to the above (6'), wherein R¹ is hydrogen, R³ is substituted methyl, substituted or unsubstituted C2-C10 alkyl, substituted or unsubstituted alkenyl or substituted or unsubstituted alkynyl, or its pharmaceutically acceptable salt.
(17') A pharmaceutical composition comprising the compound according to any one of the above (3") and (3') to (16') or its pharmaceutically acceptable salt as an active ingredient.
(18') The pharmaceutical composition according to the above (17'), which is an autotaxin inhibitor.
(19') The pharmaceutical composition according to the above (17'), for the prevention or treatment of a disease related to autotaxin.
(20') Use of a compound according to any one of (2') to (16') or its pharmaceutically acceptable salt for the manufacture of a medicament for the prevention or treatment of a disease related to autotaxin.
(21') A method for the prevention or treatment of a disease related to autotaxin, which is characterized by administering the compound according to any one of (2') to (16') or its pharmaceutically acceptable salt.

### Effect of the Invention

The compound of the present invention exhibits excellent autotaxin inhibitory activity, and is useful for the prevention or treatment of a disease, especially, related to autotaxin.

### Description of Embodiments

The definitions of the terms as used herein are as follows. Unless specified otherwise, these terms are used alone or in combination with another term in the meaning as defined.

"Halogen" includes fluorine, chlorine, bromine and.iodine. Fluorine and chlorine are particularly preferable.

"Alkyl" means a straight or branched hydrocarbon group having 1 to 10 carbon atoms, and includes alkyl of 1 to 6 carbon atoms, alkyl of 1 to 4 carbon atoms, and alkyl of 1 to 3 carbon atoms and the like. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, n-decyl and the like.

Specific examples of "C1-C2 alkyl" include methyl, and ethyl.

Specific examples of "C3-C10 alkyl" include n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, n-decyl and the like.

Specific examples of "alkyl" for R³ include methyl, ethyl, n-propyl, isopropyl, n-butyl, isobutyl, sec-butyl, tert-butyl, n-pentyl, hexyl and the like.

Specific examples of "alkyl" for R^{4a} include methyl, ethyl, n-propyl, isopropyl, n-butyl, isobutyl, sec-butyl, tert-butyl, n-pentyl, hexyl and the like. In particular, methyl, ethyl, n-propyl, n-butyl, n-pentyl, n-hexyl and the like is preferred.

Specific examples of "alkyl" for R¹² include methyl, ethyl, n-propyl, isopropyl, n-butyl, isobutyl, sec-butyl, tert-butyI and the like.

Specific examples of "alkyl" for R¹³ include methyl, ethyl, n-propyl, isopropyl, n-butyl, isobutyl, sec-butyl, tert-butyl and the like.

Specific examples of "C3-C10 alkyl" for R¹² include n-propyl, isopropyl, n-butyl, isobutyl, sec-butyl, tert-butyl, n-pentyl, n-hexyl and the like. In particular, n-propyl, n-butyl, n-pentyl, n-hexyl and the like is preferred.

The alkyl moiety of "alkyloxy", "alkyloxycarbonyl", "alkylcarbonyl", "alkylsulfinyl", "alkylsulfonyl", and "alkylthio" is as defined above "alkyl".

Examples of "alkyloxy" include methyloxy, ethyloxy, n-propyloxy, isopropyloxy, n-butyloxy, isobutyloxy, sec-butyloxy, tert-butyloxy, n-pentyloxy, n-hexyloxy, n-heptyloxy, n-octyloxy, isobutylmethylhexyloxy, n-nonyloxy and the like. In particular, methyloxy, ethyloxy, n-propyloxy, isopropyloxy and the like is preferred.

Specific examples of "alkyloxy" for R^{4a} include n-propyloxy, n-pentyloxy,and n-hexyoxyl and the like.

Examples of "alkylthio" include methylthio, ethylthio, n-propylthio, isopropylthio, n-butylthio, isobutylthio, sec-butylthio, tert-butylthio, n-pentylthio, n-hexylthio, n-heptylthio, n-octylthio, isobutylmethylhexylthio, n-nonylthio and the like. In particular, methylthio, ethylthio, n-propylthio, isopropylthio and the like is preferred.

Specific examples of "alkylthio" for R^{4a} include methylthio.

Specific examples of "alkyloxycarbonyl" for R¹² include methyloxycarbonyl, ethyloxycarbonyl, isopropyloxycarbonyl, tert-butyloxycarbonyl and the like.

Specific examples of "alkylcarbonyl" for R¹² include methylcarbonyl, ethylcarbonyl, n-propylcarbonyl, isopropylcarbonyl, n-butylcarbonyl and the like.

Specific examples of "alkylsulfonyl" for R¹² include methylsulfonyl, ethylsulfonyl and the like.

"Haloalkyl" and "haloalkyloxy" mean respectively the alkyl moiety of alkyl and alkyloxy

"Haloalkyl" and "haloalkyloxy" mean respectively alkyl and alkyloxy substituted with 1 to 5, preferably 1 to 3, "halogen" at a substitutable position.

Specific examples of "haloalkyl" for R^{4a} include monohaloalkyl, dihaloalkyl, and trihaloalkyl and the like. In particular, monofluoromethyl, monochrolomethyl, dichloromethyl, difluoromethyl, trufluoromonochloromethyl, trufluoromethyl, trifluoroethyl, trifluoropropyl, trifluorobutyl, trufluoropentyl are prefered.

"Alkenyl" means a linear or branched hydrocarbon group having 2 to 10 carbon atoms and one or more double bonds at any position, and includes alkenyl of 2 to 8 carbon atoms, alkenyl of 2 to 6. Examples include vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like.

Specific examples of "C3-C10 alkenyl" include propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like.

The alkenyl moiety of "alkenyloxy", "alkenyloxycarbonyl", "alkenyl carbonyl", "alkenylsulfinyl", "alkenylsulfonyl" and "alkenylthio" has the same meaning as defined above "alkenyl".

"Alkynyl" means a linear or branched hydrocarbon group having 2 to 10 carbon atoms and one or more triple bonds at any position, and includes alkynyl of 2 to 6 carbon atoms, alkynyl of 2 to 4 carbon atoms. Examples include ethynyl, propynyl, butynyl, pentynyl, hexynyl, heptynyl, octynyl, nonynyl, decynyl and the like. Also, the alkynyl may further have a double bond, as well as one or more triple bonds at anyl position.

Specific examples of "C3-C10 alkynyl" include propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl and the like.

The alkynyl moiety of "alkynyloxy", "alkynyloxycarbonyl", "alkynylcarbonyl", "alkynylsulfinyl", "alkynylsulfonyl" and "alkynylthio" has the same meaning as defined above "alkynyl".

"Non-aromatic carbocycle" means a saturated or unsaturated aliphatic hydrocarbon cycle which is monocyclic or fused cyclic. Examples include cyclic saturated C3 to C8 hydrocarbon (cycloalkane) or cyclic unsaturated C3 to C8 hydrocarbon (cycloalkene), or a ring wherein such cyclic hydrocarbon is fuded with further 3- to 8-membered rings one or two.

"Cycloalkane" includes monocyclic or polycyclic saturated carbocycle having 3 to 10 carbon atoms. Specific examples of the monocyclic cycloalkane include cyclopropane, cyclobutane, cyclopentane, cyclohexane, cycloheptane, cyclooctane, cycloonane, cyclodecane and the like. Specific examples of polycyclic cycloalkane include norbornane, tetrahydronaphthalene, adamantane and the like.

"Cycloalkene" includes monocyclic or polycyclic carbocycle having 3 to 10 carbon atoms and one or more double bonds at any position. Specific examples of the monocyclic cycloalkene include cyclopentene, cyclohexene and the like. Specific examples of polycyclic cycloalkene include norbornene, indene and the like.

The term "cycloalkyl" includes a monovalent group derived from "cycloalkane" as defined above. Monocyclic cycloalkyl includes, for example, cyclopropyl, cyclobutyl, cyclopentyl, cyclohexyl, cycloheptyl, cyclooctyl, cyclononyl, cyclodecyl, etc. Polycyclic cycloalkyl includes norbornyl, tetrahydronaphthalene-5-yl, tetrahydronaphthalene-6-yl, adamanty etc.

Examples of "cycloalkyl" are cycclpropyl, cyclobutyl, cyclopentyl, cyclohexyl, cyc loheptyl, cyclooctyl and the like. Especially preferable examples include C3 to C6 cycloalkyl, or C5 to C6 cycloalkyl.

The term "cycloalkenyl" includes a monovalent group derived from "cycloalkene" as defined above. Monocyclic cycloalkenyl includes cyclopentenyl, cyclohexenyl, etc. Polycyclic cycloalkenyl includes norbornenyl, indene-1-yl, indene-2-yl, indene-3-yl, etc. Especially preferable examples include C5 to C6 cycloalkenyl.

"Non-aromatic carbocyclyl" includes cyclic saturated hydrocarbon group having 3 to 8 carbon atoms, groups wherein such cyclic saturated hydrocarbon ring is fused with further one or two 3- to 8-membered rings and cyclic unsaturated aliphatic hydrocarbon groups having 3 to 8 carbon atoms, and groups wherein such cyclic unsaturated aliphatic hydrocarbon ring is fused with further one or two 3- to 8-membered rings.

Specific examples of the cyclic saturated hydrocarbon group having 3 to 8 carbon atoms include cyclopropyl, cyclobutyl, cyclopentyl, cyclohexyl, cycloheptyl, cyclooctyl. In particular, a cyclic saturated hydrocarbon group having 3 to 6 carbon atoms and a cyclic saturated hydrocarbon group having 5 or 6 carbon atoms are preferred.

Specific examples of the ring to be fused with the cyclic saturated hydrocarbon group having 3 to 8 carbon atoms include non-aromatic carbocyclic rings, such as cycloalkane ring (for example: cyclohexane, cyclopentane etc.) and cycloalkene ring (for example: cyclohexene, cyclopentene etc.); non-aromatic heterocyclic rings, such as piperidine ring, piperazine ring and morpholine ring etc. The cyclic saturated hydrocarbon group having 3 to 8 carbon atoms should be involved in the linkage of such fused ring.

Specific examples of the ring to be fused with the cyclic unsaturated aliphatic hydrocarbon group having 3 to 8 carbon atoms include carbocyclic rings: such as aromatic carbocyclic rings (for example: benzene ring, naphthalene ring) and non-aromatic carbocyclic rings (for example: cycloalkane rings such as cyclohexane ring and cyclopentane ring, cycloalkene rings such as cyclohexene ring and cyclopentene ring); and heterocyclic rings: such as aromatic heterocyclic rings (for example: pyridine ring, pyrimidine ring, pyrrole ring, imidazole ring) and non-aromatic heterocyclic rings (for example: piperidine ring, piperazine ring, morpholine ring). The cyclic unsaturated aliphatic hydrocarbon group having 3 to 8 carbon atoms should be involved in the linkage of such fused ring.

Examples of the non-aromatic carbocyclic group include the following groups. These groups may have a substituent at any substitutable position.

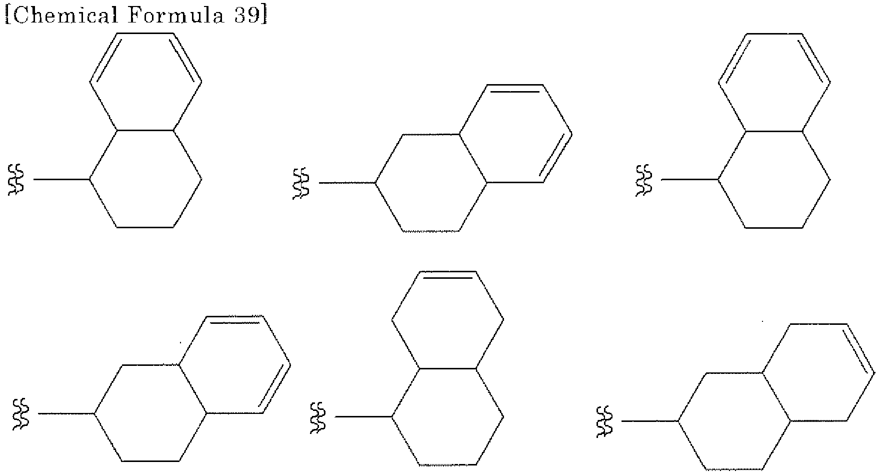

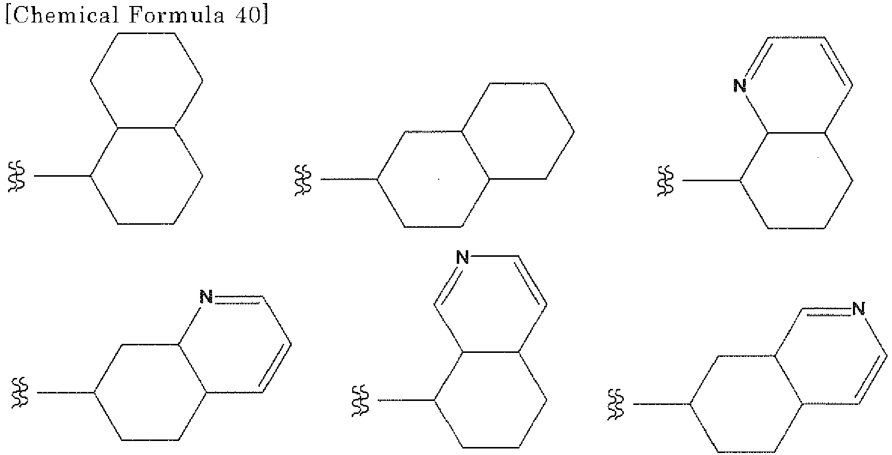

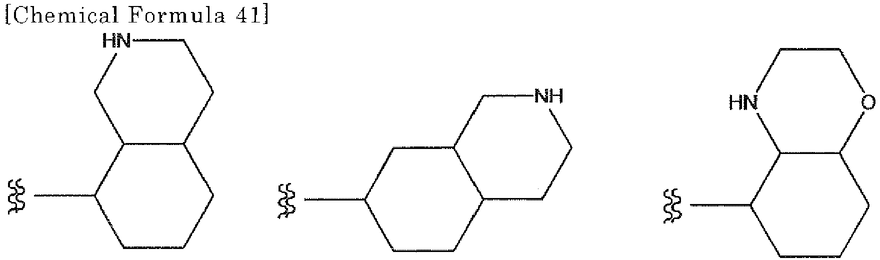

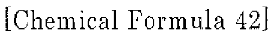

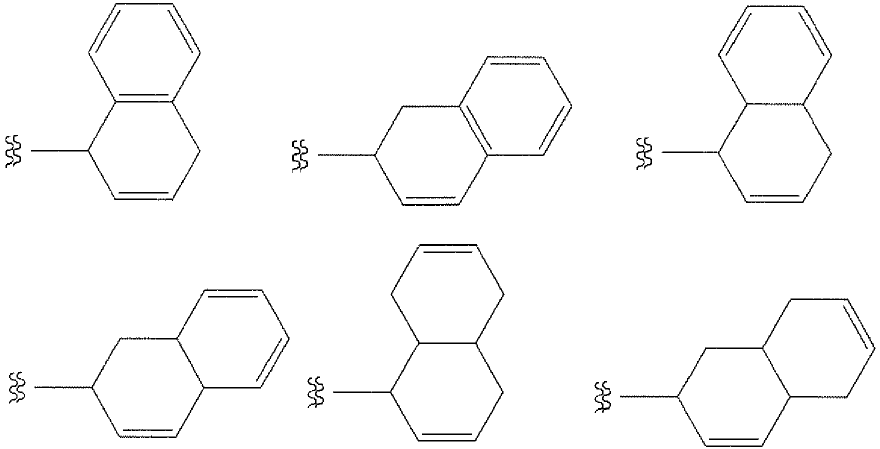

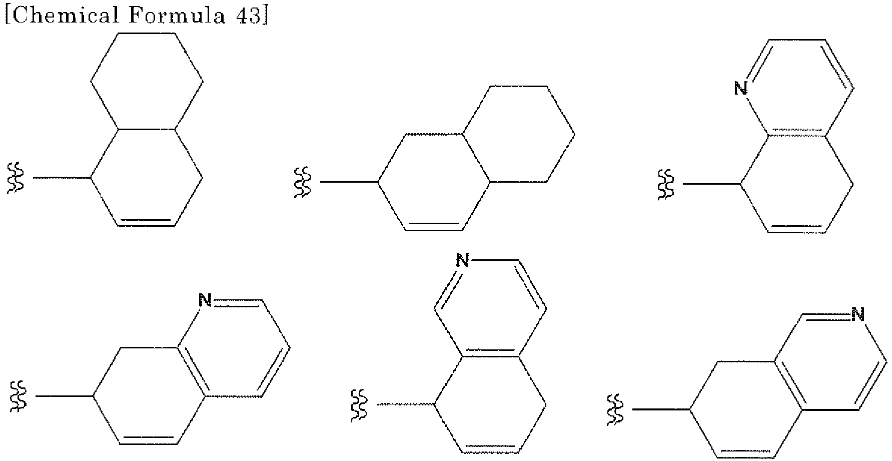

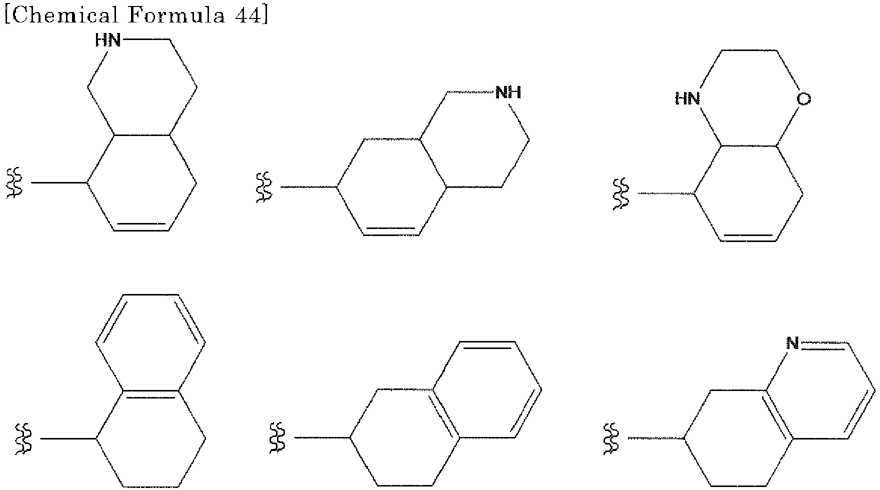

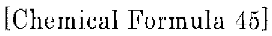

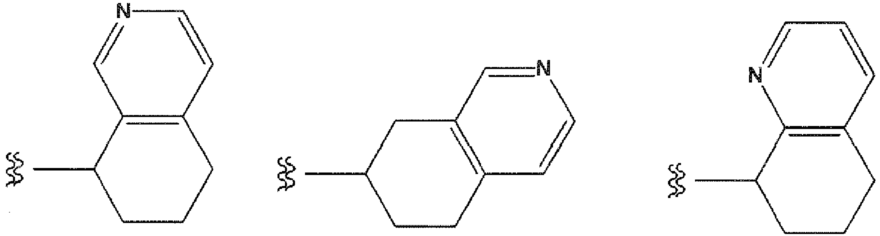

The non-aromatic carbocyclic ring moiety of "non-aromatic carbocyclyloxy", "non-aromatic carbocyclyloxycarbonyl", "non-aromatic carbocyclylcarbonyl", "non-aromatic amino", "non-aromatic carbocyclylsulfinyl", "non-aromatic carbocyclylsulfonyl", "non-aromatic carbocyclylthio", and the like is as defined above "non-aromatic carbocycle".

Spercific examples of "non-aromatic carbocyclyl" for R² include, cyclopropyl, cyclobutyl, cyclopentyl, cyclohexyl, cyclohexenyl and the like are preferable. In particular, cyclohexyl, cyclohexenyl are preferable.

Spercific examples of "non-aromatic carbocyclyloxy" for R² include, cyclopropyloxy, cyclobutyloxy, cyclopentyloxy, cyclohexyloxy, cyclohexenyloxy and the like are preferable. In particular, cyclohexyloxy, cyclohexenyloxy are preferable.

Spercific examples of "non-aromatic carbocyclylcarbonyl" for R¹¹ include, cyclopropylcarbonyl, cyclobutylcarbonyl, cyclopentylcarbonyl, cyclohexylcarbonyl, cyclohexenylcarbonyl and the like are preferable. In particular, cyclohexylcarbonyl, cyclohexenylcarbonyl are preferable.

Spercific examples of "non-aromatic carbocyclylcarbonyl" for R¹³ include, cyclopropylcarbonyl, cyclobutylcarbonyl, cyclopentylcarbonyl, cyclohexylcarbonyl, and the like are preferable.

"Aromatic carbocycle" includes monocyclic or polycyclic aromatic carbocycle. Specific examples include benzene ring, naphthalene ring, anthracene ring and phenanthrene ring. Particularly, benzene ring is preferred.

"Aromatic carbocyclyl" includes monocyclic or polycyclic aromatic carbocyclic groups and groups wherein such monocyclic or polycyclic aromatic carbocyclic ring is fused with further one or two 3- to 8-membered rings. Specific examples of the monocyclic or polycyclic aromatic carbocyclic group include phenyl, naphthyl, anthryl and phenanthryl. Particularly, phenyl is preferred.

Specific examples of the ring to be fused with the monocycli or polycyclic aromatic carbocyclic group include non-aromatic carbocycle such as cycloalkane rings (for example: cyclohexane ring, cyclopentane ring etc.), cycloalkene rings (for example: cyclohexene ring, cyclopentene ring etc.), and non-aromatic heterocycle (for example: piperidine ring, piperazine ring, morpholine ring etc). The monocyclic or polycyclic aromatic carbocyclyl should be involved in the linkage of such fused ring.

Examples of the aromatic carbocyclic groups include the following groups. These groups may have a substituent at any possible position.

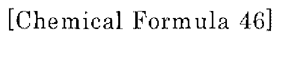

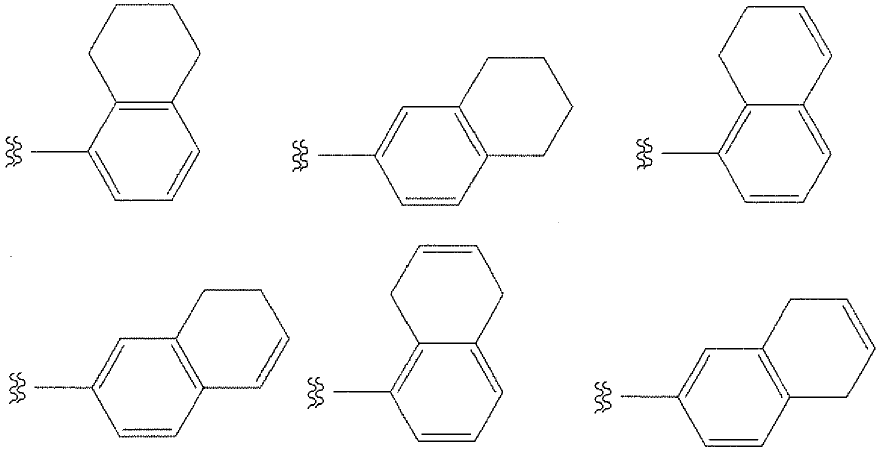

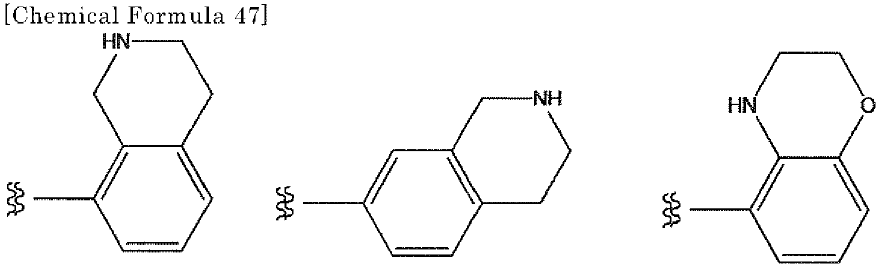

Specific examples of "aromatic carbocyclyl" for R² include phenyl, naphthyl, anthryl and phenanthryl and the like. In particular, phenyl is preferred.

Specific examples of "aromatic carbocyclyl" for R¹¹ include phenyl, naphthyl, anthryl and phenanthryl and the like. In particular, phenyl is preferred.

Specific examples of "aromatic carbocyclylcarbonyl" for R¹¹ include phenylcarbonyl, naphthylcarbonyl, anthrylcarbonyl and phenanthrylcarbonyl and the like. In particular, phenylcarbonyl is preferred.

The aromatic carbocycle moiety of "aromatic carbocyclyloxy", "aromatic carbocyclyloxycarbonyl", "aromatic carbocyclylcarbonyl", "aromatic carbocyclylamino", "aromatic carbocyclylsulfinyl", "aromatic carbocyclylsulfonyl", and "aromatic carbocyclylthio" and the like is as defined above "aromatic carbocycle".

Specific examples of "aromatic carbocyclylcarbonyl" for R¹³ include phenylcarbonyl and the like.

"Aromatic heterocycle" means 5 -6 membered aromatic ring having one or more heteroatoms selected from O, S and N in the ring.

Preferred examples of the aromatic heterocycle include monocyclic aromatic heterocycle such as pyrazole, tetrazole, furan, thiophene, pyridine, imidazole, triazole, triazine, pyridazine, pyrimidine, pyrazine, isoxazole, thiazole, isothiazole, thiadiazole, oxazole, oxadiazole and the like,
fused aromatic heterocycle such as indole, isoindole, indazole, indolizine, quinoline, isoquinoline, cinnoline, phthalazine, quinazoline, naphthyridine, quinoxaline, purine, pteridine, benzimidazole, benzisoxazole, benzoxazole, benzimidazole oxadiazole, benzisothiazole, benzothiazole, benzothiadiazole, benzofuran, isobenzofuran, benzothiophene, benzotriazole, imidazopyridine, triazolopyridine, imidazothiazole, pyradinopyridazine, benzimidazoline and the like.

Specific examples of "aromatic heterocyclyl" for R¹³ include pyrrolyl, imidazolyl, pyrazolyl, pyridyl, pyridazinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, thienyl, and the like. Pyridyl are preferred, particularly 2-pyridyl, 3-pyridyl is more preferred.

"Non-aromatic heterocycle" includes 5-7 membered non-aromatic ring having at least one heteroatoms selected from O, S and N in the ring,
non-aromatic ring wherein such non-aromatic heterocyclic rings are fused with further two or more rings, and
fused ring wherein aromatic ring having 5-7 membered aromatic ring having at least one heteroatoms selected from O, S and N in the ring is fused with one or more the above "cycloalkane" or the above "cycloalkene",
fused ring wherein non-aromatic ring having 5-7 membered aromatic ring having at least one heteroatoms selected from O, S and N in the ring is fused with one or more the above "aromatic carbocycle" or the above "aromatic heterocycle".

For example, preferred examples of the monocyclic non-aromatic heterocycle include pyrroline, pyrrolidine, imidazoline, imidazolidine, pyrazoline, pyrazolidine, piperidine, piperazine, morpholine, thiomorpholine, tetrahydropyran, dihydropyridine, dihydropyridazine, dihydropyrazine, dioxane, oxathiolane, thiane, tetrahydrofuran, tetrahydrothiazoline, tetrahydroisothiazolin.

For example, preferred examples of the fused non-aromatic heterocycle include indoline, isoindoline, benzopyran, benzodioxane, tetrahydroquinoline, benzo[d]oxazole-2(3H)-on, tetrahydrobenzothiophene.

"Aromatic heterocyclyl" means monocyclic or polycyclic aromatic heterocyclic groups having one or more heteroatoms selected from O, S and N in the ring and groups wherein such monocyclic or polycyclic aromatic heterocyclyl is fused with further one or two 3- to 8-membered rings.

Preferred examples of the monocyclic aromatic heterocyclyl include 5- or 6-membered aromatic heterocyclyl such as pyrrolyl, imidazolyl, pyrazolyl, pyridyl, pyridazinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, thienyl and the like.

Preferred examples of the polycyclic aromatic heterocyclyl include aromatic heterocyclyl fused with a 5- or 6-membered ring, such as bicyclic aromatic heterocyclyl (for example: indolyl, isoindolyl, indazolyl, indolizinyl, quinolinyl, isoquinolinyl, cinnolinyl, phthalazinyl, quinazolinyl, naphthyridinyl, quinoxalinyl, purinyl, puteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyradinopyridazinyl, oxazolopyridyl, thiazolopyridyl etc.); and tricyclic aromatic heterocyclyl (for example: carbazolyl, acridinyl, xanthenyl, phenothiazinyl, phenoxathiinyl, phenoxazinyl, dibenzofuryl etc.). Any ring of the polycyclic aromatic heterocyclyl may be involved in the linkage.

Specific examples of the ring to be fused the monocyclic or polycyclic aromatic heterocyclic groups include non-aromatic carbocycle such as cycloalkane rings (for example: cyclohexane ring, cyclopentane ring etc.), cycloalkene rings (for example: cyclohexene ring, cyclo pentene ring etc.), non-aromatic heterocycle (for example, piperidine ring, piperazine ring, morpholine ring etc.). The monocyclic or polycyclcic aromatic heterocyclyl should be involved in the linkage of such fused ring.

Examples of the aromatic heterocyclic groups include the following groups. These groups may have a substituent at any possible position.

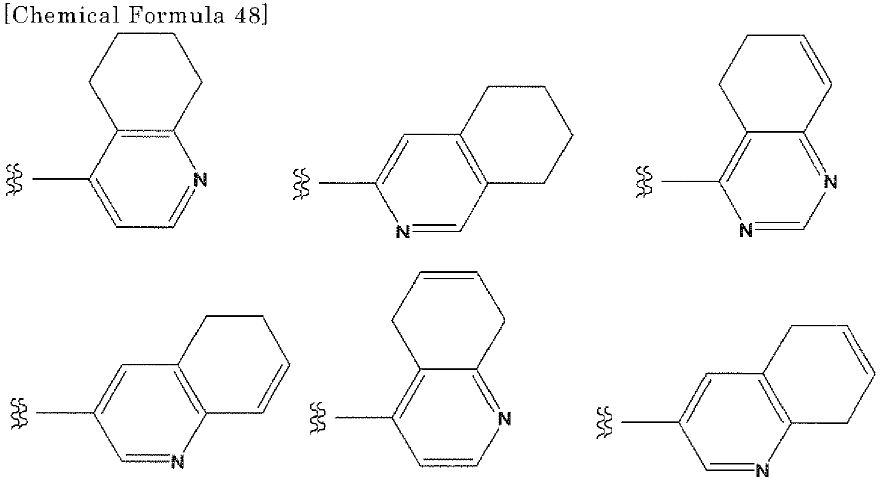

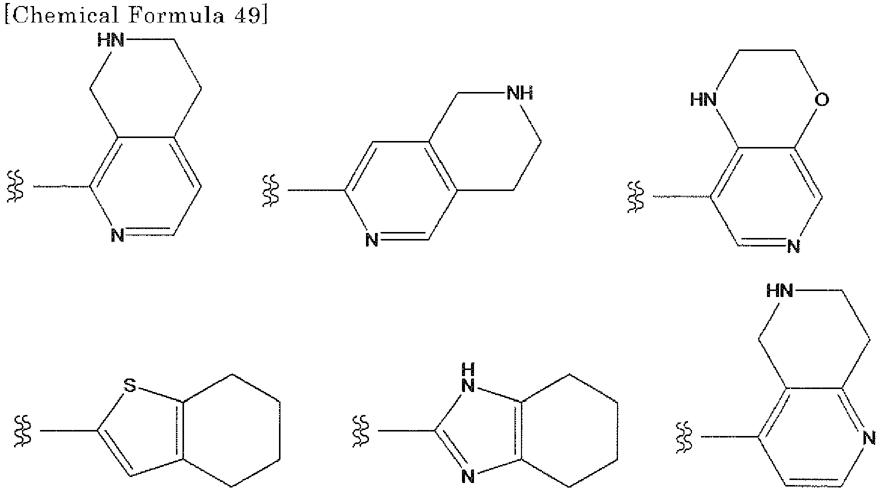

Specific examples of "aromatic heterocyclyl" for R² include pyrrolyl, imidazolyl, pyrazolyl, pyridyl, pyridadinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, thienyl and the like. In particular, pyridyl is preferred.

Specific examples of "aromatic heterocyclyl" for R¹¹ include pyrrolyl, imidazolyl, pyrazolyl, pyridyl, pyridadinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, thienyl and the like. In particular, pyridyl is preferred.

Specific examples of "aromatic heterocyclyl" for R¹⁵ include pyrrolyl, imidazolyl, pyrazolyl, pyridyl, pyridadinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl, thienyl and the like. In particular, pyridyl is preferred. Moreover, 2-pyridyl and 3-pyridyl are more preferred.

Specific examples of "aromatic heterocyclylcarbonyl" for R¹¹ include pyrrolylcarbonyl, imidazolylcarbonyl, pyrazolylcarbonyl, pyridylcarbonyl, pyridadinylcarbonyl, pyrimidinylcarbonyl, pyrazinylcarbonyl, triazolylcarbonyl, triazinylcarbonyl, tetrazolylcarbonyl, isoxazolylcarbonyl, oxazolylcarbonyl, oxadiazolylcarbonyl, isothiazolylcarbonyl, thiazolylcarbonyl, thiadiazolylcarbonyl, furylcarbonyl, thienylcarbonyl and the like. In particular, pyridylcarbonyl is preferred.

Preferrable examples of "aromatic heterocyclylcarbonyl" for R¹³ are pyrazolylcarbonyl, thienylcarbonyl is preferred.

The aromatic heterocyclyl moiety of "aromatic heterocyclyloxy", "aromatic heterocyclyloxycarbonyl", "aromatic heterocyclylcarbonyl", "aromatic heterocyclylamino", "aromatic heterocyclylsulfinyl", "aromatic heterocyclylsulfonyl", and "aromatic heterocyclylthio" and the like is as defined above "aromatic heterocycle".

"Non-aromatic heterocyclyl" means non-aromatic heterocyclyl having one or more heteroatoms selected from O, S and N in the ring and
non-aromatic group wherein such non-aromatic heterocyclyl is fused with further two 3- to 8-membered rings, and
includes monocyclic non-aromatic hetelocyclyl or molycyclic non-aromatic hetelocyclyl.

Specific examples of the monocyclic non-aromatic heterocyclyl include dioxanyl, thiiranyl, oxiranyl, oxathioranyl, azetidinyl, thianyl, pyrrolidinyl, pyrrolinyl, imidazolidinyl, imidazolinyl, pyrazolidinyl, pyrazolinyl, piperidyl, piperidino, piperazinyl, piperazino, morpholinyl, morpholino, oxadiadinyl, dihydropyridyl, thiomorpholinyl, thiomorpholino, tetrahydrofuryl, tetrahydropyranyl, tetrahydrothiazolyl, tetrahydroisothiazolyl, oxazolidyl, thiazolidyl and the like.

Specific examples of the polycyclic non-aromatic heterocyclyl include indolinyl, isoindolinyl, chromanyl, isochromanyl, isomannyl and the like. Any ring of the polycyclic non-aromatic heterocyclyl may be involved in the linkage.

Examples of the non-aromatic heterocyclyl include the following groups.

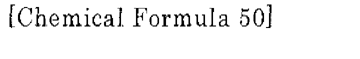

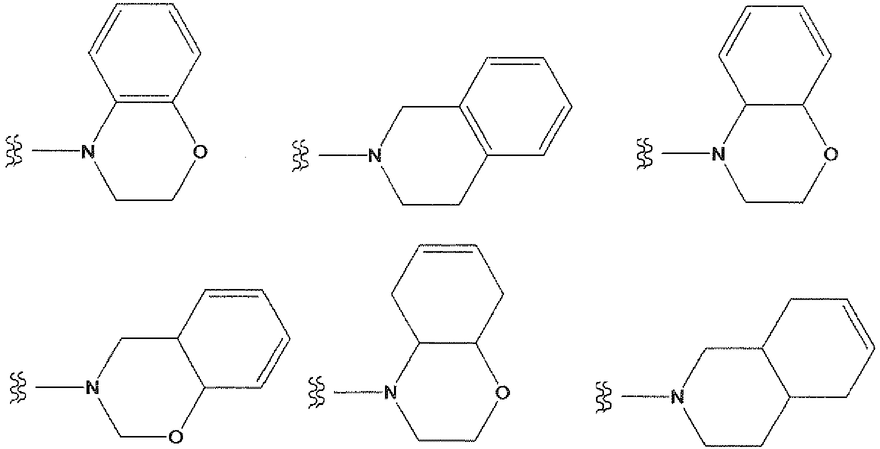

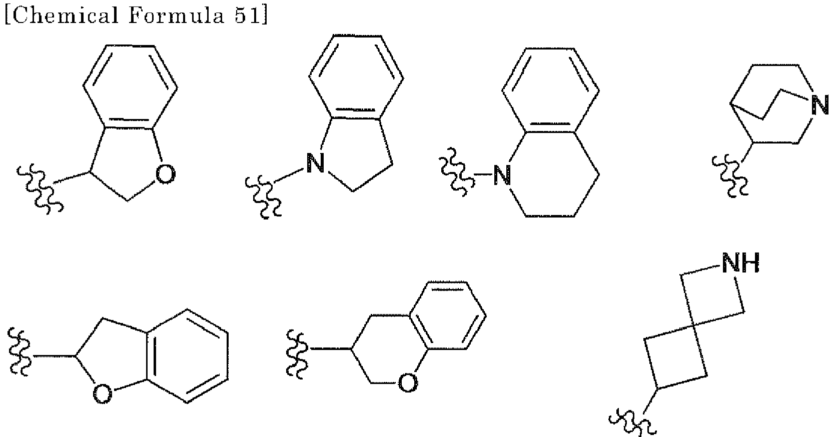

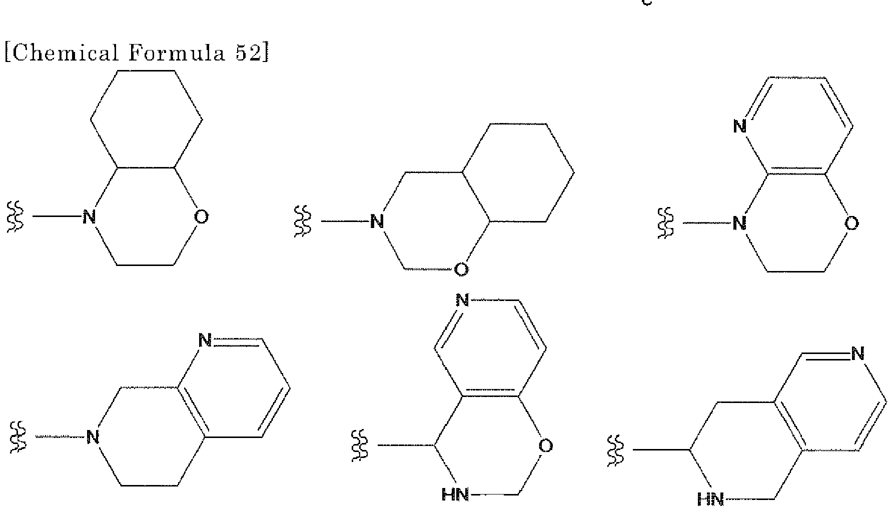

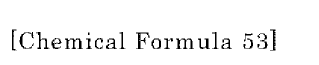

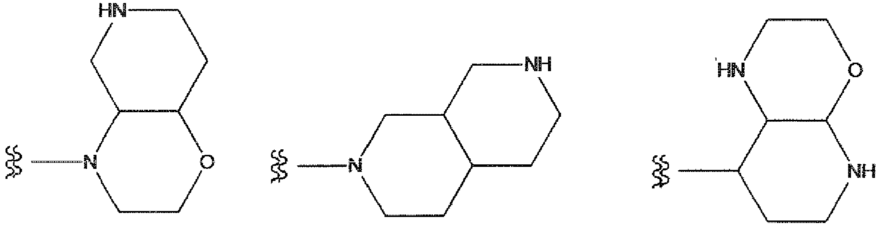

Specific examples of "non-aromatic heterocyclyl" for R¹ include azethidinyl, piperidyl, piperidino, piperazinyl, piperadino, morpholinyl, morpholino, pyrrolidinyl, azepanyl, isoindolinyl, 2-oxa-6-azaspiro [3.3] heptanyl etc.. In particular, azethidinyl, piperidyl, piperidino, piperazinyl, piperadino and the like are prefered.

Specific examples of "non-aromatic heterocyclyl" for R² include azethidinyl, piperidyl, piperidino, piperazinyl, piperadino, morpholinyl, morpholino, 2-oxa-6-azaspiro[3.3]heptanyl and the like. In particular, azethidinyl, piperidyl, piperidino, piperazinyl, piperadino and the like are prefered.

Specific examples of "non-aromatic heterocyclylcarbonyl" for R¹¹ include, azethidinylcarbonyl, piperidylcarbonyl, piperidinocarbonyl, piperazinylcarbonyl, piperadinocarbonyl, morpholinylcarbonyl, morpholinocarbonyl and the like.

In particular, pyrrolidinylcarbonyl, piperidinylcarbonyl, piperazinylcarbonyl, morpholinylcarbonyl, morpholinocarbonyl, 2-oxa-6-azaspiro [3.3] hept-ylcarbonyl and the like are preferable.

In particular, pyrrolidinylcarbonyl, piperidinylcarbonyl, piperazinylcarbonyl, morpholinylcarbonyl, 2-oxa-6-azaspiro [3.3] hept-yl carbonyl and the like are preferable.

Specific examples of "non-aromatic heterocyclylcarbonyl" for R¹³ include pyrrolidinylcarbonyl, imidazolidinylcarbonyl, tetrahydrothiophenylcarbonyl, piperidinylcarbonyl, piperazinylcarbonyl, morpholinylcarbonyl the like.

The non-aromatic heterocyclyl moiety of "non-aromatic heterocyclyloxy", "non-aromatic hetezocyclyloxycarbonyl", "non-aromatic heterocyclylamino", "non-aromatic heterocyclylsulfinyl", "non-aromatic heterocyclylsulfonyl", and "non-aromatic heterocyclylthio" and the like is as defined above "aromatic heterocycle".

The substituted or unsubstituted non-aromatic carbocyclyl or substituted or unsubstituted non-aromatic heterocyclyl are optionally substituted with one or two oxo, thioxo or substituted or unsubstituted imino.

Examples of the substitutent for "substituted alkyl", "substituted alkenyl", "substituted alkynyl" include halogen, hydroxy, mercapto, nitro, nitroso, cyano, azido, formyl, carboxy, substituted or unsubstituted alkyloxy, substituted or unsubstituted alkenyloxy, substituted or unsubstituted alkynyloxy, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted alkylcarbonyloxy, substituted or unsubstituted alkenylcarbonyloxy, substituted or unsubstituted alkynylcarbonyloxy, substituted or unsubstituted alkylthio, substituted or unsubstituted substituted alkenylthio, substituted or unsubstituted alkynylthio, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbamoyl, substituted or unsubstituted sulfamoyl, substituted or unsubstituted amino, substituted or unsubstituted non-aromatic carbocyclyl, substituted or unsubstituted aromatic carbocyclyl, substituted or unsubstituted aromatic heterocyclyl, substituted or unsubstituted non-aromatic heterocyclyl, substituted or unsubstituted non-aromatic carbocyclyloxy, substituted or unsubstituted aromatic carbocyclyloxy, substituted or unsubstituted aromatic heterocyclyloxy, substituted or unsubstituted non-aromatic heterocyclyloxy, substituted or unsubstituted non-aromatic carbocyclylcarbonyl, substituted or unsubstituted aromatic carbocyclylcarbonyl, substituted or unsubstituted aromatic heterocyclylcarbonyl, substituted or non-aromatic heterocyclylcarbonyl substituted or unsubstituted non-aromatic carbocyclyloxycarbonyl, substituted or unsubstituted aromatic carbocyclyloxycarbonyl, substituted or unsubstituted aromatic heterocyclyloxycarbonyl, substituted or unsubstituted non-aromatic heterocyclyloxycarbonyl, substituted or unsubstituted non-aromatic carbocyclylcarbonyloxy, substituted or unsubstituted aromatic carbocyclylcarbonyloxy, substituted or unsubstituted aromatic heterocyclylcarbonyloxy, non-aromatic heterocyclylcarbonyloxy and the like. One or more of these substituents may occur at any substitutable position.

Furthermore, halogen, hydroxy, mercapto, nitro, nitroso, cyano, azido, formyl, carboxy, alkyloxy, alkenyloxy, alkynyloxy, alkylcarbonyl, alkenylcarbonyl, alkynylcarbonyl, alkyloxycarbonyl, alkenyloxycarbonyl, alkynyloxycarbonyl, alkylcarbonyloxy, alkenylcarbonyloxy, alkynylcarbonyloxy, alkylthio, alkenylthio, alkynylthio, alkylsulfinyl, alkenylsulfinyl, alkynylsulfinyl, alkylsulfonyl, alkenylsulfonyl, alkynylsulfonyl, carbamoyl, substituted carbamoyl, sulfamoyl, substituted sulfamoyl, amino, substituted amino, non-aromatic carbocyclyl, aromatic carbocyclyl, aromatic heterocyclyl, non-aromatic heterocyclyl, non-aromatic carbocyclyloxy, aromatic carbocyclyloxy, aromatic heterocyclyloxy, non-aromatic heterocyclyloxy, non-aromatic carbocyclylcarbonyl, aromatic carbocyclylcarbonyl, aromatic heterocyclylcarbonyl, non-aromatic heterocyclylcarbonyl, non-aromatic carbocyclyloxycarbonyl, aromatic carbocyclyloxycarbonyl , aromatic heterocyclyloxycarbonyl, non-aromatic heterocyclyloxycarbonyl, non-aromatic carbocyclylcarbonyloxy, aromatic carbocyclylcarbonyloxy, aromatic heterocyclylcarbonyloxy, non-aromatic heterocyclylcarbonyloxy and the like are preferable. One or more of these substituents may occur at any substitutable position.

Examples of the substitutent for "substituted C3-C10 alkyl", "substituted alkyloxy", "substituted C3-C10 alkenyl", "substituted alkynyloxy", "substituted alkenyloxy", "substituted C3-C10 alkynyl" is same.

Examples of the substitutent for "substituted non-aromatic carbocyclyl", "substituted aromatic carbocyclyl", "substituted aromatic heterocyclyl" or "substituted non-aromatic heterocyclyl" include halogen, cyano , hydroxy, formyl, carboxy, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or non-aromatic heterocyclylcarbonyl unsubstituted, substituted or unsubstituted alkyloxycarbonyl, substituted or unsubstituted alkenyloxycarbonyl, substituted or unsubstituted alkynyloxycarbonyl, substituted or non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted alkylcarbonyloxy, substituted or unsubstituted alkenylcarbonyloxy, substituted or unsubstituted alkynylcarbonyloxy, substituted or unsubstituted non-aromatic carbocyclylcarbonyloxy, substituted or unsubstituted aromatic carbocyclylcarbonyloxy, substituted or unsubstituted non-aromatic heterocyclylcarbonyloxy, substituted or unsubstituted aromatic heterocyclylcarbonyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl, substituted or unsubstituted amino, substituted or unsubstituted carbamoyl and substituted or unsubstituted sulfamoyl, and the like. One or more of these substituents may occur at any substitutable position.

Examples of the substitutent for "Substituted non-aromatic carbocyclyl", "substituted aromatic carbocyclyl", "substituted aromatic heterocyclyl" or "substituted non-aromatic heterocyclyl" include halogen, hydroxy , mercapto, nitro, nitroso, cyano, azido, formyl, carboxy, alkyl, alkenyl, alkynyl, alkyloxy, alkenyloxy, alkynyloxy, alkylcarbonyl, alkenylcarbonyl, alkynylcarbonyl, alkyloxycarbonyl, alkenyloxycarbonyl, alkynyloxycarbonyl, alkylcarbonyloxy, alkenylcarbonyloxy, alkynylcarbonyloxy, alkylthio, alkenylthio, alkynylthio, alkylsulfinyl, alkenylsulfinyl, alkynylsulfinyl, alkylsulfonyl, alkenylsulfonyl, alkynylsulfonyl, carbamoyl, substituted carbamoyl, sulfamoyl, substituted sulfamoyl, amino , substituted amino, non-aromatic carbocyclyl, aromatic carbocyclyl, aromatic heterocyclyl, non-aromatic heterocyclyl, non-aromatic carbocyclyloxy, aromatic carbocyclyloxy, aromatic heterocyclyloxy, non-aromatic heterocyclyloxy, non-aromatic carbocyclylcarbonyl, aromatic carbocyclylcarbonyl, aromatic heterocyclylcarbonyl, non-aromatic heterocyclylcarbonyl, non-aromatic carbocyclyloxycarbonyl, aromatic carbocyclyloxycarbonyl , aromatic heterocyclyloxycarbonyl, non-aromatic heterocyclyloxycarbonyl, non-aromatic carbocyclylcarbonyloxy, aromatic carbocyclylcarbonyloxy, aromatic heterocyclylcarbonyloxy, non-aromatic heterocyclylcarbonyloxy and the like. One or more of these substituents may occur at any substitutable position.

Examples of the substitutent for "substituted alkyl"include cyano, hydroxy, formyl, carboxy, halogen, sulfanyl, alkyloxy, haloalkyloxy, alkylcarbonyl, carbamoyl, alkylcarbamoyl, non-aromatic carbocyclylalkyloxy and the like.

Specific examples of the substituent for "substituted alkyl" in R^{4a} and R³ include the Substituent group α (cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy, substituted or unsubstituted non-aromatic heterocyclylalkyloxy and substituted or unsubstituted aromatic heterocyclylalkyloxy) and the like.

Preferably, specific examples include Substituent group α'(cyano, halogen, alkyloxy, haloalkyloxy and non-aromatic carbocyclylalkyloxy) and the like.

Specific examples of the substituent for "substituted alkyl" in R⁷ include hydroxy, formyl, carboxy, sulfanyl, alkylcarbonyl, carbamoyl, alkylcarbamoyl and the like.

Preferable examples include hydroxy and the like.

Specific examples of the substituent for "substituted alkyloxy" in R^{4a} include Substituent group a(cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy, substituted or unsubstituted non-aromatic heterocyclylalkyloxy and substituted or unsubstituted aromatic heterocyclylalkyloxy) and the like.

Preferably, specific examples include Substituent group a'(cyano, halogen, alkyloxy, haloalkyloxy and non-aromatic carbocyclylalkyloxy) and the like.

Specific examples of the substituent for "substituted alkylthio" in R^{4a} include include the Substituent group a (cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy, substituted or unsubstituted non-aromatic heterocyclylalkyloxy and substituted or unsubstituted aromatic heterocyclylalkyloxy) and the like.

Preferably, specific examples include Substituent group a'(cyano, halogen, alkyloxy, haloalkyloxy and non-aromatic carbocyclylalkyloxy) and the like.

Specific examples of the substituent of the substituent for "substituted amino", "substituted carbamoyl", "substituted sulfamoyl", "substituted amidino" or "substituted imino" include substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted non-aromatic carbocyclylcarbonyl, substituted or unsubstituted aromatic heterocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted alkylsulfinyl, substituted or unsubstituted alkenylsulfinyl, substituted or unsubstituted alkynylsulfinyl, substituted or unsubstited non-aromatic carbocyclylsulfinyl, substituted or unsubstituted aromatic carbocyclylsulfinyl, substituted or unsubstituted non-aromatic heterocyclylsulfinyl, unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl, substituted or unsubstituted carbamoyl and substituted or unsubstituted sulfamoyl. One or two of these substituents may occur at any substitutable position.

Specific examples of the substituent of the substituent for "Substituted amino", "substituted carbamoyl", "substituted sulfamoyl", "substituted amidino" or "substituted imino" include halogen, hydroxy, mercapto, nitro, nitroso, cyano, azido, formyl, carboxy, alkyl, alkenyl, alkynyl, alkyloxy, alkenyloxy, alkynyloxy, alkylcarbonyl, alkenylcarbonyl, alkynylcarbonyl, alkyloxycarbonyl, alkenyloxycarbonyl, alkynyloxycarbonyl, alkylcarbonyloxy, alkenylcarbonyloxy, alkynyl carbonyloxy, alkylthio, alkenylthio, alkynylthio, alkylsulfinyl, alkenylsulfinyl, alkynylsulfinyl, alkylsulfonyl, alkenylsulfonyl, alkynylsulfonyl, carbamoyl, substituted carbamoyl, sulfamoyl, substituted sulfamoyl, amino, substituted amino, non-aromatic carbocyclyl, aromatic carbocyclyl, aromatic heterocyclyl, non-aromatic heterocyclyl, non-aromatic carbocyclyloxy, aromatic carbocyclyloxy, aromatic heterocyclyloxy, non-aromatic heterocyclyloxy, non-aromatic carbocyclylcarbonyl, aromatic carbocyclylcarbonyl, aromatic heterocyclylcarbonyl, non-aromatic heterocyclylcarbonyl, non-aromatic carbocyclyloxycarbonyl, aromatic carbocyclyloxycarbonyl, aromatic heterocyclyloxycarbonyl, non-aromatic heterocyclyloxycarbonyl, non aromatic carbocyclylcarbonyloxy, aromatic carbocyclylcarbonyloxy, aromatic heterocyclylcarbonyloxy, non-aromatic heterocyclylcarbonyloxy. One or two of these substituents may occur at any substitutable position.

Specific examples of the substituent for the "substituted amino" in R^{4a} include alkyl optionally substituted with one or more substitutent(s) selected from the Substituent group α (cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy, substituted or unsubstituted non-aromatic heterocyclylalkyloxy and substituted or unsubstituted aromatic heterocyclylalkyloxy), alkenyl optionally substituted with one or more substitutent(s) selected from the Substituent group α, or alkynyl optionally substituted with one or more substitutent(s) selected from the Substituent group α and the like.

Specific examples of the substituent for the "substituted amino" include alkyl optionally substituted with one or more substitutent(s) selected from the Substituent group α' (cyano, halogen, alkyloxy, haloalkyloxy and non-aromatic carbocyclylalkyloxy), alkenyl optionally substituted with one or more substitutent(s) selected from the Substituent group α' alkynyl optionally substituted with one or more substitutent(s) selected from the Substituent group α' and the like.

Specific examples of the substituent for the "substituted amino" in R⁷ include alkyl, alkenyl, alkynyl, alkylcarbonyl, alkenylcarbonyl, alkynylcarbonyl, alkyloxycarbonyl, alkenyloxycarbonyl, alkynyloxycarbonyl, non-aromatic carbocyclylcarbonyl, aromatic carbocyclylcarbonyl, non-aromatic heterocyclylcarbonyl, and aromatic heterocyclylcarbonyl and the like.

Preferable embodiments include alkyl, alkenyl, alkynyl, alkylcarbonyl, alkyloxycarbonyl, non-aromatic carbocyclylcarbonyl and the like.

Specific examples of the substituent for the "substituted amino" in R¹¹ alkyl, alkenyl, alkynyl, alkylcarbonyl, alkenylcarbonyl, alkynylcarbonyl, alkyloxycarbonyl, alkenyloxycarbonyl, alkynyloxycarbonyl, non-aromatic carbocyclylcarbonyl, aromatic carbocyclylcarbonyl, non-aromatic heterocyclylcarbonyl and aromatic heterocyclylcarbonyl and the like.

Specific examples of the substituent for the "substituted sulfamoyl" in R¹² include alkylsulfamoyl, non-aromatic carbocyclylsulfamoyl, aromatic carbocyclylsulfamoyl. In particular, methylsulfamoyl, ethylsulfamoyl, isopropylsulfamoyl, dimethylsulfamoyl, isopropyl(methyl)sulfamoyl, cyclohexylsulfamoyl, phenylsulfamoyl and the like are preferable. "Alkyl","non-aromatic carbocyclyl" and "aromatic carbocyclyl" in "alkylsulfamoyl", "non-aromatic carbocyclylsulfamoyl", "aromatic carbocyclylsulfamoyl" may be further substituted with one or more substituents as defined herein.

Specific examples of the substituent for the "substituted carbamoyl" in R¹² include alkylcarbamoyl, non-aromatic carbocyclylcarbamoyl, aromatic carbocyclylcarbamoyl.

In particular, methylcarbamoyl, ethylcarbamoyl, isopropylcarbamoyl, dimethylcarbamoyl, isopropyl(methyl)carbamoyl, cyclohexylcarbamoyl, phenylcarbamoyl and the like are preferable. "Alkyl", "non-aromatic carbocyclyl" and "aromatic carbocyclyl" in "alkylcarbamoyl", "non-aromatic carbocyclylcarbamoyl", "aromatic carbocyclylcarbamoyl" may be further substituted with one or more substituents as defined herein.

Especially preferable embodiments of the compounds of the present invention are described below. 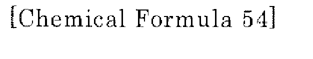 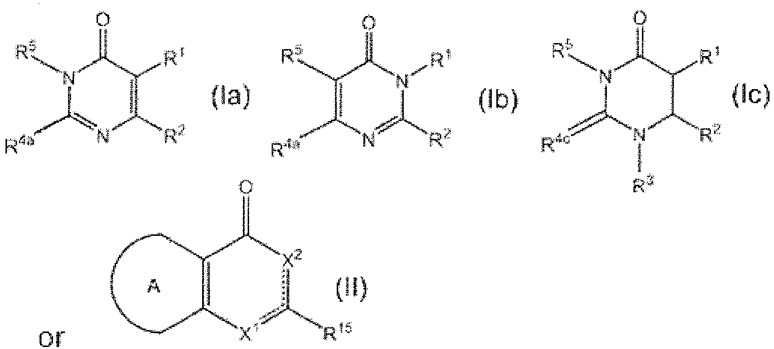 The compound represented by formula (Ia) and formula (Ib): 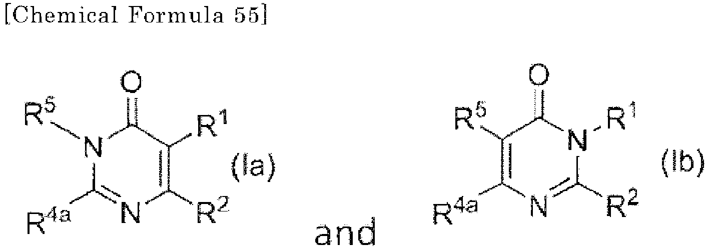 is preferable. The compound represented by formula (Ia): 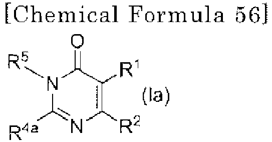 is especially preferable examples include

Preferred embodiments of R¹, R², R³, R^{4a}, R^{4b}, R^{5a} and R^{5b} in a compound represented by formula (I): 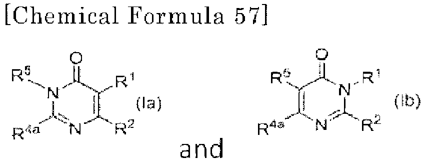 are described below. Compounds having possible combination of the substituents in the following embodiments are preferable.

R¹ includes hydrogen, halogen, hydroxy, formyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl;

Preferable embodiments of R¹ include hydrogen or halogen.

R² include a group represented by formula: 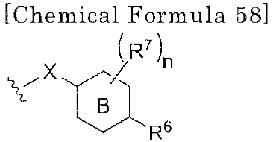
X is a single bond or O,
Ring B is a benzene ring, a 6-membered aromatic heterocycle, a non-aromatic carbocyclic or 6-membered non-aromatic heterocyclic ring,
R⁶ is halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
R⁷ includes each independently, halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl , substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl.

Preferable embodiments of R² include a group represented by formula:

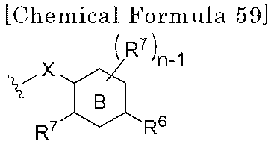

Preferable embodiments of x include a bond.

The other more preferable embodiments include O.

Preferable embodiments of Ring B include a benzene ring or 6-membered aromatic heterocycle.

The other more preferable embodiments include 6-membered non-aromatic carbocycle or 6-membered non-aromatic heterocycle.

Especially preferable embodiments include a benzene ring.

Preferable embodiments of R⁶ include halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted, or unsubstituted alkenyloxy, substituted or unsubstituted alkynyloxy, substituted or unsubstituted alkylthio, substituted or unsubstituted alkynylthio, substituted or unsubstituted alkynylthio.

The other more preferable embodiments of R ⁶ include substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl.

Especially preferable embodiments of R⁶ include halogen, cyano, alkyl, optionally substituted with halogen, or alkyloxy optionally substituted with halogen.

Preferable embodiments of R⁷ include each independently, halogen, hydroxy, cyano, formyl, carboxy, substituted or unsubstituted alkyl, substituted or unsubstituted alkynyl, substituted or unsubstituted alkynyl, substituted or unsubstituted amino , substituted or unsubstituted alkyloxy, substituted or unsubstituted alkenyloxy, substituted or unsubstituted alkynyloxy, substituted or unsubstituted alkylthio, substituted or unsubstituted alkenylthio or substituted or unsubstituted alkynylthio.

The other more preferable embodiment of R⁷ include each independently substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl.

Especially preferable embodiments of R⁷ include each independently, hydroxyalkyl, alkylcarbonylamino, non-aromatic carbocyclylcarbonylamino or alkyloxycarbonylamino.

Preferable embodiments of n include integer of 0 to 2.

The other preferable embodiments of n include integer of 1 to 2.

Preferable embodiments of R^{4a} include each independently halogen, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl substituted, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclyl.

The other more preferable embodiments of R^{4a} include substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkynylthio or substituted or unsubstituted alkynylthio.

The other more preferable embodiments of R^{4a} include alkyl optionally substituted with one or more substitutent(s) selected from the Substituent group a(cyano, halogen, substituted or unsubstituted alkyloxy, substituted or unsubstituted alkenyloxy, substituted or unsubstituted alkynyloxy, substituted or unsubstituted non-aromatic carbocyclylalkyloxy, substituted or unsubstituted aromatic carbocyclylalkyloxy), alkenyl optionally substituted with one or more substitutent(s) selected from the Substituent group α, alkynyl optionally substituted with one or more substitutent(s) selected from the Substituent group α, alkylamino optionally substituted with one or more substitutent(s) selected from the Substituent group α, alkenylamino optionally substituted with one or more substitutent(s) selected from the Substituent group α, alkynylamino optionally substituted with one or more substitutent(s) selected from the Substituent group α, alkyloxy, optionally substituted with one or more substitutent(s) selected from the Substituent group α, alkenyloxy optionally substituted with one or more substitutent(s) selected from the Substituent group α, alkynyloxy optionally substituted with one or more substitutent(s) selected from the Substituent group α, alkylthio optionally substituted with one or more substitutent(s) selected from the Substituent group α, alkenylthio optionally substituted with one or more substitutent(s) selected from the Substituent group α, alkynylthio optionally substituted with one or more substitutent(s) selected from the Substituent group α.

Especially preferable embodiments of R^{1a} include alkyl optionally substituted with one or more substitutent(s) selected from the Substituent group a(cyano, halogen, substituted or unsubstituted alkyloxy, substituted or unsubstituted alkynyloxy, substituted or unsubstituted alkynyloxy, substituted or unsubstituted non-aromatic carbocyclylalkyloxy, substituted or unsubstituted aromatic carbocyclylalkyloxy).

R⁵ is a group represented by formula: R¹¹-(C(R^{10a})(R^{10b}))m-.
R^{10a} are each independently hydrogen, halogen or substituted or unsubstituted alkyl,
R^{10b} are each independently hydrogen, halogen or substituted or unsubstituted alkyl, or
R^{10a} and R^{10b} attached to the same carbon may be taken together to form substituted or unsubstituted carbocycle or substituted or unsubstituted heterocycle, Preferable embodiments of R^{10a} and R^{10b} include hydrogen, halogen or substituted or unsubstituted alkyl.

R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, non-aromatic substituted or unsubstituted heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, substituted or unsubstituted sulfamoyl, or substituted or unsubstituted amino.

Preferable embodiments of R¹¹ include carboxy, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted non-aromatic heterocyclyloxycarbonyl, substituted or unsubstituted carbamoyl, substituted or unsubstituted sulfamoyl, or substituted or unsubstituted amino.

The other more preferable embodiments of R¹¹ include substituted or unsubstituted aromatic carbocyclyl, substituted or unsubstituted aromatic heterocyclyl, substituted or unsubstituted non-aromatic carbocyclyl or substituted or unsubstituted non-aromatic heterocyclyl.

The other more preferable embodiments of R¹¹ include aromatic carbocyclyl optionally substituted with one or more substitutent(s) selected from the Substituent group β (carboxy, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or non-aromatic carbocyclyloxycarbonyl unsubstituted, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl and substituted or unsubstituted aromatic heterocyclylsulfonyl), aromatic heterocyclyl optionally substituted with one or more substitutent(s) selected from the Substituent group β, non-aromatic carbocyclyl optionally substituted with one or more substitutent(s) selected from the Substituent group β, or non-aromatic heterocyclyl optionally substituted with one or more substitutent(s) selected from the Substituent group β,

The other more preferable embodiments of R¹¹ include substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted non-aromatic heterocyclyloxycarbonyl, substituted or unsubstituted carbamoyl or substituted or unsubstituted sulfamoyl.

The other more preferable embodiments of R¹¹ include carbamoyl optionally substituted with one or more substitutent(s) selected from the Substituent group Y (substituted or unsubstituted alkyl, substituted or unsubstituted alkynyl, substituted or unsubstituted alkynyl, substituted or unsubstituted aromatic carbocyclyl, substituted or unsubstituted aromatic heterocyclyl, substituted or unsubstituted non-aromatic carbocyclyl and substituted or unsubstituted non-aromatic heterocyclyl).

m is integer of 1 to 6.

Preferable embodiments of in include integer of 1 to 3.

The other more preferable embodiments of m include 1.

Preferred embodiments of R^{1'}, R^{4'}, X^{1'}, X^{2'} and Ring A in a compound represented by formula (II'): 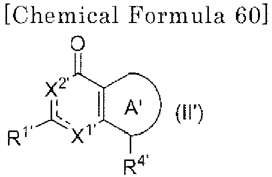 are described below. Compounds having possible combination of the substituents in the following embodiments are preferable.

R^{1'} is substituted or unsubstituted aromatic carbocyclyl, substituted or unsubstituted aromatic heterocyclyl, substituted or unsubstituted non-aromatic carbocyclyl or substituted or unsubstituted non-aromatic heterocyclyl.

Preferable embodiments of R^{1'} include substituted or unsubstituted aromatic carbocyclyl, or substituted or unsubstituted aromatic heterocyclyl.
The other more preferable embodiments of R¹¹ include substituted or unsubstituted aromatic carbocycly.
The other more preferable embodiments of R^{1'} include a group represented by formula: 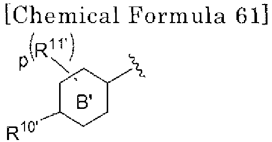
wherein Ring B' is a benzene ring, a 6-membered aromatic heterocycle, a non-aromatic carbocycle, or 6-membered non-aromatic heterocycle.
R¹⁰ is halogen, hydroxy, cyano, formyl, carboxy, substituted or unsubstituted alkyl, substituted or unsubstituted alkynyl, substituted or unsubstituted alkynyl, substituted or unsubstituted amino, substituted or unsubstituted alkyloxy, substituted or unsubstituted alkynyloxy, substituted or unsubstituted alkynyloxy,
R^{11'} are each independently, halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkynyloxy, substituted or unsubstituted alkynyloxy, substituted or unsubstituted alkylthio, substituted or unsubstituted alkenylthio, substituted or unsubstituted alkenylthio, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
p is integer of 0 to 4.

Preferable embodiments of Ring B' include a benzene ring.

The other more preferable embodiments of Rng B' include pyridine, pyrimidine or pyrazine.

Preferable embodiments of R^{10'} include halogen, cyano, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy or a substituted or unsubstituted alkynyloxy.

Preferable embodiments of R^{11'} include each dependentlly halogen, cyano, substituted or unsubstituted alkyl, substituted or unsubstituted alkynyl, substituted or unsubstituted alkynyl, substituted or unsubstituted amino, substituted or unsubstituted alkyloxy, substituted or unsubstituted alkenyloxy, substituted or unsubstituted alkynyloxy substituted or unsubstituted carbamoyl or substituted or unsubstituted sulfamoyl.

The partial structure represented by formula: 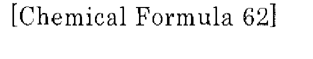 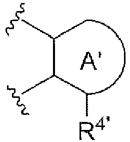 is a partial structure represented by formula: 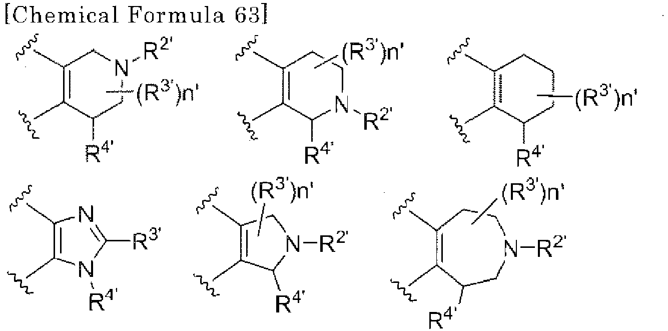 wherein the symbols in formula are the same meaning as defined above.

Preferable embodiments include a partial structure represented by formula: 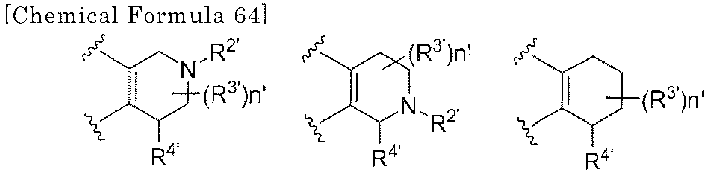 the symbols in formula are the same meaning as defined above.

The more preferable embodiments include a partial structure represented by formula: 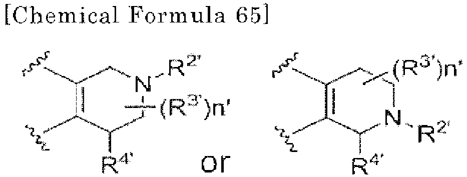 the symbols in formula are the same meaning as defined above.

R^{2'} is hydrogen, substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl or substituted or unsubstituted sulfamoyl.

Preferable embodiments of R^{2'} include substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted substituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl or substituted or unsubstituted carbamoyl.

The more preferable embodiments of R^{2'} include substituted or unsubstituted alkylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl or substituted or unsubstituted carbamoyl.

The other more preferable embodiments of R² include substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl or substituted or unsubstituted sulfamoyl.

The other more preferable embodiments of R² include substituted or unsubstituted alkylsulfonyl, or substituted or unsubstituted non-aromatic carbocyclylsulfonyl.

R^{3'} is each independently hydroxy, halogen, cyano, substituted or unsubstituted alkyl, substituted or unsubstituted alkenyl or substituted or unsubstituted alkynyl,
two R^{3'} attached to the same carbon atom may be taken together to form oxo, non-aromatic carbocycle or substituted or unsubstituted aromatic heterocycle or substituted or unsubstituted non-aromatic heterocycle, or
two R^{3'} attached to the different carbon atom may be taken together to form non-aromatic carbocycle or substituted or unsubstituted non-aromatic heterocycle.

Preferable embodiments of R^{3'} include each independently hydroxy, halogen, cyano, substituted or unsubstituted alkyl, substituted or unsubstituted alkenyl or substituted or unsubstituted alkynyl.

The more preferable embodiments of R^{3'} include halogen, cyano, substituted or unsubstituted alkyl.

The more preferable embodiments of R^{3'} include substituted or unsubstituted alkyl.

The other preferable embodiments of R^{3'} include two R^{3'} attached to the same carbon atom may be taken together to form oxo, non-aromatic carbocycle or substituted or unsubstituted aromatic heterocycle or substituted or unsubstituted non-aromatic heterocycle.

The other more preferable embodiments of R^{3'} include two R^{3'} attached to the same carbon atom may be taken together to form oxo.

The more preferable embodiments of R^{3'} include two R^{3'} attached to the different carbon atom may be taken together to form non-aromatic carbocycle or substituted or unsubstituted non-aromatic heterocycle.

The other more preferable embodiments of R^{3'} include two R^{3'} attached to the different carbon atom may be taken together to form substituted or unsubstituted non-aromatic heterocycle.

n' is integer of 0 to 4.

Preferable embodiments of n' include 0.

The other more preferable embodiments of n' include integer of 1 to 4.

R^{4'} is C1-C2 alkyl substituted with one or more substituents selected from Substituent group A consisting of substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted substituted alkynylthio, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio and substituted or unsubstituted amino, substituted or unsubstituted C3-C10 alkyl, substituted or unsubstituted C3-C10 alkenyl, substituted or unsubstituted C3-C10 alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkyloxy, substituted or unsubstituted alkenyloxy, substituted or unsubstituted alkynyloxy or a substituted or unsubstituted amino. Preferable embodiments of R^{4'} include C1-C2 alkyl substituted with one or more substituents selected from Substituent group A, substituted or unsubstituted C3-C10 alkyl, substituted or unsubstituted C3-C10 alkenyl, or substituted or unsubstituted C3-C10 alkynyl.

The more preferable embodiments of R^{4'} include substituted or unsubstituted C3-C10 alkyl, substituted or unsubstituted C3-C10 alkenyl, or substituted or unsubstituted C3-C10 alkynyl.

The more preferable embodiments of R^{4'} include substituted or unsubstituted C3-C10 alkyl.

When the partial structure represented by formula: 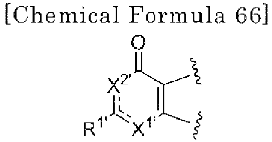 is (i) a partial structure represented by formula: 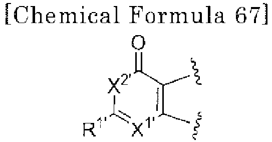
X^{1'} is N or C (R^{5'}),
R^{5'} is hydrogen, halogen, substituted or unsubstituted alkyl, substituted or unsubstituted alkenyl or substituted or unsubstituted alkynyl,
X^{2'} is N (R^{6'}) or C (R^{7'}) (R^{8'}),
R^{6'} is hydrogen, substituted or unsubstituted alkyl, substituted or unsubstituted alkenyl or substituted or unsubstituted alkynyl, and
R^{7'} and R^{8'} are each independently hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy or a substituted or unsubstituted amino, and
when the partial structure represented by the above formula is (ii) a partial structure represented by formula: 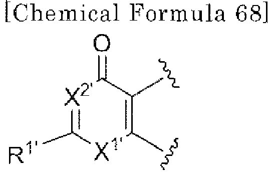
   X^{1'} is O,
   X^{2'} is N or C (R^{9'}), and
   R^{9'} is hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or substituted or unsubstituted alkynyloxy or a substituted or unsubstituted amino.

Preferable embodiments of the partial structure represented by formula: 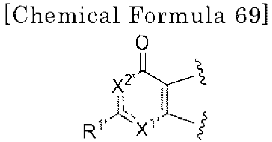 include a partial structure represented by formula: 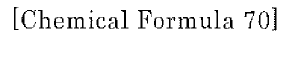 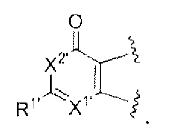
X^{1'} is N or C (R^{5'}),
R^{5'} is hydrogen, halogen, substituted or unsubstituted alkyl, substituted or unsubstituted alkenyl or substituted or unsubstituted alkynyl,
X^{2'} is N (R^{6'}) or C (R^{7'}) (R^{8'}),
R^{6'} is hydrogen, substituted or unsubstituted alkyl, substituted or unsubstituted alkenyl or substituted or unsubstituted alkynyl,
R^{7'} and R^{8'} are each independently hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy or a substituted or unsubstituted amino.

The more preferable embodiments of the partial structure represented by formula: 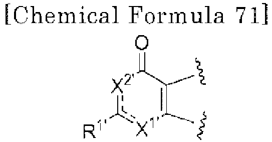 include a partial structure represented by formula: 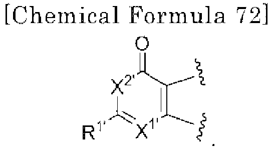
X^{1'} is N,
X^{2'} is N (R^{6'}),
R^{6'} is hydrogen, substituted or unsubstituted alkyl, substituted or unsubstituted alkenyl or substituted or unsubstituted alkynyl.

The other preferable embodiments of the partial structure represented by formula: 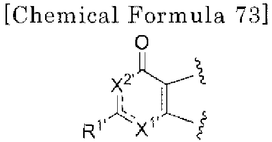 include a partial structure represented by formula: 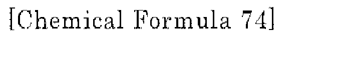 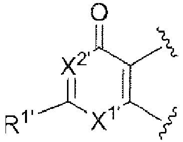
X^{1'} is O,
X^{2'} is N or C (R^{9'}),
R^{9'} is hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or substituted or unsubstituted alkynyloxy or a substituted or unsubstituted amino.

The other more preferable embodiments of the partial structure represented by formula: 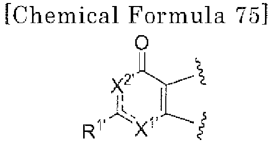 include a partial structure represented by formula: 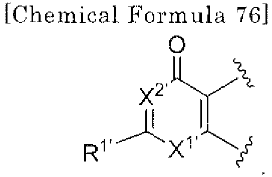
X^{1'} is O,
X^{2'} is C (R^{9'}),
R^{9'} is hydrogen.

Especially the following embodiments are preferable.
(i) A compound according to the formula (Ia'), or its pharmaceutically acceptable salt: 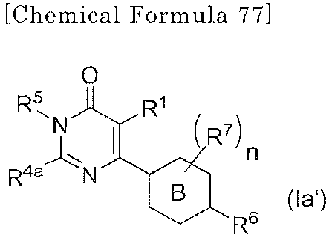 wherein
   R¹ is hydrogen,
   R⁶ is halogen, cyano, substituted or unsubstituted alkyl, or substituted or unsubstituted alkyloxy,
   R⁷ are each independently, halogen, cyano, substituted or unsubstituted alkyl, substituted or unsubstituted amino, or substituted or unsubstituted alkyloxy,
   n is integer of 0 to 4,
   R⁵ is a group represented by formula: R¹¹-(C(R^{10a})(R^{10b}))ₘ-
      wherein
      R^{10a} are each independently hydrogen, halogen or substituted or unsubstituted alkyl,
      R^{10b} are each independently hydrogen, halogen or substituted or unsubstituted alkyl,
      R¹¹ is carboxy, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, substituted or unsubstituted sulfamoyl, or substituted or unsubstituted amino,
      m is integer of 1 to 6, and
      R^{4a} is substituted or unsubstituted alkyl.
(ii) A compound according to the formula (Ia'), or its pharmaceutically acceptable salt: 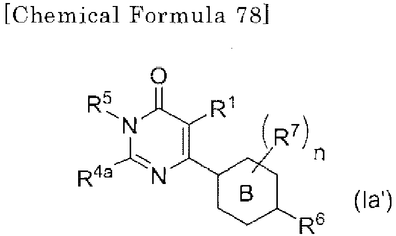
   R¹ is hydrogen,
   R⁶ is halogen, cyano, substituted or unsubstituted alkyl, or substituted or unsubstituted alkyloxy,
   R⁷ are each independently, halogen, cyano, substituted or unsubstituted alkyl, substituted or unsubstituted amino, or substituted or unsubstituted alkyloxy,
   n is integer of 0 to 4,
   R⁵ is a group represented by formula: R¹¹-(C(R^{10a})(R^{11b}))m-
      wherein
      R^{10a} are each independently hydrogen, halogen or substituted or unsubstituted alkyl,
      R^{10b} are each independently hydrogen, halogen or substituted or unsubstituted alkyl,
      R¹¹ is carboxy, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl
      m is integer of 1 to 6, and
      R^{4a} is substituted or unsubstituted alkyl.
(iii) A compound according to the formula (Ia'), or its pharmaceutically acceptable salt: 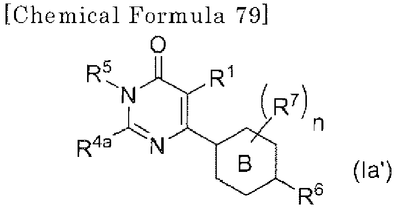
   R¹ is hydrogen,
   R⁶ is halogen, cyano, substituted or unsubstituted alkyl, or substituted or unsubstituted alkyloxy,
   R⁷ are each independently, halogen, cyano, substituted or unsubstituted alkyl, substituted or unsubstituted amino, or substituted or unsubstituted alkyloxy,
   n is integer of 0 to 4,
   R⁵ is a group represented by formula: R¹¹-(C(R^{10a})(R^{10b}))m-
      wherein
      R^{10a} are each independently hydrogen, halogen or substituted or unsubstituted alkyl,
      R^{10b} are each independently hydrogen, halogen or substituted or unsubstituted alkyl,
      R¹¹ is substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, substituted or unsubstituted sulfamoyl,
      m is integer of 1 to 6, and
   R^{4a} is substituted or unsubstituted alkyl.
(iv) A compound according to the formula (Ia'), or its pharmaceutically acceptable salt: 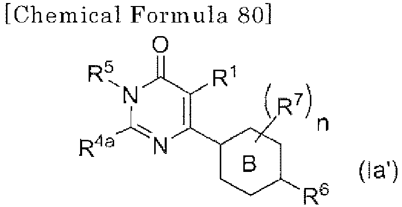
   R¹ is hydrogen,
   R⁶ is halogen, cyano, substituted or unsubstituted alkyl, or substituted or unsubstituted alkyloxy,
   R⁷ are each independently, halogen, cyano, substituted or unsubstituted alkyl, substituted or unsubstituted amino, or substituted or unsubstituted alkyloxy,
   n is integer of 0 to 4,
   R⁵ is a group represented by formula: R¹¹-(C(R^{10a})(R^{10b}))ₘ-
      wherein
      R^{10a} are each independently hydrogen, halogen or substituted or unsubstituted alkyl,
      R^{10b} are each independently hydrogen, halogen or substituted or unsubstituted alkyl,
      R¹¹ is substituted or unsubstituted amino,
      m is integer of 1 to 6, and
   R^{4a} is substituted or unsubstituted alkyl.

The compounds of this invention are not limited to a specific isomer but include all possible isomers (For example, imine-enamine isomer, geometrical isomer, diastereo isomer, optical isomer, rotamer etc.) and racemates or their mixture.

For example, the compounds of this invention include a tautomer as shown below.

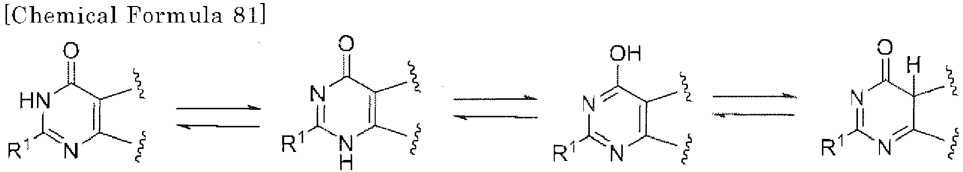

One or more hydrogen, carbon and/or other atoms in the compounds of the present invention may be replaced with isotopes of hydrogen, carbon and /or other atoms respectively. Examples of the isotopes include hydrogen, carbon, nitrogen, oxygen, phosphorus, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl respectively. The compounds of the present invention include compounds replaced with these isotopes. The compounds replaced with the above isotopes are useful as pharmaceuticals and include all of radiolabeled compounds of the compound of the present invetion. The present invention also includes a method of radiolabeling in the manufacture of the radiolabeled compounds. Such radiolabeled compounds are useful in the studies for metabolized drug pharmacokinetics and binding assay and also as a diagnostic tool.

A radiolabeled compound of the compounds of the present invention can be prepared using methods well-known in the art. For example, a tritium-labeled compound of formula (Ia), (Ib), (Ic) or (II) can be prepared by introducing a tritium into a compound of formula (Ia), (Ib), (Ic) or (II), through a catalytic dehalogenation using a tritium. This method comprises reacting with an appropriately-halogenated precursor of the compound of formula (Ia), (Ib), (Ic) or (II) with tritium gas in the presence of an appropriate catalyst, such as Pd/C, and in the presence or absent of a base. The other appropriate methods for preparing a tritium-labeled compound can be found in "Isotopes in the Physical and Biomedical Sciences, Vol.1, Labeled Compounds (Part A), Chapter 6 (1987)". A ¹⁴C-labeled compound can be prepared by using a raw material having ¹⁴C carbon.

Pharmaceutically acceptable salts of the compounds of the present invention include, for example, salts with alkaline metals such as lithium, sodium, potassium and the like; alkaline earth metals such as calcium, barium and the like; magnecium; transition metals such as zinc, iron and the like; ammonium; organic bases such as trimethylamine, trimethylamine, dicyclohexylamine, ethanolamine, diethanolamine, triethanolamine, meglumine, ethylenediamine, pyridine, picoline, quinolone and the like; amino acids; or inorganic acids such as hydrochloric acid, sulfuric acid, nitric acid, carbonic acid, hydrobromic acid, phosphoric acid, hydroiodic acid and the like; and organic acids such as formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethane sulfonic acid and the like, particularly salts with hydrochloric acid, sulfuric acid, phosphoric acid, tartaric acid and methanesulfonic acid. These salts can be formed according to conventional methods.

The compounds of the present invention or pharmaceutically acceptable salts thereof may exist in a form of solvate (e.g., hydrate and the like) and/or crystal polymorphs. The present invention encompasses those various solvates and crystal polymorphs. The "solvates" may be those wherein any numbers of solvent molecules (e.g. water molecules and the like) are coordinated with the compounds of the present invention. Whent the compounds of the present invention or pharmaceutically acceptable salts thereof are allowed to stand in the atmosphere, the compounds may absorb water, resulting in attachement of adsorbed water or formation of hydrates. Recrystallization of the compounds of the present invention or pharmaceutically acceptable salts thereof may produce crystal polymorphs.

The compounds of the present invention may form prodrugs. Such prodrugs are encompassed by the present invention. Prodrugs are derivatives of the compounds of the invention with a chemically or metabolically degradable group(s), and the compounds are converted to pharmaceutically active compounds of the invention through solvolysis or under physiological conditions in vivo. The prodrugs include compounds that are converted to a compound of the invention through enzymatic oxidation, reduction, hydrolysis or the like under physiological conditions in vivo, compounds that are converted to a compound of the invention through hydrolysis by gastric acid, and the like. Methods for selecting and preparing suitable prodrug derivatives are described in, for example, "Design of Prodrugs, Elsevier, Amsterdam, 1985". The prodrugs themselves may have some activity.

When the compound of the present invention or its pharmaceutically acceptable salt has hydroxyl group(s), the prodrugs may be acyloxy derivatives and sulfonyloxy derivatives that are prepared by, for example, reacting a compound having hydroxyl group(s) with suitable acyl halide, suitable acid anhydride, suitable sulfonyl chloride, suitable sulfonyl anhydride or mixed anhydride, or by reacting with a condensing agent. Examples include CH₃COO-, C₂H₅COO-, t-BuCOO-, C₁₅H₃₁COO-, PhCOO-, (m-NaOOCPh)COO-, NaOOCCH₂CH₂COO-, CH₃CH(NH₂)COO-, CH₂N(CH₃)₂COO, CH₃SO₃-, CH₃CH₂SO₃-, CF₃SO₃-, CH₂FSO₃-, CF₃CH₂SO₃-, p-CH₃-O-PhSO₃-, PhSO₃-, and p-CH₃PhSO₃-.

"Chronic kidney disease" means a condition where either or both of (1) kidney disorder (urine abnormalities such as proteinuria, e.g. microalbuminuria, abnormal urinary sediment, abnormal finding of clinical imaging such as single kidney and polycystic kidney disease, decreased renal function such as increased serum creatinine, electrolyte abnormalities such as hypokalemia due to tubular damage, and abnormal finding of renal tissue biopsy) and (2) deterioration in renal function less than 60 mL/min/1.73m² of GFR (glomerular filtration rate) is present for over three months.

The compounds of the present invention are produced according to general procetures as described below. Also, the compounds of the invention can be prepared according to other methods based on the knowledge in organic chemistry.

### Preparation of Compound b2

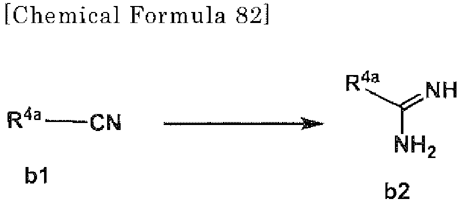

wherein R^{4a} is hydrogen, halogen, hydroxy, cyano, formyl, carboxy, substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyl, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted substituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfinyl.

The compound b2 can be obtained by reacting compound b1 with ammonium chloride or ammonium in the presence of an acid.

Examples of the solvent include toluene, methanol, ethanol, dichloroethane, DMF, THF and the like and these solvents may be used alone or in combination.

Ammonium hydroxide or ammonia may be used in 1 to 10 mole equivalents, preferably 1 to 3 mole equivalents of Compound b1.

Examples of the acid include trimethyl aluminum, hydrochloride and the like, acid may be used in 1 to 10 mole equivalents, preferably 1 to 3 mole equivalents of Compound b1.

The reaction temperature may be room temperature to 150 °C, preferably 50°C to 150°C.

The reaction time may be 1 to 24 hours, preferably 1 to 12 hours.

### Preparation of Compound b4

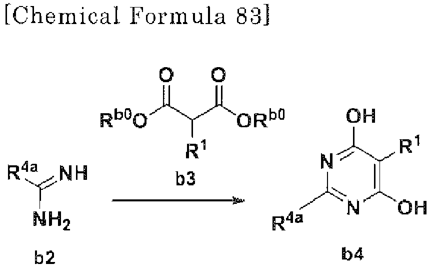

wherein R¹ is hydrogen, halogen, hydroxy, cyano, formyl, carboxy, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted amino, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylthio, substituted or unsubstituted aromatic carbocyclylthio, substituted or unsubstituted non-aromatic heterocyclylthio, substituted or unsubstituted aromatic heterocyclylthio, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted non-aromatic carbocyclylsulfinyl, substituted or unsubstituted aromatic carbocyclylsulfinyl, substituted or unsubstituted non-aromatic heterocyclylsulfinyl, substituted or unsubstituted aromatic heterocyclylsulfinyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl or substituted or unsubstituted aromatic heterocyclylsulfonyl, R^{b0} is substituted or unsubstituted alkyl, and the other symbols are as defined above.

Compound b4 can be obtained by reacting Compound b3 in the presence of base.

Examples of the reaction solvent include methanol, ethanol and the like, and the solvent may be used alone or in combination.

Examples of base may be tert-butoxy potassium, sodium methoxide, sodium ethoxide, potassium carbonate, sodium hydride and the like. The amount of the base may be 1 to 5 mole equivalents, preferably 1 to 3 mole equivalents of Compound b2.

The amount of Compound b3 may be 0.5 to 2 mole equivalents, preferably 0.5 to 1.5 mole equivalents of Compound b2.

The reaction temperature may be room temperature to 150°C, preferably 50 to 100°C.

The reaction time may be 0.1 to 12 hours, preferably 0.5 to 3 hours.

### Preparation of Compound b5

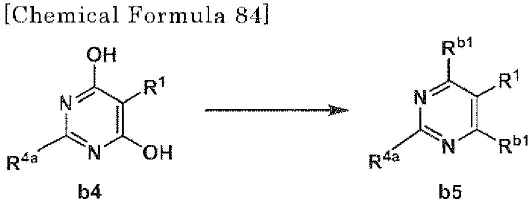

wherein R^{b1} is halogen such as chloro and bromo and the like, and the other symbols are as defined above.

Compound b5 can be obtained by reacting Compound b4 with the halogenating agent.

Examples of the halogenating agent may be phosphorus oxychloride, phosphorus oxybromide and the like The amount of the halogenating agent may be 2 mole equivalents to a large excess, preferably 5 mole equivalents to a large excess of Compound b4.

The reaction temperature may be room temperature to 150°C, preferably 50 to 100°C.

The reaction time may be 0.5 to 6 hours, preferably 1 to 3 hours.

### Preparation of Compound b7

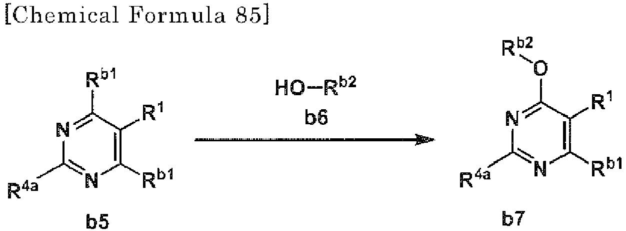

wherein R^{b2} is substituted or unsubstituted alkyl and the like, and the other symbols are as defined above.

Compound b7 can be obtained by reacting Compound b5 with Compound b6 in the presence of a base.

Examples of the reaction solvent include DMF, THF, methanol, toluene, dichloromethane, acetonitrile and the like, and the solvent may be used alone or in combination.

Examples of base may be sodium hydride, potassium carbonate, cesium carbonate, triethylamine and the like. The amount of the base may be 1 to 5 mole equivalents, preferably 1 to 3 mole equivalents of Compound b5.

Examples of Compound b6 may be p-methoxybenzyl alcohol, methanol, benzyl alcohol and the like. The amount of Compound b6 may be 1 to 5 mole equivalents, preferably 1 to 3 mole equivalents of Compound b5.

The reaction temperature may be -50°C to room temperature, preferably -30 to -10°C.

The reaction time may be 0.1 to 6 hours, preferably 0.1 to 1 hours.

### Preparation of Compound b8

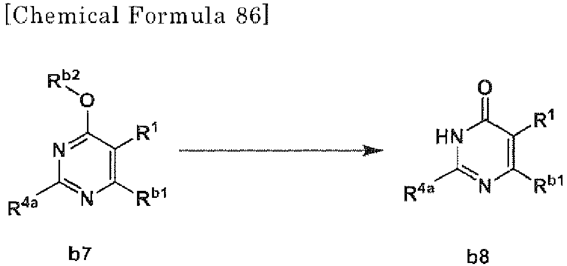

wherein each symbols is as defined above.

Compound b8 can be obtained by reacting Compound b7 with deprotecting agent.

Examples of the deprotecting agent include trifluoroacetic acid, hydrochloric acid, hydrobromic acid, ammonium hexanitratocerate (IV) and the like. The amount of the deprotecting agent may be 1 mole equivalents to a large excess, preferably 1 to 50 mole eqiovalents of Compound b7.

The reaction temperature may be -20°C to reflux temeparature, preferably under ice-cooling to room temperature.

The reaction time may be 0.1 to 6 hours, preferably 0.1 to 3 hours.

### Preparation of Compound b10

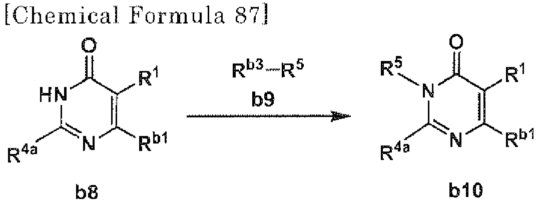

wherein R^{b3} is halogen such as chloro, bromo and the like, leaving group such as mesyl group, tosyl group and the like, R⁵ is a group of formula: R¹¹ -(C(R^{10a})(R^{10b})) m- wherein R^{10a} and R^{10b} are each independently hydrogen, halogen or substituted or unsubstituted alkyl, or R^{10a} and R^{10b} attached to the same carbon may be taken together to form substituted or unsubstituted carbocycle or substituted or unsubstituted heterocycle,
m is integer of 1 to 6,
R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubstituted aromatic heterocyclylcarbonyl, substituted or unsubstituted non-aromatic carbocyclylcarbonyl, non-aromatic substituted or unsubstituted heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted aromatic carbocyclyloxycarbonyl, substituted or unsubstituted aromatic heterocyclyloxycarbonyl, substituted or unsubstituted non-aromatic carbocyclyloxycarbonyl, substituted or unsubstituted aromatic heterocyclyloxycarbonyl unsubstituted, substituted or unsubstituted carbamoyl, substituted or unsubstituted sulfamoyl, or substituted or unsubstituted amino, and the other symbols are as defined above.

Compound b10 can be obtained by reacting Compound b8 with Compound b9 in the presence of a base.

Examples of the reaction solvent include THF, DMF, acetone, acetonitrile and the like, and the solvent may be used alone or in combination.

Examples of base may be sodium hydride, potassium carbonate, cesium carbonate, sodium methoxide, sodium ethoxide, tert-butoxy potassium and the like. The amount of the base may be 1 to 5 mole equivalents, preferably 1 to 3 mole equivalents of Compound b8.

The amount of Compound b9 may be 1 to 5 mole equivalents, preferably 1 to 3 mole equivalents of Compound b8.

The reaction temperature may be under ice-cooling to room temperature, preferably under ice-cooling to 60°C.

The reaction time may be 0.5 to 24 hours, preferably 1 to 6 hours.

### Preparation of Compound b15

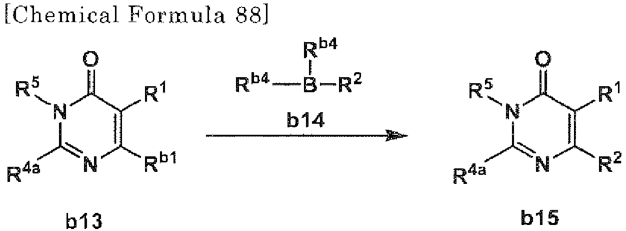

wherein R^{b4} is hydroxy, substituted or unsubstituted alkyloxy or two R^{b4} attached to the same carbon may be taken together to form substituted or unsubstituted alkylenedioxy, and the other symbols are as defined above.

Compound b15 can be obtained by reacting Compound b13 with Compound b14 in the presence of a metal catalyst and a base.

Examples of the solvent include DMF, toluene, methanol, ethanol, dioxane and the like and these solvents may be used alone or in combination.

Examples of the base include sodium carbonate, cesium carbonate, potassium carbonate, sodium hydroxide, potassium phosphate, tert-butoxy potassium and the like. The amount of the base may be 1 to 10 mole equivalents, preferably 1 to 3 mole equivalents of Compound b13.

The amount of Compound b14 may be 1 to 10 mole equivalents, preferably 1 to 3 mole equivalents of Compound b13.

Examples of the metal catalyst may be 1,1'-bis (diphenylphosphino) ferrocene palladium (II) dichloride - dichloromethane complex, chloro (2-dicyclohexyl phosphino-2',4',6'-triisopropyl-1,1'-biphenyl)[2-(2'-amino-1,1'-biphenyl)] palladium (II), tetrakis (triphenylphosphine) palladium and the like. The amount of the metal catalyst may be 0.01 to 1 mole equivalents, preferably 0.05 to 0.2 mole equivalents of Compound b13.

The reaction temperature may be 50 to 200°C, preferably 80 to 150°C.

The reaction time may be 0.1 to 12 hours, preferably 0.1 to 6 hours.

### Preparation of Compound b11

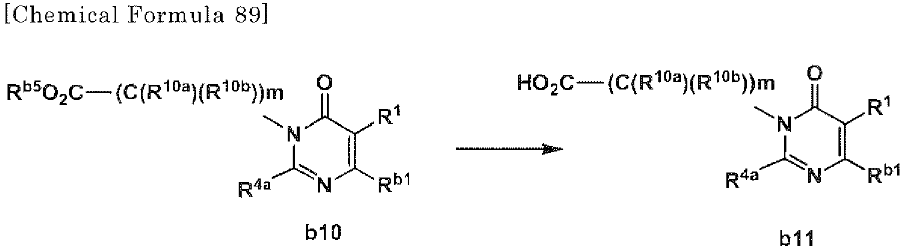

wherein R^{5b} is substituted or unsubstituted alkyl, substituted or unsubstituted alkenyl, substituted or alkynyl or unsubstituted etc., and the other symbols are as defined above.

Compound b11 can be obtained by hydrolyzing Compound b10.

Examples of the reaction solvent include THF, ethanol, water, dichloromethane, DMF, methanol, 1,4-dioxane, acetonitrile, toluene, ethyl acetate and the like, these solvents may be used alone or in combination.

Examples of the additive include sodium hydroxide, lithium hydroxide, potassium hydroxide, trimethyltin, hydrochloric acid, sulfuric acid and the like. The amount of the acid may be 1 to 10 mole equivalents, preferably 1 to 5 mole equivalents of Compound b10.

The reaction temperature may be under ice-cooling to reflux temperature, preferably room temperature.

The reaction time may be 0.1 to 24 hours, preferably 0.1 to 5 hours.

### Preparation of Compound b13

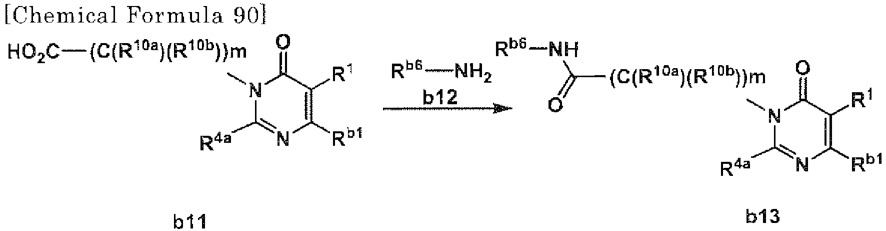

wherein R^{b6} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or substituted aromatic heterocyclyl or substituted or unsubstituted non-aromatic heterocyclyl, and the other symbols are as defined above.

Compound b13 can be obtained by reacting Compound b11 with amine b12 in the presence of base and condensing agents.

Examples of the reaction solvent include DMF, ethanol, water, dichloromethane, THF, methanol, 1,4-dioxane, acetonitrile, toluene, ethyl acetate and the like, and these solvents may be used alone or in combination.

Examples of the base include triethylamine, tert-butoxy potassium, potassium carbonate, cesium carbonate, diisopropylethylamine, DBU and the like. The amount of the base may be 1 to 10 mole equivalents, preferably 1 to 5 mole equivalents of Compound b11.

Examples of the condensing agent include HATU, WSC, DCC and the like. The amount of the condensing agent may be 1 to 10 mole equivalents, preferably 1 to 5 mole equivalents of Compound b11.

The amine b12 may be used in 1 to 10 mole equivalents, preferably 1 to 5 mole equivalents of Compound b11.

The reaction temperature may be under ice-cooling to reflux temperature, preferably room temperature.

The reaction time may be 0.1 to 24 hours, preferably 1 to 5 hours.

### Preparation of Compound b25

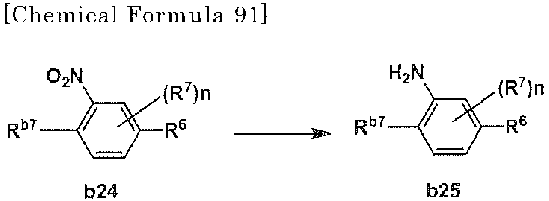

wherein R^{b7} is halogen such as chloro, bromo etc., leaving group such as mesyl, tosyl etc., R⁶ is halogen, hydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl, substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
R⁷ are each independently, halogen, bydroxy, cyano, formyl, carboxy, substituted or unsubstituted alkyl, substituted or unsubstituted alkenyl, substituted or unsubstituted alkynyl, substituted or unsubstituted amino, substituted or unsubstituted alkyloxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted alkylcarbonyl , substituted or unsubstituted alkenylcarbonyl, substituted or unsubstituted alkynylcarbonyl, substituted or unsubstituted alkyloxycarbonyl, substituted or unsubstituted alkenyloxycarbonyl, substituted or unsubstituted alkynyloxycarbonyl, substituted or unsubstituted carbamoyl , substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
n is integer of 0 to 4.

Compound b25 can be obtained by reacting Compound b24 with reductants.

Examples of the reaction solvent include ethanol, methanol, ethyl acetate, THF, dichloromethane and the like, and these solvents may be used alone or in combination.

Examples of the reductant include stannous chloride, iron, palladium - carbon and the like. The amount of the reductant may be 0.01 to 10 mole equivalents, preferably 0.05 to 5 mole equivalents of Compound b24.

The reaction temperature may be under ice-cooling to reflux temperature, preferably room temperature.

The reaction time may be 0.1 to 24 hours, preferably 0.5 to 6 hours.

### Preparation of Compound b26

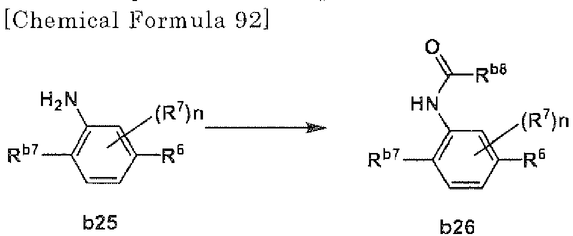

where R^{b8} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, and the other symbols are as defined above.

Compound b26 can be obtained by reacting Compound b25 with an acyl halide in the presence of base.

Examples of the reaction solvent include pyridine, dichloromethane, THF, ethyl acetate and the like, and these solvents may be used alone or in combination.

Examples of the base include sodium hydride, sodium hydroxide, potassium carbonate and the like. The amount of the base may be 1 to 5 equivalents, preferably 1 to 3 mole equivalents of Compound b25.

The reaction temperature may be under ice-cooling to reflux temperature, preferably room temperature to reflux temperature.

The reaction time may be 0.1 to 12 hours, preferably 0.5 to 3 hours.

### Preparation of Compound b28

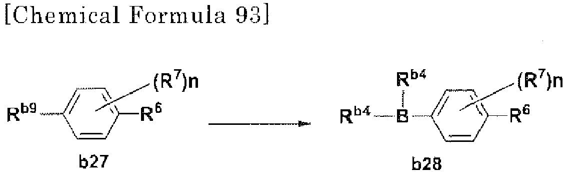

wherein R^{b9} is halogen such as chloro, bromo, etc., mesyl, leaving group such as mesyl, tosyl, etc., and the other symbols are as defined above.

Compound b28 can be obtained by reacting Compound b27 with a boronic acid ester in the presence of a base and a metal catalyst.

Examples of the solvent include dioxane, THF, toluene, DMF, dichloromethane and the like and these solvents may be used alone or in combination.

The amount of the boronic acid ester may be 1 to 10 mole equivalents, preferably 1 to 3 mole equivalents of Compound b27.

The reaction temperature may be room temperature to reflux temperature, preferably 50 °C to reflux temperature.

The reaction time may be 0.5 to 12 hours, preferably 0.5 to 6 hours.

### Preparation of Compound b30

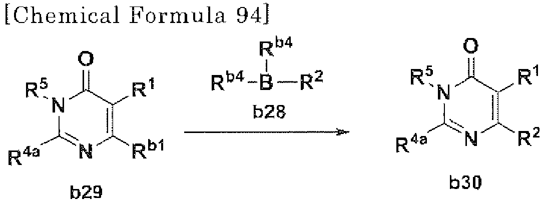

wherein R² is a group represented by formula: 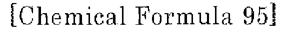 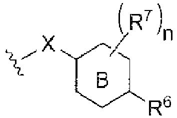
X is a single bond or O,
Ring B is a benzene ring, 6-membered aromatic heterocycle, non-aromatic carbocycle or 6-membered non-aromatic heterocycle, and the other symbols are as defined above.

Compound b30 can be obtained by reacting Compound b28 with Compound b29 in the presence of a metal catalyst and base.

Examples of the reaction solvent include DMF, THF, dioxane and the like, and the solvent may be used alone or in combination.

Examples of the base may be sodium carbonate, potassium carbonate, cesium carbonate and the like. The amount of the base may be 1 to 10 mole equivalents, preferably 1 to 3 mole equivalents of Compound b29.

Examples of the metal catalyst may be 1,1'-bis (diphenylphosphino) ferrocene - palladium (II) dichloride - dichloromethane complex, palladium acetate and the like. The amount of the metal catalyst may be 0.01 to 0.5 mole equivalents, preferably 0.05 to 0.2 mole equivalents of Compound b29.

The reaction temperature may be room temperature to reflux temperature, preferably room temperature to 100°C.

The reaction time may be 0.1 to 24 hours, preferably 1 to 12 hours.

### Preparation of Compound b35

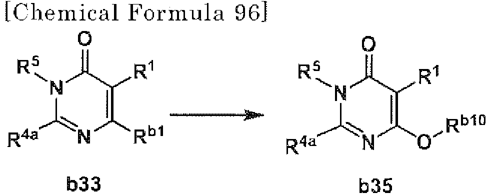

wherein R^{b10} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl, and the other symbols are the same meaning as defined above.

Compound b35 can be obtained by reacting Compound b33 with alcohol in the presence of a base.

Examples of the reaction solvent include DMF, THF, acetonitrile, dichloromethane and the like, and the solvent may be used alone or in combination.

Examples of the base may be sodium hydride, sodium hydroxide, potassium carbonate, tert-butoxy potassium, triethylamine, diisopropylethylamine and the like. The amount of the base may be 1 to 5 mole equivalents, preferably 1 to 3 mole equivalents of Compound b33.

The amount of the alcohol may be 1 to 5 mole equivalents, preferably 1 to 3 mole equivalents of Compound b33.

The reaction temperature may be room temperature to reflux temperature, preferably 50 to 100°C.

The reaction time may be 0.5 to 24 hours, preferably 1 to 6 hours.

### Preparation of Compound b41

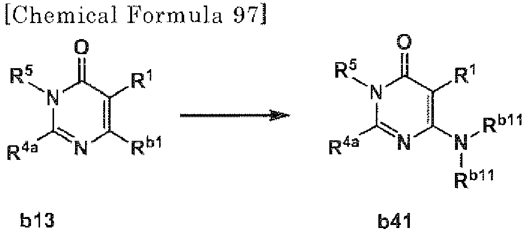

wherein R^{b11} are each independently hydrogen, substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, substituted or unsubstituted non-aromatic heterocyclyl or the two R^{b11} may be taken together to form substituted or unsubstituted non-aromatic heterocycle, and the other symbols are the same meaning as defined above.

Compound b41 can be obtained by reacting Compound b13 with amine in the presence of the base.

Examples of the reaction solvent include DMF, THF, acetonitrile, dichloromethane and the like, and the solvent may be used alone or in combination.

Examples of the base may be sodium hydride, sodium hydroxide, potassium carbonate, tert-butoxy potassium, triethylamine, diisopropylethylamine and the like. The amount of the base may be 1 to 5 mole equivalents, preferably 1 to 3 mole equivalents of Compound b13.

The amount of the amine may be 1 to 5 mole equivalents, preferably 1 to 3 mole equivalents of Compound b13.

The reaction temperature may be room temperature to reflux temperature, preferably 50 to 100°C.

The reaction time may be 0.5 to 24 hours, preferably 1 to 6 hours.

### Preparation of Compound b49

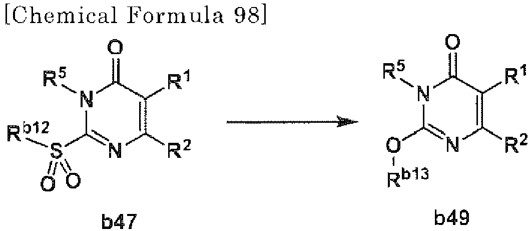

wherein R^{b12} is substituted or unsubstituted alkyl, substituted or unsubstituted alkenyl, substituted or unsubstituted alkynyl, R^{b13} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, and the other symbols are the same meaning as defined above.

Compound b49 can be obtained by reacting Compound b47 with alcohol in the presence of a base.

Examples of the reaction solvent include DMF, THF, acetonitrile, dichloromethane and the like, and the solvent may be used alone or in combination.

Examples of the base may be sodium hydride, sodium hydroxide, potassium carbonate, tert-butoxy potassium, triethylamine, diisopropylethylamine and the like. The amount of the base may be 1 to 5 mole equivalents, preferably 1 to 3 mole equivalents of Compound b47.

The amount of the alcohol may be 1 to 5 mole equivalents, preferably 1 to 3 mole equivalents of Compound b47.

The reaction temperature may be -78 °C to room temperature, preferably -78 °C to - 20 °C.

The reaction time may be 0.1 to 6 hours, preferably 0.5 to 3 hours.

### Preparation of Compound b57

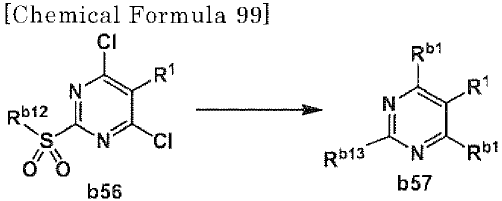

The symbols in the formula are the same meaning as defined above.

Compound b57 can be obtained by reacting Compound b56 with a metal catalyst.

Examples of the solvent include THF, benzene, toluene, acetonitrile, diethyl ether, dichloromethane and the like and these solvents may be used alone or in combination.

Examples of the metal catalyst may be alkyl magnesium bromide, aryl magnesium bromide, alkyl lithium and the like. The amount of the metal catalyst may be 1 to 5 mole equivalents, preferably 1 to 3 mole equivalents of Compound b56.

The reaction temperature may be under ice-cooling to reflux temperature,, preferably under ice-cooling to room temperature.

The reaction time may be 0.1 to 6 hours, preferably 0.5 to 3 hours.

### Preparation of Compound b68

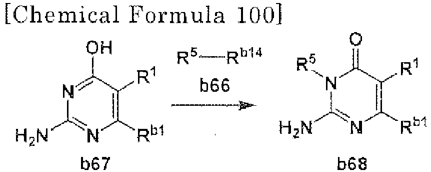

wherein R^{b14} is halogen such as chloro, bromo and the like, leaving group such as mesyl group, tosyl group and the like, and the other symbols are as defined above.

Compound b67 can be obtained by reacting Compound b67 with Compound b66 in the presence of base.

Examples of the reaction solvent include THF, DMF, acetone, acetonitrile and the like, and these solvents may be used alone or in combination.

Examples of the base include sodium hydride, potassium carbonate, cesium carbonate, sodium methoxide, sodium ethoxide, tert-butoxy potassium and the like. The amount of the base may be 1 to 5 equivalents, preferably 1 to 3 mole equivalents of Compound b67.

The reaction temperature may be under ice-cooling to 100°C, preferably under ice-cooling to 60°C.

The reaction time may be 0.5 to 24 hours, preferably 1 to 6 hours.

### Preparation of Compound b70

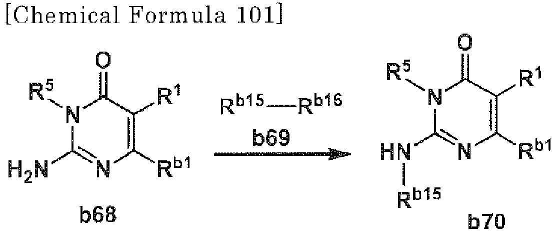

wherein R^{b15} is substituted or unsubstituted alkyl, substituted or unsubstituted alkenyl, substituted or unsubstituted alkynyl, substituted or unsubstituted aromatic carbocyclic group, a substituted or unsubstituted non-aromatic carbocyclic ring Shikimoto, a substituted or unsubstituted aromatic heterocyclic group, or a substituted or unsubstituted non-aromatic heterocyclic group, R ^{b16} is chloro, halogen bromo or mesyl leaving group, and other symbols tosyl, etc. it is as defined above.

Compound b70 can be obtained by reacting Compound b68 with Compound b69 in the presence of a base.

Examples of the reaction solvent include THF, DMF, acetone, acetonitrile and the like, and the solvent may be used alone or in combination.

Examples of the base may be sodium hydride, potassium carbonate, cesium carbonate, sodium methoxide, sodium ethoxide, tert-butoxy potassium and the like. The amount of the base may be 1 to 5 mole equivalents, preferably 1 to 3 mole equivalents of Compound b68.

The amount of Compound b69 may be 1 to 5 mole equivalents, preferably 1 to 3 mole equivalents of Compound b68.

The reaction temperature may be under ice-cooling to 100°C, preferably under ice-cooling to 60°C.

The reaction time may be 0.5 to 24 hours, preferably 1 to 6 hours.

### Preparation of Compound b70

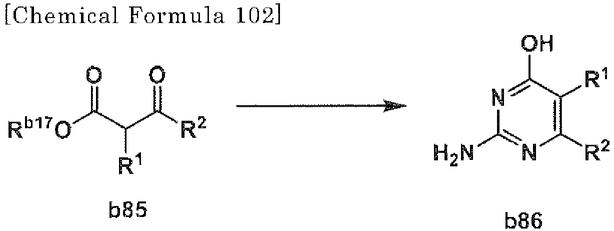

wherein R^{b17} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, and the other symbols are as defined above.

Compound b86 can be obtained by reacting Compound b85 with guanidine.

Examples of the reaction solvent include methanol, ethanol and the like, and these solvents may be used alone or in combination.

The amount of the guanidine may be 0.5 to 2 equivalents, preferably 1 to 2 mole equivalents of Compound b85.

The reaction temperature may be room temperature to 150°C, preferably 50 to 100°C.

The reaction time may be 0.1 to 12 hours, preferably 0.5 to 6 hours.

### Preparation of Compound b60

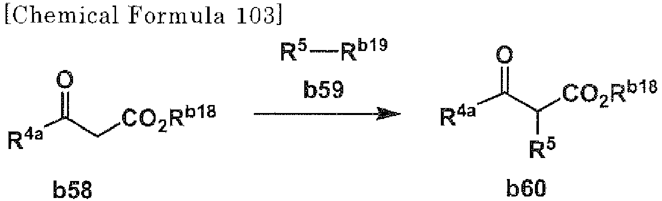

wherein R^{b18} is substituted or unsubstituted alkyl, substituted or unsubstituted alkenyl, substituted or unsubstituted alkynyl, substituted or unsubstituted aromatic carbocyclyl, substituted or unsubstituted non-aromatic carbocyclyl, substituted or unsubstituted aromatic heterocyclyl, or substituted or unsubstituted non-aromatic heterocyclyl, R^{b19} is halogen such as chloro, bromo, leaving group such as mesyl, and the other symbols are as defined above.

Compound b60 can be obtained by reacting Compound b58 with Compound b59 in the presence of base.

Examples of the reaction solvent include THF, DMF, dichloromethane and the like, and these solvents may be used alone or in combination.

Examples of the base include potassium carbonate, sodium hydride, tert-butoxy potassium, n- butyl lithium and the like. The amount of the base may be 1 to 5 equivalents, preferably 1 to 3 mole equivalents of Compound b58.

The reaction temperature may be under ice-cooling to reflux temeparature, preferably room temperature to 50°C.

The reaction time may be 1 to 24 hours, preferably 3 to 18 hours.

### Preparation of Compound b62

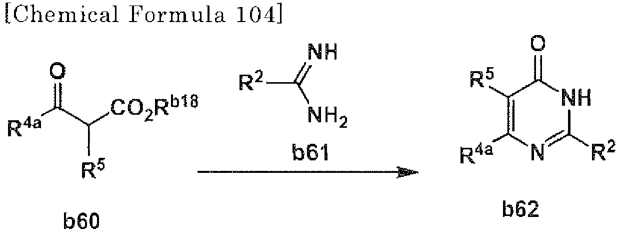

wherein each symbols is as defined above.

Compound b62 can be obtained by reacting Compound b60 with Compound b61 in the presence of base.

Examples of the reaction solvent include methanol, ethanol and the like, and these solvents may be used alone or in combination.

Examples of the base include sodium tert-butoxy potassium, sodium methoxide, sodium ethoxide, potassium carbonate, sodium hydride and the like. The amount of the base may be 1 to 5 equivalents, preferably 1 to 3 mole equivalents of Compound b60.

The reaction temperature may be room tenperature to 150°C, preferably 50 to 100°C.

The reaction time may be 0.1 to 12 hours, preferably 0.5 to 3 hours.

### Preparation of Compound b91

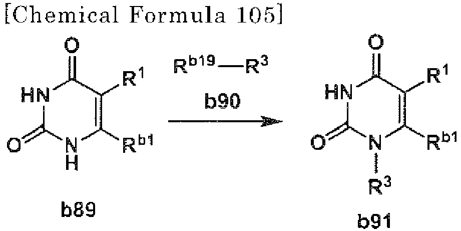

wherein R^{b19} is halogen such as chloro, bromo etc., leaving group such as tosyl, etc., R³ is substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl substituted or unsubstituted alkylcarbonyl, substituted or unsubstituted alkenylcarbonyl, substituted or unsubstituted alkynylcarbonyl, substituted or unsubstituted aromatic carbocyclylcarbonyl, substituted or unsubstituted aromatic heterocyclylcarbonyl, substituted or unsubstituted non-aromatic carbocyclylcarbonyl, substituted or unsubstituted non-aromatic heterocyclylcarbonyl, substituted or unsubstituted alkylsulfonyl, substituted or unsubstituted alkenylsulfonyl, substituted or unsubstituted alkynylsulfonyl, substituted or unsubstituted aromatic carbocyclylsulfonyl, substituted or unsubstituted aromatic heterocyclylsulfonyl, substituted or unsubstituted non-aromatic carbocyclylsulfonyl or substituted or unsubstituted non-aromatic heterocyclylsulfonyl, and the other symbols are as defined above.

Compound b91 can be obtained by reacting Compound b89 with Compound b90 in the presence of base.

Examples of the reaction solvent include THF, DMF, acetone, acetonitrile and the like, and these solvents may be used alone or in combination.

Examples of the base include sodium hydride, potassium carbonate, cesium carbonate, sodium methoxide, sodium ethoxide, tert-butoxy potassium and the like. The amount of the base may be 1 to 5 equivalents, preferably 1 to 3 mole equivalents of Compound b90.

The reaction temperature may be under ice-cooling to 100°C, preferably under ice-cooling to 60°C.

The reaction time may be 0.5 to 24 hours, preferably 1 to 6 hours.

### Preparation of Compound b93

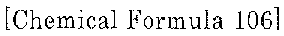

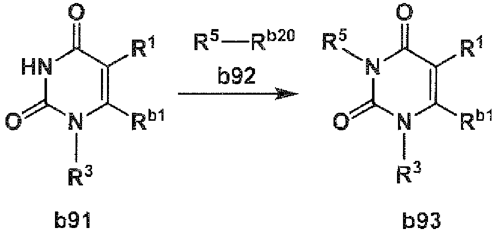

wherein R^{b20} is halogen such as chloro, bromo etc., leaving group such as mesyl, tosyl, etc., and the other symbols are as defined above.

Compound b93 can be obtained by reacting Compound b91 with Compound b92 in the presence of base.

Examples of the reaction solvent include THF, DMF, acetone, acetonitrile and the like, and these solvents may be used alone or in combination.

Examples of the base include sodium hydride, potassium carbonate, cesium carbonate, sodium methoxide, sodium ethoxide, tert-butoxy potassium and the like. The amount of the base may be 1 to 5 equivalents, preferably 1 to 3 mole equivalents of Compound b91.

The reaction temperature may be under ice-cooling to 100°C, preferably under ice-cooling to 60°C.

The reaction time may be 0.5 to 24 hours, preferably 1 to 6 hours.

### Preparation of Compound b94

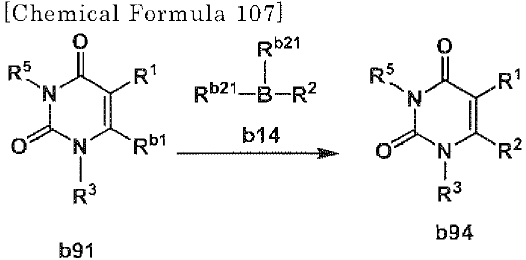

wherein R^{b21} is hydroxy, substituted or unsubstituted alkyloxy or two R^{b21} may be taken together to form substituted or unsubstituted alkylenedioxy, and the other symbols are as defined above.

Compound b94 can be obtained by reacting Compound b91 with Compound b14 in the presence of a metal catalyst and a base.

Examples of the solvent include DMF, THF, dioxane and the like and these solvents may be used alone or in combination.

Examples of the base include sodium carbonate, potassium carbonate, cesium carbonate and the like. The amount of the base may be 1 to 10 mole equivalents, preferably 1 to 3 mole equivalents of Compound b91.

The amount of Compound b14 may be 1 to 10 mole equivalents, preferably 1 to 3 mole equivalents of Compound b91.

Examples of the metal catalyst may be 1,1'-bis (diphenylphosphino) ferrocene - palladium (II) dichloride - dichloromethane, complex, palladium acetate and the like. The amount of the metal catalyst may be 0.01 to 0.5 mole equivalents, preferably 0.05 to 0.2 mole equivalents of Compound b91.

The reaction temperature may be room temperature to reflux temperature, preferably room temperature to 100°C.

The reaction time may be 0.1 to 24 hours, preferably 1 to 12 hours.

### Preparation of Compound b97

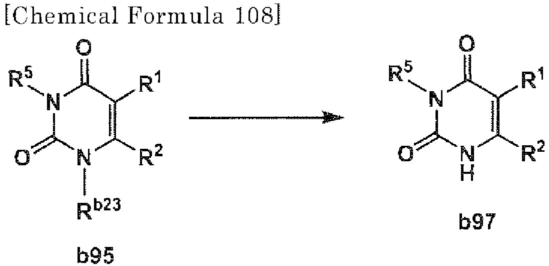

wherein R^{b23} is a protecting group such as substituted or unsubstituted phenylmethyl etc., and the other symbols are as defined above.

Compound b97 can be obtained by reacting Compound b95 with deprotecting agent.

Examples of the reaction solvent include acetonitrile, water, trifluoroacetic acid, dichloromethane, methanol, ethanol and the like, and the solvent may be used alone or in combination.

Examples of the deprotecting agent include ammonium hexanitratocerate (IV) palladium - carbon, trifluoroacetic acid, tetrabutylammonium fluoride and the like. The amount of the deprotecting agent may be 0.01 mole equivalents to a large excess, preferably 0.05 to to a large excess of Compound b95.

The reaction temperature may be under ice-cooling to 100°C, preferably room temperature to 70°C.

The reaction time may be 0.5 to 12 hours, preferably 1 to 6 hours.

### Preparation of Compound b99 and b100

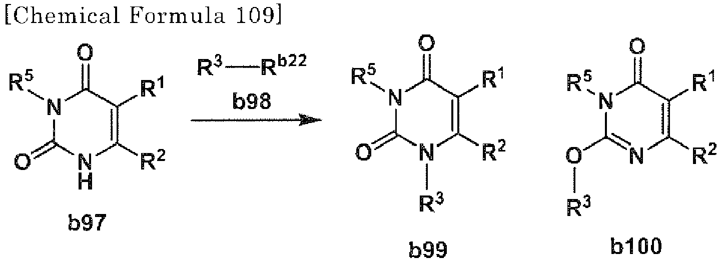

wherein each symbols is as defined above.

Compound b99 and b100 can be obtained by reacting Compound b97 with Compound b98 in the presence of a base.

Examples of the solvent include THF, DMF, acetone, acetonitrile and the like and these solvents may be used alone or in combination.

Examples of the base include triethylamine, sodium hydride, potassium carbonate, cesium carbonate, sodium methoxide, sodium ethoxide, tert-butoxy potassium and the like. The amount of the base may be 1 to 5 mole equivalents, preferably 1 to 3 mole equivalents of Compound b97.

The amount of Compound b98 may be 1 to 5 mole equivalents, preferably 1 to 3 mole equivalents of Compound b97.

The reaction temperature may be under ice-cooling to 60°C. The reaction time may be 0.5 to 24 hours, preferably 1 to 6 hours.

### Preparation of Compound c2

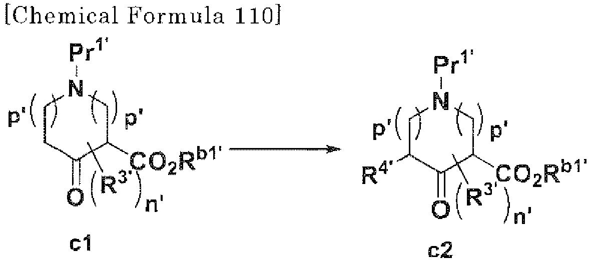

wherein R^{3'} is each independently hydroxy, halogen, cyano, substituted or unsubstituted alkyl, substituted or unsubstituted alkenyl or substituted or unsubstituted alkynyl,
two of R^{3'} attached to the same carbon atom may be taken together to form oxo, substituted or unsubstituted non-aromatic carbocycle or substituted or unsubstituted non-aromatic heterocycle, or
two of R^{3'} attached to the different carbon atom may be taken together to form substituted or unsubstituted non-aromatic carbocycle or substituted or unsubstituted non-aromatic heterocycle, Pr^{1'} is a protection group for an amino group such as Boc etc., R^{b1'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl or substituted or unsubstituted aromatic heterocyclyl, n' is an integer of 0 to 4, p' is each independently an integer of 0 to 2. Compound c2 can be obtained by reacting a solution of Compound c1 with an electrophile in the presence of base.

Examples of the reaction solvent include THF, dichloromethane, and dioxane, and the solvent may be used alone or in combination.

Examples of the electrophile include halogenated alkyl, halogenated alkenyl, haloformate, acid halides and the like. The amount of the electrophile may be 1 to 5 mole equivalents, preferably 1 to 2 mole equibalents to Compound c1.

Examples of the base include LDA, alkyl lithium, sodium hydride and the like. The amount of the base may be 1 to 5 mole equivalents, preferably 1 to 2 mole equivalents to Compound b1.

The reaction temperature may be -20°C to room temperature, preferably 0°C to room temperature.

The reaction time may be 1 to 24 hours, preferably 1 to 6 hours.

### Preparation of Compound c4

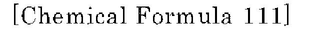

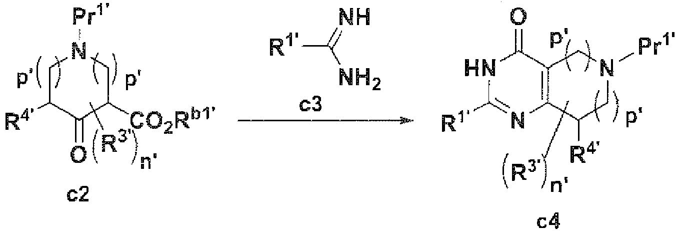

wherein R^{1'} is substituted or unsubstituted aromatic carbocyclyl, substituted or unsubstituted aromatic heterocyclyl, substituted or unsubstituted non-aromatic carbocyclyl or substituted or unsubstituted non-aromatic heterocyclyl, R^{4'} is C1-C2 alkyl substituted with one or more substituent(s) selected from the Substituent group A, substituted or unsubstituted C3-C10 alkyl, substituted or unsubstituted C3-C10 alkyl, substituted or unsubstituted C3-C10 alkenyl, substituted or unsubstituted C3-C10 alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkyloxy, substituted or unsubstituted alkenyloxy, substituted or unsubstituted alkynyloxy or substituted or unsubstituted amino, the other symbols are as defined above.

Compound c4 can be obtained by reacting a solution of Compound c2 with Compound c3 in the presence of base.

Examples of the reaction solvent include methanol, ethanol, dichloromethane, DMF, THF, dioxane and the like and the solvent may be used alone or in combination.

The amount of Compound c3 may be 1 to 5 mole equivalents, preferably 1 to 2 mole equivalents to Compound c2.

Examples of the base include potassium t-butoxide, sodium methoxide, triethylamine, potassium carbonate, pyridine and the like. The amount of the base may be 2 to 5 mole equivalents, preferably 2 to 3 mole equivalents to Compound c2.

The reaction temperature may be 0 to 100°C, preferably room temperature to 50°C.

The reaction time may be 1 to 24 hours, preferably 3 to 16 hours.

### Preparation of Compound c6

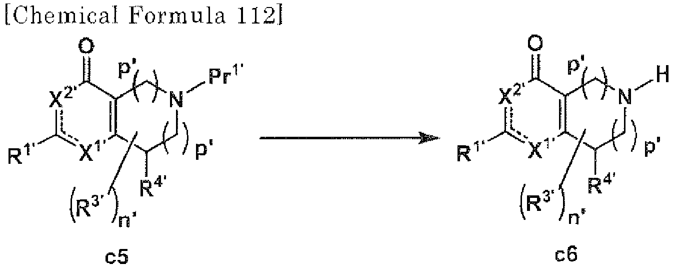

wherein X^{1'} is N or C(R^{5'}), R^{5'} is hydrogen, halogen, substituted or unsubstituted alkyl, substituted or unsubstituted alkenyl or substituted or unsubstituted alkynyl, X^{2'} is N(R^{6'}) or C(R^{7'})(R^{8'}), R^{6'} is hydrogen, substituted or unsubstituted alkyl, substituted or unsubstituted alkenyl or substituted or unsubstituted alkynyl, R^{7'} and R^{8'} are each independently hydrogen, hydroxy, halogen, cyano, substituted or unsubstituted alkyl, substituted or unsubstituted alkenyl, substituted or unsubstituted alkynyl, substituted or unsubstituted alkyloxy, substituted or unsubstituted alkenyloxy, substituted or unsubstituted alkynyloxy or substituted or unsubstituted amino, the other symbols are as defined above.

Compound c6 can be obtained by reacting a solution of Compound c5 with a deprotecting agent.

Examples of the reaction solvent include dichloromethane, dioxane, ethyl acetate, methanol, ethanol and the like, and the solvent may be used alone or in combination.

Examples of the deprotecting agent include trifluoroacetic acid, acetic acid, sulfuric acid and the like. The amount of the deprotecting agent may be used 1 mole equivalent to a large excess, preferably 1 to 5 mole equivalents to Compound c5.

The reaction temperature may be 0 to 100°C, preferably room temperature to 50°C.

The reaction time may be 0.1 to 12 hours, preferably 0.1 to 1 hour.

### Preparation of Compound c8

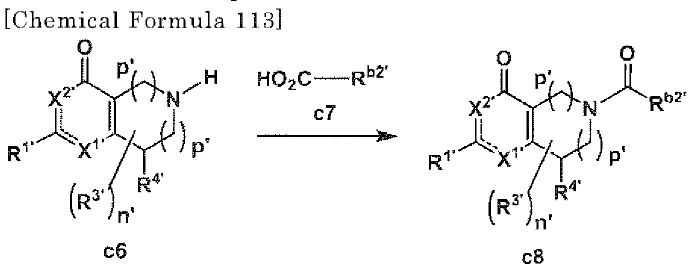

wherein R^{b2'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, substituted or unsubstituted aromatic heterocyclyl, substituted or unsubstituted alkyloxy, substituted or unsubstituted alkenyloxy, substituted or unsubstituted alkynyloxy, substituted or unsubstituted non-aromatic carbocyclyloxy, substituted or unsubstituted aromatic carbocyclyloxy, substituted or unsubstituted non-aromatic heterocyclyloxy, or substituted or unsubstituted aromatic heterocyclyloxy, the other symbols are as defined above.

Compound c8 can be obtained by reacting a solution of Compound c6 with Compound c7 in the presence of a condensation agent and base.

Examples of the reaction solvent include DMF, THF, dioxane, NMP and the like, and the solvent may be used alone or in combination.

Examples of the condensation agent include HATU, HBTU, WSC, DMT-MM, DCC, DIC and the like. The amount of the condensation agent may be 1 to 5 mole equivalents, preferably 1 to 2 mole equivalents to Compound c6.

Examples of the base include diisopropylethylamine, triethylamine, pyridine and the like. The amount of the base may be 1 to 5 mole equivalents, preferably 1 to 2 mole equivalents to Compound c6.

The amount of Compound c7 may be 1 to 5 mole equivalents, preferably 1 to 2 mole equivalents to Compound c6.

The reaction temperature may be 0 to 100°C, preferably room temperature to 50 °C.

The reaction time may be 0.1 to 24 hours, preferably 0.1 to 6 hours.

### Preparation of Compound c10

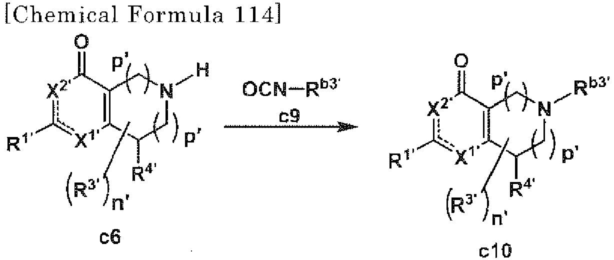

wherein R^{b3'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, or substituted or unsubstituted aromatic heterocyclyl, the other symbols are as defined above.

Compound c10 can be obtained by reacting a solution of Compound c6 with Compound c9 in the presence of base.

Examples of the reaction solvent include THF, diethyl ether, dichloromethane, methanol, ethanol and the like, and the solvent can be used alone or in combination.

Examples of the base include triethylamine, diisopropylethylamine, pyridine and the like. The amount of the base may be 1 to 5 mole equivalents, preferably 1 to 2 mole equivalents to Compound c6.

The amount of Compound c9 may be 1 to 5 mole equivalents, preferably 1 to 2 mole equivalents to Compound c6.

The reaction temperature may be 0 to 50°C, preferably room temperature to 50°C.

The reaction time may be 0.1 to 24 hours, preferably 1 to 6 hours.

### Preparation of Compound c8 (alternative method)

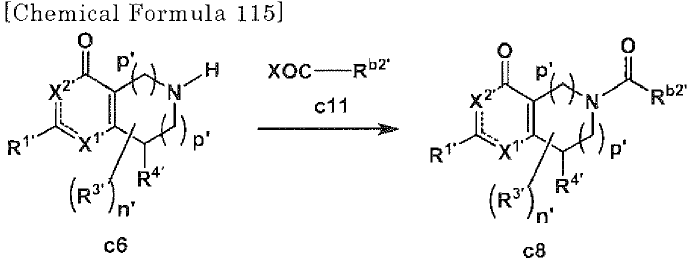

wherein X' is chloro or brome, the other symbols are as defined above.

Compound c8 can be obtained by reacting a solution of Compound c6 with Compound c11 in the presence of base.

Example of the reaction solvent include THF, dichloromethane, ethyl acetate, pyridine, DMF, water and the like, and the solvent can be used alone or in combination.

Examples of the base include triethylamine, pyridine, DIEA, sodium hydroxide, sodium hydrogen carbonate and the like. The amount of the base may be 1 mole equivalent to a large excess, preferably 1 to 2 mole equivalents to Compound c6.

The amount of Compound c11 may be 1 to 5 mole equivalents, preferably 1 to 2 mole equivalents to Compound c6.

The reaction temperature may be 0 to 100°C, preferably room temperature to 50°C.

The reaction time may be 0.1 to 24 hours, preferably 1 to 6 hours.

### Preparation of Compound c13

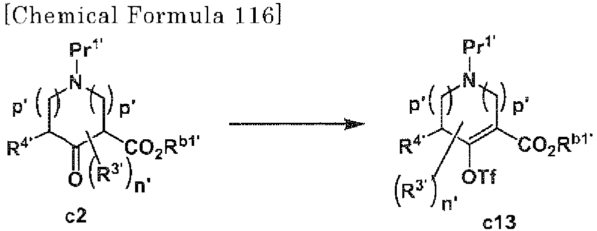

wherein the symbols are as defined above.

Compound c13 can be obtained by reacting a solution of Compound c2 with triflating agent in the presence of base.

Examples of the reaction solvent include THF, dichloromethane, toluene, DMF, hexane, methanol and the like, and the solvent may be used alone or in combination.

Examples of the base include sodium hydride, potassium hexamethyldisilazide, trimethylamine, LDA, lithium hexamethyldisilazide and the like. The amount of the base may be 1 to 5 mole equivalents, preferably 1 to 2 mole equivalents to Compound c2.

Examples of the triflating agent include N-phenyl-bis(trifluoromethane sulfonimide), triflate anhydride and the like. The amount of the triflating agent may be 1 to 5 mole equivalents, preferably 1 to 2 mole equivalents to Compound c2.

The reaction temperature may be -20 to 50°C, preferably 0°C to room temperature.

The reaction time may be 1 to 24 hours, preferably 1 to 6 hours.

### Preparation of Compound c4 (alternative method)

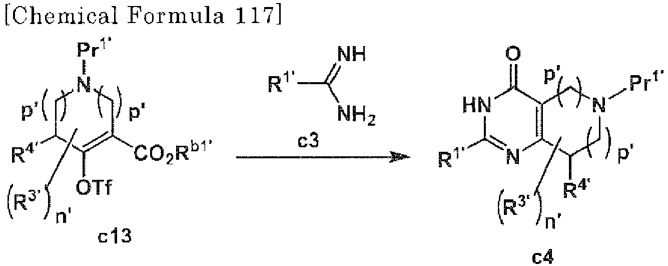

wherein the symbols are as defined above.

Compound c4 can be obtained by reacting a solution of Compound c13 with Compound c3 in the presence of base.

Examples of the reaction solvent include methanol, ethanol, dichloromethane, DMF, THF, dioxane and the like, and the solvent may be used alone or in combination.

The amount of Compound c3 may be 1 to 5 mole equivalents, preferably 1 to 2 mole equivalents to Compound c13.

Examples of the base include potassium t-butoxide, sodium methoxide, triethylamine, potassium carbonate, pyridine and the like. The amount of the base may be 2 to 5 mole equivalents, preferably 2 to 3 mole equivalents to Compound c13.

The reaction temperature may be 0 to 150°C, preferably room temperature to 120°C. The reaction may be conducted under microwave irradiation.

The reaction time may be 0.1 to 24 hours, preferably 0.1 to 12 hours.

### Preparation of Compound c15

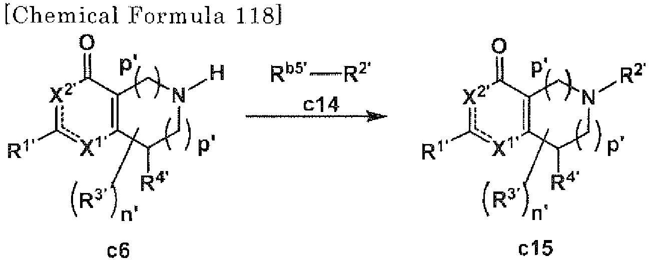

wherein R^{2'} is hydrogen, substituted or unsubstituted alkyl, substituted or unsubstituted alkenyl, substituted or unsubstituted alkynyl, substituted or unsubstituted aromatic carbocyclyl, substituted or unsubstituted aromatic heterocyclyl, substituted or unsubstituted non-aromatic carbocyclyl, substituted or unsubstituted non-aromatic heterocyclyl, substituted or unsubstituted alkylcarbonyl, substituted or unsubstituted alkenylcarbonyl, substituted or unsubstituted alkynylcarbonyl, substituted or unsubstituted non-aromatic carbocyclylcarbonyl, substituted or unsubstituted aromatic carbocyclylcarbonyl, substituted or unsubstituted non-aromatic heterocyclylcarbonyl, substituted or unsubstituted aromatic heterocyclylcarbonyl, substituted or unsubstituted alkyloxycarbonyl, substituted or unsubstituted alkenyloxycarbonyl, substituted or unsubstituted alkynyloxycarbonyl, substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocyclyloxycarbonyl, substituted or unsubstituted carbamoyl, substituted or unsubstituted alkylsulfonyl, substituted or unsubstituted alkenylsulfonyl, substituted or unsubstituted alkynylsulfonyl, substituted or unsubstituted non-aromatic carbocyclylsulfonyl, substituted or unsubstituted aromatic carbocyclylsulfonyl, substituted or unsubstituted non-aromatic heterocyclylsulfonyl, substituted or unsubstituted aromatic heterocyclylsulfonyl or substituted or unsubstituted sulfamoyl, R^{b5'} is halogen, the other symbols are as defined above.

Compound c15 can be obtained by reacting a solution of Compound c6 with Compound c14 in the presence of base.

Examples of the reaction solvent include dichloromethane, DMF, THF, diethyl ether, dioxane, and the solvent may be used alone or in combination.

Examples of Compound c14 include halogenated alkyl, halogenated alkenyl, haloformate, acid halide and the like. The amount of Compound c14 may be 1 to 5 mole equivalents, preferably 1 to 2 mole equivalents to Compound c6.

Examples of the base include triethylamine, LDA, alkyl lithium, sodium hydride, potassium carbonate and the like. The amount of the base may be 1 to 5 mole equivalents, preferably 1 to 2.5 mole equivalents to Compound c6.

The reaction temperature may be -20 to 100°C, preferably 0 to 50°C.

The reaction time may be 0.5 to 12 hours, preferably 1 to 6 hours.

### Preparation of Compound c18

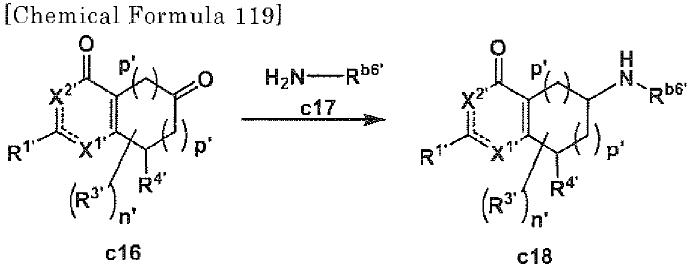

wherein R^{b6'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl, or substituted or unsubstituted aromatic heterocyclyl, the other symbols are as defined above.

Compound c18 can be obtained by reacting a solution of Compound c16 with Compound c17 and acid followed by reacting with a reductant.

Examples of the reaction solvent include dichloromethane, THF, diethyl ether, methanol, ethanol, acetic acid and the like, and the solvent may be used alone or in combination.

Examples of Compound c17 include alkylamine, aromatic carbocycle amine, non-aromatic carbocycle amine, aromatic heterocycle amine, non-aromatic heterocycle amine, ammonia and the like. The amount of Compound c17 may be 1 to 5 mole equivalents, preferably 1 to 3 mole equivalents to Compound c16.

Examples of the acid include acetic acid, formic acid, hydrochloric acid and the like. The amount of the acid may be 1 to 10 mole equivalents, preferably 1 to 3 mole equivalents to Compound c16.

Examples of the reductant include sodium triacetoxyborohydride, sodium cyanoborohydride, sodium borohydride, palladium-carbon and the like. The amount of the reductant may be 0.1 to 5 mole equivalents, preferably 1 to 3 mole equivalents to Compound c16.

The reaction temperature may be 0 to 50°C, preferably 0°C to room temperature.

The reaction time may be 0.5 to 24 hours, preferably 1 to 12 hours.

### Preparation of Compound c21

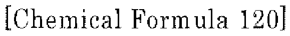

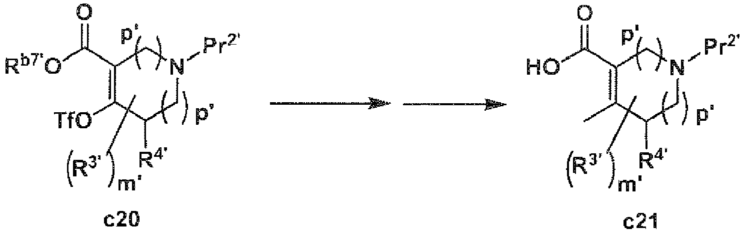

wherein R^{b7'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl or substituted or unsubstituted aromatic heterocyclyl, Pr2' is substituted or unsubstituted alkyl carbonyl, substituted or unsubstituted alkenyloxy, substituted or unsubstituted alkynyloxy, substituted or unsubstituted alkyloxycarbonyl, substituted or unsubstituted alkenyloxycarbonyl or substituted or unsubstituted alkynyloxycarbonyl, m' is an integer of 0 to 3, the other symbols are as defined above.

Compound c21 can be obtained by reacting a solution of Compound c20 with a methylating agent in the presence of a metal catalyst and a ligand, followed by hydrolyzing in the presence of base.

Examples of the reaction solvent include THF, diethyl ether, dioxane, DMF, NMP, dichloromethane and the like, and the solvent may be used alone or in combination.

Examples of the metal catalyst include tris (dibenzylideneacetone) dipalladium (0), copper iodide, tetrakis (triphenylphosphine) palladium, palladium dichloride, bis (triphenylphosphine) palladium dichloride and the like. The amount thereof may be 0.001 to 0.5 mole equivalents, preferably 0.01 to 0.1 mole equivalents to Compound c20.

Examples of the ligand include Xantphos, triphenylphosphine, dibenzylidene acetone, and the like. The amount thereof may be 0.001 to 0.5 mole equivalents, preferably 0.01 to 0.1 mole equivalents to Compound c20.

Examples of the methylating agent include dimethyl zinc, methyllithium, methyl magnesium bromide, tetramethyl tin, methylboronic acid and the like. The amount thereof include 1 to 5 mole equivalents, preferably 1 to 3 mole equivalents to Compound c20.

The reaction temperature may be -20 to 50°C, preferably 0°C to room temperature.

The reaction time may be 0.5 to 24 hours, preferably 1 to 6 hours.

Examples of the reaction solvent for the hydrolysis include methanol, ethanol, water, THF, dioxane and the like, and the solvent may be used alone or in combination.

Examples of the base include potassium hydroxide, sodium hydroxide and the like. The amount thereof may be 1 to 10 mole equivalents, preferably 1 to 3 mole equivalents to Compound c20.

The reaction temperature may be 0°C to reflux temperature, preferably room temperature to reflux temperature.

The reaction time may be 0.5 to 12 hours, preferably 1 to 6 hours.

### Preparation of Compound c2

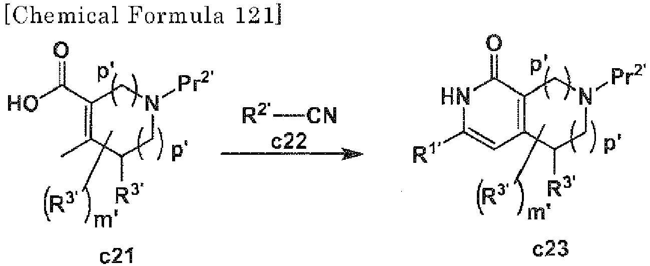

wherein the symbols are as defined above.

Compound c23 can be obtained by reacting a solution of Compound c21 with Compound c22 in the presence of base.

Examples of the reaction solvent include THF, dichloromethane, hexane, DMF, water, acetone and the like, and the solvent may be used alone or in combination.

Examples of the base include LDA, n-butyl lithium, DIEA, potassium carbonate, pyridine and the like. The amount thereof may be 1 to 10 mole equivalents, preferably 1 to 3 mole equivalents to Compound c21.

Examples of Compound c22 include alkyl nitriles, aromatic carbocyclic nitriles, nonaromatic carbocyclic nitriles, aromatic heterocyclic nitrile, nonaromatic heterocyclic nitrile and the like. The amount thereof may be 1 to 10 mole equivalents, preferably 1 to 3 mole equivalents to Compound c21.

The reaction temperature may be 0 to 50°C, preferably 0°C to room temperature.

The reaction time may be 1 to 24 hours, preferably 1 to 12 hours.

### Preparation of Compound c25

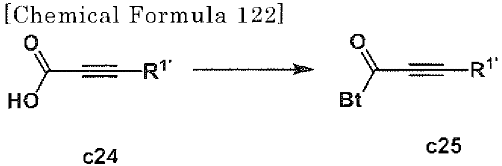

wherein the symbols are as defined above.

Compound c25 can be obtained by reacting a suspension of 1H-benzo[d][1,2,3]triazole with a halogenating agent, followed by reacting with Compound c24.

Examples of the reaction solvent include dichloromethane, THF, diethyl ether and the like, and the solvent may be used alone or in combination.

Examples of the halogenating agent include thionyl chloride, hydrochloric acid and the like. The amount thereof may be 1 to 5 mole equivalents, preferably 1 to 2 mole equivalents to Compound c24.

The reaction temperature may be 0 to 50°C, preferably 0°C to room temperature.

The reaction time may be 1 to 24 hours, preferably 1 to 12 hours.

### Preparation of Compound c27

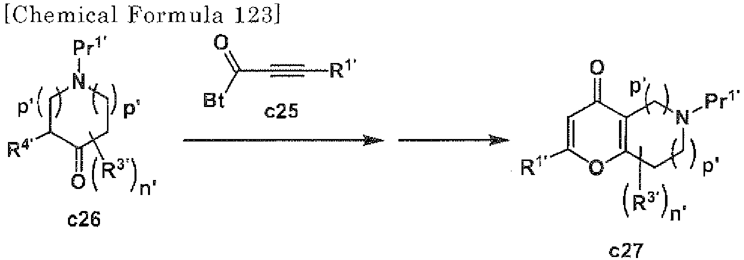

wherein the symbols are as defined above.

Compound c27 can be obtained by reacting a solution of Compound c26 with base, followed by reacting with Compound c25 in the presence or absence of HMPA, further cyclizing in the presence of base.

Examples of the reaction solvent include THF, diethyl ether, dioxane, hexane, dichloromethane and the like, and the solvent may be used alone or in combination.

Examples of the base include LDA, sodium hydride, sodium methoxide, lithium hexamethyldisilazide and the like. The amount thereof may be 1 to 5 mole equivalents, preferably 1 to 2 mole equivalents to Compound c26.

The reaction temperature may be -78°C to room temperature, preferably -60°C. to -20°C.

The reaction time may be 1 to 12 hours, preferably 1 to 6 hours.

Examples of the solvent for the cyclization reaction include DMF, NMP, dichloromethane, THF, dioxane and the like, and the solvent may be used alone or in combination.

Examples of the base include N,N-dimethyl amino pyridine, pyridine, DIEA, triethylamine, potassium t-butoxide and the like. The amount thereof may be 1 to 10 mole equivalents, preferably 1 to 3 mole equivalents to Compound c26.

The reaction temperature may be room temperature to 100°C, preferably 50 to 80°C.

The reaction time may be 0.5 to 12 hours, preferably 1 to 5 hours.

### Preparation of Compound c31

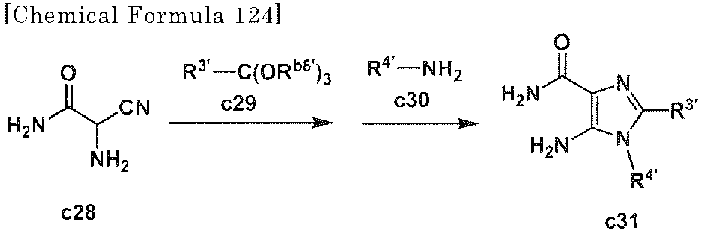

wherein R^{b8'} is substituted or unsubstituted alkyl, substituted or unsubstituted alkenyl, substituted or unsubstituted alkynyl, substituted or unsubstituted non-aromatic carbocyclyl, substituted or unsubstituted aromatic carbocyclyl, substituted or unsubstituted non-aromatic heterocyclyl or substituted or unsubstituted aromatic heterocyclyl, the other symbols are as defined above.

Compound c31 can be obtained by reacting a solution of Compound c28 with Compound c29, followed by reacting with Compound c30.

Examples of the reaction solvent include acetonitrile, DMF, THF, dichloromethane, ethyl acetate, ethanol and the like, and the solvent may be used alone or in combination.

Examples of Compound c29 inlcude 1,1,1-trialkoxyalkane, 1,1,1-trialkoxy aromatic carbocycle, 1,1,1-trialkoxy non-aromatic carbocycle, 1,1,1-trialkoxy aromatic heterocycle, 1,1,1-trialkoxy non-aromatic heterocycle and the like. The amount thereof may be 1 to 5 mole equivalents, preferably 1 to 3 mole equivalents to Compound c28.

Examples of Compound c30 include ammonia, primary amine and the like. The amount thereof may be 1 to 5 mole equivalents, preferably 1 to 3 mole equivalents to Compound c28.

The reaction temperature may be room temperature to 100°C, preferably 50 to 100°C.

The reaction time may be 0.1 to 12 hours, preferably 0.1 to 5 hours.

### Preparation of Compound c33

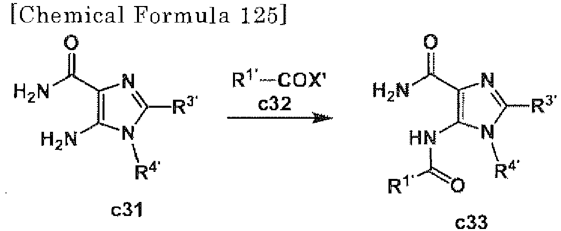

wherein the symbols are as defined above.

Compound c33 can be obtained by reacting a solution of Compound c31 with Compound c32 in the presence of base.

Examples of the reaction solvent include THF, dichloromethane, ethyl acetate, pyridine, DMF, and water, and the solvent may be used alone or in combination.

Examples of the base include triethylamine, pyridine, DIEA, sodium hydroxide, sodium hydrogen carbonate and the like. The amount thereof may be 1 mole equivalent to a large excess, preferably, preferably 1 to 2 mole equivalents to Compound c31.

The amount of Compound c32 may be 1 to 5 mole equivalents, preferably 1 to 2 mole equivalents to Compound b31.

The reaction temperature may be 0 to 100°C, preferably room temperature to 50°C.

The reaction time may be 0.1 to 24 hours, preferably 1 to 6 hours.

### Preparation of Compound c34

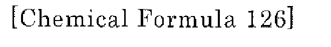

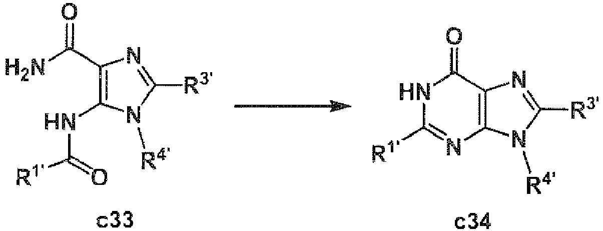

wherein the symbols are as defined above.

Compound c34 can be obtained by reacting a solution of Compound c33 in the presence of base.

Examples of the reaction solvent include t-butanol, ethanol, dioxane, dichloromethane, DMF, methanol, THF, water and the like, and the solvent may be used alone or in combination.

Examples of the base include potassium t-butoxide, sodium carbonate, potassium carbonate, potassium hydroxide, trimethylamine and the like. The amount thereof may be 1 to 10 mole equivalents, preferably 1 to 5 mole equivalents to Compound c33. It can also be used as a solvent.

The reaction temperature may be room temperature to reflux temperature, preferably 50°C to reflux temperature.

The reaction time may be 1 to 48 hours, preferably 1 to 10 hours.

The compound of the present invention thus obtained may be purified and crystallized in a variety of solvents. Examples of the solvent to be used include alcohols (methanol, ethanol, isopropylalcohol, n-butanol etc.), ether (diethylether, diisopropylether etc.), methyl acetate, ethyl acetate, chloroform, methylene chloride, tetrahydrofuran, N,N-dimethylformamide, toluene, benzene, xylene, acetonitrile, hexane, dioxane, dimethoxyethane, water or a mixture thereof. The compound may be dissolved in the solvent under heating, and the impurities are removbed. The solution is then gradually cooled and filtered to collect the precipitated solid or crystal.

The compound of the present invention has autotaxin inhibitory acivity. Accordingly, the pharmaceutical composition containing the compound of the present invention is useful as a therapeutic and/or prophylactic agent for diseases related to autotaxin. The diseases related to autotaxin include, for example, urinary extraction failure, chronic kidney disease or renal fibrosis, interstitial pneumonitis or pulmonary fibrosis, scleroderma, pain, fibromyalgia, rheumatoid arthritis, angiogenesis, cancer, formation, growth and propagation of tumor, arteriosclerosis, ocular diseases, choroidal neovascularization and diabetic retinopathy, inflammatory diseases, arthritis, neurodegeneration, restenosis, wound healing, transplant rejection, endometriosis and the like. The pharmaceutical composition containing the compound the the present invention is usefule as a therapeutic agent and/or preventive agent for these diseases. More preferably, the pharmaceutical composition containing the compound of the present invention is useful as a therapeutic agent and/or preventive agent for urinary extraction failure, interstitial lung disease or fibroid lung, renal fibrosis, hepatic fibrosis, pachyderma, pain, fibromyalgia syndrome, arthsitis and rheumatism, disseminated sclerosis or endometriosis and the like. The compound of the present invention may have a utility as as pharmaceutical, as well as autotaxin inhibitory effect, characterized by any of or all of the features as follows:
a) weak inhibitory effect on CYP enzyme (e.g. CYP1A2, CYP2C9, CYP3A4, etc.);
b) good pharmacokinetics, such as high bioavailability and appropriate clearance;
c) low toxicity (e.g. anemia-induced action);
d) high metabolic stability;
e) high water solubility;
f) high brain migration;
g) free of gastrointestinal disorders (e.g., hemorrhagic enteritis,
gastrointestinal ulcers, gastrointestinal bleeding, etc.).

Also, the compound of the present invention has low affinity for ENPP1, ENPP3 to 7 receptors and high selectivity for ENPP2 receptor.

The pharmaceutical composition of the present invention may be administered orally in a formulation as conventionally used including tablets, granules, powders, capsules, pills, solution, syrups, buccal or sublingual. The pharmaceutical composition may be administered parentally in a formulation as conventionally used including injections such as intramuscular or intravenous injection, suppositories, transdermal absorbents, inhalants, etc.

The pharmaceutical composition may be prepared by mixing an effective amount of the compound of the invention with various pharmaceutical additives suitable for the formulation, such as excipients, binders, moistening agents, disintegrants, lubricants, diluents and the like. For injections, an active ingredient together with a suitable carrier may be sterilized to obtaine a pharmaceutical composition.

Examples of the excipients include lactose, saccharose, glucose, starch, calcium carbonate, crystalline cellulose and the like. Examples of the binders include methylcellulose, carboxymethylcellulose, hydroxypropylcellulose, gelatin, polyvinylpyrrolidone and the like. Examples of the disintegrants include carboxymethylcellulose, sodium carboxymethylcellulose, starch, sodium alginate, agar, sodium lauryl sulfate and the like. Examples of the lubricants include talc, magnesium stearate, macrogol and the like. For base materials of suppositories, cacao oil, macrogol, methylcellulose and the like may be used. Solubilizing agents, suspending agents, emulsifiers, stabilizers, preservatives, isotonic agents and the like, which are commonly used, may be added when the composition is prepared as solutions, emulsified or suspended injections. Sweetening agents, flavors and the like, which are commonly used, may be added for oral formulation.

The dosage of the pharmaceutical composition of the invention is determined in the light of the age and weight of the patient, the type and severity of the disease to be treated, and the route for administration and the like. In the case of oral administration to adults, the dosage is usually in the range of 0.05 to 100mg/kg/day, preferably 0.1 to 10mg/kg/day. In the case of parenteral administration, the dosage is variable depending on the administration route, but is usually 0.005 to 10mg/kg/day, preferably in the range of 0.01 to 1mg/kg/day. The dosage may be administered in single or divided doses.

### Examples

The present invention is further explained by the following Examples and Test Examples, which are not intented to limit the scope of the present invention.

The abbreviations as used herein represent the following meaning.
Me: methyl
Et: ethyl
Bu: butyl
Ph: phenyl
PPh₃, TPP: triphenylphosphine
AcOEt: ethyl acetate
DMF: N, N- dimethylformamide
TFA: trifluoroacetic acid
DMSO: dimethyl sulfoxide
THF: tetrahydrofuran
DIEA, Hunig's Base : N, N- diisopropylethylamine
TBAF: tetrabutylammonium fluoride
SEM: 2- (trimethylsilyl) ethoxymethyl
OAc: acetic acid group
mCPBA: metachloroperbenzoic acid
NMP: 1- methylpyrrolidin-2-one
LAH: lithium aluminum hydride
DBU : 1,8-diazabicyclo [5.4.0] undec-7-ene
DCM: methylene chloride
TEA: trimethylamine
TMS: tetramethylsilane
HATU: O- (7- azabenzotriazol 1-yl) -N, N, N', N', - tetramethyluronium hexafluorophosphate
DPPA: diphenylphosphoryl azide

NMR analysis of the compounds obtained in the Example was carried out at 400MHz, using deuterated dimethyl sulfoxide (d₆-DMSO) or deuterochloroform (CDCl₃).

LC/MS was measured under the following conditions.

### (Method A)

Column: ACQUITY UPLC BEH C18 (1.7µm i.d.2.1x50mm) (Waters)
Flow rate: 0.8mL/min
UV detection wavelength: 254nm
Mobile phase:
   [A] 0.1% formic acid in water
   [B] 0.1% formic acid in acetonitrile
Gradient: linear gradient from 10% to 100% [B] over 3.5 minutes, and then 100% [B] was maintained for 0.5 minutes.

### (Method B)

Column: Shim-pack XR-ODS (2.2µm, i.d.50x3.0mm) (Shimadzu)
Flow rate: 1.6mL/min
UV detection wavelength: 254nm
Mobile phase:
   [A] 0.1% formic acid in water
   [B] 0.1% formic acid in acetonitrile
Gradient: linear gradient from 10% to 100% [B] over 3 minutes, and then 100% [B] was maintained for 0.5 minute.

### (Method C)

Column: Boston ODS-3 250x4.6mm
flow rate: 1.0 mL/min
UV detection wavelength: 246nm

### Mobile phase:

[A] water
[B] acetonitrile

Gradient: linear gradient from 10% to 100% [B] over 3 minutes, and then 100% [B] was maintained for 0.5 minute.

### (Method D)

Column: Waters X Bridge C18 (3.5µm 50x4.6mm)
Flow rate: 2.0mL/min
UV detection wavelength: 254nm

### Mobile phase:

[A] 0.05% TFA in water
[B] 0.05% TFA in acetonitrile

Gradient: linear gradient from 5% to 100% [B] over 1.6 minutes, and then 100% [B] was maintained for 1.4 minutes.

### (Method E)

Column: ACQUITY UPLC BEH C18 (1.7µm i.d.2.1x50mm) (Waters)
Flow rate: 0.55mL/min
UV detection wavelength: 254nm

### Mobile phase:

[A] 0.1% formic acid in water
[B] 0.1% formic acid in acetonitrile

Gradient: linear gradient from 5% to 100% [B] over 3.5 minutes, and then 100% [B] was maintained for 0.5 minutes.

### (Method F)

Column: Waters X Bridge C18 (3.5µm 50x4.6mm)
Flow rate: 2.0mL/min
UV detection wavelength: 254nm

### Mobile phase:

[A] 0.01% NH₄HCO₃aq in water
[B] acetonitrile

Gradient: linear gradient from 5% to 100% [B] over 1.6 minutes, and then 100% [B] was maintained for 1.4 minutes.

### Example 1: Preparation of Compound a15 (I-0218)

### Step 1: Preparation of Compound a2

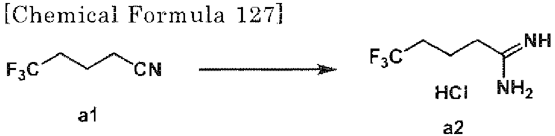

To a solution of ammonium chloride (6.73g, 126mmol) in toluene (93mL) was dropped 2mol/L trimethyl aluminum n-hexane solution (58.5 mL, 117 mmol) under ice-cooling, and the mixture was stirred for 30 minutes under ice-cooling. After Compound a1 (9.32g, 68mmol) was added under ice-cooling, the solution was stirred at 80°C overnight. The reaction solution was filtered, washed with methanol (150mL) / methylene chloride (150mL) solution, and concentrated under reduced pressure to yield Compound a2 hydrochloride (9.5g, yield: 73%) as white oil.
¹H-NMR (DMSO-D₆) δ: 4.17 (s, 1H), 3.16 (s, 3H), 2.50-2.46 (m, 3H), 2.32-2.27 (m, 2H), 1.85-1.83 (m, 2H).

### Step 2: Preparation of Compound a4

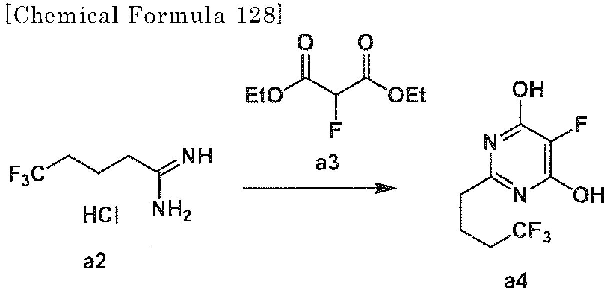

To a solution of Compound a2 hydrochloride (1.0g, 5.25mmol) in methanol (10mL) was added potassium tert-butoxide (1.07g, 9.54mmol), the mixture was stirred 10 minutes under ice-cooling. After added Compound a3 (739ul, 4.77mmol), the solution was stirred at 60°C for 1 hour. After added 2mol/L hydrochloric acid aqueous solution, methanol was removed under reduced pressure. The precipitated solid was filtered, washed with water, and concentrated under reduced pressure to yield Compound a4 (648.8mg, yield: 57%) as pale pink solid.
LC-MS: m/z=241. [M+H]⁺
LC/MS method: Method A, retention time: 0.74 min

### Step 3: Preparation of Compound a5

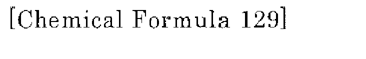

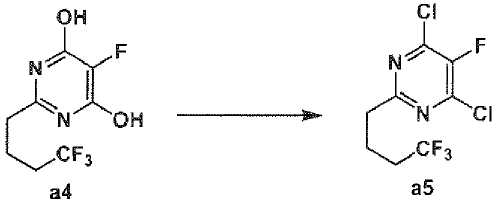

A solution of Compound a4 (648mg, 2.70mmol) in phosphorus oxychloride (6mL) was stirred at 80°C for one and a half hours. After removed the excess phosphorus oxychloride, to the reaction solution was added ice. To the residue was added water, the solution was extracted with ethyl acetate twice. The organic layer was washed with water, saturated sodium bicarbonate aqueous solution and brine, dried over anhydrous sodium sulfate, and concentrated under reduced pressure to yield Compound a5 (519.3mg, yield : 70%) as pale brown oil.
¹ H-NMR (CDCl₃) δ: 2.56 (t, 2H, J = 7.5 Hz), 2.33-2.29 (m, 2H), 1.86-1.84 (m, 2H).

### Step 4: Preparation of Compound a7

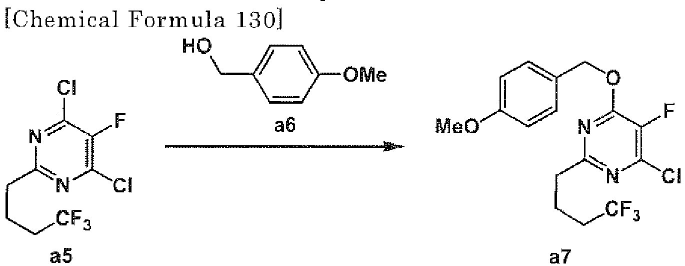

To a solution of Compound a6 (252uL, 2.03mmol) in DMF (2mL) was added sodium hydride (81mg, 2.025mmol) under salt ice bath cooling to prepare the solution A. To a solution of Compound a5 (510mg, 1.84mmol) in DMF (3mL) was dropped the solution A under salt ice bath cooling, and the mixture was stirred for 30 minutes. To the reaction solution was added to ice-water, and the solution was extracted with ethyl acetate twice. The organic layer was washed with water and brine, dried over anhydrous sodium sulfate and concentrated under reduced pressure. The obtained residue was purified by silica gel column chromatography (ethyl acetate / hexane) to yield Compound a7 (343mg, yield: 49%).
LC-MS: m/z=379. [M+H]⁺
LC/MS method: Method A, retention time: 2.84 min

### Step 5: Preparation of Compound a8

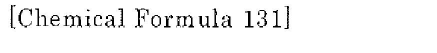

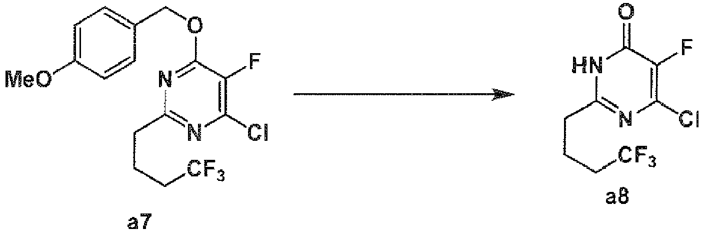

A solution of Compound a7 (343mg, 1.84mmol) in trifluoroacetic acid (3mL) was stirred for 20 minutes under ice-cooling. To the reaction solution was added water, and the solution was extracted with chloroform twice. The organic layer was washed with water,dried over anhydrous sodium sulfate and concentrated under reduced ressure to yield Compound a8 (351.5mg, yield: quant) as a white solid.
LC-MS: m/z=259. [M+H]⁺
LC/MS method: Method A, retention time: 1.65 min

### Step 6: Preparation of Compound a10

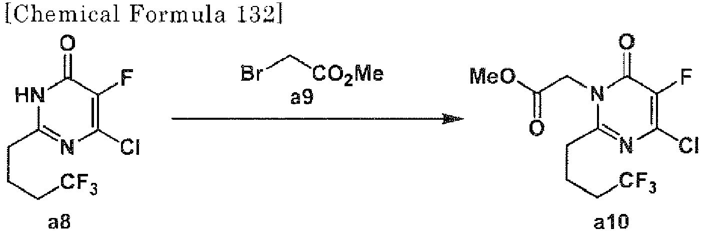

To a solution of Compound a8 (350mg, 1.35mmol) in THF (3.5mL) was added sodium hydride (70.4mg, 1.759mmol) under ice-cooling, and the solution was stirred for 10 minutes. After Compound a9 (162ul, 1.76mmol) was added to the reaction solution, the solution was stirred for 60°C for 2 hours. To the reaction solution was added water, and the solution was extracted with ethyl acetate twice. The organic layer was washed with water and brine, dried over anhydrous sodium sulfate and concentrated under reduced pressure. The obtained residue was purified by silica gel column chromatography (ethyl acetate / hexane) to yield Compound a10 (243mg, yield : 54%) as colorless oil.
LC-MS: m/z=331. [M+H]⁺
LC/MS method: Method A, retention time: 2.05 min

### Step 7: Preparation of Compound a11

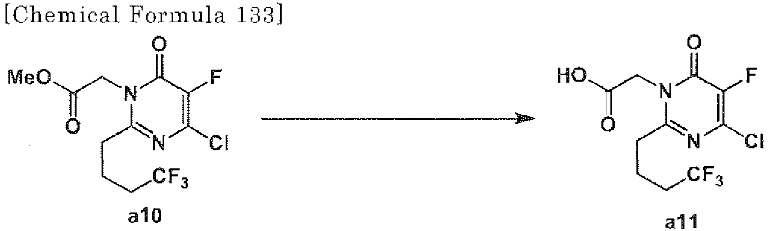

To a solution of Compound a10 (233mg, 0.71mmol) in methanol / THF (1:1, 2.3mL) was added 2mol/L sodium hydroxide aqueous solution (1.06ml, 2.11mmol), and the solution was stirred at room temperature for 20 minutes. To the reaction solution was added water, and the solution was extracted with chloroform twice. The organic layer was washed with brine, anhydrous sodium sulfate, and concentrated under reduced pressure. The obtained crude product was directly used in the next step.

### Step 8: Preparation of Compound a13

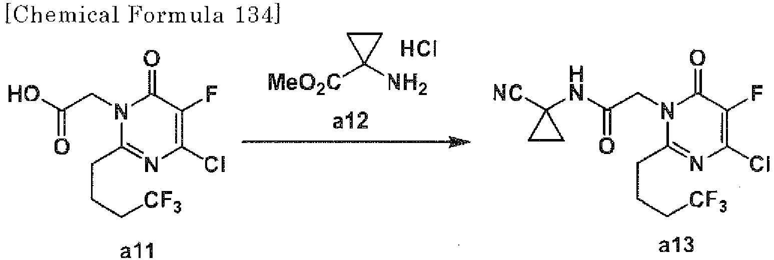

To a solution of Compound a11 in DMF (2.3mL) was added HATU (322mg, 0.85mmol), and added Compound a12 hydrochloride (100mg, 0.85mmol) and trimethylamine (147ul, 1.06mmol) under ice-cooling, and the mixture was stirred for 3 hours under ice-cooling. To the reaction solution was added water, and the solution was extracted with ethyl acetate twice. The organic layer was washed with water and brine, dried over anhydrous sodium sulfate and concentrated under reduced pressure. The residue was purified by silica gel column chromatography (ethyl acetate / hexane) to yield Compound a13 (178.3mg, yield: 67%) as a pale brown solid.
LC-MS: m/z=381. [M+H]⁺
LC/MS method: Method A, retention time: 1.87 min

### Step 9: Preparation of Compound a15 (I-0218)

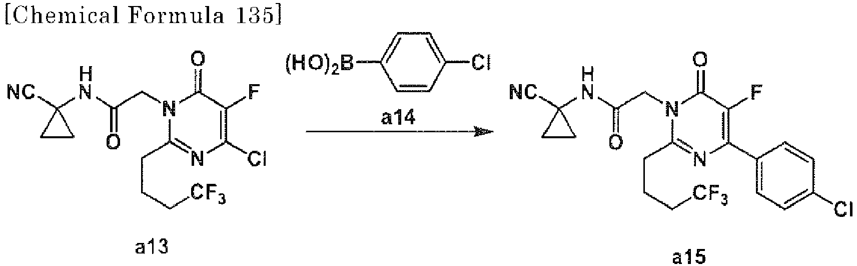

To a solution of Compound a13 (65mg, 0.17mmol) in DMF (650ul) was added Compound a14 (29.4mg, 0.19mmol), 1,1'-bis(diphenylphosphino)ferrocen-palladium(II)dichloride-dichloromethane complex (13.9mg, 0.017mmol) and 2mol/L sodium carbonate aqueous solution (94µl, 0.188mmol), and the mixture was stirred at 150°C for 10 minutes under microwave irradiation. To the reaction solution was added water, and the solution was extracted with chloroform twice. The organic layer was washed with water and brine, dried over anhydrous sodium sulfate and concentrated under reduced pressure. The obtained residue was purified by reversed phase silica gel column chromatography to yield Compound a15 (I-0218) (2.4mg, yield: 3%) as a pale broen solid.
¹H-NMR (CDCl₃) δ: 7.99 (d, 2H, J = 7.8 Hz), 7.69 (s, 1H), 7.47 (d, 2H, J = 7.8 Hz), 4.73 (s, 2H), 2.97-2.93 (m, 2H), 2.33-2.18 (m, 4H), 1.57-1.56 (m, 2H), 1.31-1.29 (m, 2H).

### Example 2: Preparation of Compound a23 (I-0112)

### Step 1: Preparation of Compound a17

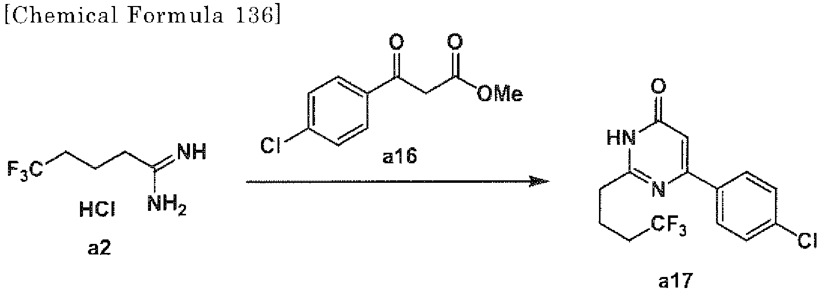

Compound a17 was obtained by the method similar to the preparation of Compound a4.
LC-MS: m/z=317. [M+H]⁺

### Step 2: Preparation of Compound a18 (I-0254)

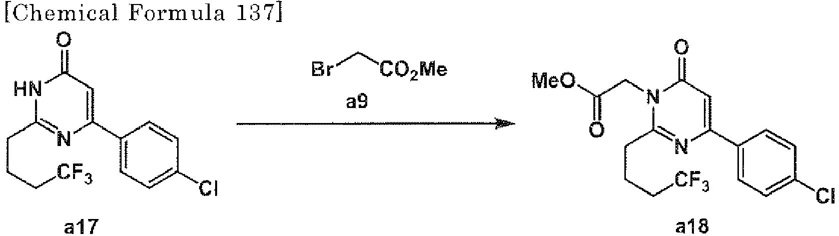

To a solution of Compound a17 (1.0g, 3.16mmol) in THF (10mL) was added potassium carbonate (655mg, 4.74mmol), and added Compound a9 (389ul, 4.10mmol) under ice-cooling, and the mixture was stirred for 7 hours under ice-cooling. The reaction solution was added water, and extracted with ethyl acetate. The organic layer was washed with brine, dried over anhydrous sodium sulfate, and concentrated under reduced pressure. The obtained residue was purified by silica gel column chromatography (ethyl acetate / hexane) to yield Compound a18 (I-0254) (134.2mg, yield: 86%) as a pale brown solid.
LC-MS: m/z=389. [M+H]⁺

### Step 3: Preparation of Compound a19 (I-0110)

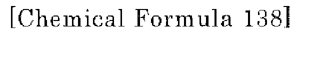

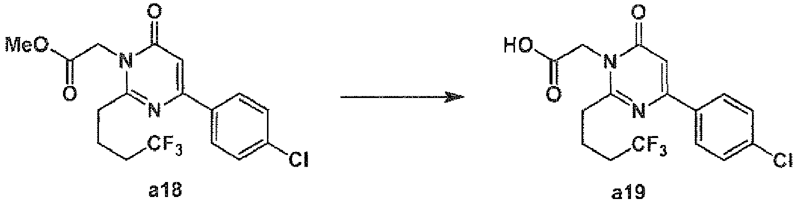

Compound a19(I-0110) was obtained by the method similar to the preparation of Compound a11.
¹H-NMR (DMSO-D₆) δ: 8.11 (d, 2H, J = 7.3 Hz), 7.57 (d, 2H, J = 7.3 Hz), 6.94 (s, 1H), 4.64 (s, 2H), 2.86 (t, 2H, J = 7.0 Hz), 2.43-2.36 (m, 2H), 2.07-2.00 (m, 2H).

### Step 4: Preparation of Compound a21 (I-0111)

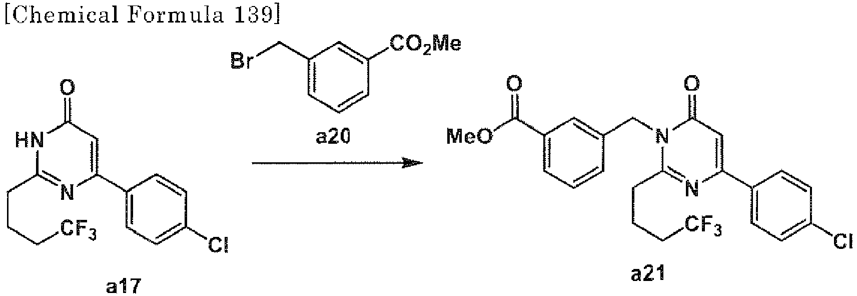

Compound a21(I-0111) was obtained by the method similar to the preparation of Compound a18.
¹H-NMR (CDCl₃) δ: 7.97 (dd, 3H, J = 14.4, 7.7 Hz), 7.89 (s, 1H), 7.46-7.38 (m, 4H), 6.89 (s, 1H), 5.38 (s, 2H), 3.91 (s, 3H), 2.79 (t, 2H, J = 6.5 Hz), 2.24-2.11 (m, 4H).

### Step 5: Preparation of Compound a23 (I-0112)

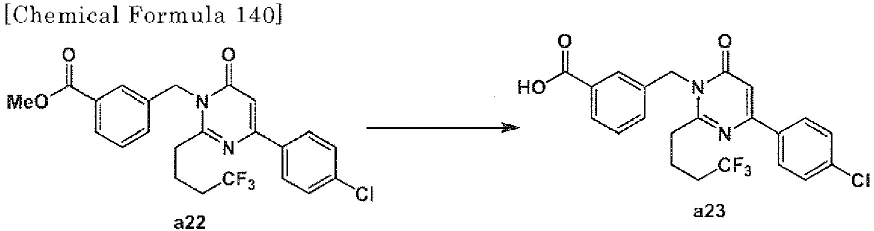

Compound a23 (I-0112) was obtained by the method similar to the preparation of Compound a11.
¹H-NMR (DMSO-D₆) δ: 8.15 (d, 2H, J = 8.5 Hz), 7.86 (d, 1H, J = 7.4 Hz), 7.76 (s, 1H), 7.58 (d, 2H, J = 8.5 Hz), 7.50-7.42 (m, 2H), 7.08 (s, 1H), 5.38 (s, 2H), 2.87 (t, 2H, J = 7.0 Hz), 2.39-2.27 (m, 2H), 2.01-1.93 (m, 2H).

### Example 3: Preparation of Compound a32 (I-0205)

### Step 1: Preparation of Compound a25

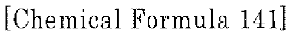

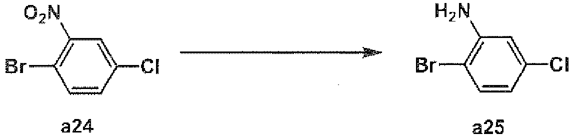

To a solution of Compound a24 (1.5g, 6.34mmol) in ethanol (15mL) was added tin chloride dihydrate (5.73g, 25.4mmol), and the solution was stirred at 60°C for 1 hour. To the reaction solution was added ethyl acetate and 4mol/L sodium hydroxide aqueous solution (12.69ml, 50.8mmol), the precipitated solid was filtered. The organic layer was concentrated under reduced pressure to yield Compound a25 (1.29g, yield : 98%) as a pale brown solid.
¹H-NMR (CDCl₃) δ: 7.30 (d, 1H, J = 8.5 Hz), 6.75 (s, 1H), 6.59 (d, 1H, J = 8.5 Hz), 4.15 (br s, 2H).

### Step 2: Preparation of Compound a26

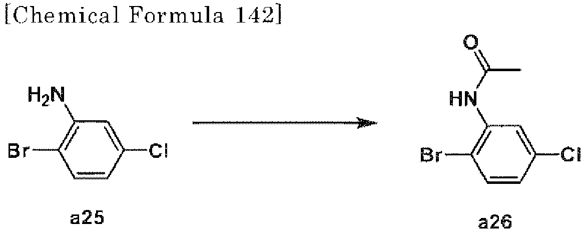

To a solution of Compound a25 (1g, 4.84mmol) in pyridine (8mL) was added sodium hydride (0.21g, 5.33mmol) and acetyl chloride (0.42ml, 5.81mmol), and the solution was stirred at 80°C for 1 hour. To the reaction solution was added water, and the solution was extracted with ethyl acetate twice. The organic layer was washed with water and brine, dried over anhydrous sodium sulfate and concentrated under reduced pressure. To a solution of the obtained residue in THF / methanol (1:1, 9ml) was added 2mol/L sodium hydroxide aqueous solution (2.6ml, 5.16mmol), and the solution was stirred at room temperature for 30 minutes. To the reaction solution was added water, and the solution was extracted with ethyl acetate twice. The organic layer was washed with water and brine, dried over anhydrous sodium sulfate and concentrated under reduced pressure. To the obtained residue was added ethyl acetate (5ml), and the mixture was dissolved in a water bath of 80°C. After cooled to room temperature, hexane (5ml) was added to the solution, and the precipitated solid was filtered, washed with isopropyl ether (5ml), and dried under reduced pressure to Compound a26 (674.4mg, yield: 53%) as a pale brown solid.
¹H-NMR (CDCl₃) δ: 8.46 (s, 1H), 7.59 (s, 1H), 7.45 (d, 1H, J = 8.5 Hz), 6.97 (d, 1H, J = 8.5 Hz), 2.25 (s, 3H).

### Step 3: Preparation of Compound a28

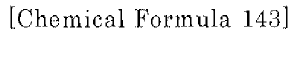

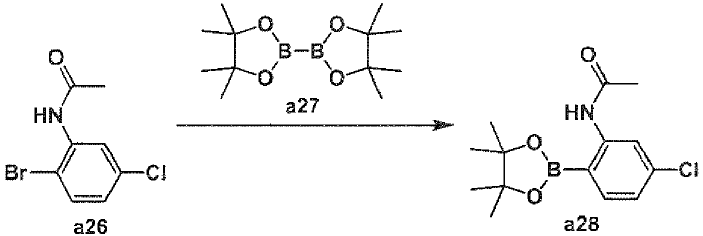

To a solution of Compound a26 (300mg, 1.21mmol) in 1,4-dioxane (3mL) was added Compound a27 (368g, 1.45mmol), potassium acetate (355mg, 3.62mmol) and 1,1'-bis(diphonylphosphino)ferrocene-palladium(II) dichloride-dichloromethane complex (99mg, 0.121mmol), and the mixture was stirred at 100°C for 5 hours. The reaction mixtute was purified by silica gel column chromatography (ethyl acetate / hexane) to yield Compound a28 (101mg, yield: 29%) as a broen oil.
¹H-NMR (CDCl₃) δ: 9.50 (s, 1H), 8.50 (s, 1H), 7.66 (d, 1H, J = 8.3 Hz), 7.04 (d, 1H, J = 8.0 Hz), 2.15 (s, 3H), 1.37 (s, 13H).

### Step 4: Preparation of Compound a30

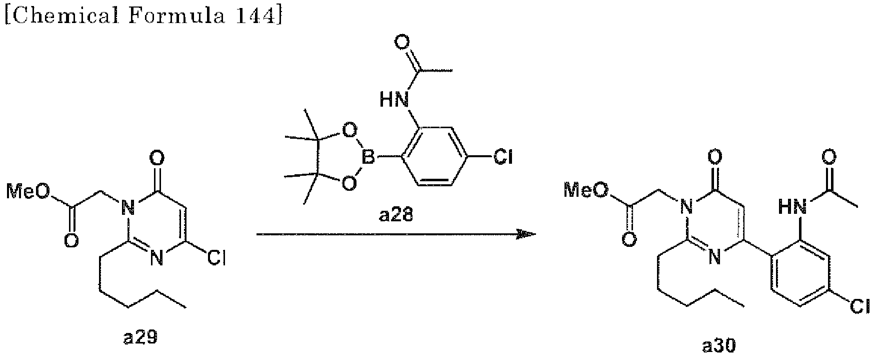

Compound a30 was obtained by the method similar to the preparation of Compound all and a15.
LC/MS method: Method A, retention time: 2.31 min

### Step 5: Preparation of Compound a31

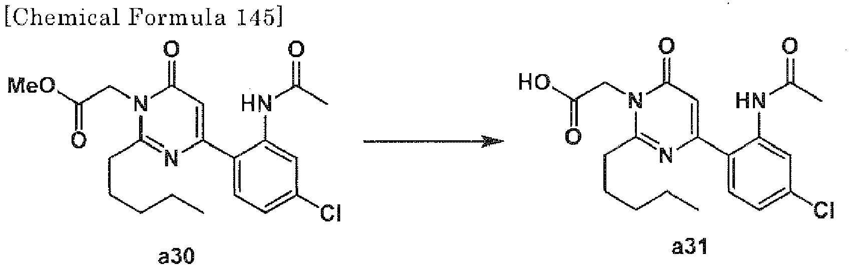

Compound a31 was obtained by the method similar to the preparation of Compound a11.

### Step 6: Preparation of Compound a32 (I-0205)

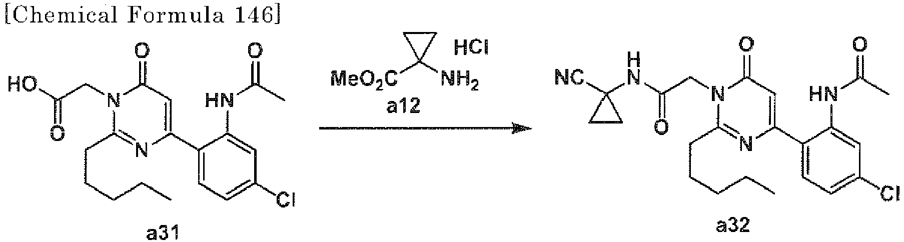

Compound a32 (I-0205) was obtained by the method similar to the preparation of Compound a13.
LC/MS method: Method A, retention time: 2.11 min

### Example 4: Preparation of Compound a35 (I-0208)

### Step 1: Preparation of Compound a33

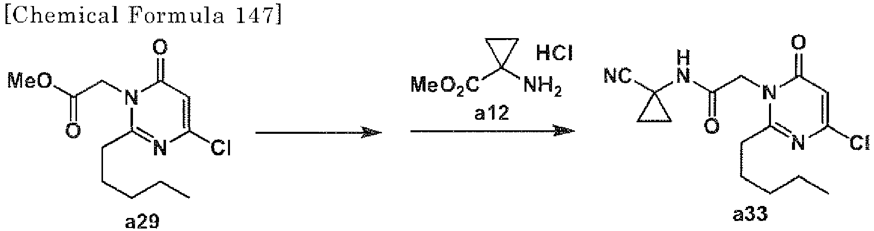

Compound a33 was obtained by the method similar to the preparation of Compound a13.
LC/MS method: Method A, retention time: 1.77 min

### Step 2: Preparation of Compound a35 (I-0208)

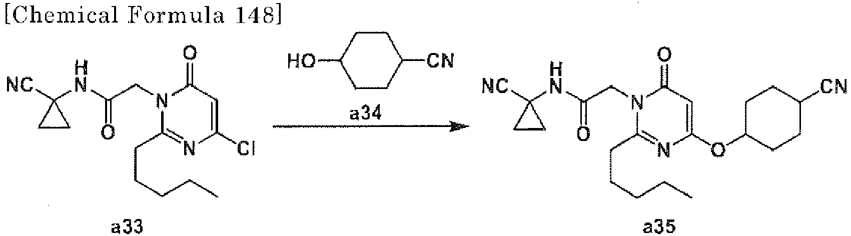

To a solution of Compound a34 (80mg, 0.64mmol) in DMF (200ul) was added sodium hydride (30.7mg, 0.77mmol) under ice-cooling, and the mixture was stirred for 10 minutes. Compound a33 (206mg, 0.64mmol) and DMF (600ul) was added to the solution, and the solution was stirred at 60°C for 2 hours, and stirred at 80°C for 1 hour. To the reaction solution was added water, and the solution was extracted with ethyl acetate twice. The organic layer was washed with water and brine, dried over anhydrous sodium sulfate and concentrated under reduced pressure. The obtained residue was purified by reversed-phase silica gel column chromatography to yield Compound a35 (5.1mg, yield: 2%) as a white solid.
LC/MS method: Method A, retention time: 1.85 min

### Example 5: Preparation of Compound a39 (I-0255)

### Step 1: Preparation of Compound a38 (I-0254)

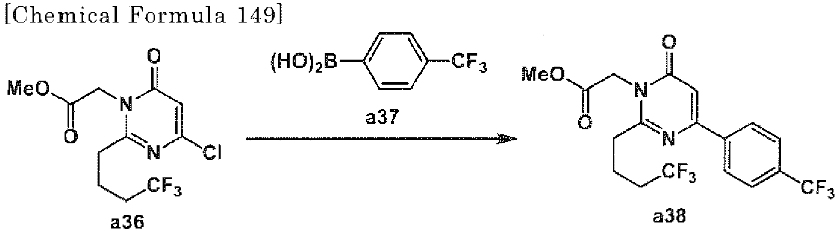

Compound a38 (I-0254) was obtained by the method similar to the preparation of Compound a15.
LC-MS: m/z=423. [M+H]⁺
LC/MS method: Method B, retention time: 2.37 min

### Step 2: Preparation of Compound a39 (I-0255)

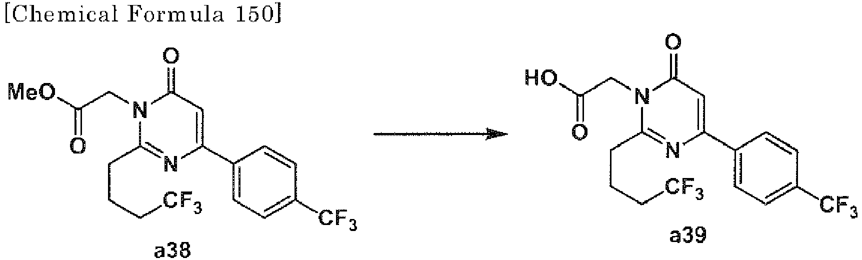

Compound a39 (I-0255) was obtained by the method similar to the preparation of Compound a11.
LC-MS: m/z=409. [M+H]⁺
LC/MS method: Method B, retention time: 2.18 min

### Example 6: Preparation of Compound a41 (I-0254)

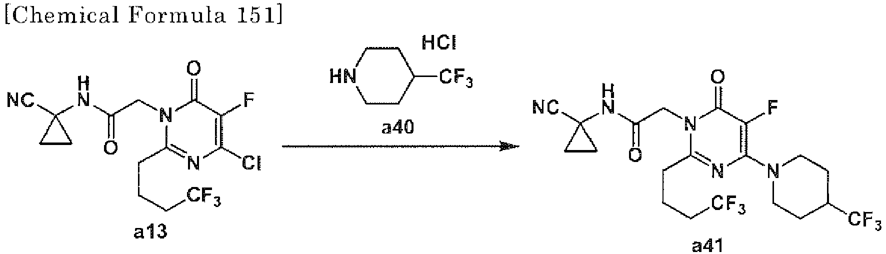

To a solution of Compound a40 hydrochloride (112mg, 0.59mmol) in DMF (200ul) was added sodium hydride (47.1mg, 1.18mmol) under ice-cooling, and the solution was stirred for 10 minutes. Compound a13 (112mg, 0.29mmol) in DMF (1ml) was added to the solution under ice-cooling, and the solution was stirred at 60°C for 1 hour. To the reaction solution was added water, and the solution was extracted with ethyl acetate twice. The organic layer was washed with water and brine, dried over anhydrous sodium sulfate and concentrated under reduced pressure. The obtained residue was purified by reversed-phase silica gel column chromatography to yield Compound a41 (15.8mg, yield: 11%) as a white solid. LC/MS method: Method A, retention time: 2.19 min

### Example 7: Preparation of Compound a52 (I-0242)

### Step 1: Preparation of Compound a43

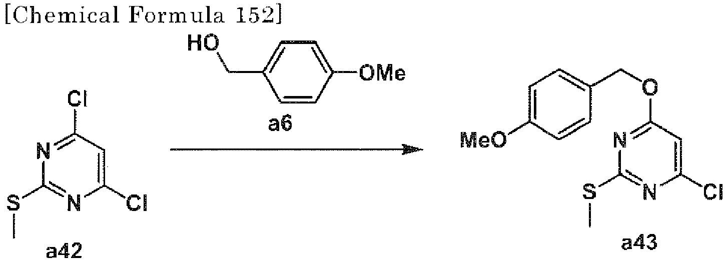

Compound a43 was obtained by the method similar to the preparation of Compound a7.
¹H-NMR (CDCl₃) δ: 7.35 (2H, d, J = 8.4 Hz), 6.91 (2H, d, J = 8.4 Hz), 6.42 (1H, s), 5.35 (2H, s), 3.82 (3H, s), 2.56 (3H, s).
LC-MS: m/z=297. [M+H]⁺

### Step 2: Preparation of Compound a44

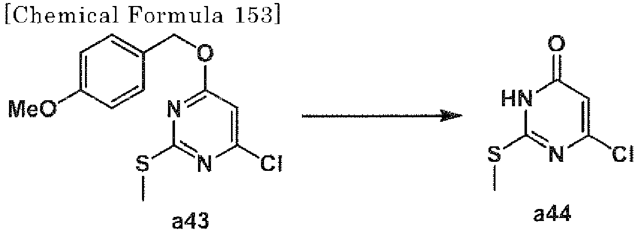

Compound a44 was obtained by the method similar to the preparation of Compound a8.
¹H-NMR (DMSO-D₆) δ: 8.26 (1H,s), 6.30 (1H, s), 2.49 (3H, s).
LC-MS: m/z=177. [M+H]⁺

### Step 3: Preparation of Compound a45

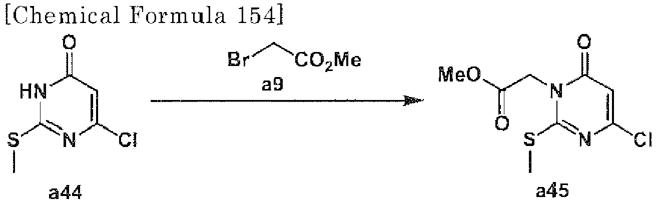

Compound a45 was obtained by the method similar to the preparation of Compound a10.
¹H-NMR (CDCl₃) δ: 6.31 (1H, s), 4.81 (2H, s), 3.80 (3H, s), 2.62 (3H, s).
LC-MS: m/z=249. [M+H]⁺

### Step 4: Preparation of Compound a46

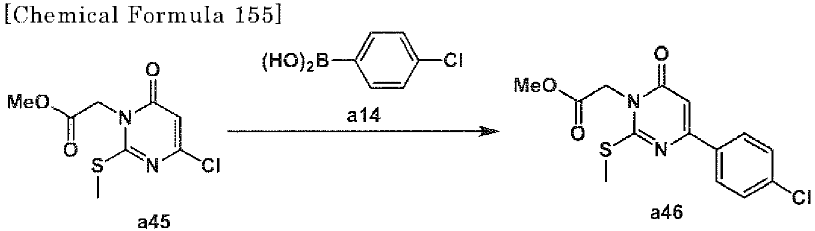

To a solution of Compound a45 (500mg, 2.01mmol) in THF (4mL) was added Compound a14 (472mg, 3.02mmol), chloro(2-dicyclohexylphosphino-2',4',6'-triisopropyl-1,1'-biphenyl)[2-(2'-amino-1,1'-biphenyl)]palladium(II) (31.7mg, 0.040mmol) and 2mol/L potassium phosphate aqueous solution (8.04ml), and the mixture was stirred at 60°C for 1 hour. To the reaction solution was added water, and the solution was extracter with ethyl acetate twice. The organic layer was washed with water and brine, dried over anhydrous sodium sulfate and concentrated under reduced pressure. The residue was purified by silica gel column chromatography (ethyl acetate / hexane) to yield Compound a46 (580mg, yield: 89%) as a white solid.
¹H-NMR (CDCl₃) δ: 7.93 (2H, d, J = 8.4 Hz), 7.43 (2H, d, J = 8.3 Hz), 6.68 (1H, s), 4.89 (2H, s), 3.81 (3H, s), 2.71 (3H, s).
LC-MS: m/z=325. [M+H]⁺
LC/MS method: Method B, retention time: 1.85 min

### Step 5: Preparation of Compound a47

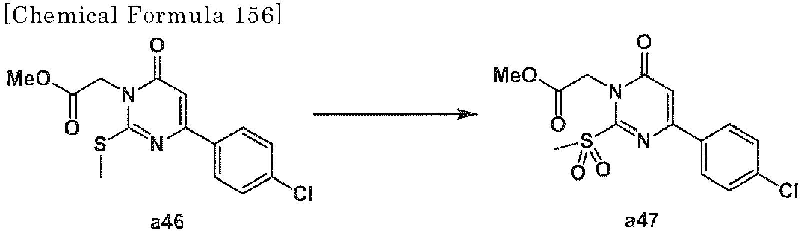

To a solution of Compound a46 (200mg, 0.62mmol) in dichloromethane (5mL) was added m-chloroperbanzoic acid (425mg, 1.85mmol), and the mixture was stirred at room temperature for 3 hours. To the reaction solution was added 10% sodium bisulfite aqueous solution, and the solution was extracted with chloroform twice. The organic layer was washed with water and brine, dried over anhydrous sodium sulfate and concentrated under reduced pressure. The residue was purified by silica gel column chromatography (ethyl acetate / hexane) to yield Compound a47 (110mg, yield: 50%) as colorless oil.
¹H-NMR (CDCl₃) δ: 7.83 (2H, d, J = 8.4 Hz), 7.48 (2H, d, J = 8.3 Hz), 6.90 (1H, s),
5.19 (2H, s), 3.80 (3H, s), 3.56 (3H, s).
LC-MS: m/z=357. [M+H]⁺
LC/MS method: Method B, retention time: 1.90 min

### Step 6: Preparation of Compound a49

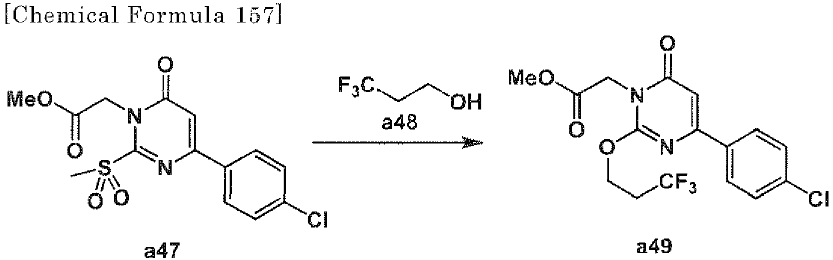

To a solution of Compound a48 (19.2mg, 0.17mmol) in THF (250uL) was added potassium tert-butoxide (18.9mg, 0.17mmol), and the solution was stirred for 30 minutes under ice-cooling. To the reaction solution was added a solution of Compound a47 (50mg, 0.14mmol) in THF (250ul), and the solution was stirred at - 78°C for 1 hour. To the reaction solution was added 1mol/L hydrochloric acid aqueous solution, the solution was extracted with ethyl acetate twice. The organic layer was washed with water and brine, dried over anhydrous sodium sulfate and concentrated under reduced pressure. The residue was purified by silica gel column chromatography (ethyl acetate / hexane) to yield Compound a49 (42mg, yield: 77%) as a white solid.
¹H-NMR (CDCl₃) δ: 7.88 (2H, d, J = 8.4 Hz), 7.43 (2H, d, J = 8.3 Hz), 6.63 (1H, s), 4.82-4.75 (4H, m), 3.78 (3H, s), 2.68-2.62 (2H, m).
LC-MS: m/z=391. [M+H]⁺
LC/MS method: Method B, retention time: 2.25 min

### Step 7: Preparation of Compound a50

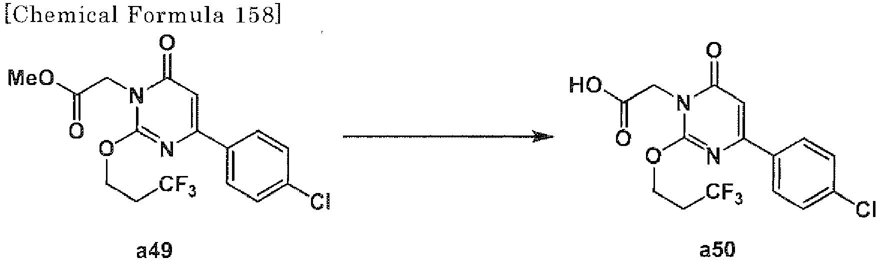

Compound a50 was obtained by the method similar to the preparation of Compound a11.

### Step 8: Preparation of Compound a52 (I-0242)

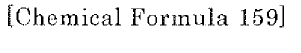

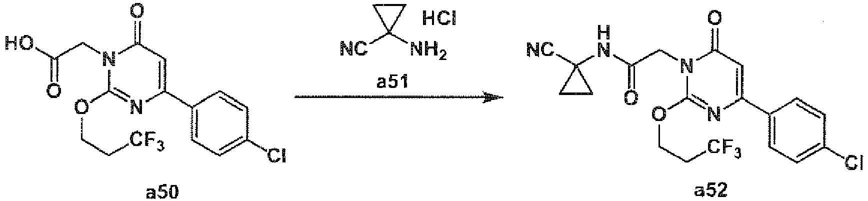

Compound a52 (I-0242) was obtained by the method similar to the preparation of Compound a13.
¹H-NMR (CDCl₃) δ: 7.86 (2H, d, J = 7.8 Hz), 7.43 (1H, d, J = 7.8 Hz), 6.84 (2H, d, J = 7.9 Hz),6.61 (1H, s,), 4.70 (1H, t, J =7.8hz), 4.66 (1H, s), 2.70 (2H, t, J = 7.8Hz), 1.57-1,56 (m, 2H), 1.31-1.29 (m, 2H).
LC-MS: m/z=441. [M+H]⁺
LC/MS method: Method B, retention time: 1.77 min

### Example 8: Preparation of Compound a55 (I-0243)

### Step 1: Preparation of Compound a53

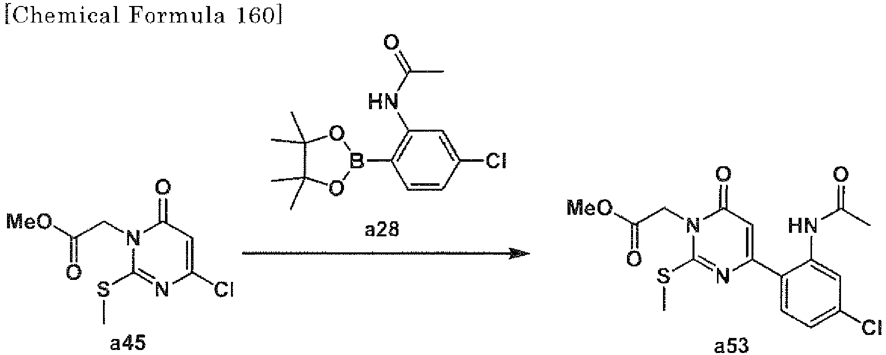

Compound a53 was obtaine by the method similar to the preparation of Compound a46.
LC/MS method: Method B, retention time: 1.60 min

### Step 2: Preparation of Compound a54

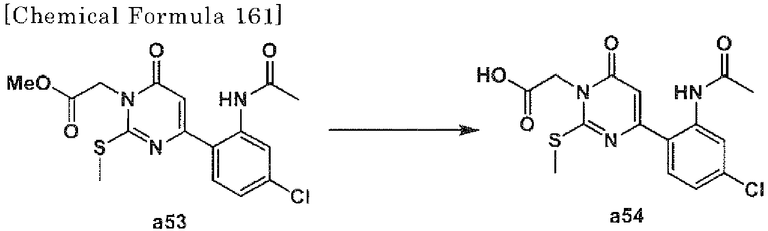

To a solution of Compound a53 (20mg, 0.052mmol) in 1,2-dichloroethane (1ml) was added hydroxy trimethyltin (95mg, 0.52mmol), and the solution was stirred at 70°C for 2 hours. To the reaction solution was added 1mol/L hydrochloric acid aqurous solution, and the solution was extracted with ethyl acetate, dried over anhydrous sodium sulfate and concentrated under reduced pressure. The obtained Compound a54 was directly used in the next step.

### Step 3: Preparation of Compound a55 (I-0243)

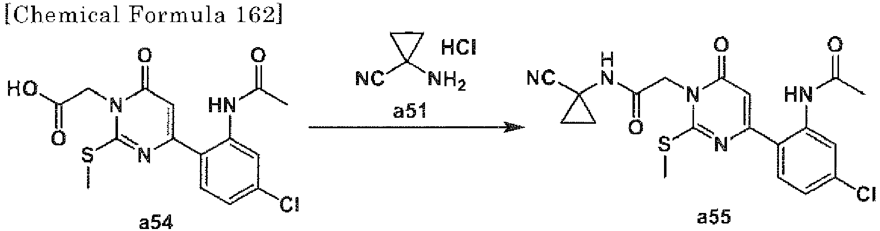

Compound a55 (I-0243) was obtained by the method similar to the preparation of Compound a13.
¹H-NMR (DMSO-D₆) δ: 9.88 (1H, s), 9.29 (1H, s), 8.01 (1H, s), 7.65 (1H, d, J = 8.5 Hz), 7.30 (1H, d, J = 8.5 Hz), 6.46 (1H, s), 4.66 (2H, s), 2.58 (3H, s), 2.01 (3H, s), 1.52-1.51 (2H, m), 1.16-1.14 (2H, m).
LC-MS: m/z=432. [M+H]⁺

### Example 9: Preparation of Compound a57

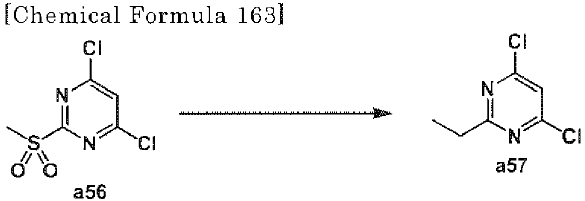

To a solution of Compound a56 (1g, 4.40mmol) in THF (7ml) was added 3mol/L ethyl magnesium bromide diethyl ether solution (2.9ml, 8.81mmol) under ice-cooling, and the solution was stirred for 1 hour under ice-cooling. To the reaction solution was added 1mol/L hydrochloric acid aqueous solution, and the solution was extracted with ethyl acetate. The organic layer was washed saturated sodium hydrogen carbonate solution, dried over anhydrous sodium sulfate and concentrated under reduced pressure to yield Compound a57 (730mg, yield: 94%) as yellow oil.
LC/MS method: Method B, retention time: 1.97 min

### Example 10: Preparation of Compound a63 (I-0002)

### Step 1: Preparation of Compound a60

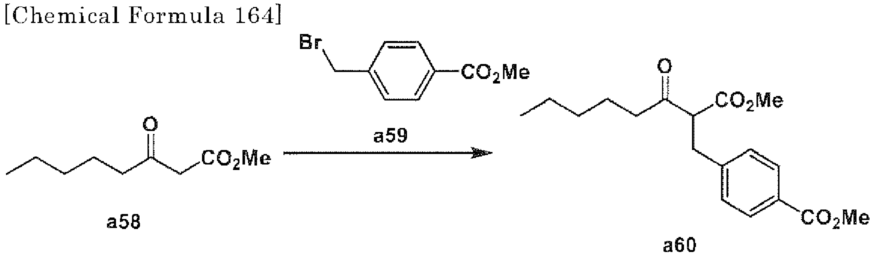

To a solution of Compound a58 (1.0g, 5.81mmol) in acetone (20mL) was added potassium carbonate (1.6g, 11.6mmol) and Compound a59 (1.46g, 6.39mmol), and the solution was stirred at 40°C for 18 hours. To the reaction solution was added saturated ammonium chloride aqueous solution, and the solution was extracted with ethyl acetate three times. The organic layer was concentrated under reduced pressure, the residue was purified by silica gel column chromatography (ethyl acetate / petroleum ether) to yield Compound a60 (530mg, yield: 28%) as colorless oil.

### Step 2: Preparation of Compound a62

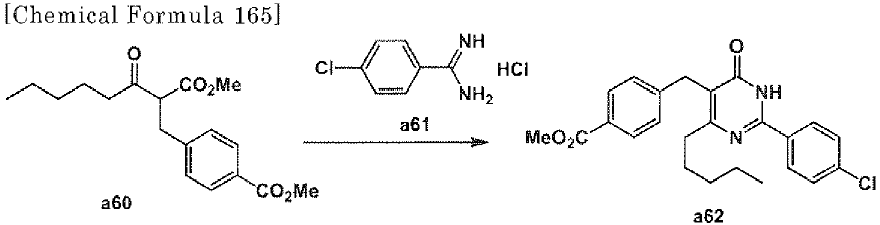

Compound a62 was obtained by the method similar to the preparation of Compound a4.

### Step 3: Preparation of Compound a63 (I-0002)

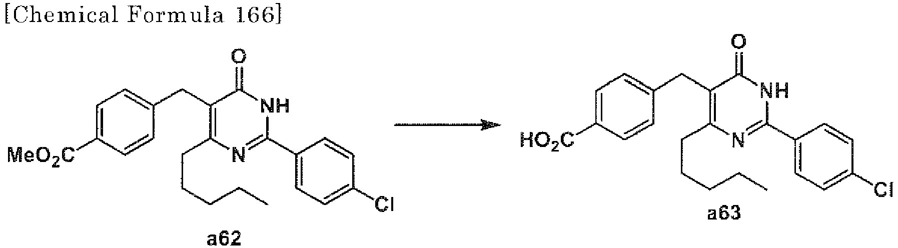

To a solution of Compound a62 (150mg, 0.35mmol) in 1,4-dioxane / water (3:1, 4mL) was added lithium hydroxide (14.7mg, 0.61mmol), and the mixture was stirred at 40°C for 18 hours. The solvent was removed under reduced pressure and the residue was adjusted to pH5 to 7 by adding 1mol/L hydrochloric acid aqueous solution. To the reaction solution was added water, and the solution was extracted with dichloromethane three times. The organic layer was washed with brine, dried over anhydrous sodium sulfate and concentrated under reduced pressure to yield Compound a63 (I-0002) (33mg, yield: 23%) as colorless oil.
LC-MS: m/z=411. [M+H]⁺

### Example 11: Preparation of Compound a68

### Step 1: Preparation of Compound a66

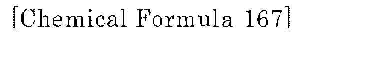

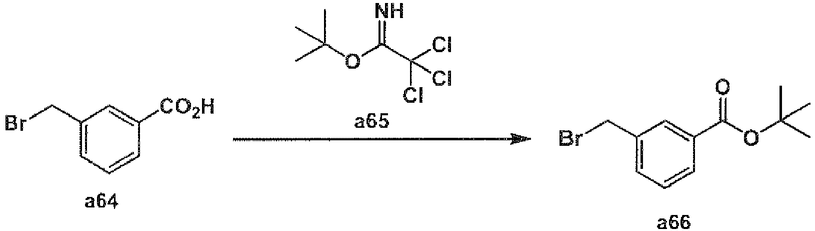

To a solution of Compound a64 (2.0g, 9.3mmol) in dichloromethane (10mL), cyclohexane (18.5ml) and THF (1ml) was added a solution of Compound a65 (4.07g, 18.6mmol) in cyclohexane (6ml) under ice-cooling. To the reaction solution was dropped boron trifluoride-diethyl ether complex (0.26g, 1.86mmol) under ice-cooling, and the mixture was stirred at room temperature overnight. To the reaction solution was added sodium hydrogen carbonate (2g), and the mixture was stirred at room temperature for 30 minutes. The insoluble material was filtered out, and the filtrate was concentrated under reduced pressure. The residue was purified by silica gel column chromatography (ethyl acetate / petroleum ether) to yield Compound a66 (2.2g, yield: 88%) as colorless oil.
LC-MS: m/z=271. [M+H]⁺

### Step 2: Preparation of Compound a68

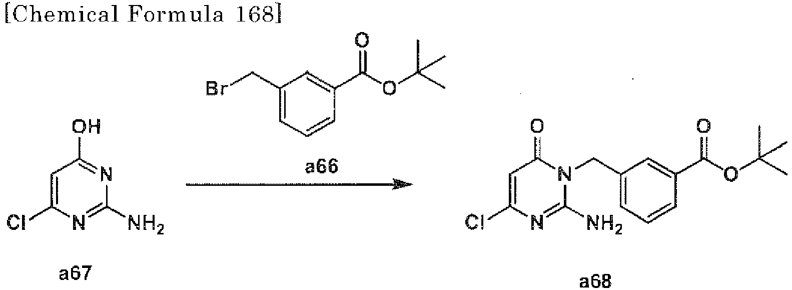

To a solution of Compound a67 (2.0g, 13.8mmol) in DMF (18mL) was added sodium hydride (0.55g, 13.8mmol) slowly, and the solution was added at room temperature for 30 minutes. A solution of Compound a66 (3.73g, 13.8mmol) in DMF (11mL) was dropped into the reaction solution, and the mixture was stirred at room temperature overnight. To the reaction solution was added saturated ammonium chloride aqueous solution and water, and the solution was extracted with ethyl acetate three times. The organic layer was washed with water and brine, dried over anhydrous sodium sulfate and concentrated under reduced pressure. The residue was purified by silica gel column chromatography (ethyl acetate / petroleum ether) to yield Compound a68 (1g, yield: 22%) as a white solid.
LC-MS: m/z=336. [M+H]⁺

### Example 12: Preparation of Compound a74 (I-0014)

### Step 1: Preparation of Compound a70

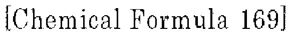

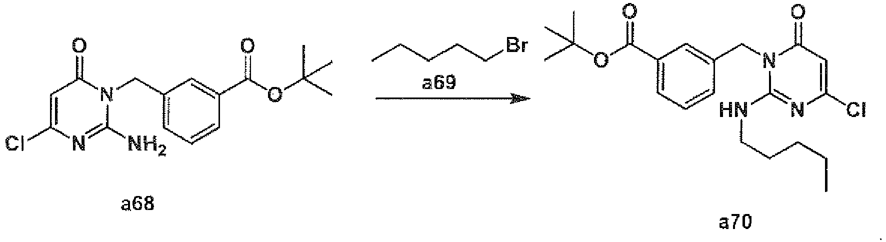

To a solution of Compound a68 (1.0g, 2.98mmol) in DMF (10mL) was added sodium hydride (0.14g, 3.57mmol) slowly, and the solution was added at room temperature for 15 minutes. To the reaction solution was added Compound a69 (0.47g, 3.13mmol), and the solution was stirred at 60°C overnight. To the reaction solution was added saturated ammonium chloride aqueous solution, and the solution was extracted with ethyl acetate three times. The organic layer was washed with water and brine, dried over anhydrous sodium sulfate and concentrated under reduced pressure to yiled Compound a70 (305mg, yield: 25%) as a gray solid.
LC-MS: m/z=406 [M+H]⁺
¹H-NMR (CDCl₃): 7.96 (d, J=8.0 Hz, 1H), 7.89 (s, 1H), 7.44 (t, J=7.6 Hz, 1H), 7.36 (d, J=7.6 Hz, 1H), 6.03 (s, 1H), 5.21 (s, 2H), 4.59 (b, 1H), 3.31 (q, J=6.8 Hz, 2H), 1.43-1.38 (m, 2H), 1.21-1.16 (m, 2H), 1.02-0.96 (m, 2H), 0.79 (t, J=7.2 Hz, 3H)

### Step 2: Preparation of Compound a71

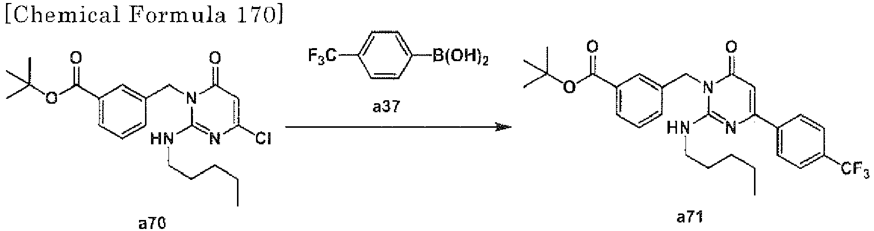

Compound a71 was obtained by the method similar to the preparation of Compound a15.
LC-MS: m/z=516. [M+H]⁺
¹H-NMR (CDCl₃): 8.09 (d, J=8.0 Hz, 2H), 7.95 (d, J=8.4 Hz, 2H), 7.70 (d, J=8.0 Hz, 2H), 7.47-7.41 (m, 2H), 6.51 (s, 1H), 5.31 (s, 2H), 4.60 (b, 1H), 3.44 (q, J=6.8 Hz, 2H), 1.60 (s, 9H), ,1.52-1.45 (m, 2H), 1.25-1.18 (m, 2H), 1.09-1.02 (m, 2H), 0.81 (t, J=7.2 Hz, 3H)

### Step 3: Preparation of Compound a72

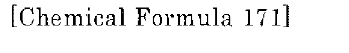

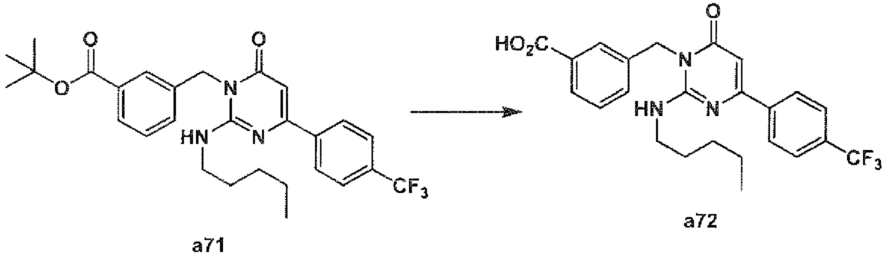

To a solution of Compound a71 (418mg, 0.81mmol) in chloroform (13mL) was dropped trifluoroacetic acid (5mL) under ice-cooling, and the solution was stirred at room temperature for 4 hours. The reaction solution was concentrated under reduced pressure to yield Compound a72 (317mg, yield: 85%) as a brown solid. LC-MS: m/z=460. [M+H]⁺
¹H-NMR (CDCl₃): 8.26 (d, J=8.0 Hz, 2H), 7.83 (J=7.6 Hz, 4H), 7.48 (t, J=8.4 Hz, 2H), 7.36 (b, 1H), 6.48 (s, 1H), 5.31 (s, 2H), 3.42 (d, J=5.6 Hz, 2H), 1.55-1.49 (m, 2H), 1.23-1.16 (m, 2H), 1.11-1.05 (m, 2H), 0.76 (t, J=7.2 Hz, 3H)

### Step 4: Preparation of Compound a74 (I-0014)

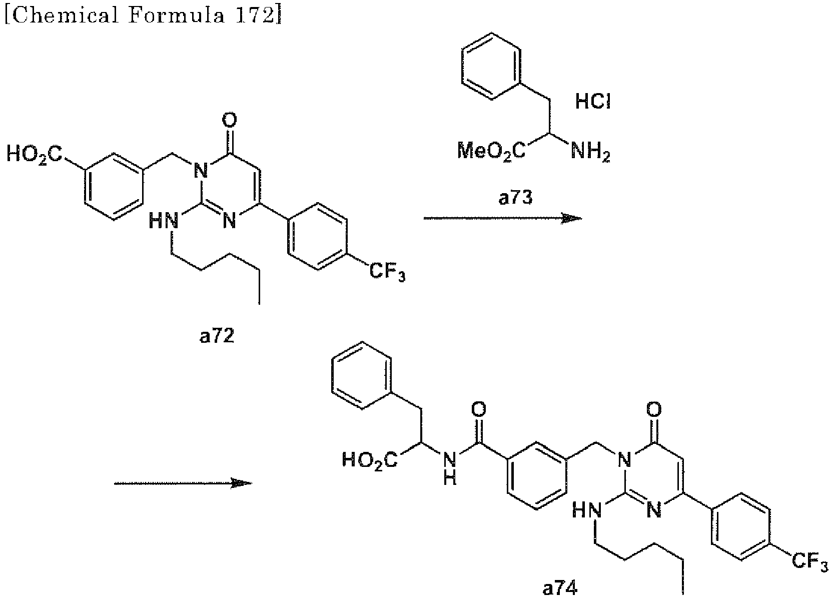

Compound a74 (I-0014) was obtained by the method similar to the preparation of Compound all and a13.
¹H-NMR (DMSO-D₆) δ: 12.7 (brs, 1H), 8.65 (d, 1H, J = 8.0 Hz), 8.25 (d, 2H, J = 8.2 Hz), 7.83 (d, 2H, J = 8.3 Hz), 7.69 (t, 2H, J = 6.8 Hz), 7.41 (t, 1H, J = 7.7 Hz), 7.28-7.22 (m, 6H), 7.14 (t, 1H, J = 7.0 Hz), 6.45 (s, 1H), 5.27 (s, 2H), 4.59-4.57 (m, 1H), 3.39 (q, 1H, J = 6.2 Hz), 3.17 (dd, 1H, J = 13.7, 4.5 Hz), 3.07-3.04 (m, 1H), 1.51-1.50 (m, 2H), 1.19-1.12 (m, 4H), 0.76 (t, 3H, J = 7.2 Hz).LC-MS: m/z=607. [M+H]⁺

### Example 13: Preparation of Compound a80 (I-0205)

### Step 1: Preparation of Compound a77

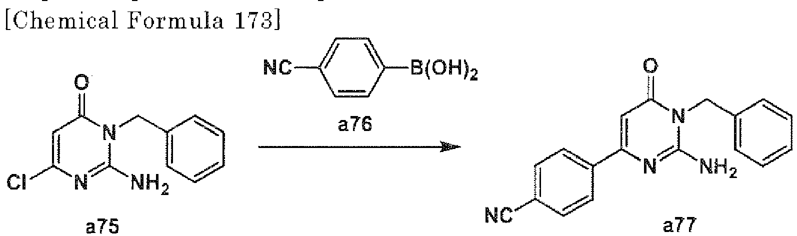

Compound a77 was obtained by the method similar to the preparation of Compound a 15.
LC-MS: m/z=303. [M+H]⁺
LC/MS method: Method B, retention time: 1.61 min

### Step 2: Preparation of Compound a80 (I-0205)

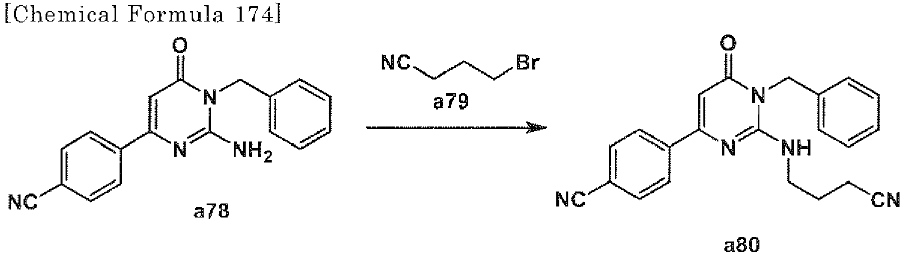

Compound a80 (1-0205) was obtained by the method similar to the preparation of Compound a70.
¹H-NMR (CDCl₃) δ: 8.06 (d, 2H, J = 8.4 Hz), 7.73 (d, 2H, J = 8.4 Hz), 7.43-7.36 (m, 3H), 7.28 (d, 3H, J = 6.9 Hz), 6.54 (s, 1H), 5.30 (s, 2H), 4.84 (s, 1H), 3.61 (q, 2H, J = 6.2 Hz), 2.11 (t, 2H, J = 6.9 Hz), 1.89-1.87 (m, 2H).
LC-MS: m/z=370 [M+H]⁺

### Example 14: Preparation of Compound a84 (I-0201)

### Step 1: Preparation of Compound a82

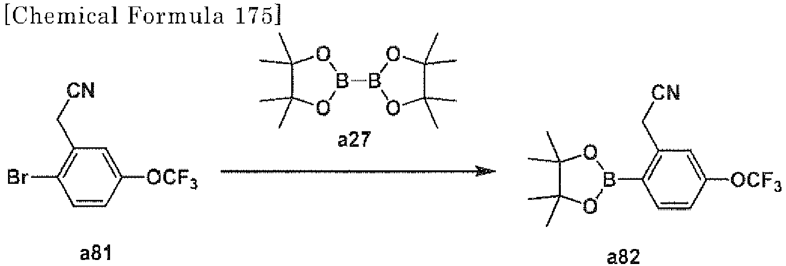

Compound a81 was obtained by the method similar to the preparation of Compound a28.
¹H-NMR (CDCl₃) δ: 7.92 (d, 1H, J = 8.3 Hz), 7.30 (s, 1H), 7.19 (s, 1H), 4.11 (s, 2H), 3.74 (s, 1H), 1.35 (s, 13H).

### Step 2: Preparation of Compound a84 (I-0201)

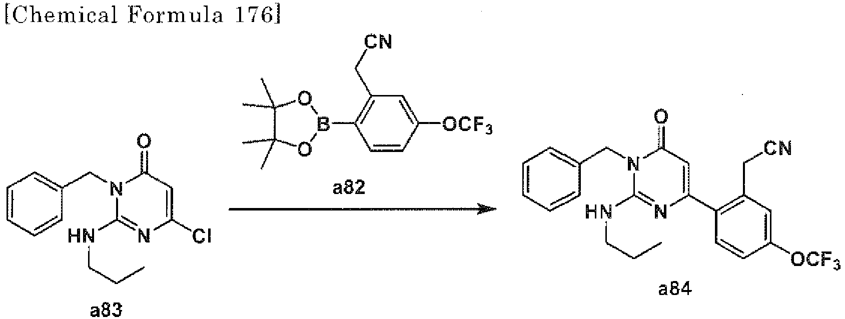

Compound a84 (1-0201) was obtained by the method similar to the preparation of Compound a15.
¹H-NMR (CDCl₃ ) δ: 7.56 (d, 1H, J = 8.3 Hz), 7.42-7.37 (m, 4H), 7.30-7.27 (m, 4H), 6.13 (s, 1H), 5.27 (s, 2H), 4.64 (s, 1H), 4.11 (s, 2H), 3.34-3.32 (m, 2H), 1.38-1.37 (m, 2H), 1.11-1.08 (m, 1H), 0.81 (t, 3H, J = 7.3 Hz).

### Example 15: Preparation of Compound a88 (I-0001)

### Step 1: Preparation of Compound a86

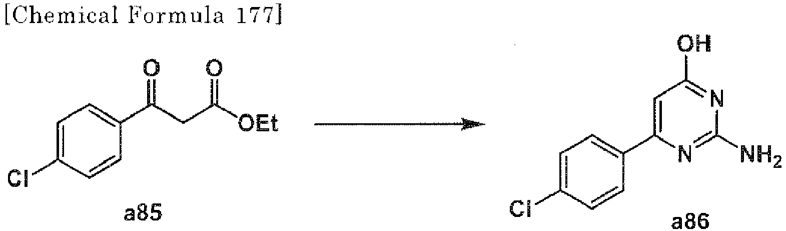

To a solution of Compound a85 (3g, 13.24mmol) in ethanol (24mL) was added guanidine hemi-carbonate (2.39g, 13.24mmol), and the solution was refluxed for 5 hours. After ethanol was removed under reduced pressrure, water was added to the reaction solution, and the precipitated solid was filtered. The solid washed with water and hexane, and dried under reduced pressure to yield Compound a86 (1.41g, yield: 48%) as a yellow solid.
¹H-NMR (DMSO-D₆) δ: 10.94 (1H, br s), 7.97 (2H, d, J = 8.8 Hz), 7.49 (2H, d, J = 8.5 Hz), 6.67 (2H, br s), 6.13 (1H, s).
LC-MS: m/z=222. [M+H]⁺

### Step 2: Preparation of Compound a87

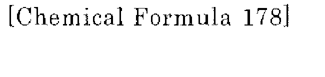

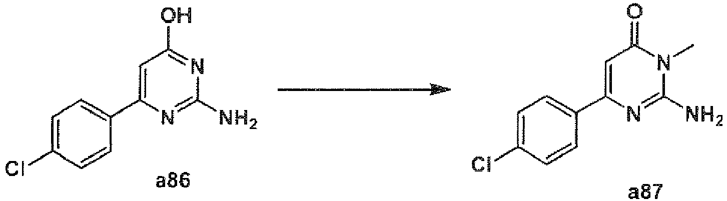

Compound a87 was obtained by the method similar to the preparation of Compound a68.
¹H-NMR (DMSO-D₆) δ: 7.99 (2H, d, J = 8.3 Hz), 7.51 (2H, d, J = 8.4 Hz), 7.28 (2H, br s), 6.24 (1H, s), 3.29 (3H, s).
LC-MS: m/z=236. [M+H]⁺

### Step 3: Preparation of Compound a88 (I-0001)

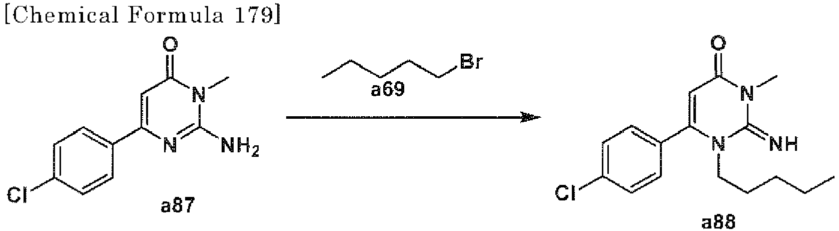

Compound a88 (I-0001) was obtained by the method similar to the preparation of Compound a70.
LC-MS: m/z=306 [M+H]⁺
LC/MS method: Method B, retention time: 2.49 min

### Example 16: Preparation of Compound a96 (I-0107)

### Step 1: Preparation of Compound a91

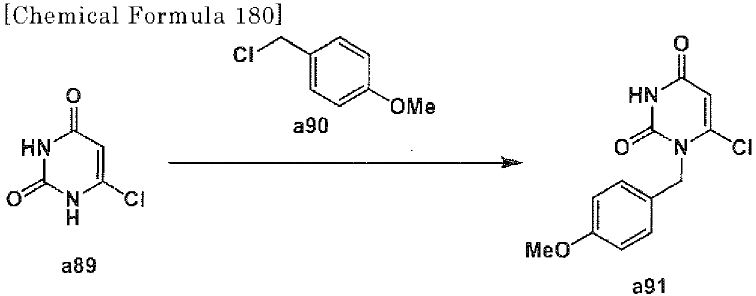

To a solution of Compound a89 (400mg, 2.73mmol) in DMF (4mL) was added potassium carbonate (189mg, 1.37mmol) and Compound a90 (483ul, 3.55mmol), and the solution was stirred at 70°C for 2 hours. After DMF was removed under reduced pressure, the residue was purified by silica gel column chromatography (chloroform / methanol) to yield Compound a91 (287.7mg, yield: 40%) as a white solid.
LC-MS: m/z=265 [M-H]⁺
LC/MS method: Method B, retention time: 1.52 min

### Step 2: Preparation of Compound a92

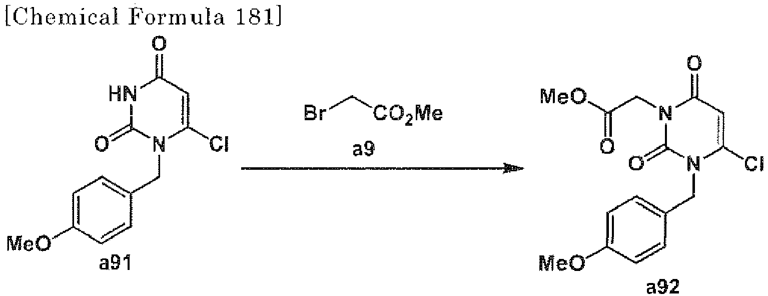

Compound a92 was obtained by the method similar to the preparation of Compound a18.
LC-MS: m/z=339 [M+H]⁺
LC/MS method: Method B, retention time: 1.84 min

### Step 3: Preparation of Compound a93

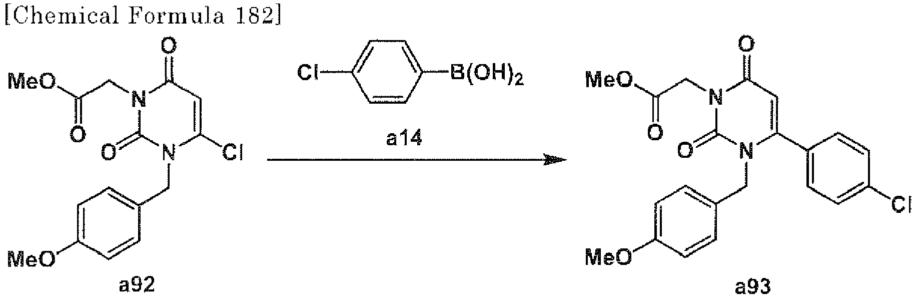

Compound a93 was obtained by the method similar to the preparation of Compound a15.
LC-MS: m/z=415 [M+H]⁺
LC/MS method: Method B, retention time: 2.17 min

### Step 4: Preparation of Compound a94

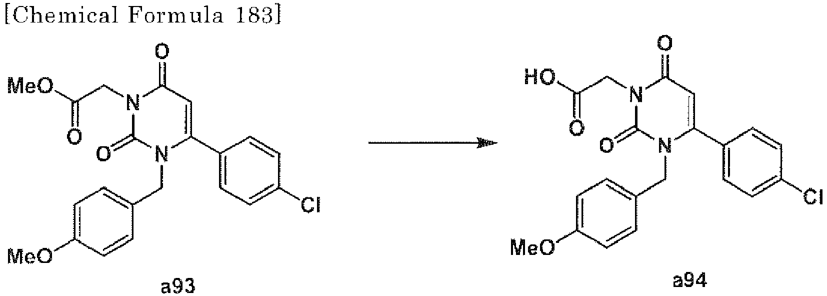

Compound a94 was obtained by the method similar to the preparation of Compound a11.
LC-MS: m/z=401. [M+H]⁺
LC/MS method: Method B, retention time: 2.00 min

### Step 5: Preparation of Compound a95

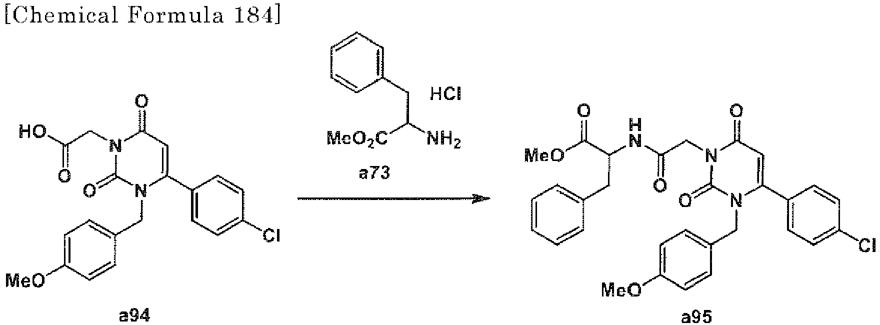

Compound a95 was obtained by the method similar to the preparation of Compound a13.
LC-MS: m/z=562 [M+H]⁺
LC/MS method: Method B, retention time: 2.32 min

### Step 6: Preparation of Compound a96 (I-0107)

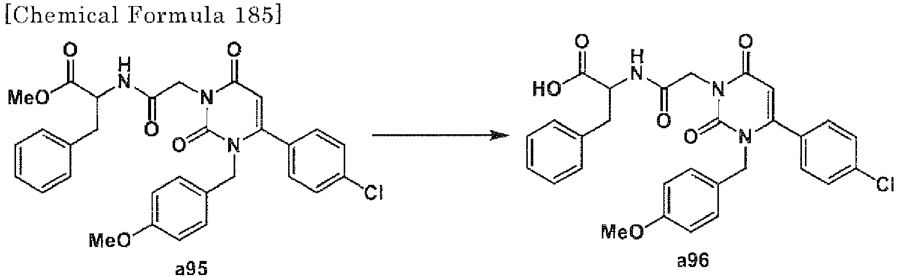

Compound a96 (I-0107) was obtained by the method similar to the preparation of Compound a11.
LC-MS: m/z=548 [M+H]⁺
LC/MS method: Method B, retention time: 2.13 min
¹H-NMR (CDCl₃) δ: 7.35 (d, 2H, J = 7.8 Hz), 7.21-7.19 (m, 3H), 7.08 (d, 2H, J = 7.8 Hz), 6.79-6.74 (m, 4H), 6.51 (br s, 1H), 5.67 (s, 1H), 4.86 (s, 3H), 4.69 (s, 2H), 3.75 (s, 3H), 3.25-3.13 (m, 2H), 1.26 (s, 4H), 0.88 (t, 2H, J = 6.3 Hz).

### Example 17: Preparation of Compound a100 (I-0115) and a101 (1-0116)

### Step 1: Preparation of Compound a97

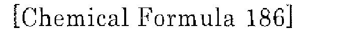

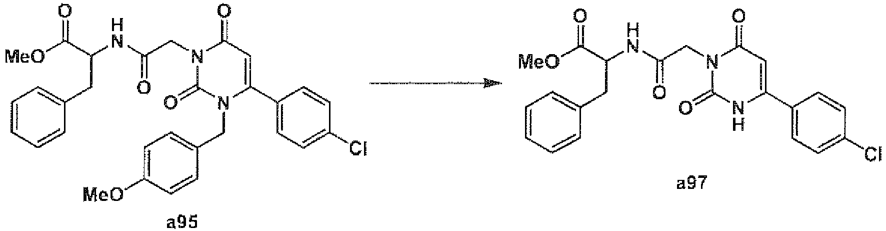

To a solution of Compound a95 (150mg, 0.27mmol) in acetonitrile / water (9:1, 1.5mL) was added ammonium hexanitrato cerium(IV) (293mg, 0.53mmol), and the mixture was stirred at 50°C for 3 hours. To the reaction solution was added water, and the solution was extracted with ethyl acetate twice. The organic layer was washed with water and brine, dried over anhydrous sodium sulfate and concentrated under reduced pressure. Diisopropyl ether was added to the residue, and the precipirated solid was filtered to yield Compound a97 (64mg, yield: 54%) as a pale brown solid.
¹H-NMR (DMSO-D₆) δ: 8.57 (s, 1H), 7.80 (d, 2H, J = 8.3 Hz), 7.57-7.55 (m, 2H), 7.29-7.27 (m, 2H), 7.23 (s, 3H), 5.96 (s, 1H), 4.46-4.42 (m, 4H), 3.58 (s, 3H), 3.17 (d, 1H, J = 4.8 Hz), 3.00-2.94 (m, 2H), 2.09 (s, 3H).

### Step 2: Preparation of Compound a98 and a99

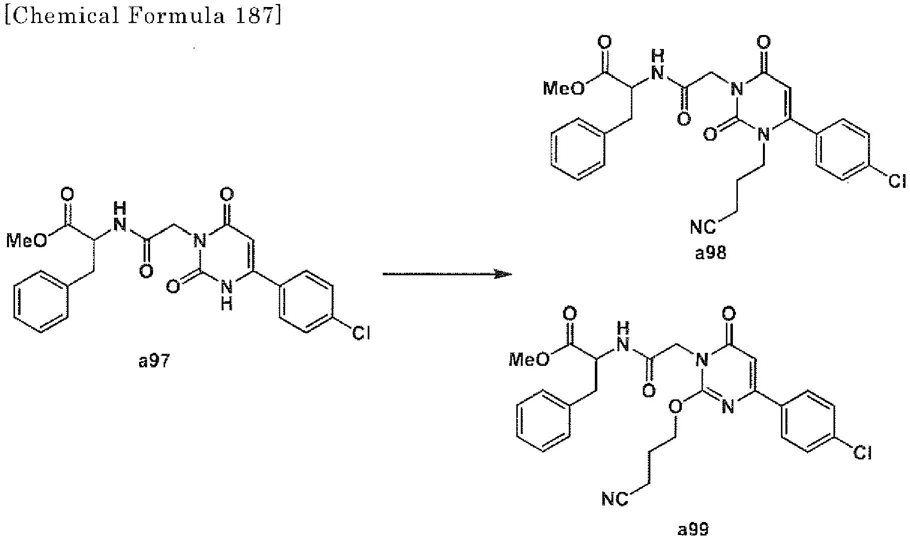

To a solution of Compound a97 (45mg, 0.10mmol) in DMF (450uL) was added triethylamine (16.9ul, 0.12mmol) and 4-bromobutane nitrile (12.1ul, 0.12mmol), and the solution was stirred at 80°C for 2 hours. The reaction solution was purified by silica gel column chromatography (chloroform / methanol) to yield a mixture of Compound a98 and a99 (100mg) as a brown oil.
LC-MS: m/z=509 [M+H]⁺
Compound a98
LC/MS method: Method B, retention time: 1.95 min
Compound a99
LC/MS method: Method B, retention time: 2.16 min

### Step 3: Preparation of Compound a100 (1-0115) and a101 (1-0116)

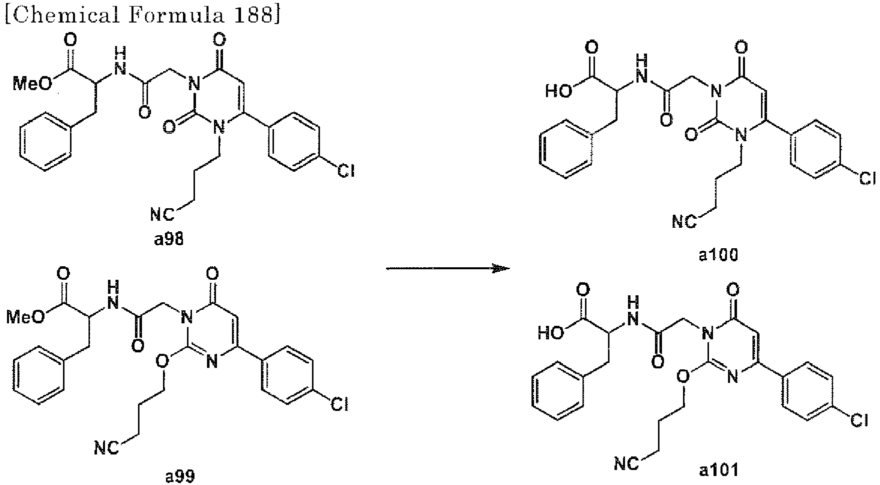

Compound a 100 (1-0115) and a101 (I-0116) was obtained by the method similar to the preparation of Compound a54.
a compound a100
¹H-NMR (CDCl₃) δ: 7.47 (d, 2H, J = 7.8 Hz), 7.25-7.20 (m, 8H), 7.08 (br s, 1H), 5.63 (s, 1H), 4.75-4.73 (m, 1H), 4.63-4.57 (m, 2H), 4.18 (br s, 2H), 3.76-3.74 (m, 2H), 3.48 (s, 1H), 3.21-3.19 (m, 1H), 3.08-3.06 (m, 1H), 2.64 (s, 1H), 2.19 (t, 2H, J = 6.8 Hz), 1.75 (t, 2H, J = 6.9 Hz).
Compound a101
¹H-NMR (CDCl₃) δ: 7.72 (d, 3H, J = 60.5 Hz), 7.24-7.19 (m, 9H), 6.40 (s, 1H), 4.71 (s, 2H), 4.48-4.27 (m, 4H), 3.26 (s, 1H), 2.94 (s, 1H), 2.63 (s, 1H), 2.26 (s, 2H), 1.87 (s, 2H).

### Example 18: Preparation of Compound d6

### Step 1: Preparation of Compound d3

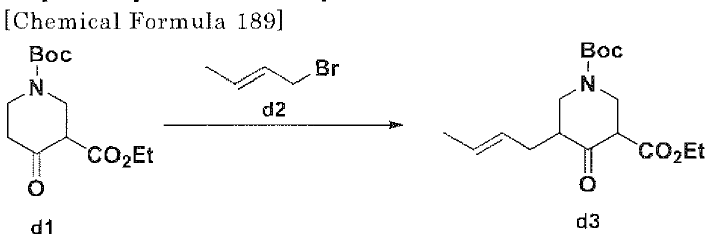

N-butyl lithium (2.5mol/L, 9.73mL, 24.3mmol) was dropped into a solution of diisopropyl amine (2.462g, 24.3mmol) in THF (15mL) at -10°C under nitrogen atomosphere, and the solution was stirred at -10°C for 15 minutes. A solution of Compound dl(3g, 11.1mmol) in THF (15mL) was dropped into the solution at 0°C, and the solution was stirred at 0°C for 1 hour. After Compound d2 (1.717g, 12.7mmol) was dropped, the solution was warmed to room temperature and stirred overnight. Saturated ammonium chloride aqueous solution was added to the reaction solution, and the solution was extracted with ethyl acetate three times. The organic layer was washed with brine, and concentrated under reduced pressure. The residue was purified by silica gel chromatography to yield Compound d3 (1.6g, yield: 44%) as a colorless oil.
MS: 270[M-56+H]⁺, RT: 2.27, LC/MS method: F

### Step 2: Preparation of Compound d4

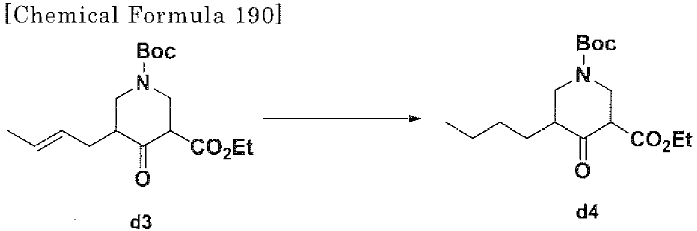

To a solution of Compound d3 (2g, 6.15mmol) in ethanol (20mL) was added 5% palladium-carbon (200mg), and the solution was stirred for 9 hours under hydrogen atomosphere. The reaction solution was filtered through celite, and the filtrate was concentrated under reduced pressure to yield Compound d4 (1.8g, yield: 89%) as a colorless oil.
MS: 272[M-56+H]⁺, RT: 2.33, LC/MS method: F

### Step 3: Preparation of Compound d6

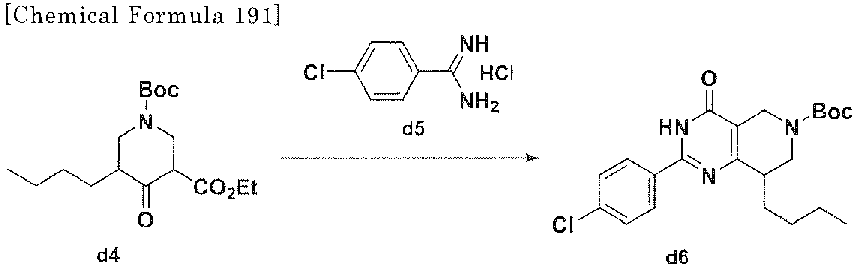

To a solution of Compound d4 (1.345g, 4.11mmol) in methanol (13.4mL) was added Compound d5(0.824g, 4.31mmol) and potassium t-butoxide (1.383g, 12.32mmol), and the solution was stirred at room temperature for 16 hours. After the reaction solution was concentrated under reduced pressure, the solution was added water (5mL) and neutralized to pH7 with 1mol/L hydrochloride aqueous solution. The precipitated solid was filtered and dried to yield Compound d6 (602mg, yield: 35%) as a gray solid.
MS: 418[M+H]⁺, RT: 2.21, LC/MS method: F

### Example 19: Preparation of Compound d11

### Step 1: Preparation of Compound d8

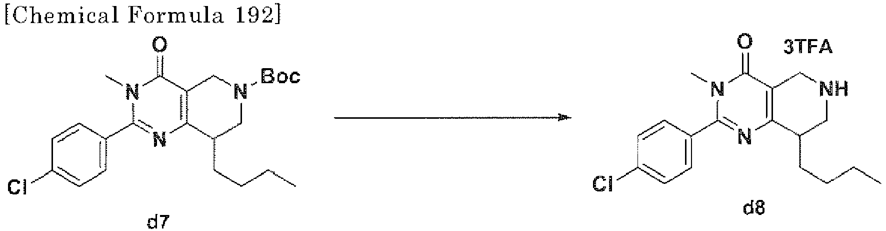

To a solution of Compound d7 (130.1mg, 0.301mmol) in dichloromethane (2mL) was added trifluoroacetic acid (2mL), and the solution was stirred at room temperature for 15 minutes. The reaction solution was concentrated under reduced pressure, and the residue was co-evaporated with methanol three times to yield a trifluoroacetic acid salt of Compound d8 as oil. The obtained crude product was used in the next step.
MS: 418[M+H]⁺, RT: 2.74, LC/MS method: E

### Step 2: Preparation of Compound d10

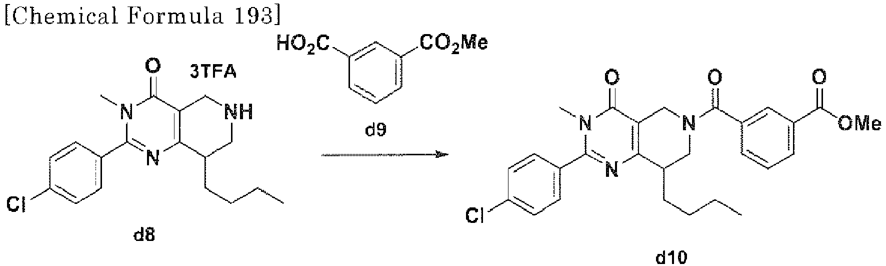

To a solution of Compound d8 (50.0mg, 0.074mmol) in DMF (2mL) was added DIEA (65ul, 0.371-mmol). To the solution was added a solution of Compound d9 (16.0mg, 0.089mmol), HATU (33.9mg, 0.089mmol) and DIEA (26ul, 0.148mmol) in DMF (2mL) which is separately prepared, and the obtained solution was stirred at room temperature for 15 minutes. Water was added to the reaction solution, and the mixture was extracted with ethyl acetate. The organic layer was washed with water three times, and concentrated under reduced pressure. The obtained residue was purified by silica gel chromatography (ethyl acetate / hexane) to yield Compound d10 (28mg, yield: 75%) as colorless oil.
MS: 494[M+H]⁺, RT: 2.47, LC/MS method: E

### Step 3: Preparation of Compound d11

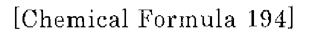

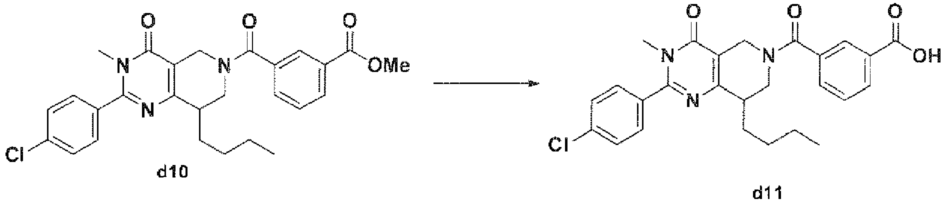

To a solution of Compound d10 (36.4mg, 0.074mmol) in THF/methanol (2:1, 1.5mL) was added 2mol/L sodium hydroxide aqueous solution (0.147ml, 0.295mmol), and the solution was stirred at 50°C for 20minutes. After the reaction solution was concentrated under reduced pressure, water was added to the residue and the mixture was adjusted to pH4 with 2mol/L hydrochloric acid aqueous solution, and extracted with ethyl acetate. The organic layer was washed with water, and concentrated under reduced pressure. The residue was purified by silica gel column chromatography (methanol / chloroform) and solidified with ethyl acetate / hexane to yield Compound d11 (24mg, yield: 67%) as a white solid.
MS: 480[M+H]⁺, RT: 2.19, LC/MS method: E

### Example 20: Preparation of Compound d14

### Step 1: Preparation of Compound d13

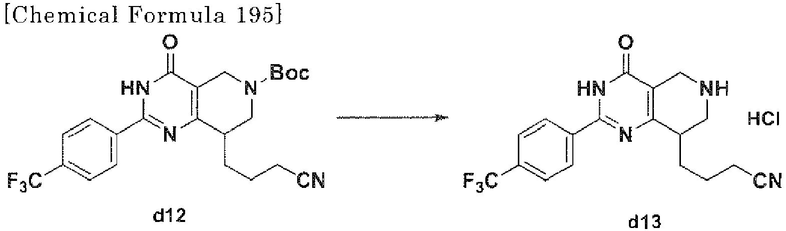

To a solution of Compound d12 (430mg, 0.930mmol) in 1,4-dioxane (3mL) was added 4mol/L hydrochloric acid / dioxane solution (1 ml, 4.00 mmol), and the mixture was stirred at room temperature for 2hours 30 minutes. After the reaction solution was concentrated under reduced pressure, ethyl acetate was added, and the precipitated solid was filtered to yield Compound d13 (289mg, yield: 78%) as a white solid.
MS: 363 [M+H]⁺, RT: 1.38, LC/MS method: E
¹H-NMR (DMSO-D₆)δ: 9.54 (2H, s), 8.32 (2H, d, J = 7.7 Hz), 7.92 (2H, d, J = 8.3 Hz), 3.99 (2H, dd, J = 21.9, 16.6 Hz), 3.53 (1H, dd, J = 12.5, 5.4 Hz), 3.23 (1H, dd, J = 12.6, 8.0 Hz), 3.06 (1H, s), 2.57 (2H, t, J = 6.1 Hz), 2.12-2.10 (1H, m), 1.81-1.72 (3H, m).

### Step 2: Preparation of Compound d14

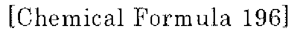

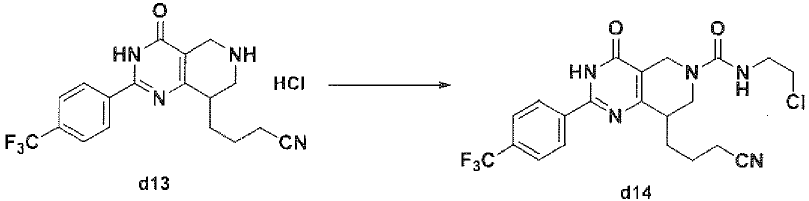

To a solution of Compound d13 (30mg, 0.075mmol) in THF (1mL) was added trimethylamine (31.5µL, 0.226mmol) and 1-chloro-2-isocyanato ethane (9.68µL, 0.113mmol), and the mixture was stirred at room temperature overnight. After to the reaction solution was added methanol and the mixture was concentrated under reduced pressure, the residue was purified by silica gel column chromatography (methanol / chloroform), and then purified by suspending with ethyl acetate / hexane to yield Compound d14 (8.1mg, yield: 23%) as a white solid.
MS: 468 [M+H]⁺, RT: 1.88, LC/MS method: E
¹H-NMR (DMSO-D₆) δ: 8.29 (2H, d, J = 8.0 Hz), 7.90 (2H, d, J = 8.0 Hz), 7.08-7.05 (1H, m), 4.37 (1H, d, J = 17.6 Hz), 4.12 (1H, d, J = 18.1 Hz), 3.79-3.76 (1H, m), 3.61 (2H, t, J = 6.4 Hz), 2.72-2.70 (1H, m), 2.56 (2H, t, J = 7.0 Hz), 1.84-1.81 (3H, m), 1.57-1.55 (1H, m).

### Example 21: Preparation of Compound d15

### Step 1: Preparation of Compound d15

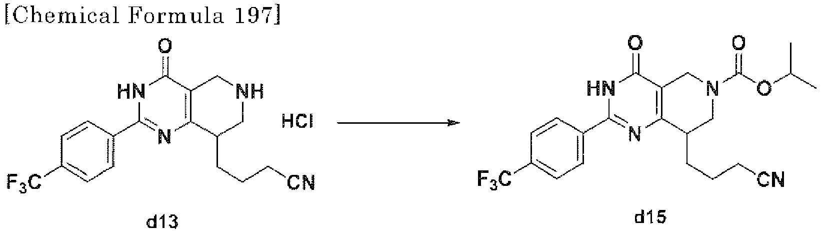

To a solution of Compound d13 (22mg, 0.055mmol) in THF (1mL) was added trimethylamine (23.07µL, 0.165mmol) and isopropylcarbochloridate (11.37µL, 0.083mmol), and the mixture was stirred at room temperature for 3 hours 30 minnutes. After methanol was added to the reaction solution and the mixture was concentrated under reduced pressure, the residue was purified by silica geI column chromatography (methanol / chloroform) to yield Compound d15 (22mg, yield: 89%) as a white solid.
MS: 449 [M+H]⁺, RT: 2.03, LC/MS method: E
¹H-NMR (DMSO-D₆) δ: 8.29 (2H, d, J = 8.2 Hz), 7.90 (2H, d, J = 8.3 Hz), 4.88-4.82 (1H, m), 4.50-4.42 (1H, m), 4.05-3.96 (2H, m), 2.73 (1H, s), 2.58-2.57 (2H, m), 1.91-1.70 (3H, m), 1.53-1.51 (1H, m), 1.23 (6H, d, J = 6.1 Hz).

### Example 22: Preparation of Compound d18

### Step 1: Preparation of Compound d18

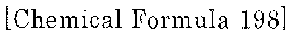

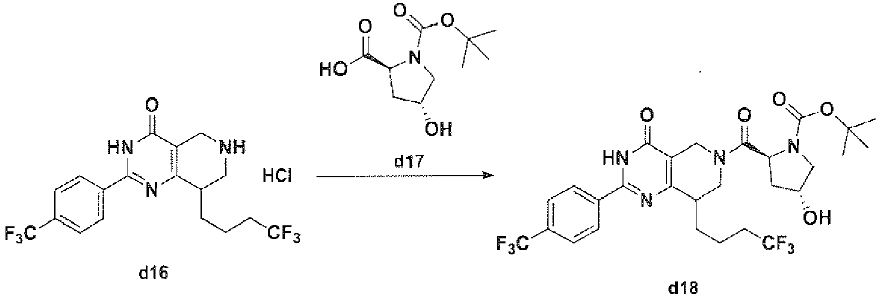

To a solution of Compound d16 (100mg, 0.226mmol) in DMF (1mL) was added Compound d17 (62.8mg, 0.272mmol), HATU (103mg, 0.272mmol) and trimethylamine (94ul, 0.679mmol), and the mixture was stirred at room temperature for 40minutes. Methanol and water was added to the reaction solution, and the mixture was extracted with ethyl acetate. The organic layer was dried over anhydrous sodium sulfate and concentrated under reduced pressure. The residue was purified by silica gel column chromatography (chloroform / methanol) to yield Compound d18 (135mg, yield: 96%) as a white solid.
MS: 619 [M+H]⁺, RT: 2.23, LC/MS method: E

### Example 23: Preparation of Compound d21

### Step 1: Preparation of Compound d20

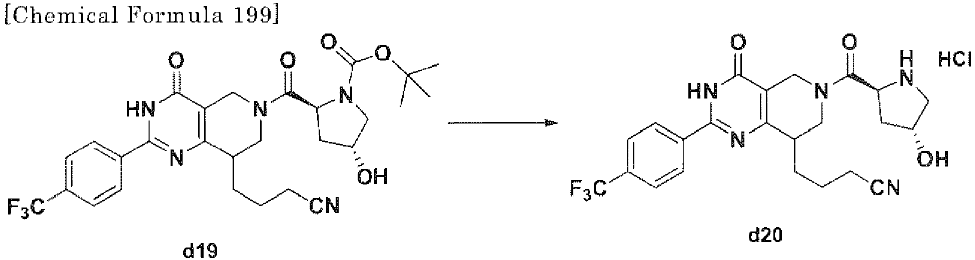

To a solution of Compound d19 (85mg, 0.148mmol) in 1,4-dioxane (1mL) was added 4mol/L hydrochloric acid / dioxane solution (0.5ml, 2.00mmol), and the mixture was stirred at room temperature for 1hours. After the reaction solution was concentrated under reduced pressure, ethyl acetate was added to the mixture. The precipitated solid was filtered to yield Compound d20 (72mg, yield: 95%) as a white solid.
MS: 476 [M+H]⁺, RT: 1.41, LC/MS method: E

### Step 2: Preparation of Compound d21

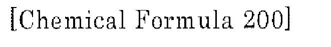

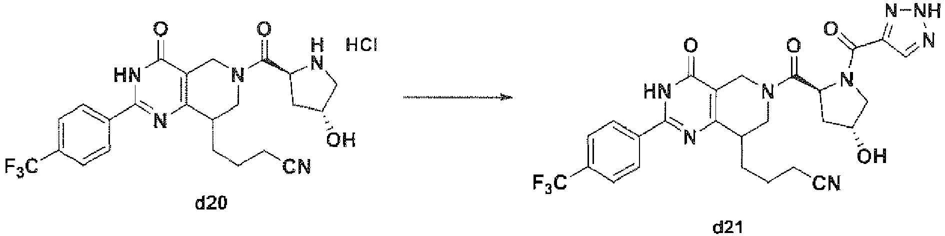

To a solution of Compound d20 (50mg, 0.098mmol) in DMF (1mL) was added 1H-1,2,3-triazole-4-carboxylic acid (13.3mg, 0.117mmol), HATU (44.6mg, 0.117mmol) and trimethylamine (41ul, 0.293mmol), and the mixture was stirred at room temperature overnight. Methanol and water was added to the reaction solution, and the mixture was extracted with ethyl acetate. The organic layer was dried over anhydrous sodium sulfate and concentrated under reduced pressure. The residue was purified by silica gel column chromatography (chloroform / methanol) to yield Compound d21 (49mg, yield: 88%) as a white solid.
MS: 571 [M+H]⁺, RT: 1.58, LC/MS method: E

### Example 24: Preparation of Compound d23

### Step 1: Preparation Compound d23

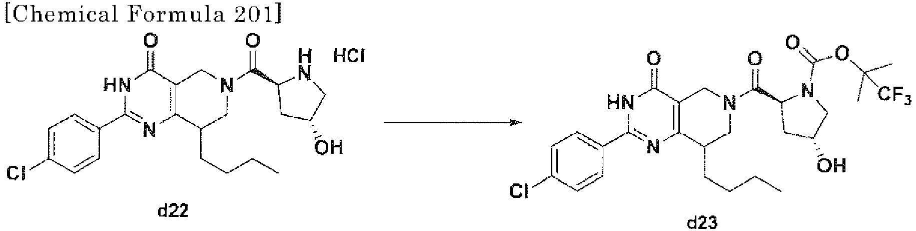

To a solution of 1,1,1-trifluoro-2-methylpropane-2-ol (0.322ml, 3.00mmol) in dichloromethane (1mL) was added 4-nitrophenyl carbonochloridate (91mg, 0.449mmol) and DMAP (183mg, 1.498mmol) at -40°C, and the mixture was warmed to 0°C and stirred for 1hours. Compound d22 (50mg, 0.098mmol) was added to the mixture at -40°C, and the mixture was stirred at room temperature for 1hour30minutes. To the reaction mixture was added methanol and sodium hydroxide aqueous solution, and the mixture was extracted with ethyl acetate. The organic layer was washed with brine, dried over anhydrous sodium sulfate, and concentrated under reduced pressure. The residue was purified by silica gel column chromatography (chloroform / methanol) to yield Compound d23 (51mg, yield: 58%) as a white solid.
MS: 585 [M+H]⁺, RT: 2.34, LC/MS method: E

### Example 25: Preparation of Compound d26

### Step 1: Preparation of Compound d25

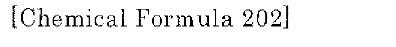

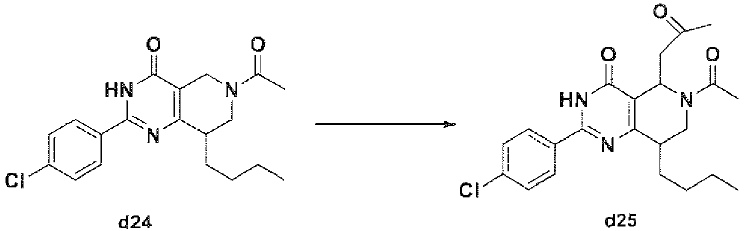

To a solution of Compound d24 (150mg, 0.417mmol) in 1,2-dichloroethane (3mL) was added trimethyl (1-propene-2-yl oxy) silane (1.38mL, 8.336mmol), copper (I) bromide (23.92mg, 0.167mmol) and t-butyl hydroperoxide (400µL, 2.000mmol), and the mixture was stirred at 80°C for 6hours. To the reaction solution was added water, and the solution was extracted with ethyl acetate. The organic layer was washed with brine, dried over anhydrous sodium sulfate, and concentrated under reduced pressure. The residue was purified by silica gel column chromatography (chloroform / methanol) to yield Compound d25 (58mg, yield: 34%) as a yellow solid. MS: 416 [M+H]⁺, RT: 2.12, 2.21, LC/MS method: E

### Step 2: Preparation of Compound d26

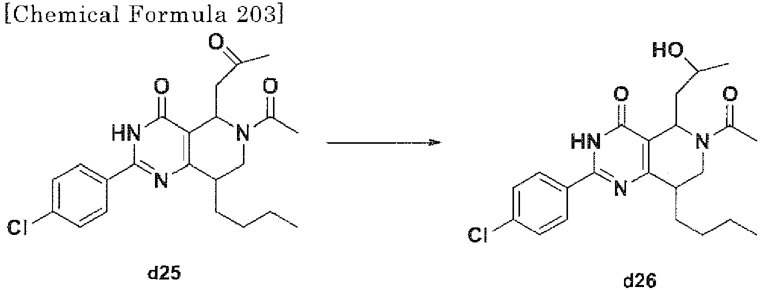

To a solution of Compound d25 (50mg, 0.120mmol) in ethanol (2mL) was added sodium borohydride (22.74mg, 0.601mmol) under ice-cooling, and the mixture was stirred for 45 minutes. To the reaction solution was added saturated ammonium chloride aqueous solution, and the solution was extracted with ethyl acetate. The organic layer was dried over anhydrous sodium sulfate and concentrated under reduced pressure. The residue was purified by silica gel column chromatography (chloroform / methanol) to yield Compound d26 (5mg, yield: 10%) as colorless oil. MS: 418 [M+H]⁺, RT: 2.09, 2.17, 2.28, LC/MS method: E

### Example 26: Preparation of Compound d31

### Step 1: Preparation of Compound d28

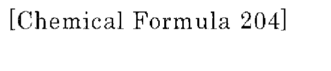

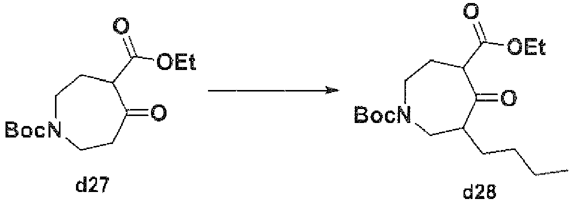

To a solution of diisopropyl amine (1.460ml, 10.24mmol) in THF (20mL) was added n-butyt lithium / hexane solution (3.72ml, 10.02mmol) in the ice / methanol. After cooling to -50°C, a solution of Compound d27 (1.299g, 4.55mmol) in THF (8mL) was dropped into the solution, and the mixture was stirred for 30 minutes. To the reaction solution was added a solution of HMPA (3.96ml, 22.76mmol) and methyl iodide (1005mg, 5.46mmol) in THF (5mL), and the mixture was stirred for 40 minutes. After stirred overnight, saturated ammonium chloride aqueous solution was added to the solution, and the solution was extracted with ethyl acetate. The organic layer was washed with water three times, and concentrated under reduced pressure. The obtained residue was purified by silica gel column chromatography (ethyl acetate / hexane) to yield Compound d28 (685mg, yield: 44%) as colorless oil. MS: 342 [M+H]⁺, RT: 2.91, LC/MS method: E

### Step 2: Preparation of Compound d29

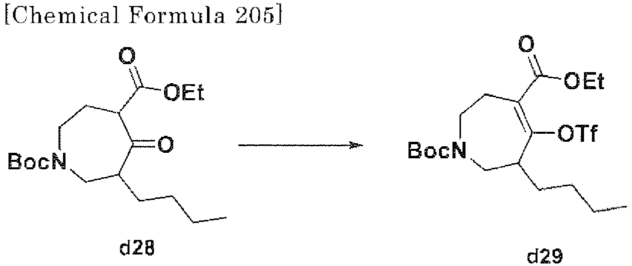

To a solution of Compound d28 (682.1mg, 1.998mmol) in THF (20mL) was added N-phenyl-bis(trifluoromethane sulfonimide) (0.785g, 2.197mmol) and sodium hydride (0.120g, 3.00mmol), the mixture was stirred at room temperature overnight. To the reaction solution was added saturated ammonium chloride aqueous solution, the solution was extracted with ethyl acetate, and the organic layer was concentrated under reduced pressure. The obtained residue was purified by silica gel column chromatography (ethyl acetate / hexane) to yield Compound d29 (1.05g, yield: 65%, purity: 59%) as yellow oil.
MS: 418 [M+H]⁺, RT: 2.91, LC/MS method: E

### Step 3: Preparation of Compound d30

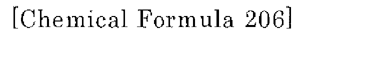

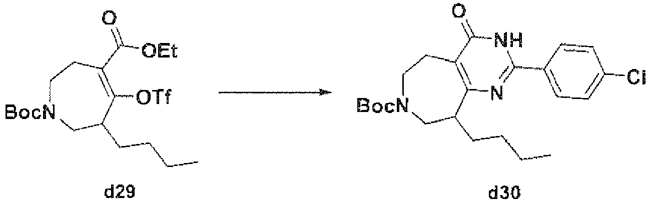

To a solution of Compound d29 (500.0mg, 0.623mmol) in 1,4-dioxane (10mL) was added 4-chlorobenzimidate hydroiodide (264mg, 0.935mmol), Pd₂ (dba)₃ (28.5mg, 0.031mmol), xantphos (36.0mg, 0.062mmol) and cesium carbonate (812mg, 2.492mmol), and the mixture was stirred at 130°C for 15 minutes under microwave irradiation. To the reaction solution was added water, and the solution was extracted with ethyl acetate, and the organic layer was concentrated under reduced pressure. The obtained residue was purified by silica gel column chromatography (ethyl acetate / hexane) and purified by suspending with ethyl acetate / hexane to yield Compound d30 (202mg, yield: 75%) as a white solid.
MS: 432 [M+H]⁺ RT: 2.74, LC/MS method: E
¹H-NMR (CDCl₃) δ: 0.90-0.91 (3H, m), 1.26-1.85 (15H, m), 2.79-2.82 (1H, m), 3.04-3.36 (3H, m), 3.51-3.55 (1H, m), 3.76-3.90 (2H, m), 7.49 (2H, d, J = 8.7 Hz), 8.18 (2H, d, J = 8.7 Hz), 12.75-12.80 (1H, m).

### Step 4: Preparation of Compound d31

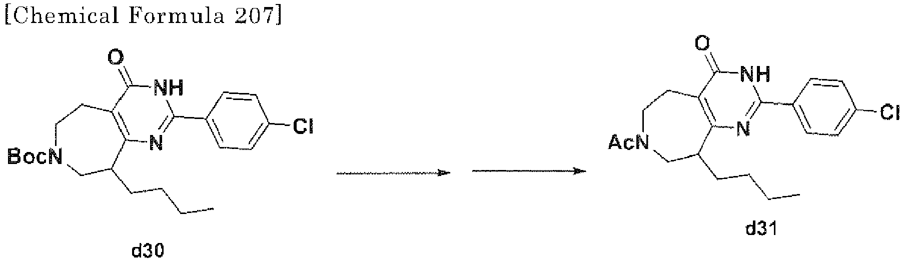

To a solution of Compound d30 (34.0mg, 0.079mmol) in dichloromethane (0.2mL) was added trifluoroacetic acid (0.2mL), and the mixture was stirred at room temperature for 20minutes. The reaction solution was concentrated under reduced pressure, and the residue was co-evaporated with methanol three times. To the residue was added 2mol/L sodium hydroxide aqueous solution (0.118mL, 0.236mmol) and anhydrous acetic acid (0.382mL, 0.094mmol), and the mixture was stirred at room temperature overnight. To the reaction solution was added water, and the solution was extracted with ethyl acetate. The aqueous layer was extracted with dichloromethane, and the organic layer was combined and concentrated under reduced pressure. The obtained residue was purified by silica gel column chromatography (methanol / chloroform) to yield Compound d31 (21mg, yield: 70%) as a white solid.
MS: 374 [M+H]⁺, RT: 2.06, LC/MS method: E
¹H-NMR (CDCl₃) δ: 0.87-0.94 (3H, m), 1.26-1.48 (4H, m), 1.62-1.96 (2H, m), 2.15-2.22 (3H, m), 2.79-3.99 (7H, m), 7.51-7.53 (2H, m), 8.16-8.21 (2H, m), 12.63-12.77 (1H, m).

### Example 27: Preparation of Compound d32

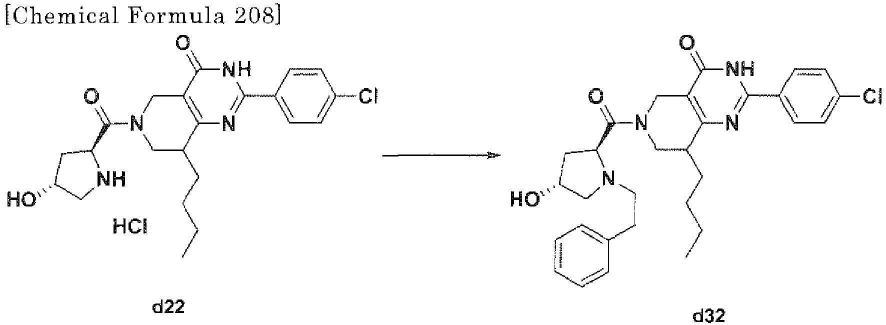

To a solution of Compound d22 (20mg, 0.043mmol) in dichloromethane (1mL) was added trimethylamine (0.018ml, 0.128mmol) and (2-bromoethyl)benzene (9.50mg, 0.051mmol), and the mixture was stirred at 60°C for 3hours 30minutes. To the reaction solution was added water, and the solution was extracted with ethyl acetate. The organic layer was washed with brine, dried over anhydrous sodium sulfate, and concentrated under reduced pressure. The obtained residue was purified by silica gel column chromatography (methanol / chloroform) and purified by suspending with ethyl acetate / hexane to yield Compound d32 (10mg, yield: 44%) as a white solid. MS: 535 [M+H]⁺, RT: 1.94, 1.97, LC/MS method: E

### Example 28: Preparation of Compound d35

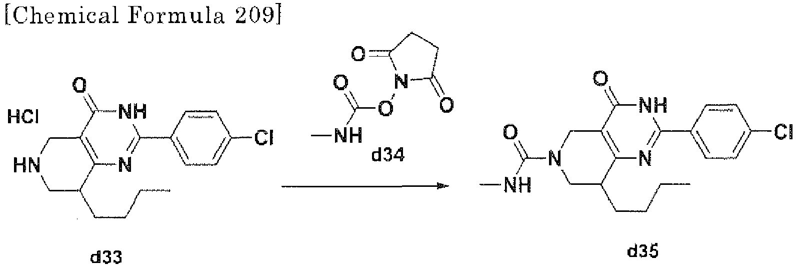

To a solution of Compound d33 (30mg, 0.085mmol) in THF (1mL) was added Compound d34 (35mg, 0.204mmol) and DIEA (74.0µL, 0.423mmol), and the mixture was stirred at room temperature for 2 hours. To the reaction solution was added water, and the solution was extracted with ethyl acetate. The organic layer was washed with brine, dried over anhydrous sodium sulfate, and concentrated under reduced pressure. The obtained residue was purified by suspending with ethyl acetate / hexane to yield Compound d35 (20.6mg, yield: 65%) as a white solid.
MS: 375 [M+H]⁺, RT: 2.04, LC/MS method: E
¹H-NMR (CDCl₃) δ: 8.08 (2H, d, J = 8.8 Hz), 7.49 (2H, d, J = 8.5 Hz), 4.70-4.68 (1H, m), 4.37 (1H, d, J = 17.1 Hz), 4.25 (1H, d, J = 17.1 Hz), 3.92 (1H, dd, J = 13.3, 4.3 Hz), 3.46 (1H, dd, J = 13.3, 4.0 Hz), 2.90 (3H, d, J = 4.5 Hz), 2.76-2.73 (1H, m), 1.88-1.85 (1H, m), 1.61-1.37 (7H, m), 0.94 (3H, t, J = 7.2 Hz).

### Example 29: Preparation of Compound d43

### Step 1: Preparation of Compound d37

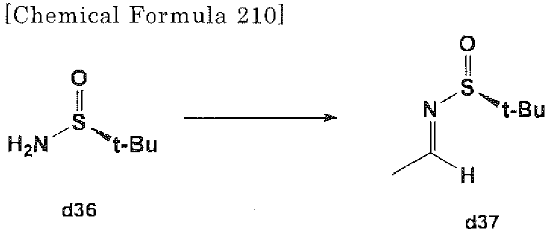

To a solution of Compound d36 (2.000g, 16.50mmol) in dichloromethane (10mL) was added acetaldehyde (1.845ml, 33.0mmol) and magnesium sulfate (7.94g, 66.0mmol), and the mixture was stirred at room temperature for 24 hours. After the insoluble material was filtered out, the filtrate was concentrated under reduced pressure, the obtained residue was purified by silica gel column chromatography (ethyl acetate / hexane) to yield Compound d37 (1.91g, yield : 79%) as colorless oil.

### Step 2: Preparation of Compound d38

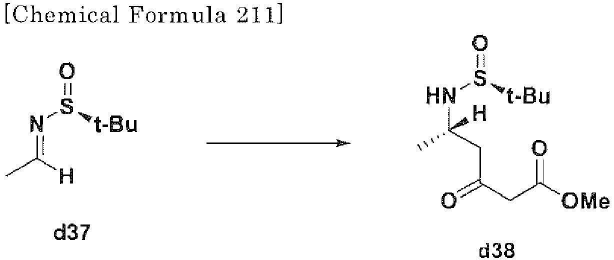

To a solution of sodium bis(trimethylsilyl)amide (71.3ml, 78mmol) in THF (68mL) was added methyl acetate (5.16ml, 64.8mmol) at -78°C, and the mixture was stirred for 15 minutes. To the reaction solution was added ether (37mL), and a solution of Compound d37 (1.908g, 12.96mmol) in ether (8mL) was dropped into the solution. After warmed to -50°C and stirred for 1 hour 30 minutes, to the reaction solution was added saturated ammonium chloride aqueous solution, and the solution was extracted with ethyl acetate and concentrated under reduced pressure. The obtained residue was purified by silica gel column chromatography (ethyl acetate / hexane) to yield Compound d38 (1.75g, yield: 46%) as colorless oil.
MS: 264 [M+H]⁺, RT: 1.41, LC/MS method: E

### Step 3: Preparation of Compound b39

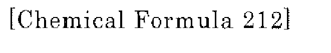

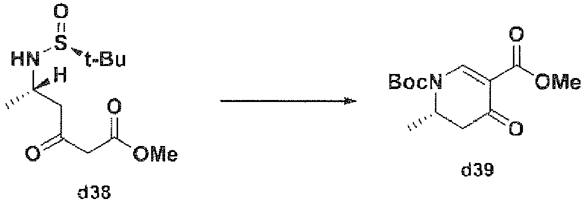

To a solution of Compound d38 (2.191g, 8.32mmol) in N,N-dimethylformamide dimethyl acetal (2.228ml, 16.64mmol), and the mixture was stirred at 80°C for 1 hour 30 minutes. The reaction solution was concentrated under reduced pressure, 4mol/L hydrochloric acid / 1,4-dioxane (20.80ml, 83mmol) was added to the solution, and the mixture was stirred at room temperature for 1 hour, and concentrated under reduced pressure. To a solution of the residue in acetonitrile, (22 mL) was added trimethylamine (5.77ml, 41.6mmol), N,N-dimethyl amino pyridine (0.508g, 4.16mmol) and di-carbonate-tert-butyl (2.318ml, 9.98mmol), and the mixture was stirred at room temperature for 1 hour 30 minutes. Water was added to the reaction solution, and the solution was extracted with ethyl acetate, and concentrated under reduced pressure. The obtained residue was purified by silica gel column chromatography (ethyl acetate / hexane) to yield Compound d39 (1.87g, yield: 70%, purity: 84wt%). ¹H-NMR (CDCl₃) δ: 1.24 (3H, d, J = 6.8 Hz), 1.58 (9H, s), 2.38 (1H, dd, J = 15.8, 1.5 Hz), 2.86 (1H, dd, J = 15.8, 6.8 Hz), 3.81 (3H, s), 4.66 (1H, dt, J = 8.5, 3.0 Hz), 8.74 (1H, d, J = 1.3 Hz).

### Step 4: Preparation of Compound d40

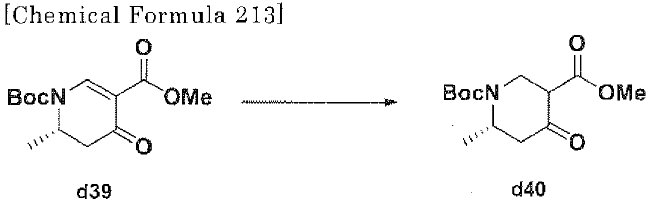

To a solution of Compound d39 (1.000g, 2.91mmol) in ethanol (10mL) was added sodium borohydride (28.0mg, 0.741mmol), and the mixture was stirred for 15 minutes. After the reaction solution was concentrated under reduced pressure, water was added to the residue and adjusted to pH5.5 with 2mol/L hydrochloric acid aqueous solution, and the mixture was extracted with ethyl acetate. The organic layer was concentrated under reduced pressure, and the obtained residue was purified by silica gel column chromatography (ethyl acetate / hexane) to yield Compound d40 (713mg, yield: 90%) as colorless oil.
¹H-NMR (CDCl) δ: 1.15 (3H, t, J = 7.1 Hz), 1.48 (9H, s), 2.02-2.09 (1H, m), 2.65-2.71 (1H, m), 3.62 (1H, d, J = 15.8 Hz), 3.78 (3H, s), 4.42-4.48 (1H, m), 4.57-4.63 (1H, m), 11.97 (1H, s).

### Step 5: Preparation of Compound d41

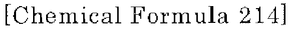

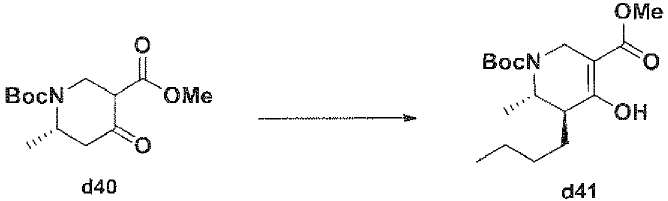

To a solution of diisopropylamine (0.839ml, 5.89mmol) in THF (2mL) was added n-butyl lithium solution (3.64ml, 5.82mmol) at 0°C, and the mixture was stirred for 20 minutes. After cooled to -45°C, a solution of Compound d40 (710mg, 2.62mmol) in THF (8mL) was dropped into the solution, and the solution was warmed to -30°C. After the reaction solution was cooled to -45°C, HMPA (2.73ml, 15.70mmol) and butyl iodide (578mg, 3.14mmol) was dropped into the solution, and the solution was stirred for 2 hours. To the reaction solution was added saturated ammonium chloride aqueous solution, and the mixture was extracted with ethyl acetate. The organic layer was washed with water, concentrated under reduced pressure, and the obtained residue was purified by silica gel column chromatography (ethyl acetate / hexane) to yield Compound d41 (488mg, yield: 57%) as colorless oil. ¹H-NMR (CDCl₃) δ: 0.91 (3H, s), 1.12 (3H, d, J = 6.9 Hz), 1.21-1.51 (16H, m), 2.02-2.04 (1H, m). 3.57-3.77 (4H, m), 4.33-4.54 (2H, m), 11.98 (1H, d, J = 0.4 Hz).

### Step 6: Preparation of Compound d42

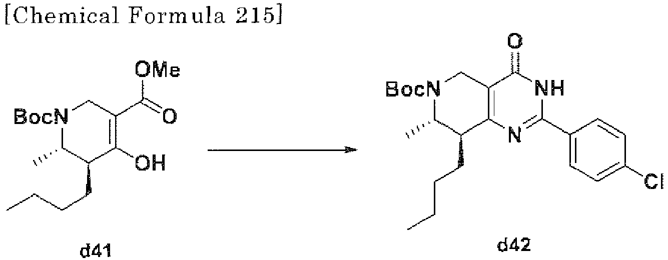

To a solution of Compound d41 (486.0mg, 1.484mmol) in methanol (13mL) was added 4-chlorobenzimidate hydrochloride (503mg, 1.781mmol) and potassium tert-butoxide (500mg, 4.45mmol), and the mixture was stirred at 135°C for 1 hour under microwave irradiation. To the reaction solution was added water, and the solution was adjusted to pH6 with 2mol/L hydrochloric acid aqueous solution, extracted with ethyl acetate, and then the organic layer was washed with water, and concentrated under reduced pressure. The obtained residue was purified by silica gel column chromatography (ethyl acetate / hexane) to yield Compound d42 (34mg, yield: 5%) as a white solid.
MS: 432, RT: 2.78, LC/MS method: E
¹H-NMR (CDCl₃) δ: 0.86-1.02 (4H, m), 1.13 (3H, d, J = 6.3 Hz), 1.24-1.73 (23H, m), 2.41-2.55 (1H, m), 3.94-4.12 (1H, m), 4.56-4.94 (2H, m), 7.51 (2H, d, J = 8.5 Hz), 8.22 (2H, d, J = 7.2 Hz), 12.77-12.90 (1H, m).

### Step 7: Preparation of Compound d43

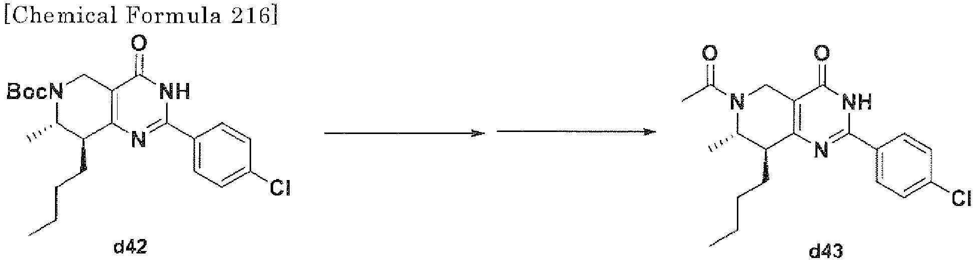

To a solution of Compound d42 (17.0mg, 0.039mmol) in dichloromethane (1mL) was added trifluoroacetic acid (1mL), and the mixture was stirred at room temperature for 15 minutes. The reaction solution was concentrated under reduced pressure, and the residue was co-evaporated with ethanol twice. To a solution of the residue in THF (2mL) was added 2mol/L sodium hydroxide aqueous solution (0.098ml, 0.197mmol) and a drop of acetic anhydride was added, and the mixture was stirred for 1 hour at room temperature. After the reaction mixture was concentrated under reduced pressure, water was added to the residue, the solution was extracted with dichloromethane and the organic layer was concentrated under reduced pressure. The obtained residue was purified by silica gel column chromatography (ethyl acetate / hexane) to yield Compound d43 (6.5mg, yield: 44%) as a white solid.
MS: 374 [M+H]⁺, RT: 2.07, LC/MS method: E
¹H-NMR (CDCl₃) δ: 0.91-1.62 (19H, m), 2.24 (3H, s), 2.55-2.62 (1H, m), 3.94-4.58 (2H, m), 5.12-5.21 (1H, m), 7.53 (2H, d, J = 7.3 Hz), 8.14-8.22 (2H, m), 12.61 (1H, s).

### Example 30: Preparation of Compound d48

### Step 1: Preparation of Compound d45

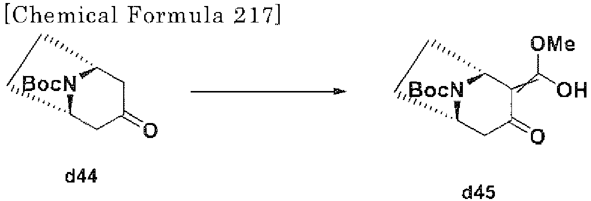

Sodium bis(trimethylsilyl)amide (14ml, 15.40mmol) was dropped into a solution of Compound d44 (3.000g, 13.32mmol) in THF (45mL) under ice-cooling. After the reaction mixture was cooled to -78°C, methyl cyanoformate (1.473g, 17.31mmol) was added to the reaction mixture, and the solution was warmed to room temperature. To the reaction solution was added saturated ammonium chloride aqueous solution, and the mixture was extracted with ethyl acetate, and concentrated under reduced pressure. The obtained residue was purified by silica gel column chromatography (ethyl acetate / hexane) to yield Compound d45 (1.85g, yield: 49%) as colorless oil. MS: 284 [M+H]⁺ RT: 2.21, LC/MS method: E

### Step 2: Preparation of Compound d46

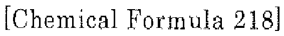

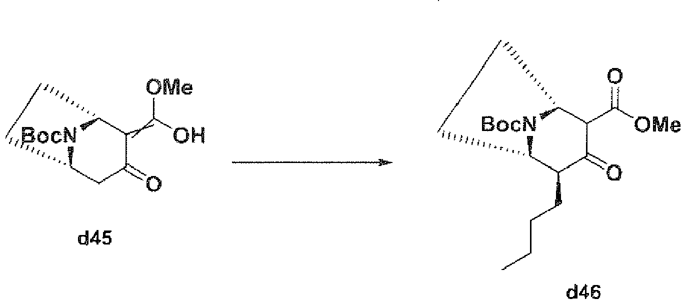

To a solution of diisopropylamine (2.089ml, 14.66mmol) in THF (20mL) was added n-butyl lithium / hexane solution (9.05ml, 14.48mmol) at 0°C, and the mixture was stirred for 1 hour. After the reaction solution was cooled to -45°C, a solution of Compound d45 (1845.5mg, 6.51mmol) in THF (21mL) was dropped into the solution, and the mixture was warmed to -30°C. After the reaction mixture was cooled to 45°C, a solution of HMPA (6.80ml, 39.1mmol) and butyl iodide (1.438g, 7.82mmol) in THF (4mL) was dropped into the solution, and the mixture was stirred for 1 hour 30 minutes. To the reaction solution was added saturated ammonium chloride aqueous solution, and the mixture was extracted with ethyl acetate. The organic layer was washed with water twice, and concentrated under reduced pressure, and the obtained residue was purified by silica gel column chromatography (ethyl acetate / hexane) to yield Compound d46 (1.91g, yield: 86%) as colorless oil.
MS: 340 [M+H]⁺, RT: 2.80, LC/MS method: E

### Step 3: Preparation of Compound d47

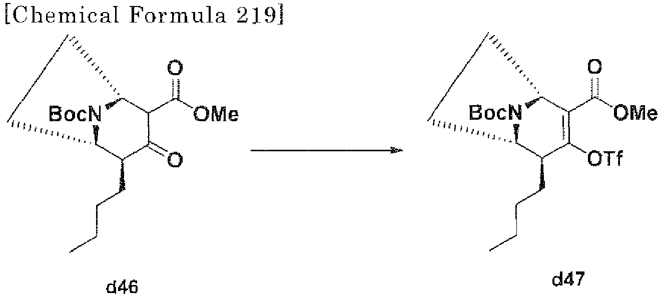

To a solution of Compound d46 (518.0mg, 1.52mmol) in THF (8mL) was added sodium hydride (76mg, 1.89mmol) and N-phenyl-bis(trifluoromethanesulfonimide) (654mg, 1.831mmol) at -30°C, and the mixture was stirred at room temperature overnight. To the reaction mixture was added water, and the solution was extracted with ethyl acetate, the organic layer was washed with water, and concentrated under reduced pressure. The obtained residue was purified by silica gel column chromatography (ethyl acetate / hexane) to yield a crude product of Compound d47 (997mg) as colorless oil.
MS: 472 [M+H]⁺, RT: 2.95, LC/MS method: E

### Step 4: Preparation of Compound d48

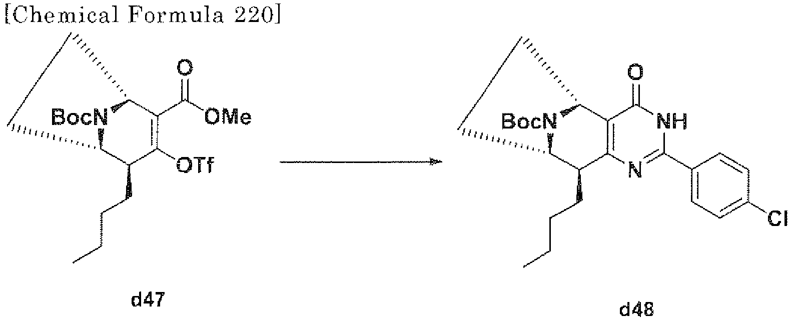

To a solution of Compound d47 (505.2mg, 0.773mmol) in 1,4-dioxane (4mL) was added 4-chlorobenzimidate hydroiodide (262mg, 0.928mmol), Pd₂(dba)₃ (35.4mg, 0.0339mmol), xantphos (44.7mg, 0.077mmol) and cesium carbonate (856mg, 2.63mmol), and the mixture was stirred at 130°C for 15 minutes under microwave irradiation. To the reaction solution was added water, the solution was extracted with ethyl acetate, and the organic layer was concentrated under reduced pressure. The obtained residue was purified by suspending with ethyl acetate to yield Compound d48 (175mg, yield: 51%) as a white solid.
MS: 444, RT: 2.80, LC/MS method.: E
¹H-NMR (CDCl₃) δ: 0.98 (3H, d, J = 6.7 Hz), 1.33-1.73 (2()H, m), 1.84-2.41 (6H, m), 4.59 (1H, s), 5.40 (1H, s), 7.50 (2H, d, J = 8.5 Hz), 8.18 (2H, d, J = 8.4 Hz), 12.81 (1H, s).

### Example 31: Preparation of Compound d56

### Step 1: Preparation of Compound d50

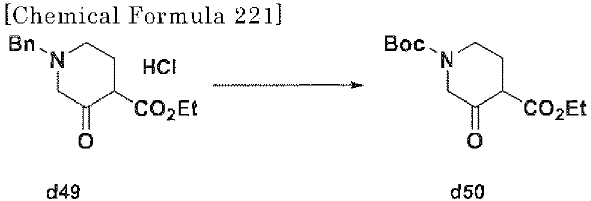

To a solution of Compound d49 (15g, 50.4mmol) in ethanol (400mL) was added dicarbonate-tert-butyl (13.2g, 60.5mmol), trimethylamine (10.2g, 101mmol) and palladium hydroxide-carbon (1.50g, 10%w), and the mixture was stirred at 16 atm for 4 hours under hydrogen atmosphere. The reaction solution was filtered through celite, and the filtrate was concentrated under reduced pressure. The obtained residue was purified by silica gel column chromatography (ethyl acetate / petroleum ether) to yield Compound d50 (24.2g, yield: 88%) as colorless oil.

### Step 2: Preparation of Compound d52

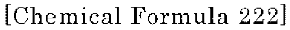

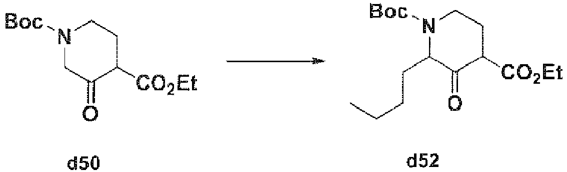

A hexane solution of 2mol/L lithium diisopropylamide (46mL), 92mmol) was dropped into a solution of Compound d50 (10.0g, 36.9mmol) in THF (200mL) at -10°C, and the mixture was stirred for 1 hour under nitrogen atmosphere. To the reaction solution was added 1-ioclebutane (7.56g, 40.5mmol), and the mixture was stirred at 15°C for 18 hours. To the reaction solution was added saturated ammonium chloride aqueous solution, and the solution was extracted with ethyl acetate. The organic layer was washed with brine, dried over anhydrous sodium sulfate, and concentrated under reduced pressure. The obtained residue was purified by silica gel column chromatography (ethyl acetate / petroleum ether) to yield Compound d52 (6.30g, yield: 52%) as pale yellow oil.

### Step 3: Preparation of Compound d54

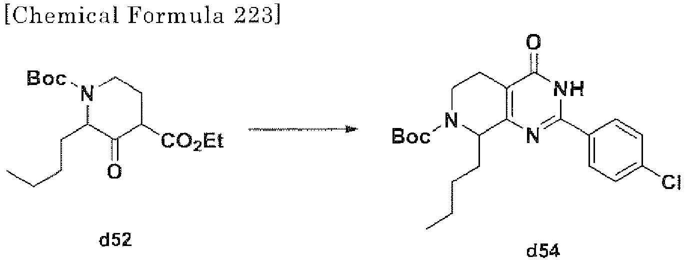

To a solution of Compound d52 (2.50g, 7.64mmol) in methanol (50mL) was added 4-chlorobenzimidamide (1.41g, 9.16mmol) and potassium tert-butoxide (2.14g, 19.1mmol), and the mixture was stirred at 20°C for 18 hours. The reaction solution was filtered through celite, and the filtrate was concentrated under reduced pressure. The obtained residue was purified by silica gel column chromatography (ethyl acetate / petroleum ether) to yield Compound d54 (1.50g, yield: 47%) as a white solid.

### Step 4: Preparation of Compound d55

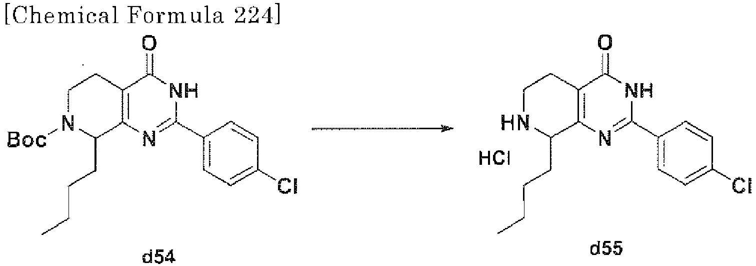

4mol/L Hydrochloric acid / ethyl acetate solution (20mL) was dropped into a solution of Compound d54 (1.5g, 3.60mmol) in dichloromethane (40mL), and the mixture was stirred at 15°C for 3 hours. The precipitated solid was filtered and dried under reduced pressure to yield Compound d55 (1.20g, yield: 96%) as a white solid.

### Step 5: Preparation of Compound d56

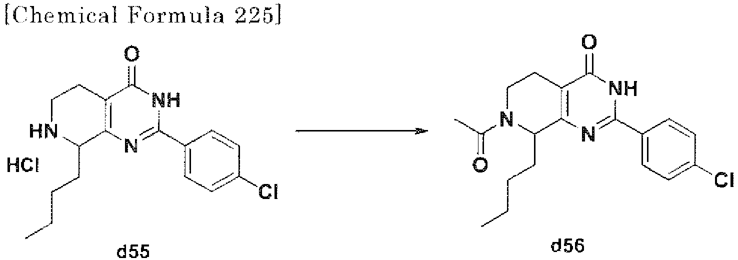

To a solution of Compound d55 (200mg, 0.568mmol) in dichloromethane (5mL) was added triethylamine (171mg, 1.69mmol) and acetic anhydride (160mg, 0.734mmol) under ice-cooling, and the mixture was stirred at 15°C for 18 hours. To the reaction solution was added methanol (10mL) and potassium carbonate (171mg, 1.24mmol), and the mixture was stirred at 13°C for 10 hours. The reaction solution was concentrated under reduced pressure, and the obtained residue was purified by reverse phase silica gel column chromatography and lyophilized to yield Compound d56 (45mg, yield: 22%) as a white solid.
MS: 360.0 [M+H]⁺, RT: 2.01, LC/MS method: A

### Example 32: Preparation of Compound d57

### Step 1: Preparation of Compound d57

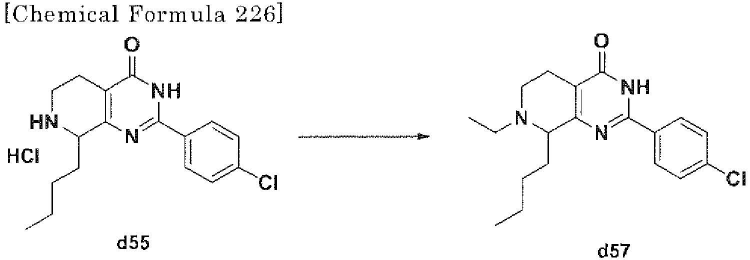

To a solution of Compound d55 (30mg, 0.085mmol) in dichloromethane (1mL) was added triethylamine (0.059ml, 0.423mmol) and ethyl iodide (7.45µl, 0.093mmol), and the mixture was stirred at 50°C for 2 hours 30 minutes. To the reaction solution was added water, and the solution was ethyl acetate. The organic layer was washed with brine and dried over anhydrous sodium sulfate and concentrated under reduced pressure. The obtained residue was purified by silica gel column chromatography (methanol / chloroform) to yield Compound d57 (8.3mg, yield: 28%).
MS: 346 [M+H]⁺, RT: 1.75, LC/MS method: E
¹H-NMR (CDCl₃) δ: 8.15 (2H, d, J = 8.3 Hz), 7.49 (2H, dt, J = 9.1, 2.3 Hz), 3.45 (1H, t, J = 5.8 Hz), 3.17-3.10 (1H, m), 2.88-2.86 (1H, m), 2.72-2.62 (3H, m), 2.51-2.46 (1H, m), 1.88-1.19 (2H, m), 1.53-1.31 (7H, m), 1.14 (3H, t, J = 7.0 Hz), 0.92 (3H, t, J = 7.2 Hz).

### Example 33: Preparation of Compound d63 and d64

### Step 1: Preparation of Compound d59

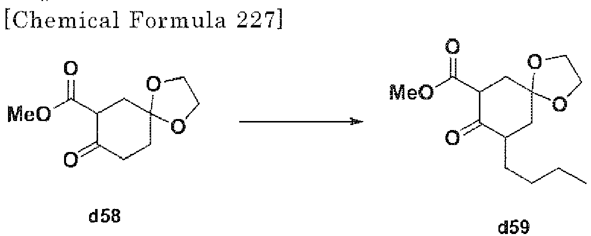

To a solution of diisopropylamine (2.245ml, 15.76mmol) in THF (15mL) was added n-butyl lithium / hexane solution (5.73ml, 15.40mmol) at 0°C, and the mixture was stirred for 1 hour. After the reaction solution was cooled to -78°C, a solution of Compound d58 (1.500g, 7.00mmol) in THF (21mL) was dropped into the reaction solution. After the reaction solution was cooled to -45°C, a solution of HMPA (6.09ml, 35.0mmol) and butyl iodide (1482mg, 8.05mmol) in THF (4 mL) was dropped into the reaction solution, and the mixture was stirred for 2 hours. To the reaction solution was added saturated ammonium chloride aqueous solution, and the mixture was extracted with ethyl acetate. The organic layer was washed with water, concentrated under reduced pressure, the obtained residue was purified by silica gel column chromatography (ethyl acetate / hexane) to yield Compound d59 (825mg, yield: 44%) as yellow oil.
MS: 271 [M+H]⁺, RT: 2.46, LC/MS method: E

### Step 2: Preparation of Compound d60

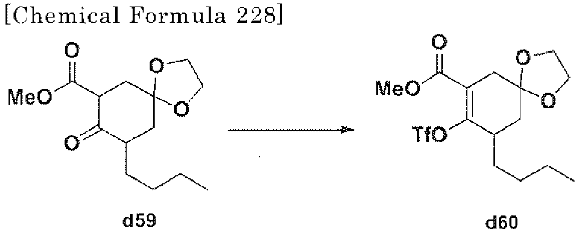

To a solution of Compound d59 (823.0mg, 3.04mmol) in THF (8mL) was added N-phenyl-bis (trifluoromethanesulfonimide) (1.305g, 3.65mmol) and sodium hydride (201.7mg, 5.04mmol), and the mixture was stirred at room temperature overnight. To the reaction solution was added saturated ammonium chloride aqueous solution, and the mixture was extracted with ethyl acetate, the organic layer was washed with water and concentrated under reduced pressure. The obtained residue was purified by silica gel column chromatography (ethyl acetate / hexane) to yield Compound d60 (1.10g, yield: 90%) as colorless oil.
MS: 402 [M+H]⁺, RT: 2.61, LC/MS method: E

### Step 3: Preparation of Compound d61

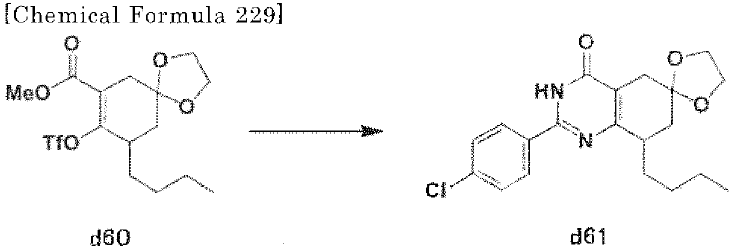

To a solution of Compound d60 (1.100g, 2.73mmol) in 1,4-dioxane (11mL) was added 4-chlorobanzimidamide hydroiodide (927mg, 3.28mmol), Pd₂(dba)₃ (125mg, 0.137mmol), xantphos (158mg, 0.273 mmol) and cesium carbonate (3028mg, 9.29mmol), and the mixture was stirred at 130°C for 15 minutes under microwave irradiation. To the reaction solution was added water, the mixture was extracted with ethyl acetate, and the organic layer was concentrated under reduced pressure. The obtained residue was purified by silica gel column chromatography (methanol / chloroform) and purified by suspending with ethyl acetate to yield Compound d61 (627mg, yield: 61%) as a white solid.
MS: 375 [M+H]⁺, RT: 2.46, LC/MS method: E
¹H-NMR (CDCl₃) δ: 0.94 (3H, t, J = 6.6 Hz), 1.35-1.44 (4H, m), 1.69 (1H, dd, J = 12.5, 6.7 Hz), 1.81 (1H, t, J = 11.8 Hz), 2.08-2.13 (1H, m), 2.19-2.24 (1H, m), 2.73 (1H, d, J = 17.6 Hz), 2.91 (1H, d, J = 17.8 Hz), 3.00 (1H, d, J = 3.8 Hz), 4.00-4.14 (4H, m), 7.48 (2H, d, J = 8.5 Hz), 8.19 (2H, d, J = 8.5 Hz), 12.77 (1H, s).

### Step 4: Preparation of Compound d62

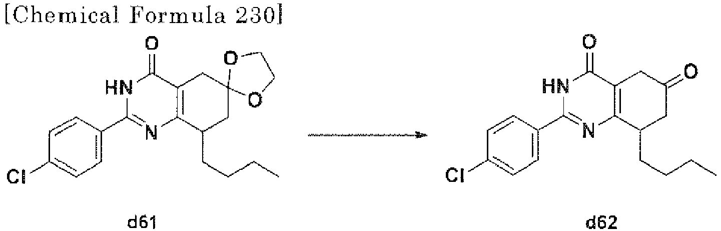

To a suspension of Compound d61 (626.5mg, 1.671mmol) in THF (13mL) was added 2mol/L hydrochloric acid aqueous solution (4.18ml, 8.36mmol) and acetone (20mL), and the mixture was refluxed for 4 hours. The reaction solution was concentrated under reduced pressure, and the residue was purified by suspending with ethyl acetate / hexane to yield Compound d62 (543mg, yield: 98%) as a white solid.
MS: 331 [M+H]⁺, RT: 2.24, LC/MS method: E
¹H-NMR (DMSO-D₆) δ: 0.86 (3H, t, J = 6.7 Hz), 1.29-1.46 (5H, m), 1.71 (1H, d, J = 13.2 Hz), 2.46 (1H, d, J = 3.9 Hz), 2.84 (1H, dd, J = 14.9, 6.1 Hz), 3.07-3.14 (2H, m), 3.31-3.36 (1H, m), 7.62 (2H, d, J = 8.5 Hz), 8.14 (2H, d, J = 8.3 Hz), 12.81 (1H, s).

### Step 5: Preparation of Compound d63 and d64

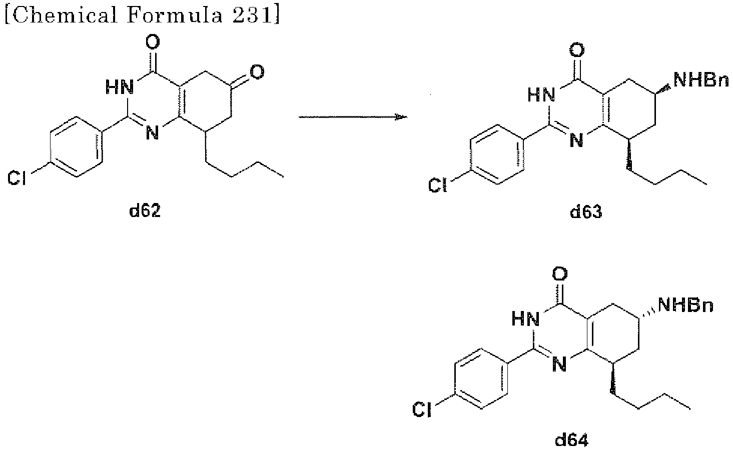

To a solution of Compound d62 (70.0mg, 0.212mmol) in dichloromethane (12mL) was added benzylamine (0.070ml, 0.634mmol) and acetic acid (0.034ml, 0.592mmol). To the reaction solution was added sodium triacetoxyborohydride (134.6mg, 0.634mmol), and the mixture was stirred at room temperature for 7 hours. To the reaction solution was added water, the mixture was extracted with ethyl acetate, and the organic layer was washed with water, and concentrated under reduced pressure. The obtained residue was purified by silica gel column chromatography (methanol / chloroform) to yield Compound d63 (47mg, yield: 53%) and Compound d64 (15mg, yield: 17%) as respectively white solid.
MS: 331 [M+H]⁺, RT: 2.24, LC/MS method: E
¹H-NMR (DMSO-D₆) δ: 0.89 (3H, t, J = 6.3 Hz), 1.21-1.34 (5H, m), 1.53 (1H, s), 1.99 (1H, dd, J = 15.9, 8.0 Hz), 2.10-2.22 (2H, m), 2.62-2.74 (2H, m), 2.91 (1H, d, J = 16.3 Hz), 3.81 (2H, d, J = 3.5 Hz), 7.21 (1H, t, J = 7.5 Hz), 7.31 (2H, t, J = 7.5 Hz), 7.38 (2H, d, J = 7.5 Hz), 7.58 (2H, d, J = 8.0 Hz), 8.10 (2H, d, J = 8.0 Hz).
MS: 331 [M+H]⁺, RT: 2.24, LC/MS method: E
¹H-NMR (DMSO-D₆) δ: 0.89 (3H, t, J = 6.4 Hz), 1.20-1.39 (5H, m), 1.64 (1H, s), 1.86 (2H, d, J = 10.8 Hz), 2.17 (1H, dd, J = 17.3, 7.8 Hz), 2.61-2.75 (2H, m), 2.87 (1H, s), 3.80 (2H, d, J = 14.3 Hz), 7.21 (1H, t, J = 7.0 Hz), 7.30 (2H, t, J = 7.3 Hz), 7.36 (2H, d, J = 7.3 Hz), 7.58 (2H, d, J = 8.0 Hz), 8.10 (2H, d, J = 8.0 Hz).

### Example 34: Preparation of Compound d71

### Step 1: Preparation of Compound d66

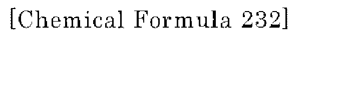

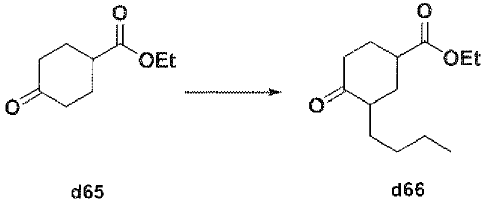

A solution of lithium hexamethyldisilazane (35.3ml, 35.3mmol), HMPA (20.44ml, 118mmol) and 1-iodebutane (6.49g, 35.3mmol) in THF (6mL) was dropped into a solution of Compound d65 (5.000g, 29.4mmol) in THF (50mL) at -78°C, and the solution was warmed at room temperature. To the reaction solution was added saturated ammonium chloride aqueous solution, the mixture was extracted with ethyl acetate, and the organic layer was washed with water and concentrated under reduced pressure. The obtained residue was purified by silica gel column chromatography (ethyl acetate / hexane) to yield Compound d66 (1.03g, yield: 16%) as colorless oil.
¹H-NMR (CDCl₃) δ: 0.86-0.92 (3H, m), 1.17-1.35 (8H, m), 1.74-2.07 (3H, m), 2.22-2.50 (5H, m), 2.74-2.88 (1H, m), 4.12-4.25 (2H, m).

### Step 2: Preparation of Compound d67

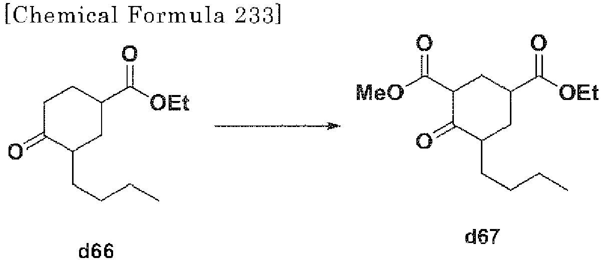

Hexamethyldisilazane lithium (5.22ml, 5.22mmol) and cyanofarmate methyl (0.433ml, 5.45mmol) was dropped into a solution of Compound d66 (1.028g, 4.54mmol) in THF (10mL) at -78°C, and the solution was warmed to room temperature. To the reaction solution was added saturated ammonium chloride aqueous solution, and the solution was extracted with ethyl acetate, and concentrated under reduced pressure. The obtained residue was purified by silica gel column chromatography (ethyl acetate / hexane) to yield Compound d67 (762mg, yield: 59%) as colorless oil.

### Step 3: Preparation of Compound d68

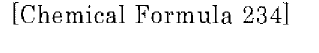

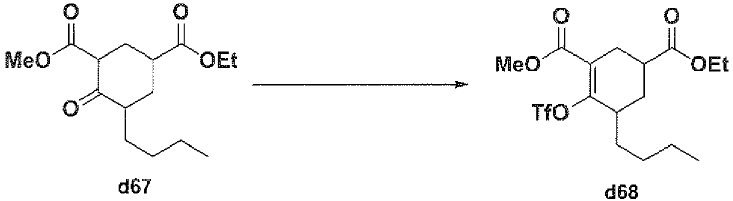

To a solution of Compound d67 (759.8mg, 2.67mmol) in THF (10mL) was added N-phenyl-bis(trifluoromethanesulfonimide) (1145mg, 3.21mmol) and sodium hydride (139mg, 3.47mmol), and the mixture was stirred at room temperature overnight. To the reaction solution was added saturated ammonium chloride aqueous solution, and the solution was extracted with ethyl acetate, the organic layer was washed with water, and concentrated under reduced pressure. The obtained residue was purified by silica gel column chromatography (ethyl acetate / hexane) to yield Compound d68 (570mg, yield: 51%) as colorless oil.
MS: 417 [M+H]⁺, RT: 2.74, LC/MS method: E

### Step 4: Preparation of Compound d69

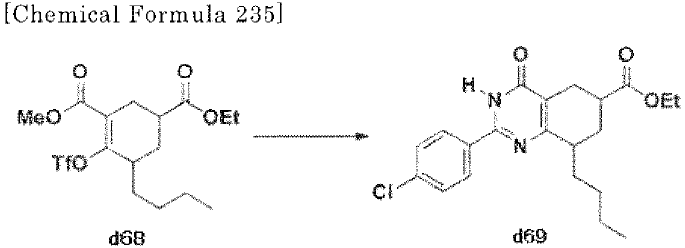

To a solution of Compound d68 (570mg, 1.37mmol) in 1,4-dioxane (12mL) was added 4-chlorobenzimidamide hydroiodide (464mg, 1.64mmol), Pd₂ (dba)₃ (62.7mg, 0.068mmol), xantphos (79mg, 0.14mmol) and cesium carbonate (1517mg, 4.66mmol), and the mixture was stirred at 100°C for 30 minutes under microwave irradiation. To the reaction solution was added water, and the solution was extracted with ethyl acetate, the organic layer was concentrated under reduced pressure. The obtained residue was purified by suspending with ethyl acetate to yield Compound d69 (288mg, yield: 54%) as a pale yellow solid.
MS: 389 [M+H]⁺, RT: 2.65, 2.69, LC/MS method: E
¹H-NMR (DMSO-D₆) δ: 0.91 (3H, t, J = 6.4 Hz), 1.17-1.58 (9H, m), 1.88-2.40 (4H, m), 2.57-2.84 (4H, m), 4.07-4.17 (2H, m), 7.60 (2H, d, J = 8.3 Hz), 8.11 (2H, t, J = 7.3 Hz), 12.65 (1H, s).

### Step 5: Preparation of Compound d70

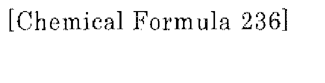

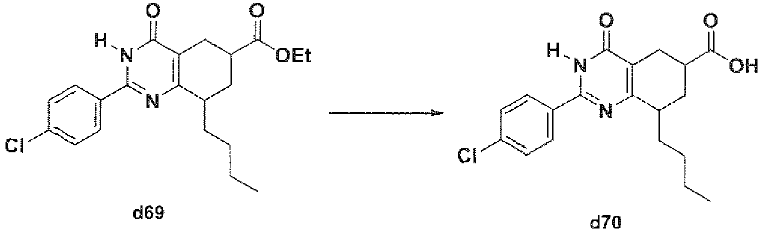

To a mixed solution of Compound d69 (285.7mg, 0.735mmol) in methanol (6mL) / water (3mL) was added potassium hydroxide (194mg, 2.94mmol), and the mixture was refluxed for 1 hour 30 minutes. The reaction solution was concentrated under reduced pressure, and 2mol/L, hydrochloric acid aqueous solution (1.469ml, 2.94mmol) and water was added. The precipitated solid was filtered to yield Compound d70 (229mg, yield: 86%) as yellow solid.
MS: 361 [M+H]⁺, RT: 2.21, 2.25, LC/MS method: E

### Step 6: Preparation of Compound d71

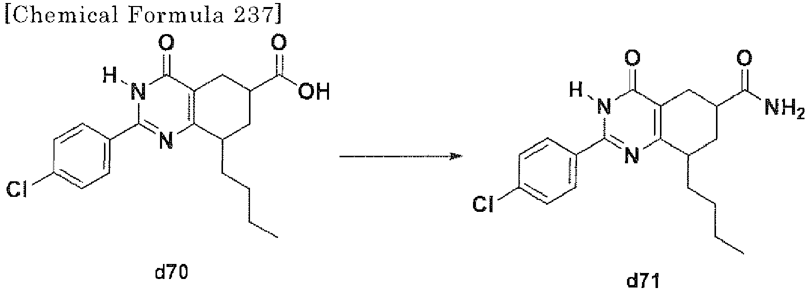

To a solution of Compound d70 (45.8mg, 0.127mmol) in DMF (1mL) was added HATU (50.7mg, 0.133mmol), DIEA (0.067ml, 0.381mmol) and ammonium chloride (13.58mg, 0.254 mmol), and the mixture was stirred at room temperature for 5 minutes. To the reaction solution was added water. The precipitated solid was filtered to yield Compound d71 (41mg, yield: 89%) as a white solid.
MS: 360, RT: 2.01, 2.05, LC/MS method.: E

### Step 7: Preparation of Compound d72

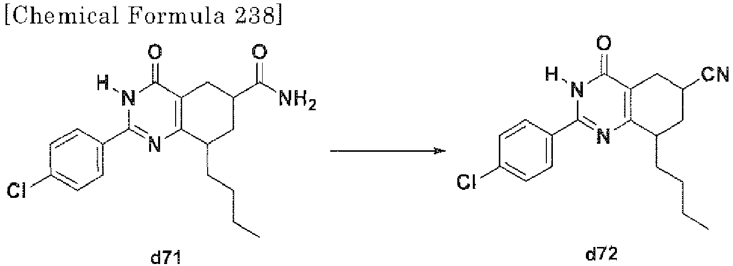

To a solution of Compound d71 (40.5mg, 0.113mmol) in THF (2mL) was added pyridine (0.072ml, 0.900mmol) and trifluoroacetic anhydride (0.064ml, 0.452mmol), and the mixture was stirred at room temperature for 3 hours 30 minutes. To the reaction solution was added water, and the precipitated solid was filtered. The obtained solid was purified by silica gel column chromatography (ethyl acetate / hexane) to yield Compound d72 (13mg, yield: 32%).
¹H-NMR (CDCl₃) δ: 0.95 (3H, t, J = 6.1 Hz), 1.37-1.45 (4H, m), 1.61-1.68 (1H, m), 1.83 (1H, q, J = 11.9 Hz), 2.20-2.30 (1H, m), 2.48 (1H, d, J = 13.1 Hz), 2.70-2.85 (3H, m), 3.19 (1H, t, J = 8.5 Hz), 7.53 (2H, d, J = 7.8 Hz), 8.18 (2H, d, J = 7.8 Hz), 12.77 (1H, s).

### Example 35: Preparation of Compound d79

### Step 1: Preparation of Compound d74

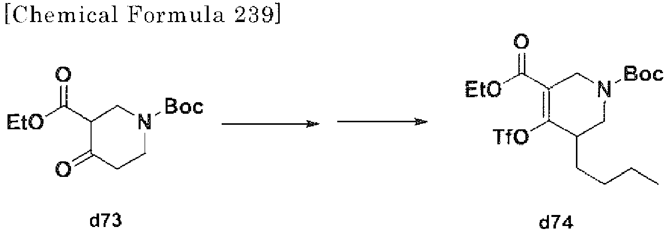

A n-butyl lithium / hexane solution (9.74ml, 15.58mmol) was dropped into a solution of diisopropylamine (2.272ml, 15.94mmol) in THF (20mL) at -30°C, and the mixture was warmed to -20°C. After the reaction solution was cooled to -45°C, a solution of Compound d73 (1.922g, 7.08mmol) in THF (10mL) was dropped into the solution. A solution of HMPA (6.16ml, 35.4mmol) and butyl iodide (1.499g, 8.15mmol) in THF (4mL) was dropped into the reaction solution, and the mixture was stirred for 3 hours 30 minutes. To the reaction solution was added saturated ammonium chloride aqueous solution, and the solution was extracted with ethyl acetate. The organic layer was washed with water twice, concentrated under reduced pressure. A solution of the residue in THF (20mL) was added N-phenyl-bis(trifluoromethanesulfonimide) (3.04g, 8.50mmol) and sodium hydride (0.425g, 10.63mmol), and the mixture was stirred at room temperature for 30 minutes. To the reaction solution was added water, and the solution was extracted with ethyl acetate. The organic layer was washed with water three times, concentrated under reduced pressure, and the obtained residue was purified by silica gel column chromatography (ethyl acetate / hexane) to yield Compound d74 (1.78g, yield: 55%) as a white solid.
MS: 482 [M+Na]⁺, RT: 2.95, LC/MS method: E

### Step 2: Preparation of Compound d75

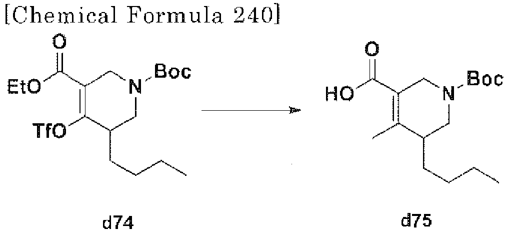

To a solution of Compound d74 (400.0mg, 0.871mmol) in THF (8mL) was added Pd₂(dba)₃ (39.9mg, 0.044mmol), xantphos (50.4mg, 0.087mmol) and dimethyl zinc (2237µl, 2.351mmol), and the mixture was stirred at room temperature overnight. To the reaction mixture was added ice, and the solution was adjusted to pH3 with 2mol/L hydrochloric acid aqueous solution and extracted with ethyl acetate, and the organic layer was washed with water, and concentrated under reduced pressure. To a mixed solution of the obtained residue in methanol (6mL) / water (1mL) was added potassium hydroxide (127.5mg, 1.932mmol), and the solution was refluxed for 2 hours 30 minutes. The reaction solution was concentrated under reduced pressure, water was added to the obtained residue, and the solution was extracted with ether. The aqueous layer was adjusted to pH3 with 2mol/L hydrochloric acid aqueous solution, extracted with ethyl acetate, and the organic layer was washed with water and concentrated under reduced pressure. The obtained residue was purified by silica gel column chromatography (methanol / chloroform) to yield Compound d75 (220mg, yield: 85%) as a white solid.
MS: 298 [M+H]⁺, RT: 2.38, LC/MS method: E

### Step 3: Preparation of Compound d76

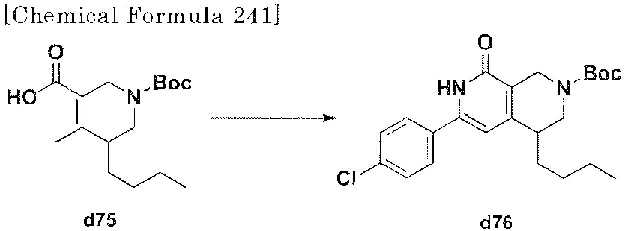

N-butyl lithium / hexane solution (0.70ml, 1.120mmol) was dropped into a solution of diisopropylamine (0.165ml, 1.160mmol) in THF (3mL) at -10°C. After cooled to -45°C, a solution of Compound d75 (150.0mg, 0.504mmol) in THF (4mL) was dropped into the solution, and the mixture was stirred for 1 hour. To the reaction solution was added 4-chlorobanzonitrile (153mg, 1.110mmol), and the solution was stirred at 0°C for 2 hours, and stirred at room temperature overnight. The reaction solution was adjusted to pH4 with 2mol/L hydrochloric acid aqueous solution, and extracted with ethyl acetate. The organic layer was washed with water twice, concentrated under reduced pressure, and the residue was purified by silica gel column chromatography (ethyl acetate / hexane) to yield Compound d76 (65mg, yield: 21%) as a white solid.
MS: 417, RT: 2.64, LC/MS method: E

### Step 4: Preparation of Compound d77

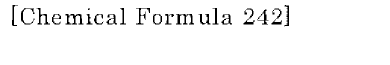

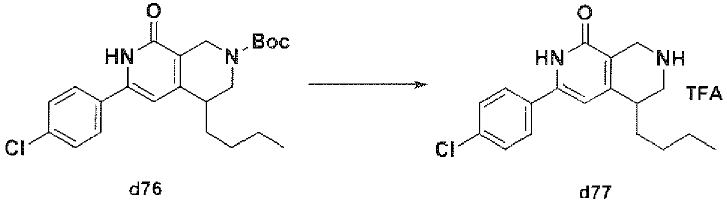

To a solution of Compound d76 (64.6mg, 0.155mmol) in dichloromethane (2mL) was added trifluoroacetic acid (2mL), and the mixture was stirred at room temperature for 15 minutes. The reaction solution was concentrated under reduced pressure, and co-evaporated with methanol three times, and the residue was purified by suspending with ethyl acetate - hexane to yield Compound d77 (49mg, yield: 74%) as a yellow solid.
MS: 317 [M+H]⁺, RT: 1.54, LC/MS method: E

### Step 5: Preparation of Compound d78

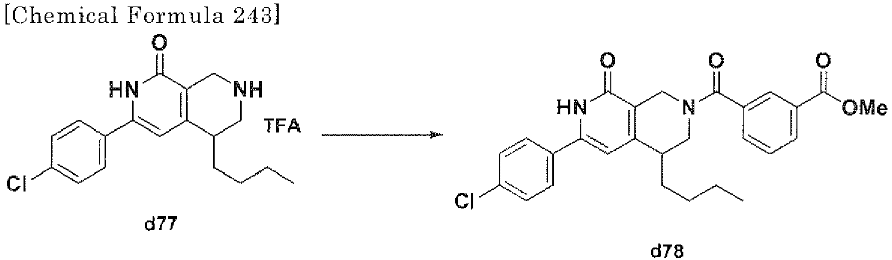

To a solution of Compound d77 (22mg, 0.051mmol) in DMF, (2mL) was added DIVA (18ul, 0.102mol). A solution of 3-(methoxycarbonyl)benzoic acid (12.0mg, 0.066mmol). HATU (25.2mg, 0.066mmol) and DIEA (18ul, 0.102mmol) in DMF (2mL), which is separately prepared, was added to the solution, and the mixture was stirred at room temperature for 15 minutes. To the reaction solution was added water, and the solution was extracted with ethyl acetate. The organic layer was washed with water three times, and concentrated under reduced pressure. The obtained residue was purified by silica gel column chromatography (ethyl acetate / hexane) to yield Compound d78 (35mg, yield: 89%) as colorless oil. MS: 479 [M+H]⁺, RT: 2.38, LC/MS method: E

### Step 6: Preparation of Compound d79

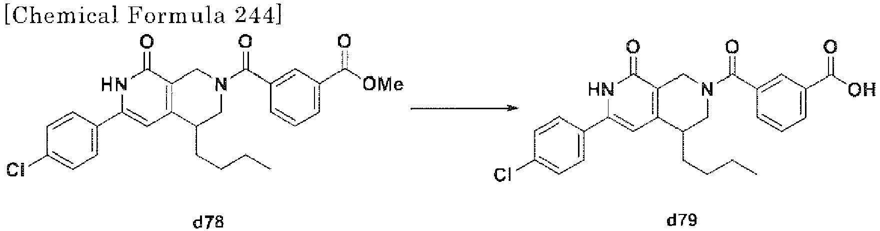

To a mixed solution of Compound d78 (35.4mg, 0.074mmol) in methanol (4mL) / THF (2mL) was added 2mol/L sodium hydroxide aqueous solution (0.148ml, 0.296mmol), and the solution was stirred at 50°C for 4 hours. The reaction solution was concentrated under reduced pressure, and water was added to the residue. The solution was adjusted to pH4 with 2mol/L hydrochloric acid aqueous solution (1.469ml, 2.94mmol), and extracted with ethyl acetate. The organic layer was washed with water, and concentrated under reduced pressure. The residue was purified by suspending with ethyl acetate to yield Compound d79 (32mg, yield: 93%) as a white solid.
MS: 465 [M+H]⁺, RT: 2.13, LC/MS method: E
¹H-NMR (DWSO-D₆) δ: 0.70-1.63 (10H, m), 2.63-2.73 (1H, m), 3.59 (1H, s), 4.14-5.00 (3H, m), 6.54 (1H, s), 7.53 (2H, d, J = 8.4 Hz), 7.62 (1H, t, J = 7.5 Hz), 7.68 (1H, d, J = 7.3 Hz), 7.75 (2H, s), 7.94 (1H, s), 8.05 (1H, d, J = 7.5 Hz), 11.85 (1H, s).

### Example 36: Preparation of Compound d85

### Step 1: Preparation of Compound d81

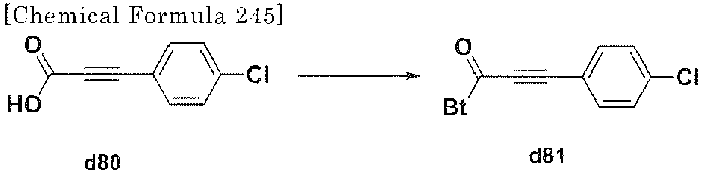

To a suspension of 1H-benzo[d][1,2,3]triazole (6.53g, 54.8mmol) in dichloromethane (17mL) was dropped thionyl chloride (1ml, 13.70mmol), and the mixture was stirred at room temperature for 30 minutes. To the reaction solution was added Compound d80 (2.474g, 13.70mmol), and the solution was stirred at room temperature overnight. After the insoluble materials was removed, the organic layer was washed with 2mol/L sodium hydroxide aqueous solution three times and washed with brine, and concentrated under reduced pressure. The residue was purified by suspending with ethyl acetate to yield Compound d81 (2.32g, yield: 60%) as a white solid.
¹H-NMR (CDCl₃) δ: 7.45 (2H, d, J = 8.4 Hz), 7.56 (1H, t, J = 7.7 Hz), 7.72 (3H, dd, J = 16.0, 8.2 Hz), 8.17 (1H, d, J = 8.2 Hz), 8.31 (1H, d, J = 8.2 Hz).

### Step 2: Preparation of Compound d83

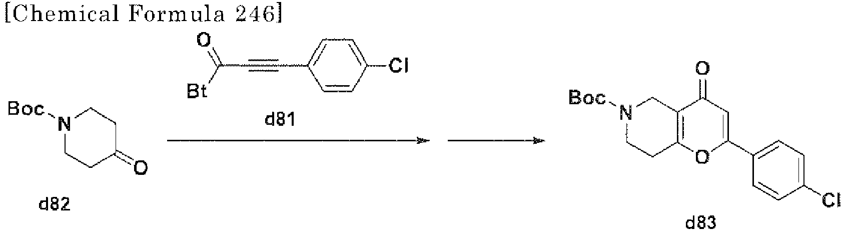

To a solution of diisopropylamine (0.858ml, 6.02mmol) in THF (10mL) was dropped n-butyl lithium / hexane solution (3.61ml, 5.77mmol) at -30°C. After the reaction solution was warmed to 0°C, the solution was cooled to -40°C and a solution of Compound d82 (1.000g, 5.02mmol) in THF (8mL) was dropped into the solution. To the reaction solution was added HMPA (3.49ml, 20.08mmol) and a compound d81 (1.626g, 5.77mmol), and the solution was stirred for 2 hours 30 minutes. To the reaction solution was added saturated ammonium chloride aqueous solution, and the solution was extracted with ethyl acetate. The organic layer was washed with water, concentrated under reduced pressure, and the residue was purified by silica gel column chromatography (ethyl acetate / hexane) to yield an intermediate (1816mg) as brown amorphous. To a solution of this intermediate (1816mg, 5.02mmol) in DMF (10mL) was added N,N-dimethyl amino pyridine (613mg, 5.02mmol), and the mixture was stirred at 80°C for 1 hour 30 minutes. The reaction mixture was concentrated under reduced pressure, and 2mol/L sodium hydroxide aqueous solution was added to the residue. The solution was extracted with dichloromethane. The organic layer was washed with water, and concentrated under reduced pressure, and the obtained residue was purified by silica gel column chromatography (ethyl acetate / hexane) and purified by suspending with ethyl acetate / hexane to yield Compound d83 (505mg, yield: 28%) as a brown solid.
MS: 362 [M+H]⁺, RT: 2.31, LC/MS method: E
¹H-NMR (CDCl₃) δ: 1.49 (9H, s), 2.78 (2H, s), 3.76 (2H, t, J = 5.6 Hz), 4.36 (2H, s), 6.71 (1H, s), 7.47 (2H, d, J = 8.5 Hz), 7.70 (2H, d, J = 8.5 Hz).

### Step 3: Preparation of Compound d84

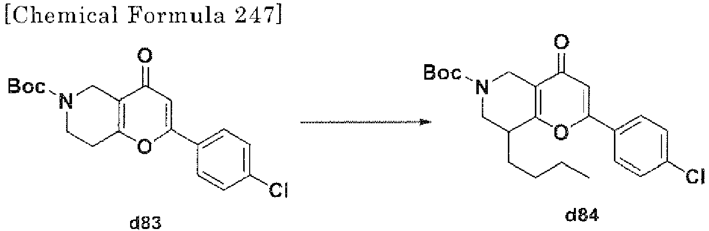

To a solution of Compound d83 (300.0mg, 0.829mmol) in THF (6mL) was dropped sodium bis(trimethylsilyl)amide (0.905ml, 0.995mmol) at -78°C. To the reaction solution was added 1-iodebutane (229mg, 1.244mmol), and the reaction solution was warmed at 0°C and stirred at 3 hours. To the reaction solution was added saturated ammonium chloride aqueous solution and the solution was extracted with ethyl acetate. The organic layer was washed with water, and concentrated under reduced pressure. The obtained residue was purified by silica gel column chromatography (ethyl acetate / hexane) to yield Compound d84 (5.7mg, yield : 2%) as a white solid.
MS: 418 [M+H]⁺, RT: 2.82, LC/MS method: E
¹H-NMR (CDCl₃) δ: 0.92-0.96 (3H, m), 1.32-1.86 (24H, m), 2.68-2.84 (1H, m), 3.33-3.57 (1H, m), 3.80-4.27 (2H, m), 4.40-4.70 (1H, m), 6.69 (1H, s), 7.46-7.48 (2H, m), 7.64-7.69 (2H, m).

### Step 4: Preparation of Compound d85

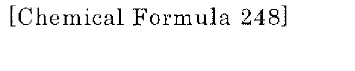

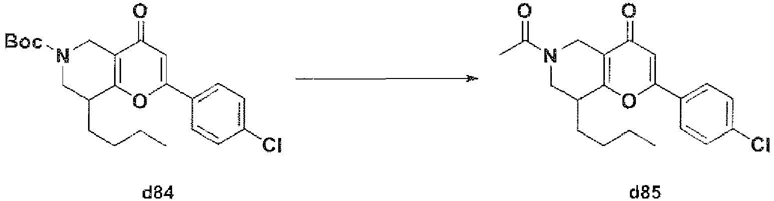

To a solution of Compound d84 (28.2mg. 0.067mmol) in THF (4mL) was added 2mol/L sodium hydroxide aqueous solution (0.101ml, 0.202mmol) and acetic anhydride (0.019ml, 0.202mmol), and the mixture was stirred at room temperature for 20 minutes. The reaction solution was concentrated under reduced pressure, and the obtained residue was purified by silica gel column chromatography (methanol / chloroform) to yield Compound d85 (15mg, yield: 63%) as a brown solid.
MS: 360 [M+H]⁺, RT: 2.22, LC/MS method: E
¹H-NMR (CDCl₃) δ: 0.95 (3H, t, J = 15.4 Hz), 1.36-1.83 (6H, m), 2.21 (3H, s), 2.78-2.88 (1H, m), 3.60 (1H, dd, J = 13.2, 3.6 Hz), 4.09-4.14 (1H, m), 4.39 (2H, dd, J = 44.3, 17.2 Hz), 6.71 (1H, s), 7.49 (2H, d, J = 7.8 Hz), 7.69 (2H, d, J = 7.8 Hz).

### Example 37: Preparation of Compound d89

### Step 1: Preparation of Compound d87

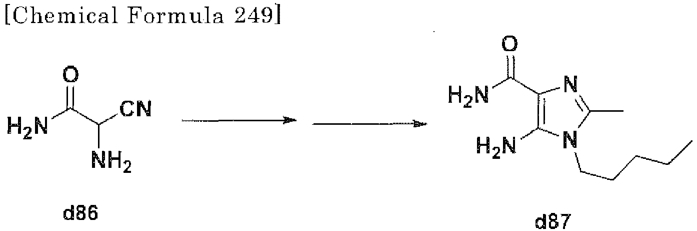

To a solution of Compound d87 (18.5g, 187mmol) in acetonitrile (200mL) was added 1,1,1-triethoxyethane (30.3g, 187mmol), and the mixture was stirred at 80°C for 4 hours. To the reaction solution was added 1-aminopentane (16.3g, 187mmol), and the solution was stirred at 80°C for 15 minutes. The reaction solution was cooled with ice-bath, and the precipitated solid was filtered to yield Compound d87 (20.4g, yield: 52%) as a brown solid.

### Step 2: Preparation of Compound d89

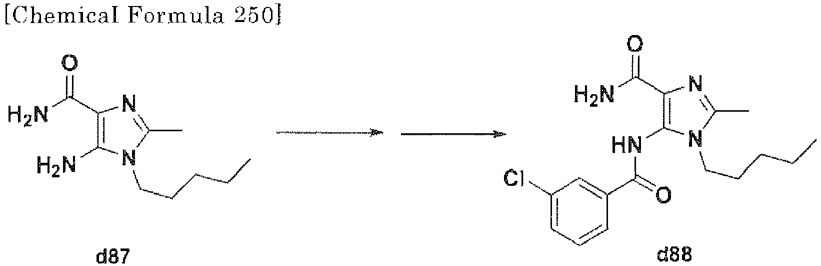

To a solution of Compound d87 (1.00g, 4.76mmol) in pyridine (5mL) was added 3-chlorobenzoyl chloride (1.25g, 7.14mmol), and the solution was stirred at 11°C for 16 hours, and concentrated under reduced pressure. To the residue was added methanol (15mL) and potassium tert-butoxide (1.60g, 14.3mmol), and the solution was stirred at 40°C for 16 hours. The reaction solution was concentrated under reduced pressure to yield a crude product of Compound d88 (1.66g).

### Step 3: Preparation of Compound d89

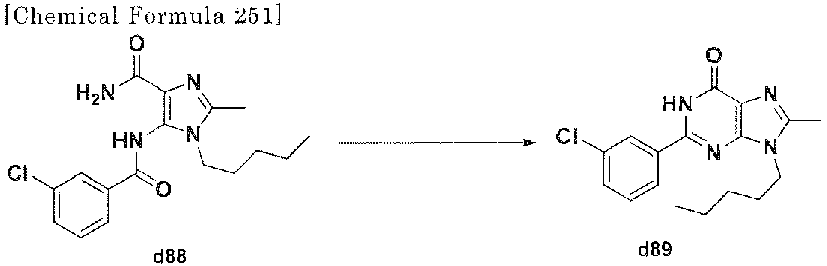

To a solution of Compound d88 (1.66g, 4.76mmol) in tert-butanol (15mL) was added potassium tert-butoxide (2.13g, 19.0mmol), and the solution was refluxed for 48 hours. To the reaction solution was added water, and the solution was extracted with ethyl acetate. The organic layer was washed with brine, dried over anhydrous sodium sulfate, and concentrated under reduced pressure. The obtained residue was purified by silica gel column chromatography (ethyl acetate / methanol) to yield Compound d89 (460mg, yield: 29%) as a white solid.
MS: 330.9 [M+H]⁺, RT: 2.13, LC/MS method: A

Similarly, the following compounds were synthesized.

**[Table 1]**

| Example No | Structure |
|---|---|
| I-0001 | 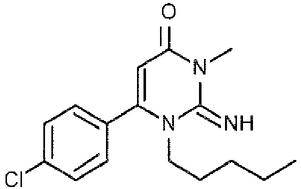 |
| I-0002 | 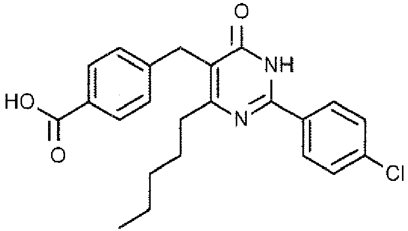 |
| I-0003 | 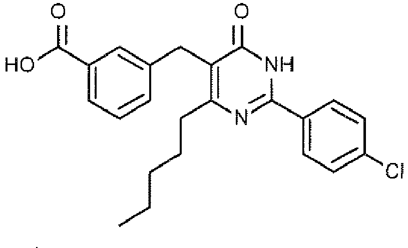 |
| I-0004 | 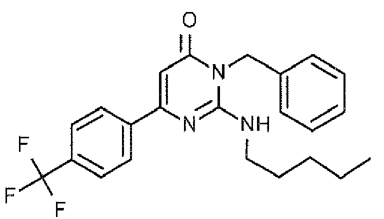 |
| I-0005 | 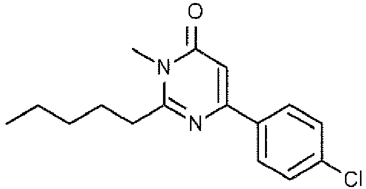 |
| I-0006 | 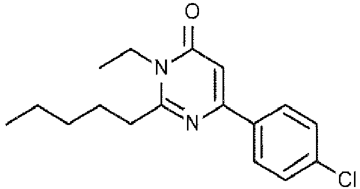 |

**[Table 2]**

| | |
|---|---|
| I-0007 | 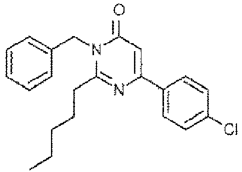 |
| I-0008 | 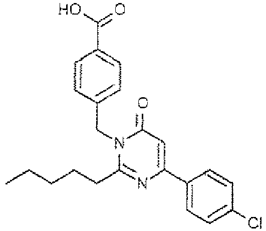 |
| I-0009 | 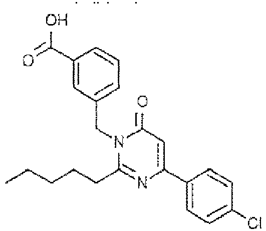 |
| I-0010 | 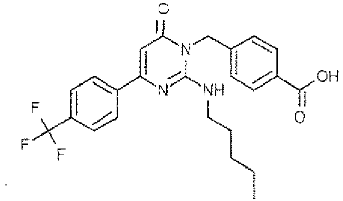 |
| I-0011 | 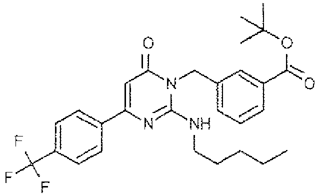 |
| I-0012 | 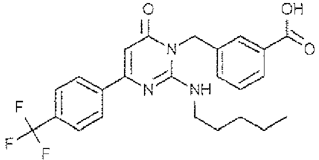 |

**[Table 3]**

| | |
|---|---|
| I-0013 | 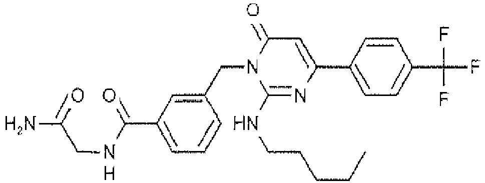 |
| I-0014 | 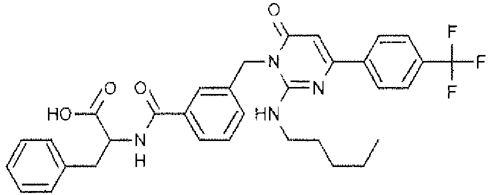 |
| I-0015 | 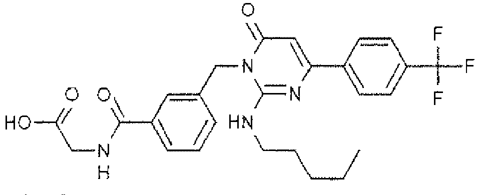 |
| I-0016 | 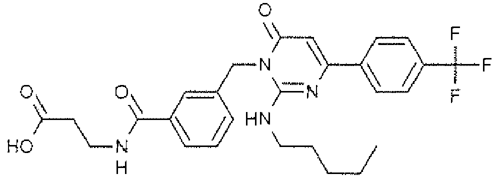 |
| I-0017 | 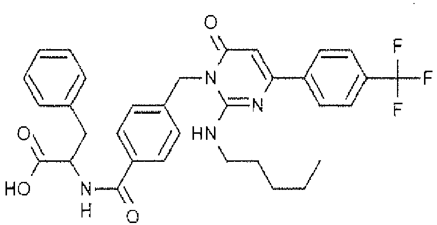 |
| I-0018 | 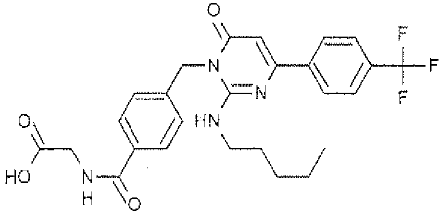 |

**[Table 4]**

| | |
|---|---|
| I-0019 | 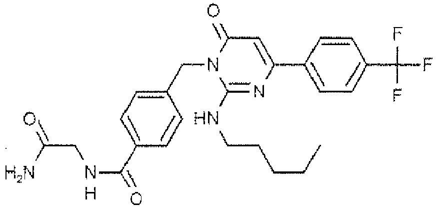 |
| I-0020 | 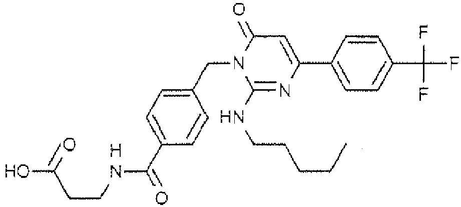 |
| I-0021 | 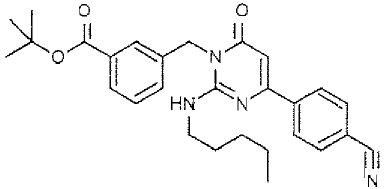 |
| I-0022 | 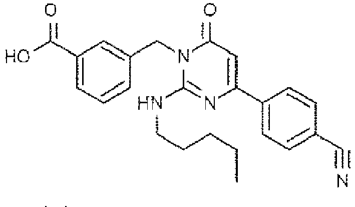 |
| I-0023 | 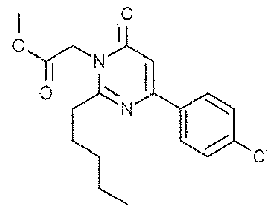 |
| I-0024 | 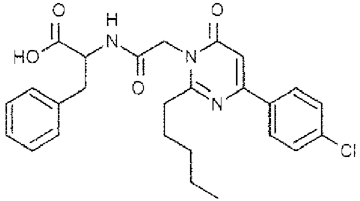 |

**[Table 5]**

| | |
|---|---|
| I-0025 | 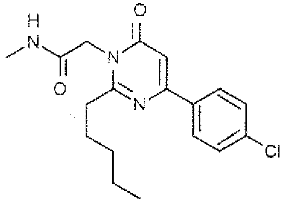 |
| I-0026 | 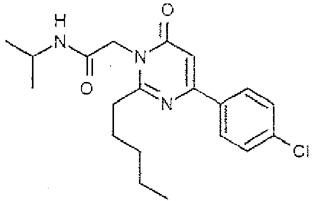 |
| I-0027 | 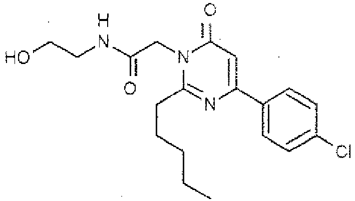 |
| I-0028 | 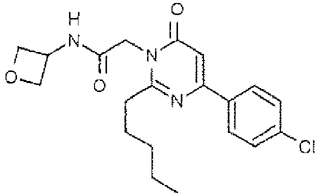 |
| I-0029 | 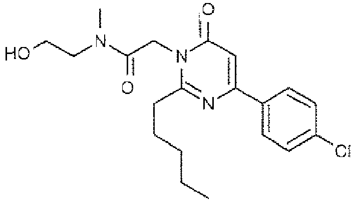 |
| I-0030 | 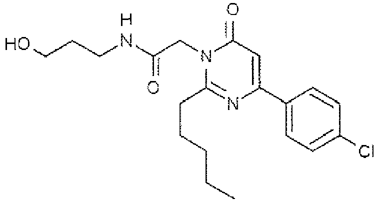 |

**[Table 6]**

| | |
|---|---|
| I-0031 | 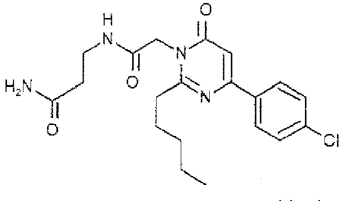 |
| I-0032 | 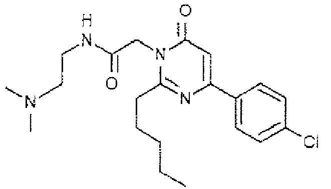 |
| I-0033 | 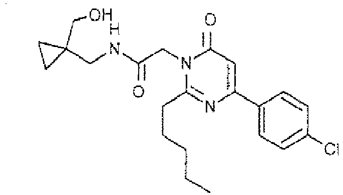 |
| I-0034 | 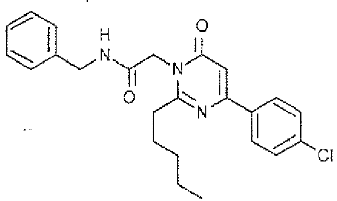 |
| I-0035 | 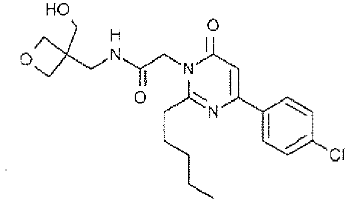 |
| I-0036 | 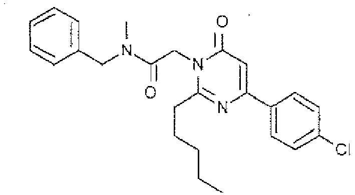 |

**[Table 7]**

| | |
|---|---|
| I-0037 | 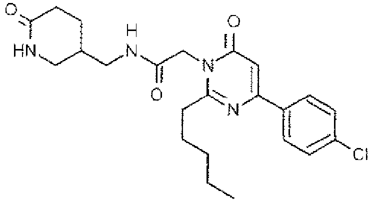 |
| I-0038 | 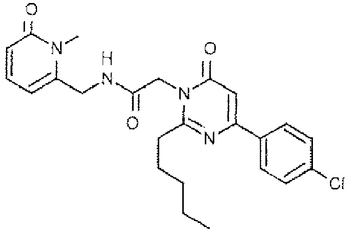 |
| I-0039 | 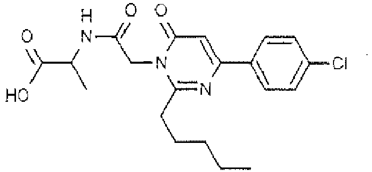 |
| I-0040 | 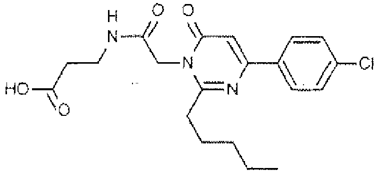 |
| I-0041 | 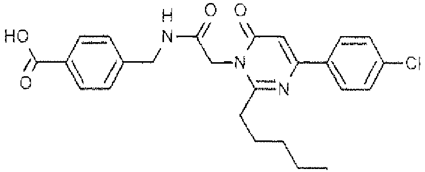 |
| I-0042 | 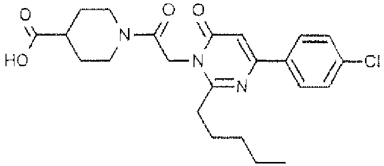 |

**[Table 8]**

| | |
|---|---|
| I-0043 | 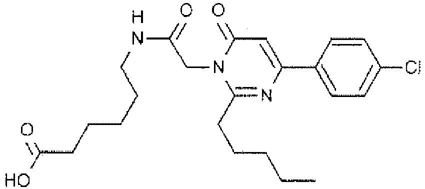 |
| I-0044 | 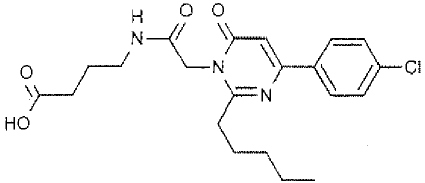 |
| I-0045 | 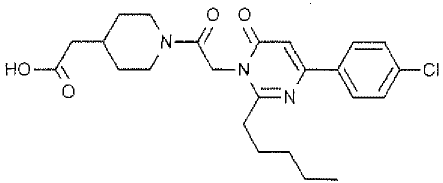 |
| I-0046 | 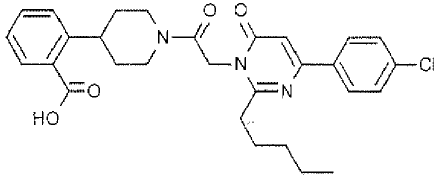 |
| I-0047 | 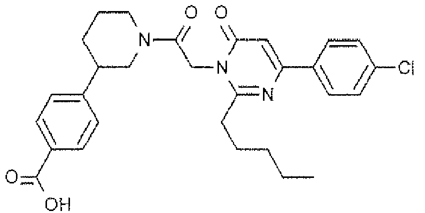 |
| I-0048 | 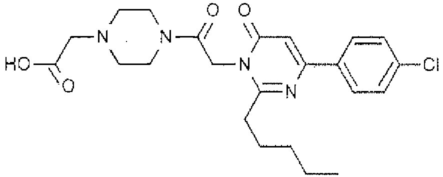 |

**[Table 9]**

| | |
|---|---|
| I-0049 | 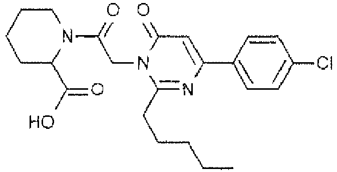 |
| I-0050 | 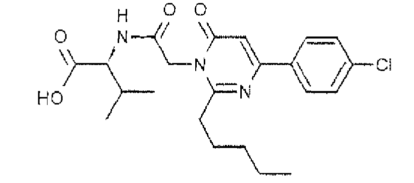 |
| I-0051 | 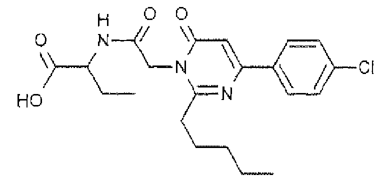 |
| I-0052 | 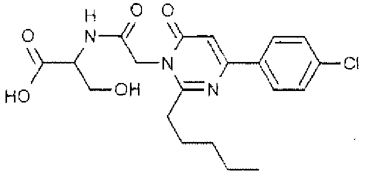 |
| I-0053 | 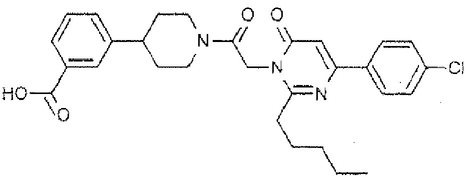 |
| I-0054 | 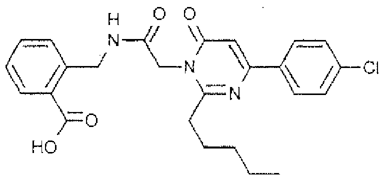 |

**[Table 10]**

| | |
|---|---|
| I-0055 | 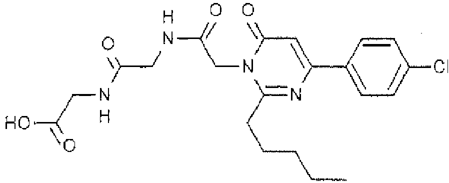 |
| I-0056 | 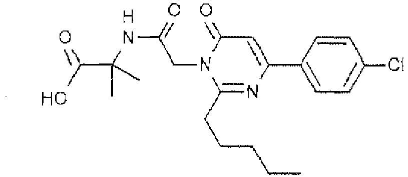 |
| I-0057 | 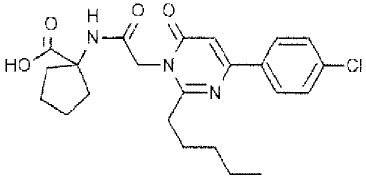 |
| I-0058 | 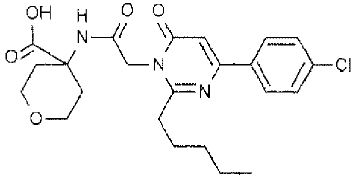 |
| I-0059 | 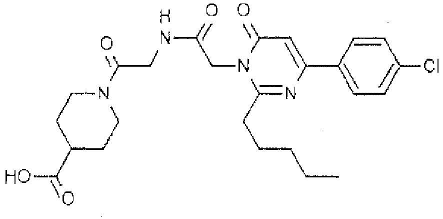 |
| I-0060 | 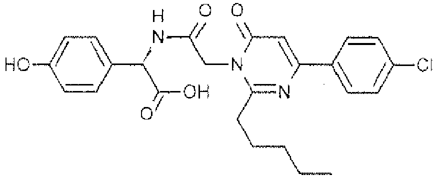 |

**[Table 11]**

| | |
|---|---|
| I-0061 | 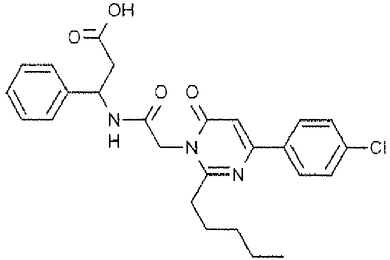 |
| I-0062 | 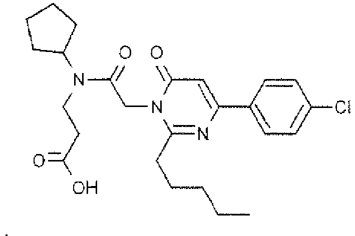 |
| I-0063 | 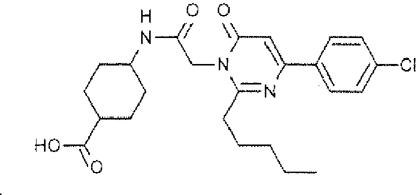 |
| I-0064 | 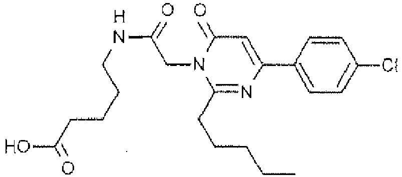 |
| I-0065 | 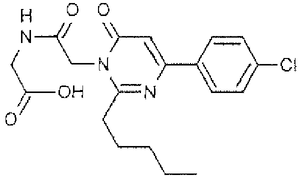 |
| 1-0066 | 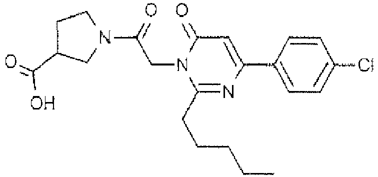 |

**[Table 12]**

| | |
|---|---|
| I-0067 | 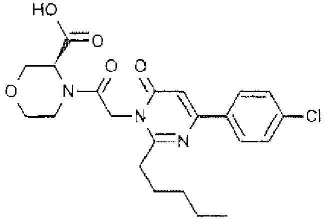 |
| I-0068 | 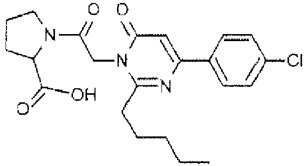 |
| I-0069 | 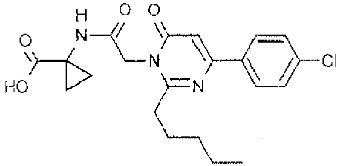 |
| I-0070 | 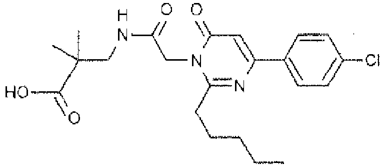 |
| I-0071 | 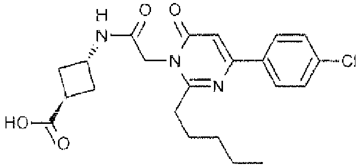 |
| I-0072 | 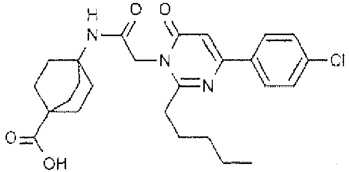 |

**[Table 13]**

| | |
|---|---|
| I-0073 | 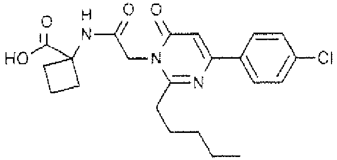 |
| I-0074 | 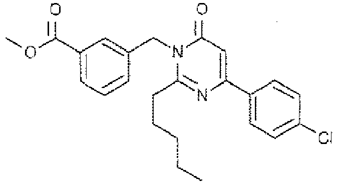 |
| I-0075 | 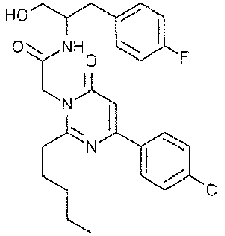 |
| I-0076 | 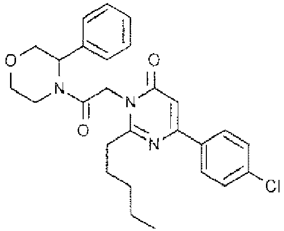 |
| I-0077 | 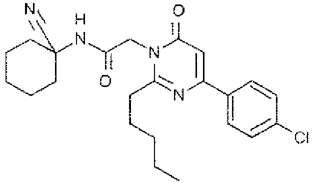 |
| I-0078 | 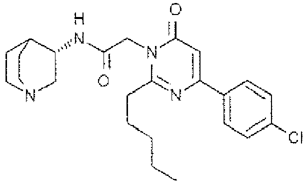 |

**[Table 14]**

| | |
|---|---|
| I-0079 | 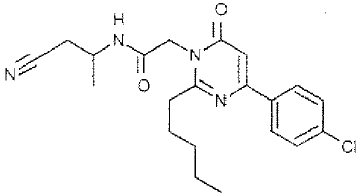 |
| I-0080 | 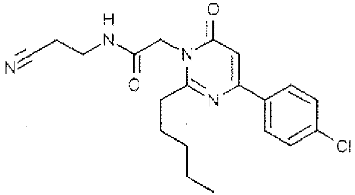 |
| I-0081 | 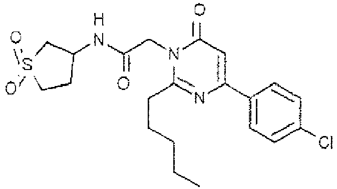 |
| I-0082 | 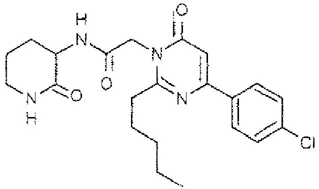 |
| I-0083 | 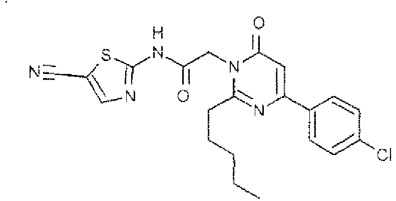 |
| I-0084 | 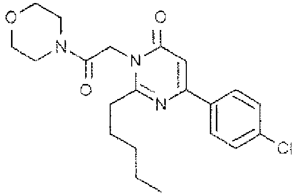 |

**[Table 15]**

| | |
|---|---|
| I-0085 | 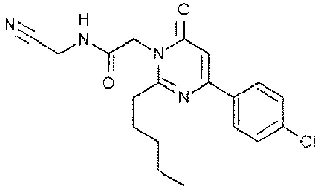 |
| I-0086 | 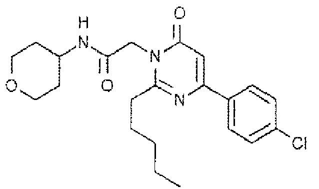 |
| I-0087 | 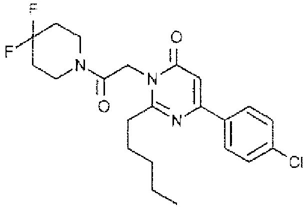 |
| I-0088 | 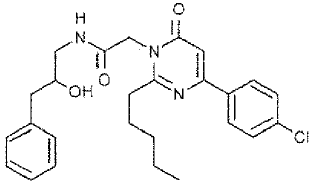 |
| I-0089 | 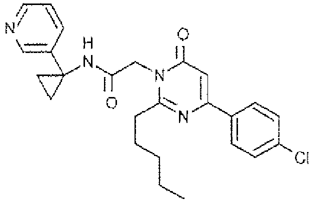 |
| I-0090 | 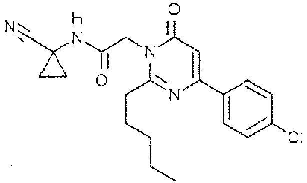 |

**[Table 16]**

| | |
|---|---|
| I-0091 | 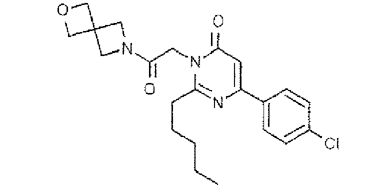 |
| I-0092 | 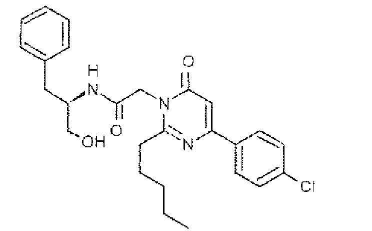 |
| I-0093 | 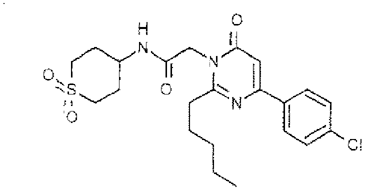 |
| I-0094 | 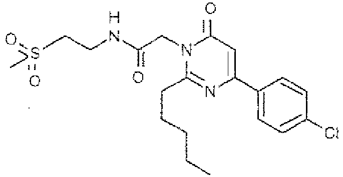 |
| I-0095 | 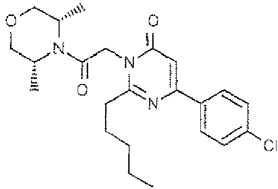 |
| I-0096 | 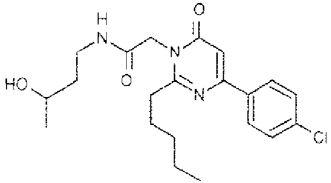 |

**[Table 17]**

| | |
|---|---|
| I-0097 | 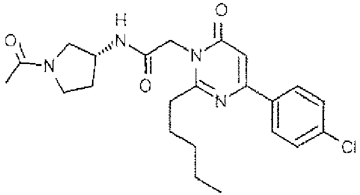 |
| I-0098 | 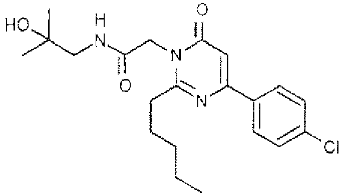 |
| I-0099 | 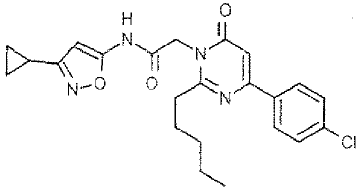 |
| I-0100 | 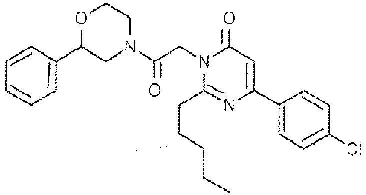 |
| I-0101 | 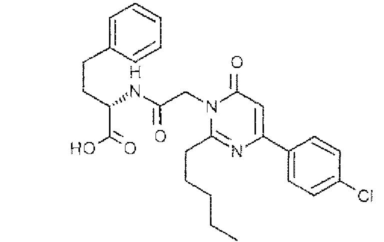 |
| I-0102 | 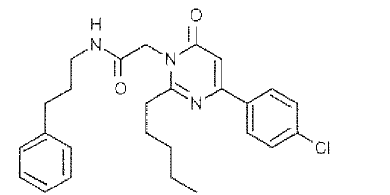 |

**[Table 18]**

| | |
|---|---|
| I-0103 | 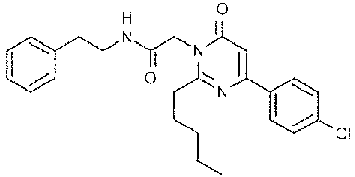 |
| I-0104 | 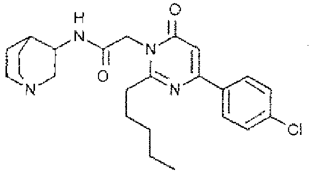 |
| I-0105 | 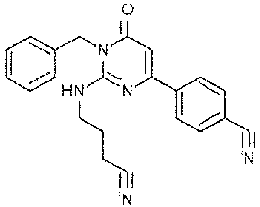 |
| I-0106 | 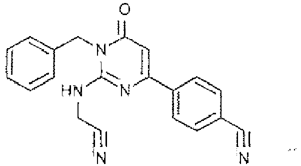 |
| I-0107 | 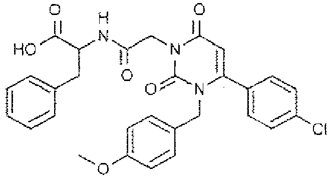 |
| I-0108 | 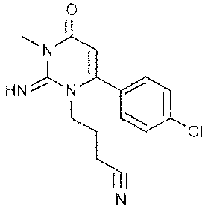 |

**[Table 19]**

| | |
|---|---|
| I-0109 | 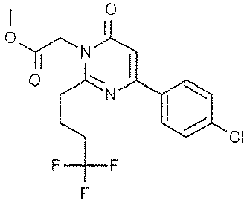 |
| I-0110 | 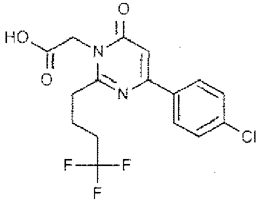 |
| I-0111 | 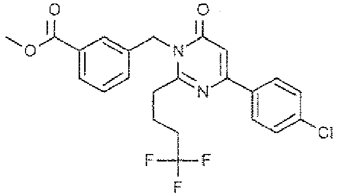 |
| I**-**0112 | 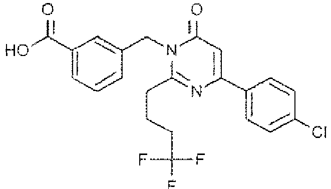 |
| I-0113 | 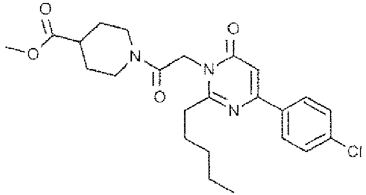 |
| I-0114 | 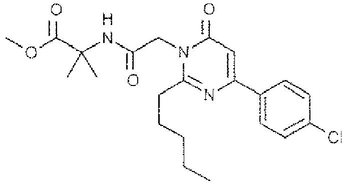 |

**[Table 20]**

| | |
|---|---|
| I-0115 | 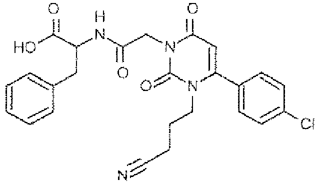 |
| I-0116 | 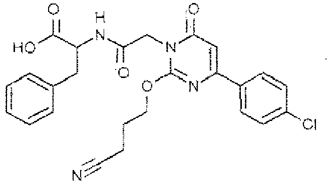 |
| I-0117 | 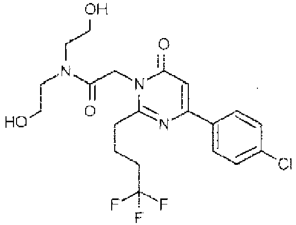 |
| I-0118 | 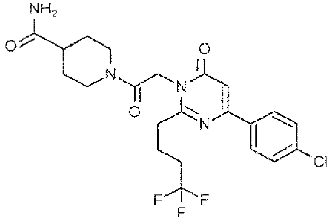 |
| I-0119 | 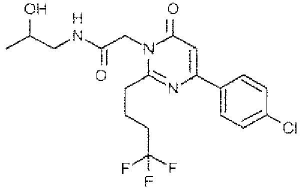 |
| I-0120 | 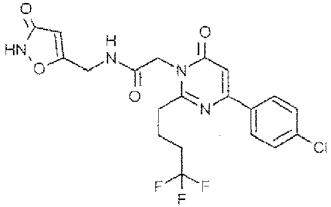 |

**[Table 21]**

| | |
|---|---|
| I-0121 | 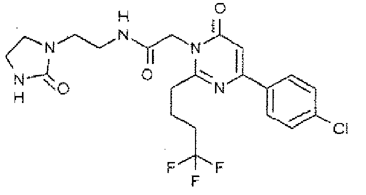 |
| I-0122 | 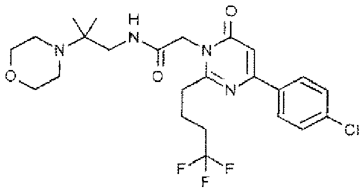 |
| I-0123 | 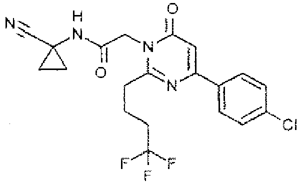 |
| I-0124 | 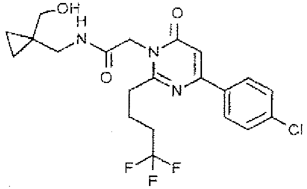 |
| I-0125 | 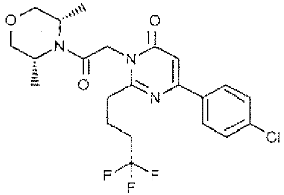 |
| I-0126 | 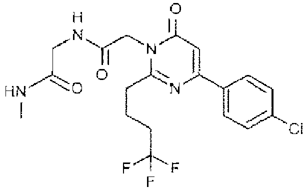 |

**[Table 22]**

| | |
|---|---|
| I-0127 | 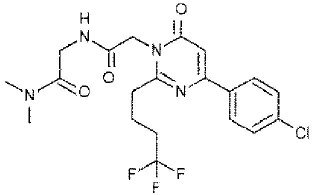 |
| I-0128 | 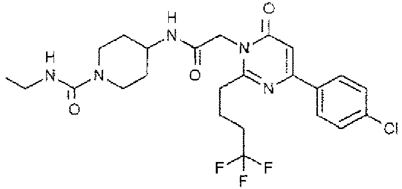 |
| I-0129 | 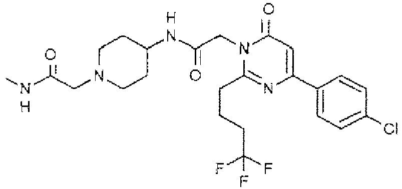 |
| I-0130 | 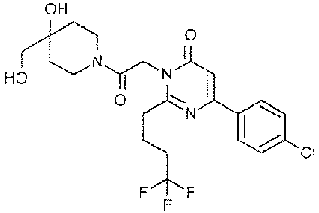 |
| I-0131 | 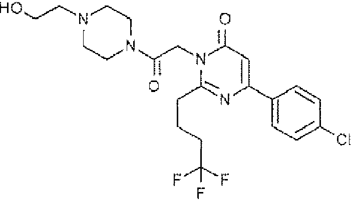 |
| I-0132 | 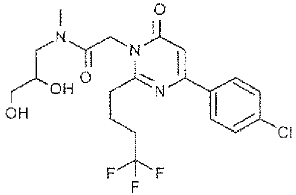 |

**[Table 23]**

| | |
|---|---|
| I-0133 | 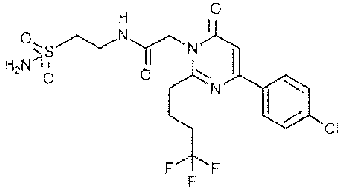 |
| I-0134 | 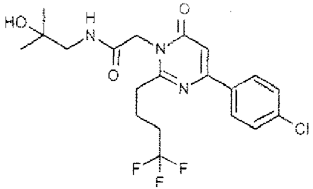 |
| I-0135 | 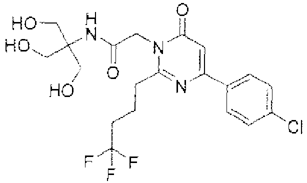 |
| I-0136 | 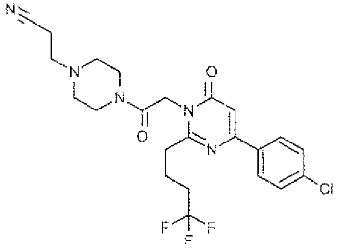 |
| I-0137 | 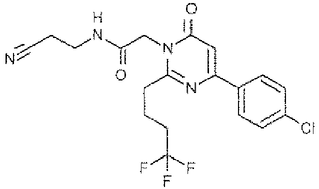 |
| I-0138 | 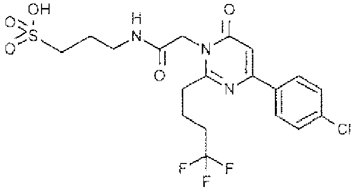 |

**[Table 24]**

| | |
|---|---|
| I-0139 | 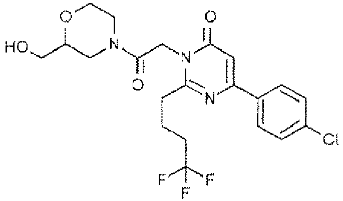 |
| I-0140 | 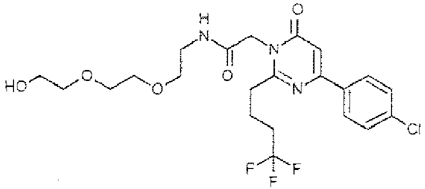 |
| I-0141 | 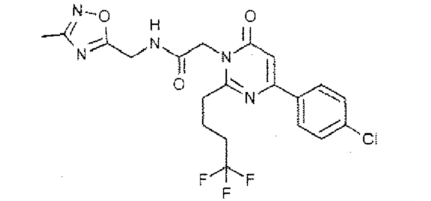 |
| I**-**0142 | 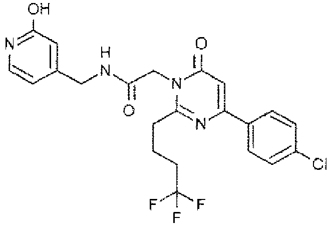 |
| I-0143 | 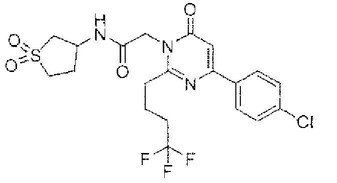 |
| I-0144 | 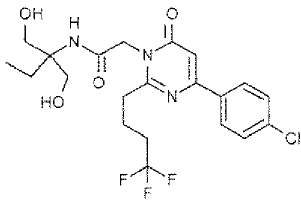 |

**[Table 25]**

| | |
|---|---|
| I-0145 | 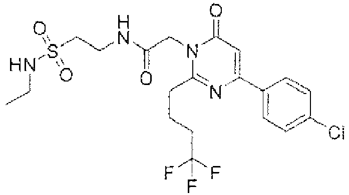 |
| I-0146 | 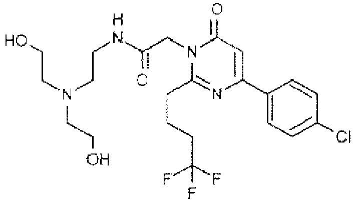 |
| I-0147 | 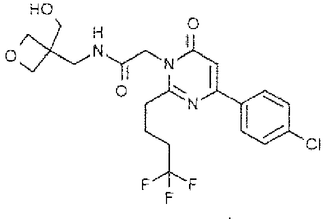 |
| I-0148 | 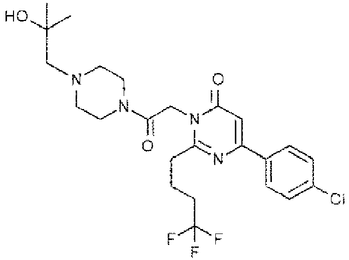 |
| I-0149 | 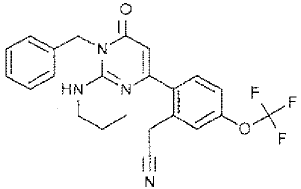 |
| I-0150 | 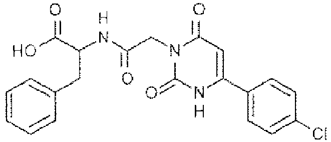 |

**[Table 26]**

| | |
|---|---|
| I-0151 | 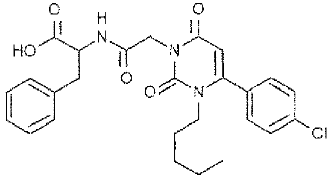 |
| I-0152 | 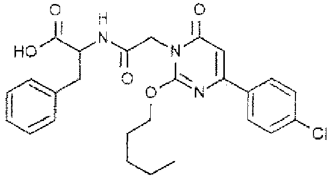 |
| I-0153 | 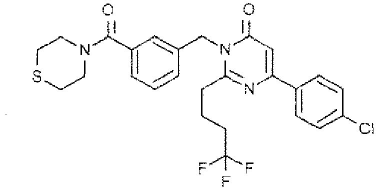 |
| I-0154 | 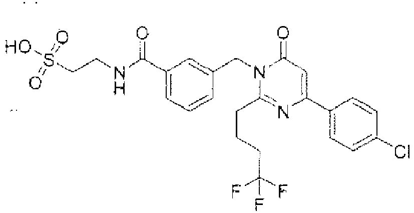 |
| I-0155 | 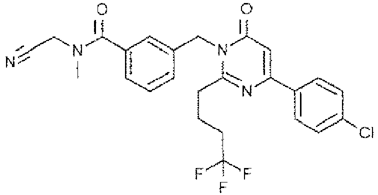 |
| I-0156 | 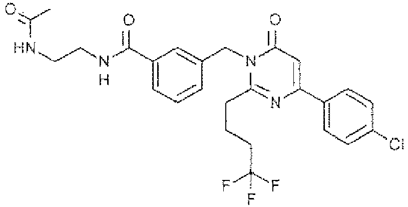 |

**[Table 27]**

| | |
|---|---|
| I-0157 | 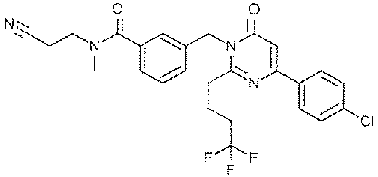 |
| I-0158 | 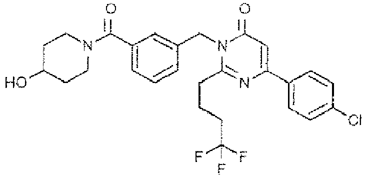 |
| I-0159 | 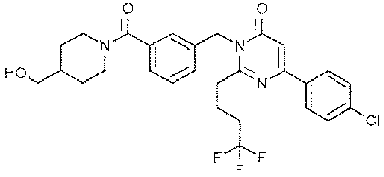 |
| I-0160 | 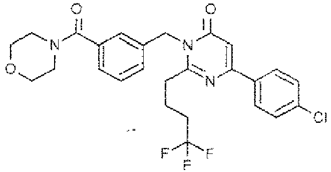 |
| I-0161 | 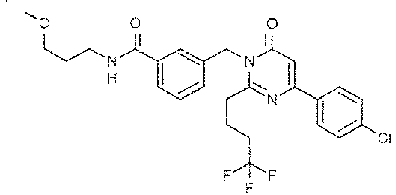 |
| I-0162 | 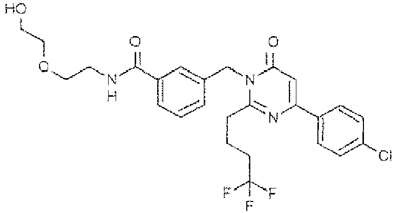 |

**[Table 28]**

| | |
|---|---|
| I-0163 | 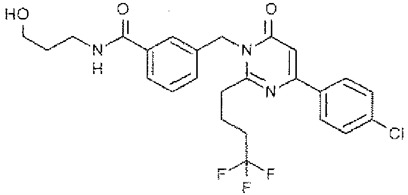 |
| I-0164 | 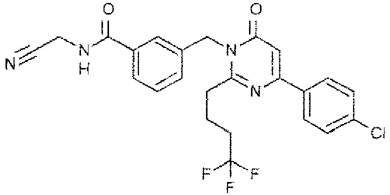 |
| I-0165 | 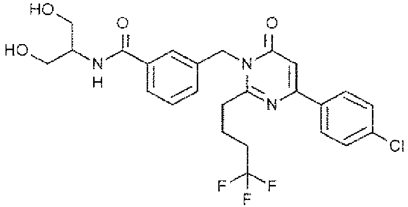 |
| I-0166 | 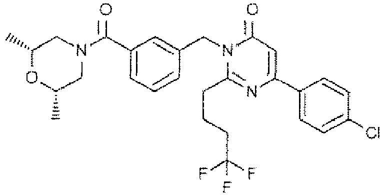 |
| I-0167 | 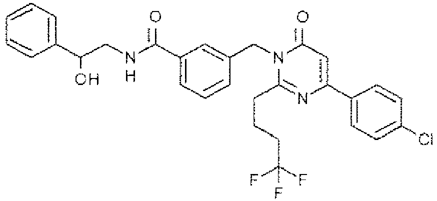 |
| I-0168 | 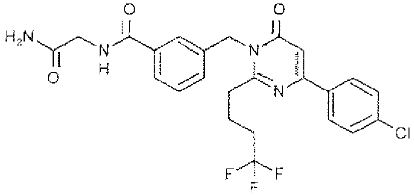 |

**[Table 29]**

| | |
|---|---|
| I-0169 | 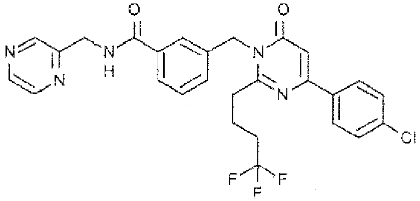 |
| I-0170 | 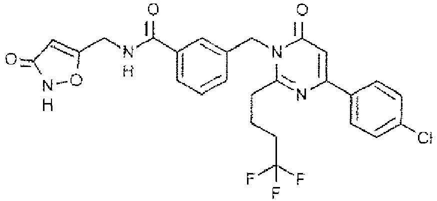 |
| I-0171 | 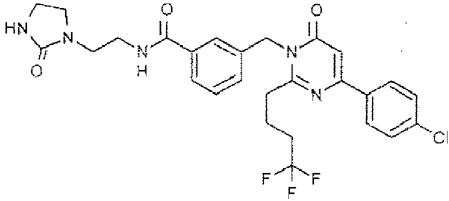 |
| I-0172 | 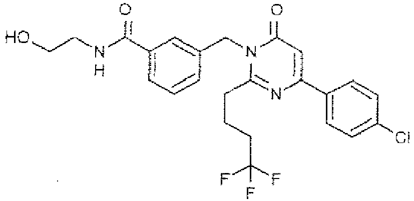 |
| I-0173 | 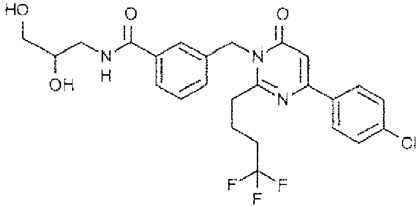 |
| I-0174 | 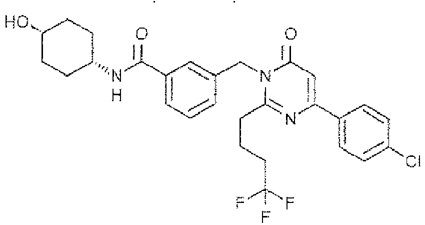 |

**[Table 30]**

| | |
|---|---|
| I-0175 | 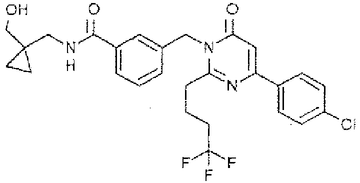 |
| I-0176 | 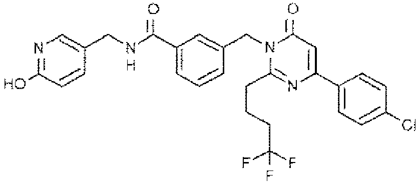 |
| I-0177 | 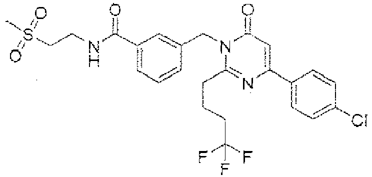 |
| I-0178 | 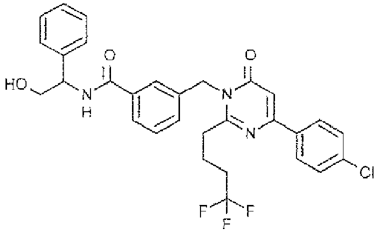 |
| I-0179 | 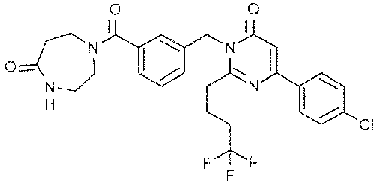 |
| I-0180 | 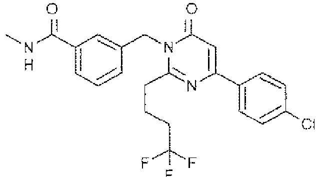 |

**[Table 31]**

| | |
|---|---|
| I-0181 | 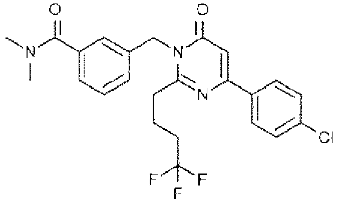 |
| I-0182 | 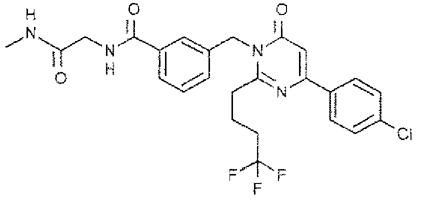 |
| I-0183 | 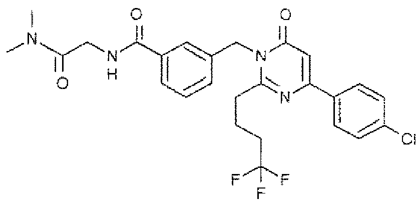 |
| I-0184 | 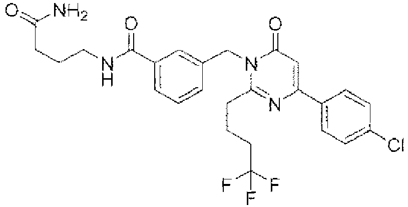 |
| I-0185 | 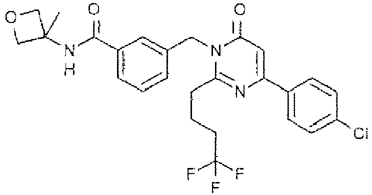 |
| I-0186 | 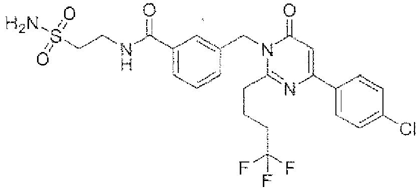 |

**[Table 32]**

| | |
|---|---|
| I-0187 | 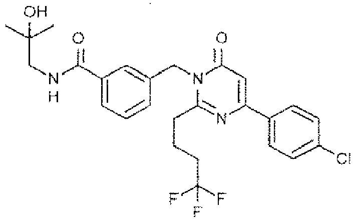 |
| I-0188 | 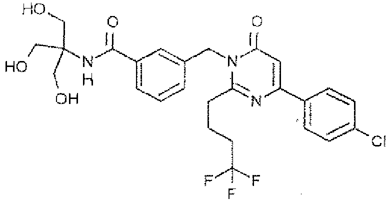 |
| I-0189 | 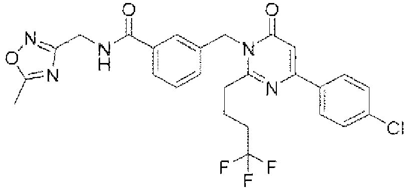 |
| I-0190 | 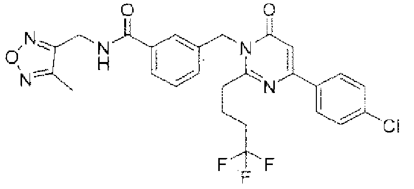 |
| I-0191 | 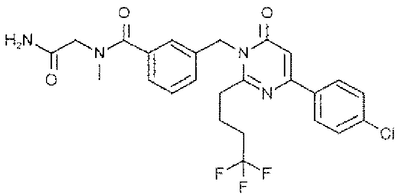 |
| I-0192 | 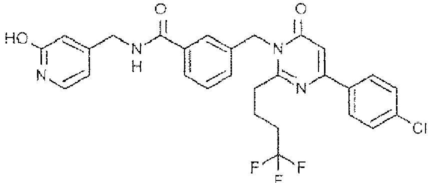 |

**[Table 33]**

| | |
|---|---|
| I-0193 | 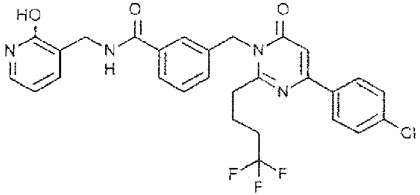 |
| I-0194 | 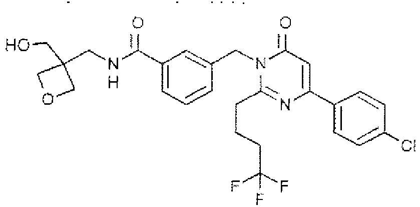 |
| I-0195 | 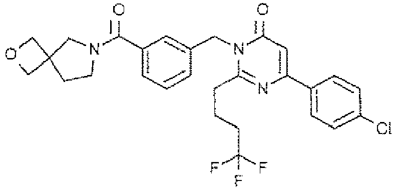 |
| I-0196 | 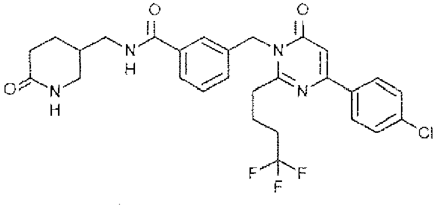 |
| I-0197 | 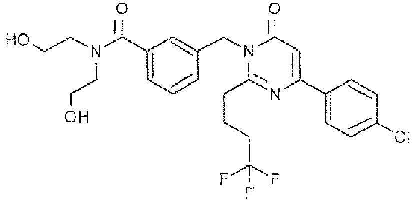 |
| I-0198 | 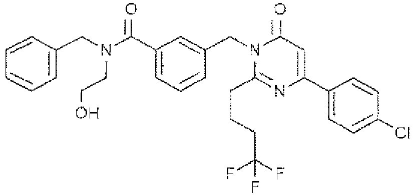 |

**[Table 34]**

| | |
|---|---|
| I-0199 | 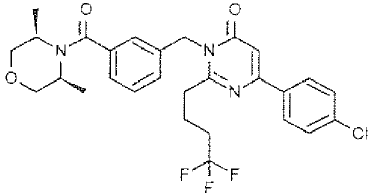 |
| I-0200 | 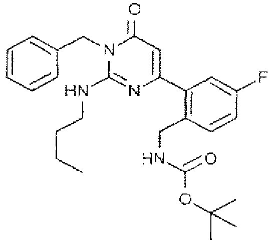 |
| I-0201 | 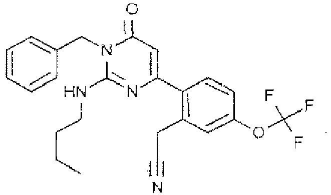 |
| I-0202 | 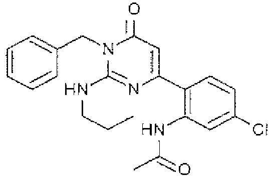 |
| I-0203 | 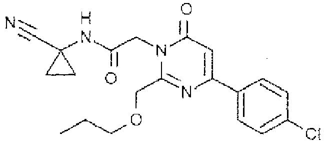 |
| I-0204 | 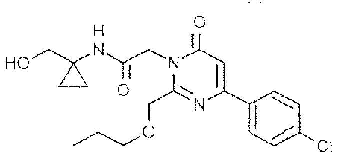 |

**[Table 35]**

| | |
|---|---|
| I-0205 | 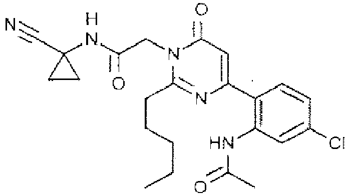 |
| I-0206 | 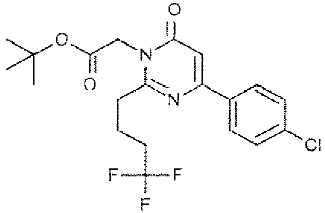 |
| I-0207 | 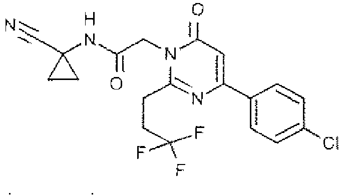 |
| I-0208 | 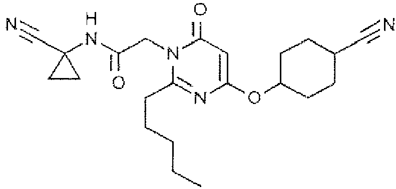 |
| I-0209 | 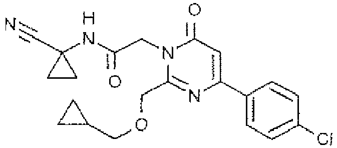 |
| I-0210 | 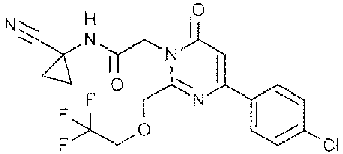 |

**[Table 36]**

| | |
|---|---|
| I-0211 | 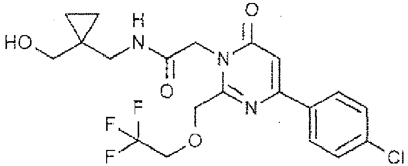 |
| I-0212 | 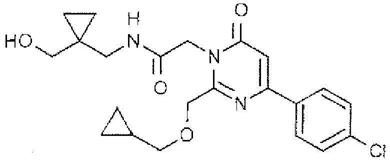 |
| I-0213 | 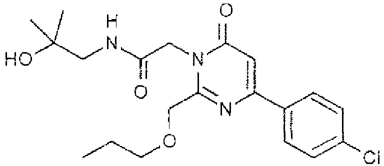 |
| I-0214 | 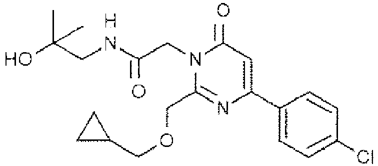 |
| I-0215 | 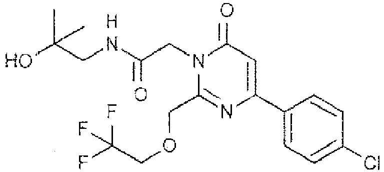 |
| I-0216 | 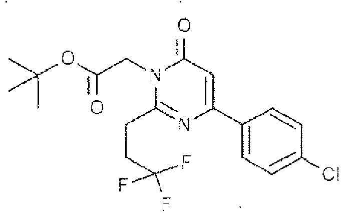 |

**[Table 37]**

| | |
|---|---|
| I-0217 | 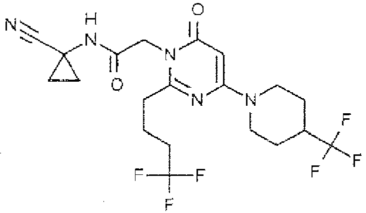 |
| I-0218 | 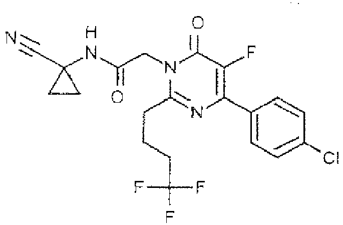 |
| I-0219 | 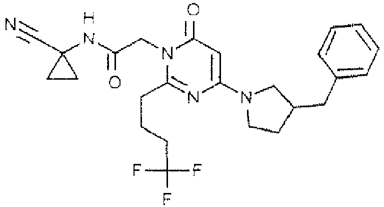 |
| I-0220 | 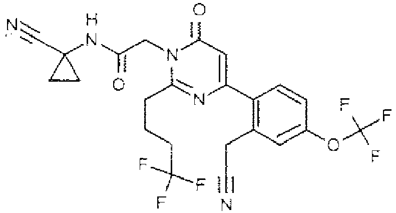 |
| I-0221 | 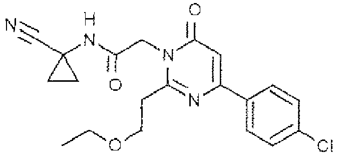 |
| I-0222 | 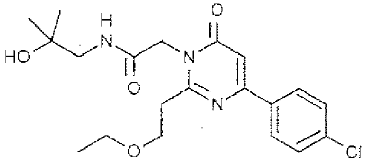 |

**[Table 38]**

| | |
|---|---|
| I-0223 | 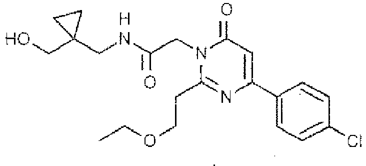 |
| I-0224 | 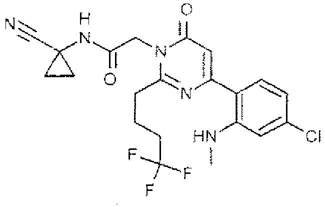 |
| I-0225 | 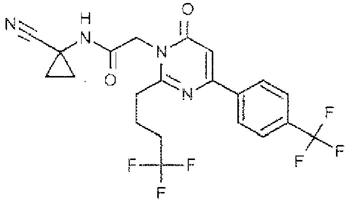 |
| I-0226 | 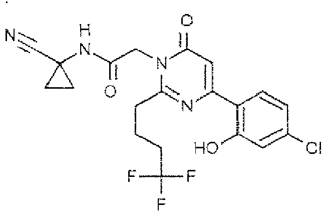 |
| I-0227 | 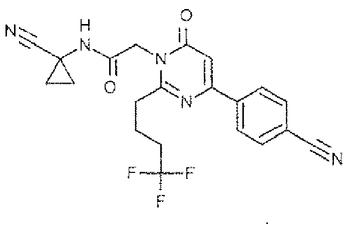 |
| I-0228 | 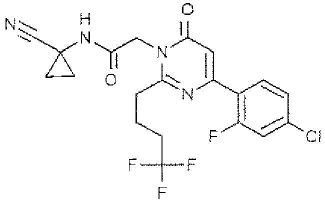 |

**[Table 39]**

| | |
|---|---|
| I-0229 | 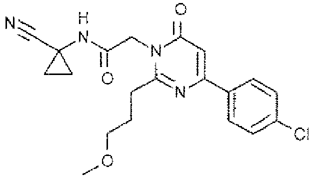 |
| I-0230 | 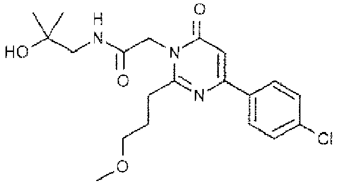 |
| I-0231 | 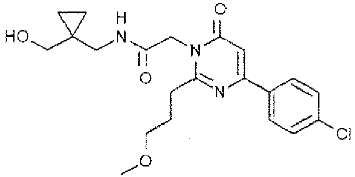 |
| I-0232 | 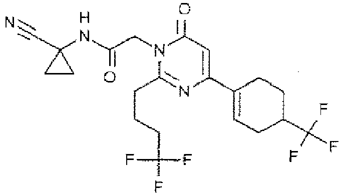 |
| I-0233 | 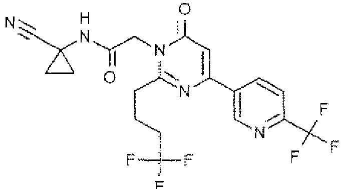 |
| I-0234 | 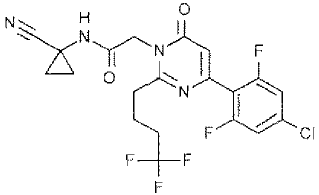 |

**[Table 40]**

| | |
|---|---|
| I-0235 | 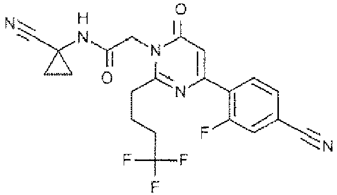 |
| I-0236 | 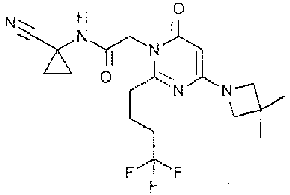 |
| I-0237 | 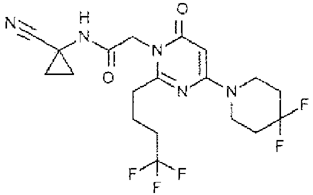 |
| I-0238 | 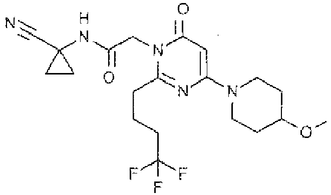 |
| I-0239 | 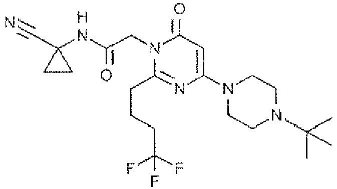 |
| I-0240 | 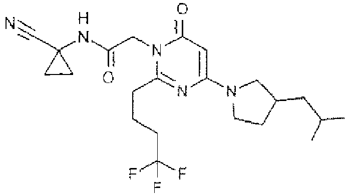 |

**[Table 41]**

| | |
|---|---|
| I-0241 | 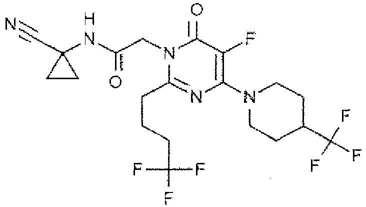 |
| I-0242 | 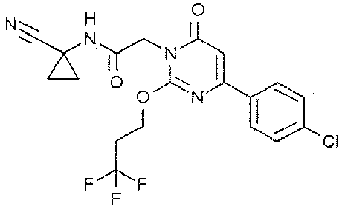 |
| I-0243 | 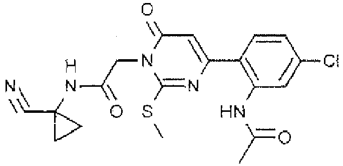 |
| I-0244 | 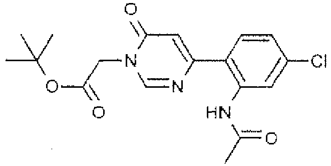 |
| I-0245 | 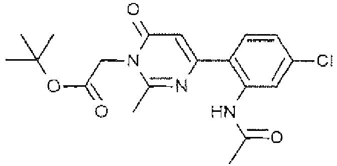 |
| I-0246 | 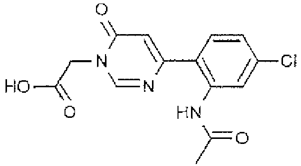 |

**[Table 42]**

| | |
|---|---|
| I-0247 | 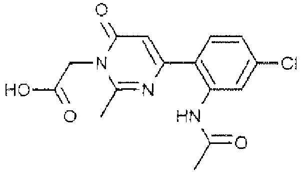 |
| I-0248 | 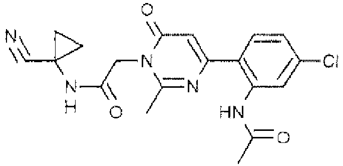 |
| I-0249 | 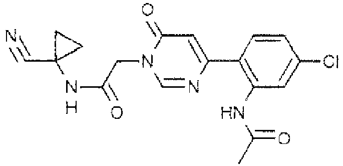 |
| I-0250 | 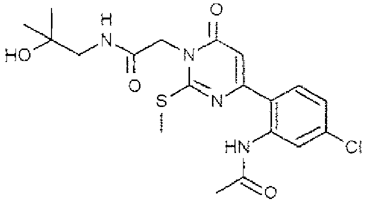 |
| I-0251 | 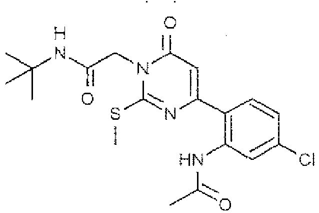 |
| I-0252 | 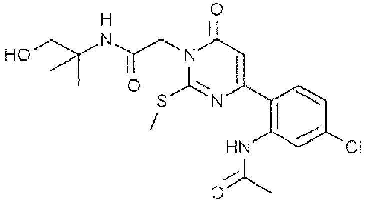 |

**[Table 43]**

| | |
|---|---|
| I-0253 | 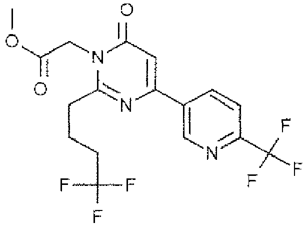 |
| I-0254 | 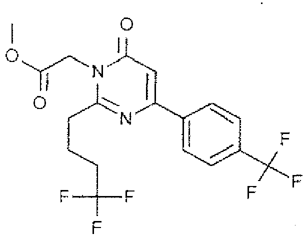 |
| I-0255 | 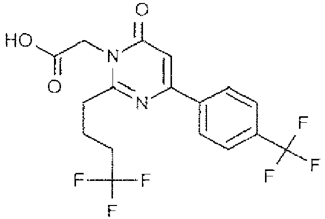 |
| I-0256 | 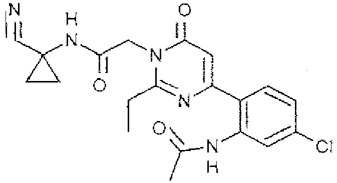 |
| I-0257 | 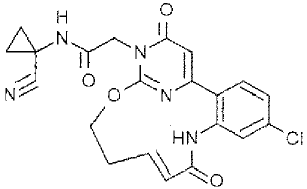 |

**[Table 44]**

| | |
|---|---|
| I-0258 | 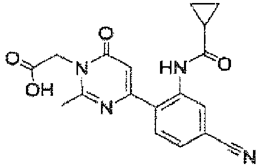 |
| I-0259 | 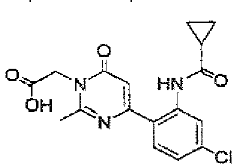 |
| I-0260 | 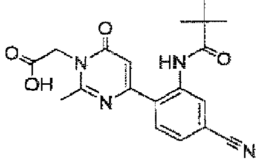 |
| I-0261 | 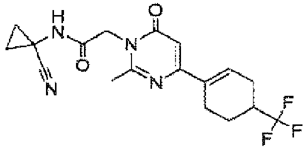 |
| I-0262 | 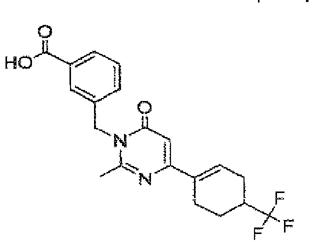 |
| I-0263 | 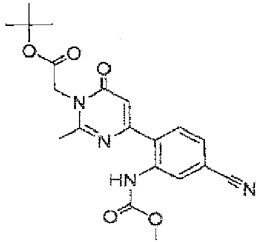 |

**[Table 45]**

| | |
|---|---|
| I-0264 | 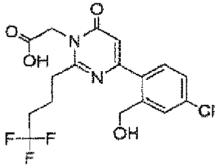 |
| I-0265 | 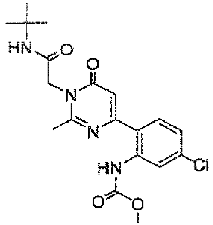 |
| I-0266 | 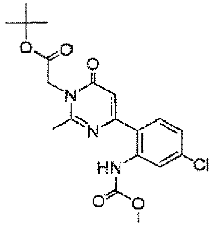 |
| I-0267 | 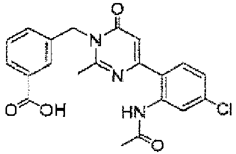 |
| I-0268 | 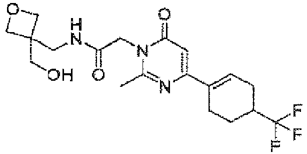 |
| I-0269 | 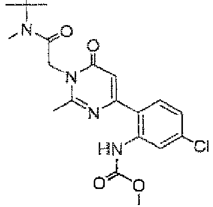 |

**[Table 46]**

| | |
|---|---|
| I-0270 | 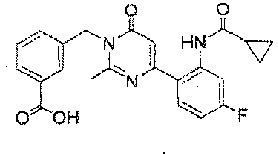 |
| I-0271 | 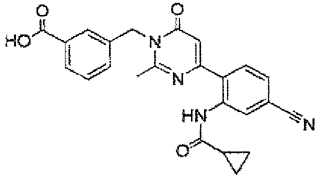 |
| I-0272 | 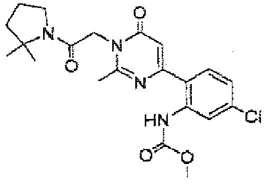 |
| I-0273 | 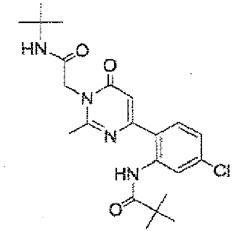 |
| I-0274 | 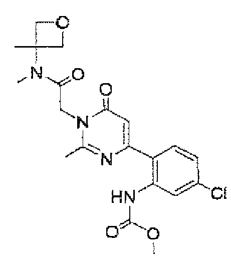 |
| I-0279 | 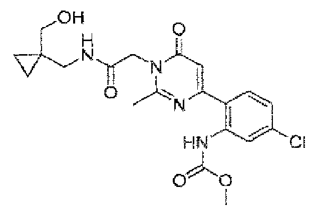 |

**[Table 47]**

| | |
|---|---|
| I-0276 | 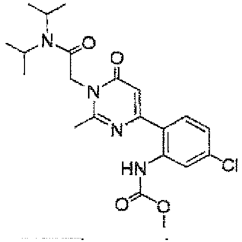 |
| I-0277 | 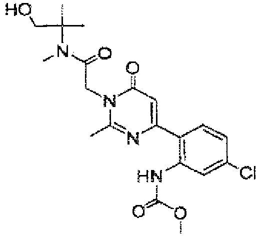 |
| I-0278 | 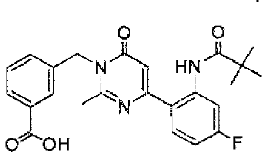 |
| I-0279 | 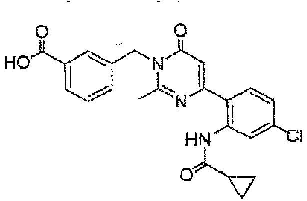 |
| I-0280 | 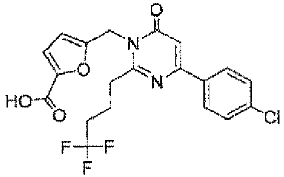 |
| I-0281 | 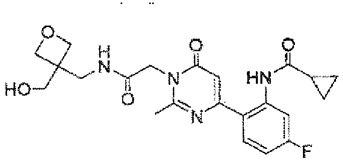 |

**[Table 48]**

| | |
|---|---|
| I-0282 | 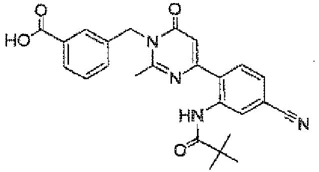 |
| I-0283 | 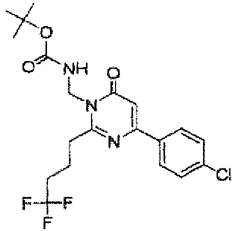 |
| I-0284 | 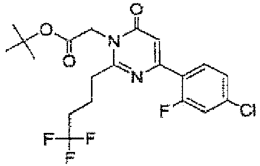 |
| I-0285 | 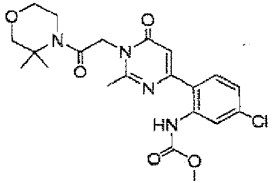 |
| I-0286 | 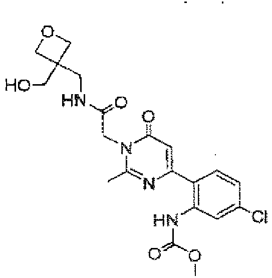 |

**[Table 49]**

| | |
|---|---|
| I-0287 | 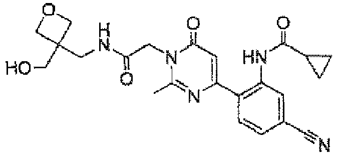 |
| I-0288 | 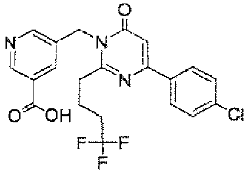 |
| I-0289 | 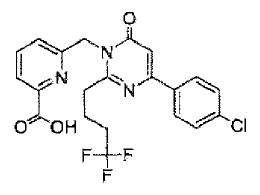 |
| I-0290 | 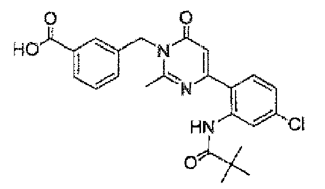 |
| I-0291 | 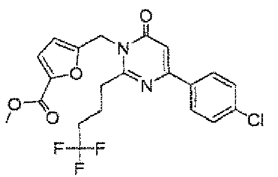 |
| I-0292 | 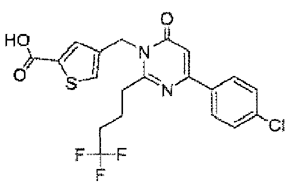 |

**[Table 50]**

| | |
|---|---|
| I-0293 | 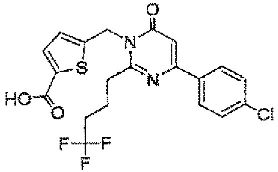 |
| I-0294 | 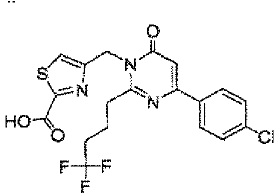 |
| I-0295 | 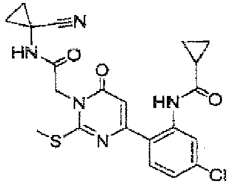 |
| I-0296 | 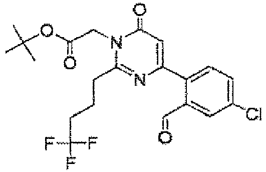 |
| I-0297 | 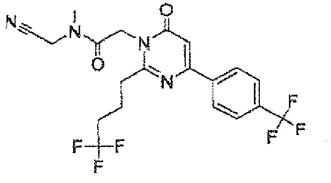 |
| I-0298 | 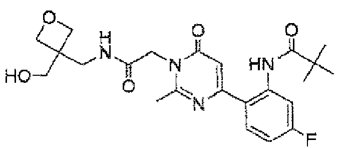 |

**[Table 51]**

| | |
|---|---|
| I-0199 | 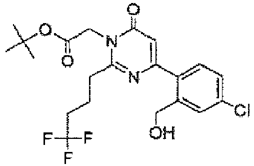 |
| I-0300 | 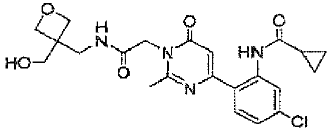 |
| I-0301 | 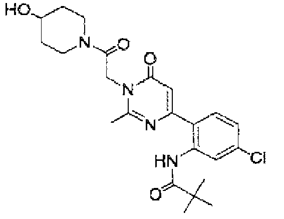 |
| I-0302 | 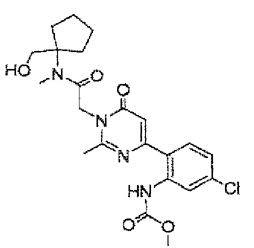 |
| I-0303 | 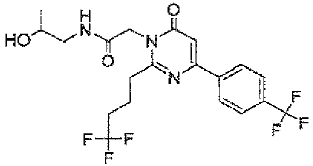 |
| I-0304 | 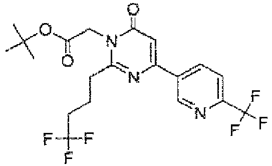 |

**[Table 52]**

| | |
|---|---|
| I-0305 | 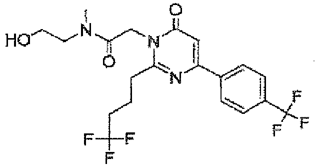 |
| I-0306 | 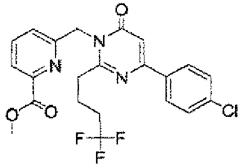 |
| I-0307 | 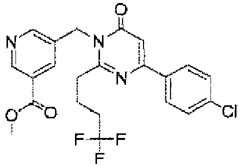 |
| I-0308 | 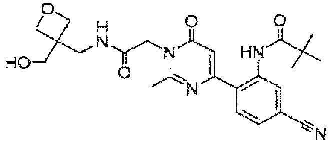 |
| I-0309 | 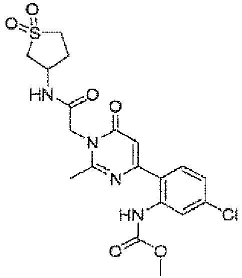 |

**[Table 53]**

| | |
|---|---|
| I-0310 | 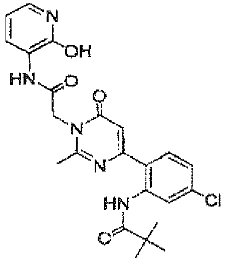 |
| I-0311 | 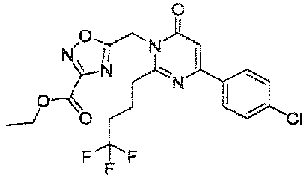 |
| I-0312 | 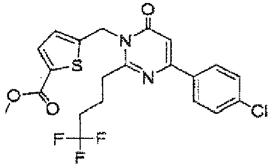 |
| I-0313 | 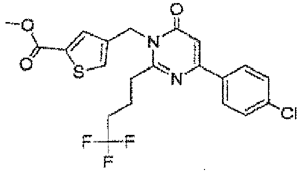 |
| I-0314 | 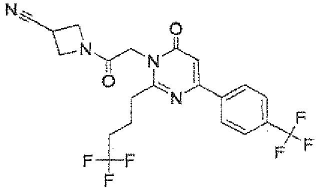 |
| I-0315 | 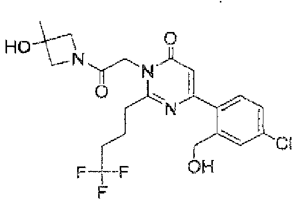 |

**[Table 54]**

| | |
|---|---|
| I-0316 | 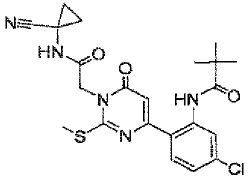 |
| I-0317 | 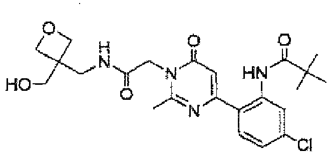 |
| I-0318 | 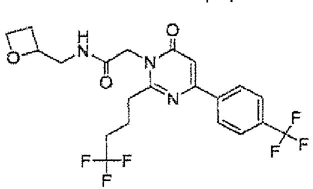 |
| I-0319 | 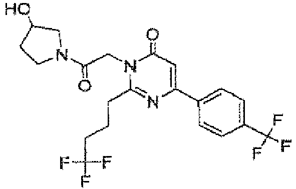 |
| I-0320 | 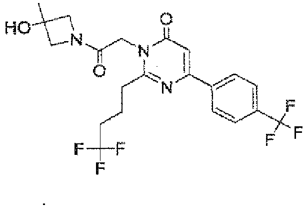 |
| I-0321 | 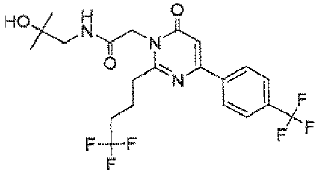 |

**[Table 55]**

| | |
|---|---|
| I-0322 | 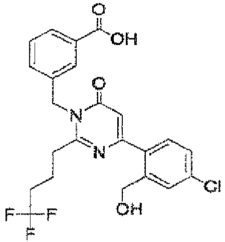 |
| I-0323 | 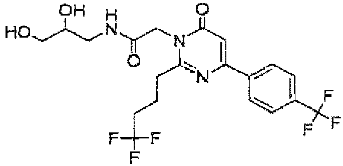 |
| I-0324 | 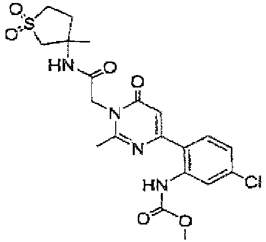 |
| I-0325 | 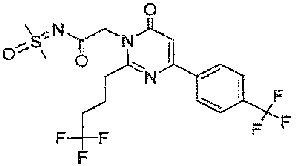 |
| I-0326 | 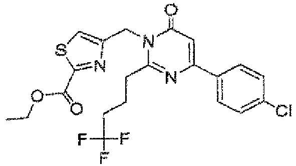 |

**[Table 56]**

| | |
|---|---|
| I-0327 | 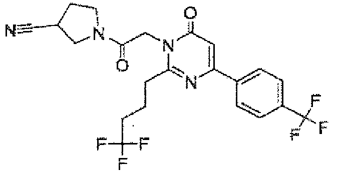 |
| I-0328 | 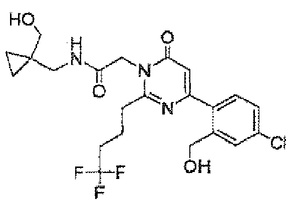 |
| I-0329 | 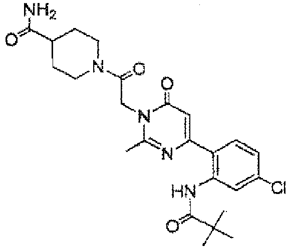 |
| I-0330 | 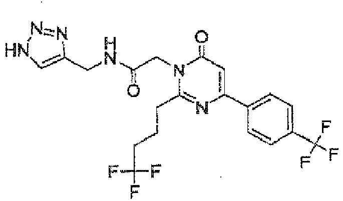 |
| I-0331 | 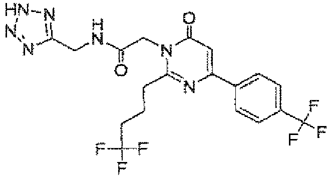 |

**[Table 57]**

| | |
|---|---|
| I-0332 | 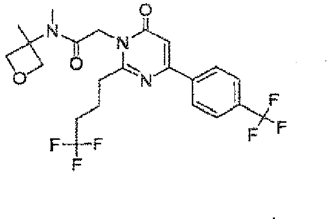 |
| I-0333 | 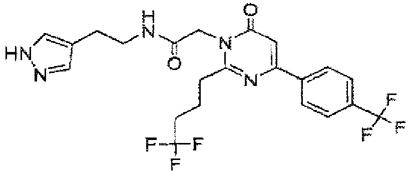 |
| I-0334 | 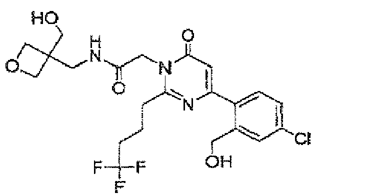 |
| I-0335 | 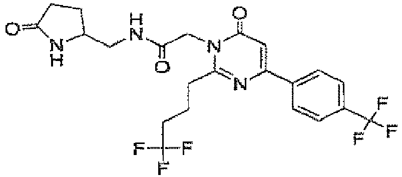 |
| I-0336 | 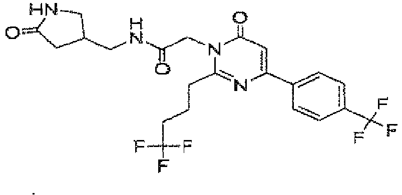 |
| I-0337 | 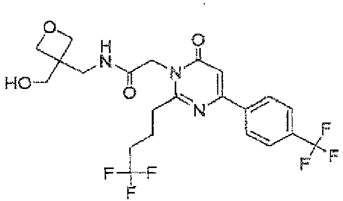 |

**[Table 58]**

| | |
|---|---|
| I-0338 | 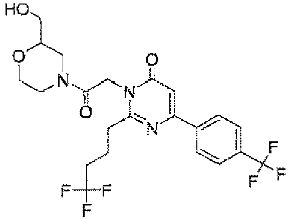 |
| I-0339 | 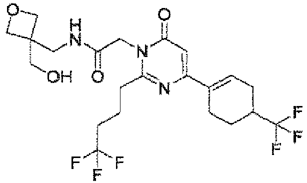 |
| I-0340 | 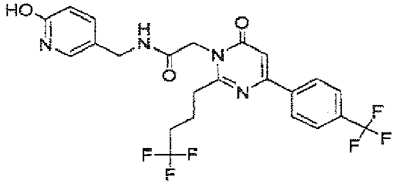 |
| I-0341 | 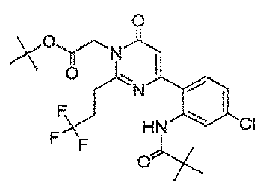 |
| I-0342 | 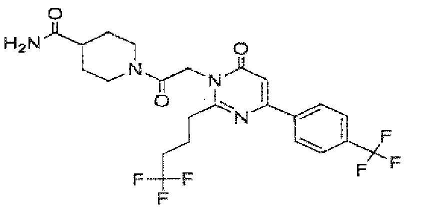 |

**[Table 59]**

| | |
|---|---|
| I-0343 | 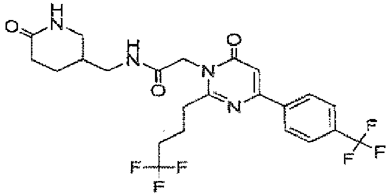 |
| I-0344 | 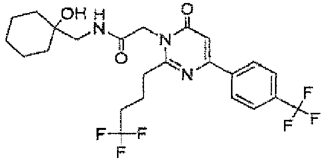 |
| I-0345 | 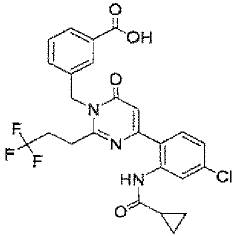 |
| I-0346 | 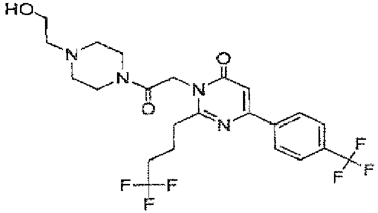 |
| I-0347 | 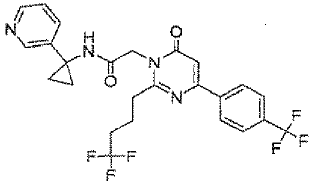 |

**[Table 60]**

| | |
|---|---|
| I-0348 | 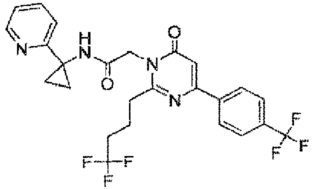 |
| I-0349 | 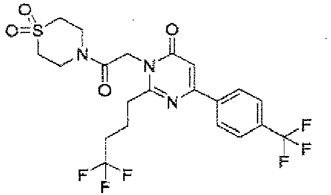 |
| I-0350 | 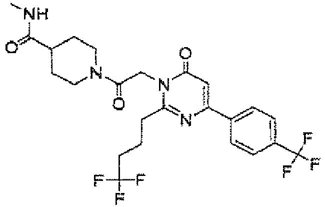 |
| I-0351 | 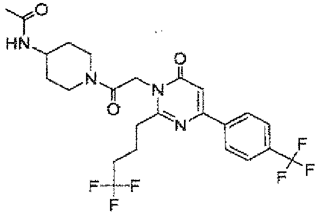 |
| I-0352 | 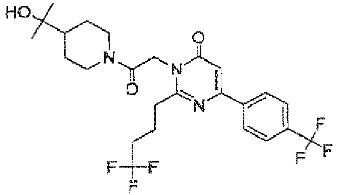 |

**[Table 61]**

| | |
|---|---|
| I-0353 | 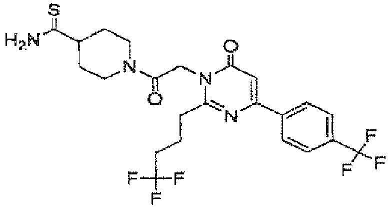 |
| I-0354 | 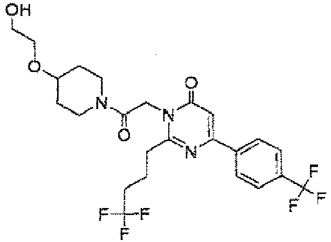 |
| I-0355 | 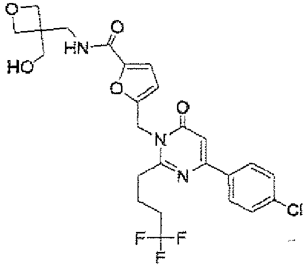 |
| I-0356 | 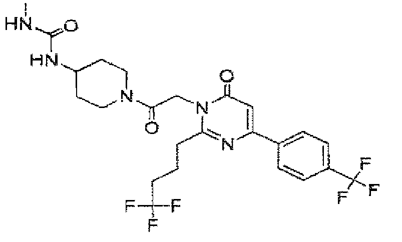 |
| I-0357 | 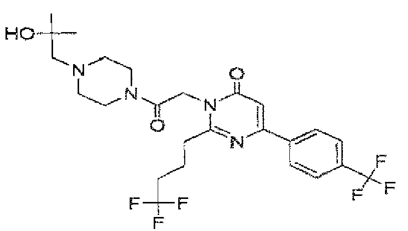 |

**[Table 62]**

| | |
|---|---|
| I-0358 | 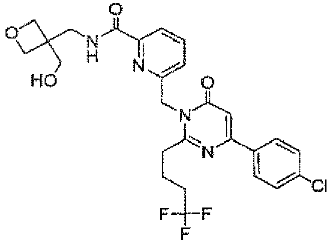 |
| I-0359 | 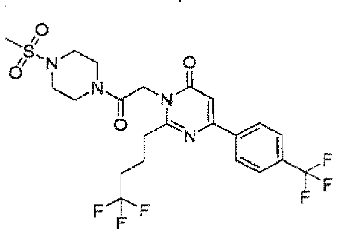 |
| I-0360 | 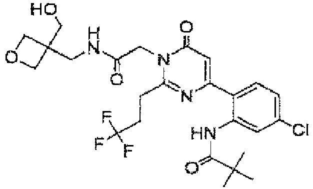 |
| I-0361 | 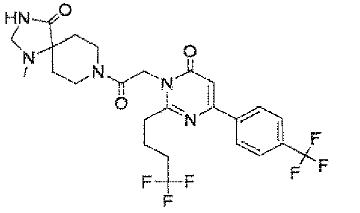 |
| I-0362 | 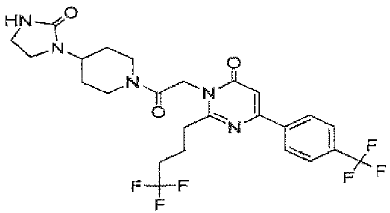 |

**[Table 63]**

| | |
|---|---|
| I-0363 | 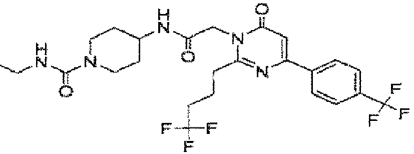 |
| I-0364 | 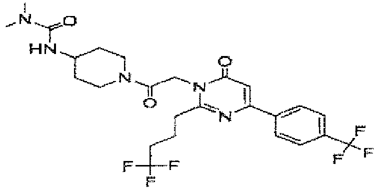 |
| I-0365 | 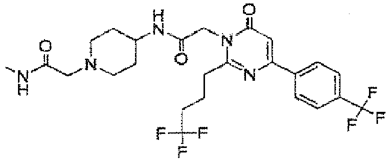 |
| I-0366 | 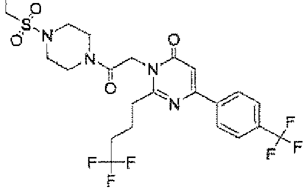 |
| I-0367 | 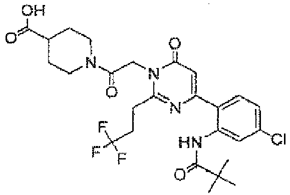 |
| I-0368 | 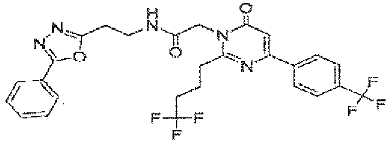 |

**[Table 64]**

| | |
|---|---|
| I-0369 | 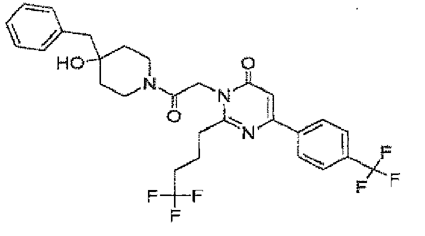 |
| I-0370 | 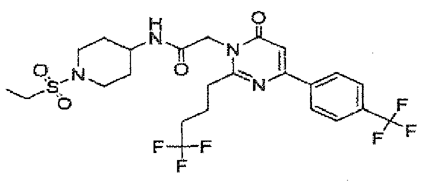 |

**[Table 65]**

| | |
|---|---|
| I-0371 | 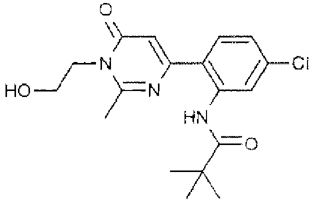 |
| I-0372 | 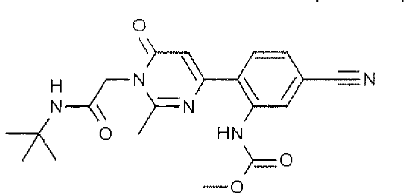 |
| I-0373 | 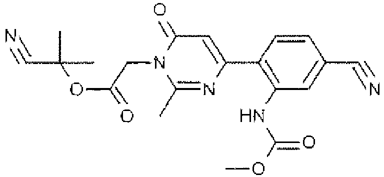 |
| I-0374 | 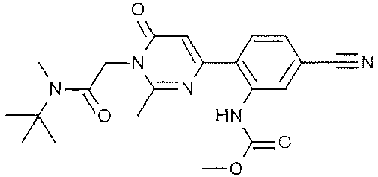 |
| I-0375 | 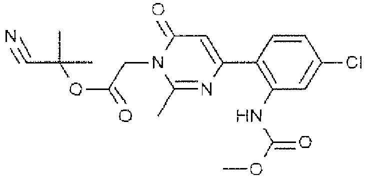 |
| I-0376 | 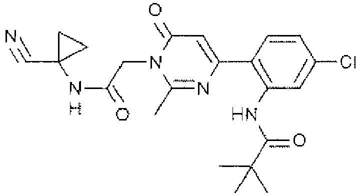 |

**[Table 66]**

| | |
|---|---|
| I-0377 | 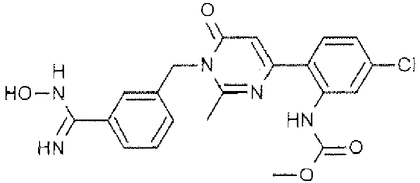 |
| I-0378 | 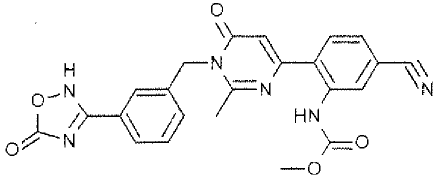 |
| I-0379 | 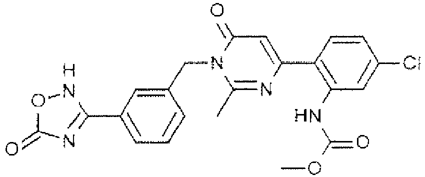 |
| I-0380 | 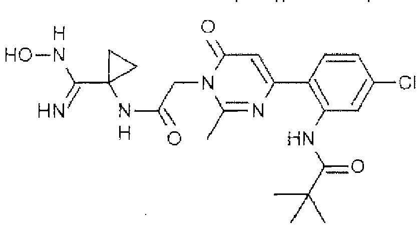 |
| I-0381 | 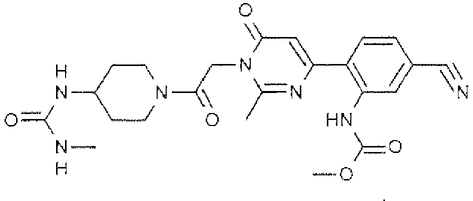 |
| I-0382 | 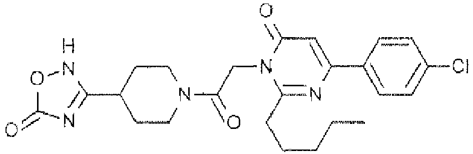 |

**[Table 67]**

| | |
|---|---|
| I-0383 | 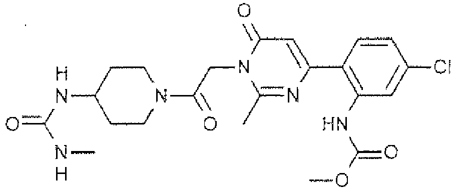 |
| I-0384 | 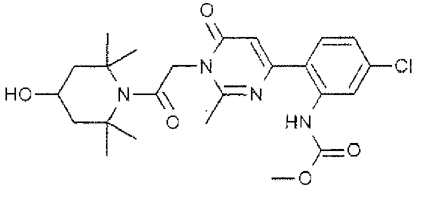 |
| I-0385 | 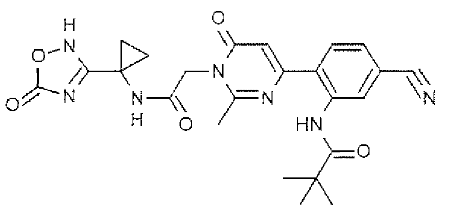 |
| I-0386 | 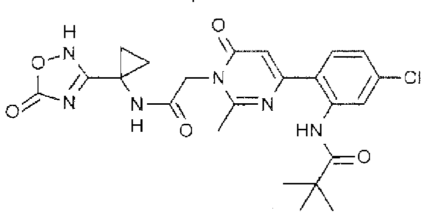 |
| I-0387 | 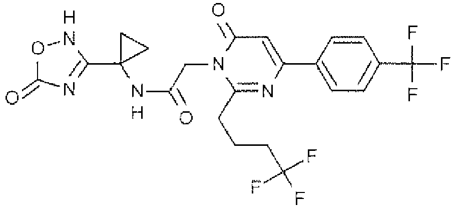 |
| I-0388 | 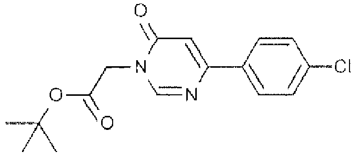 |

**[Table 68]**

| | |
|---|---|
| I-0389 | 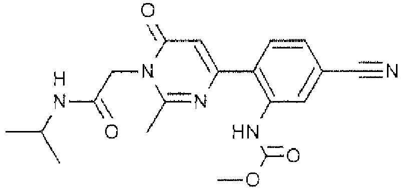 |
| I-0390 | 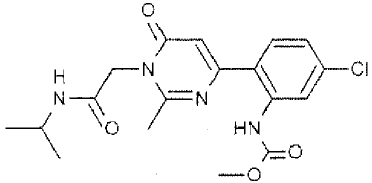 |
| I-0391 | 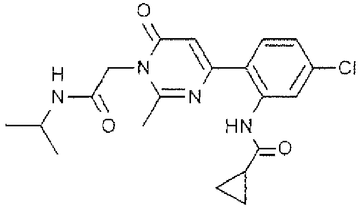 |
| I-0392 | 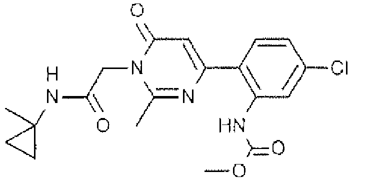 |
| I-0393 | 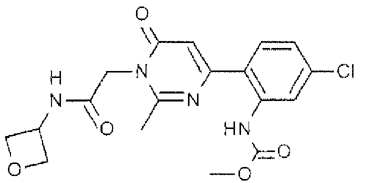 |
| I-0394 | 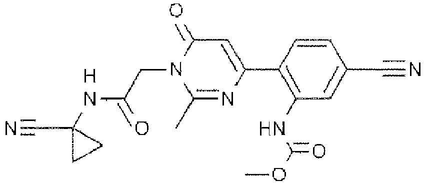 |

**[Table 69]**

| | |
|---|---|
| I-0395 | 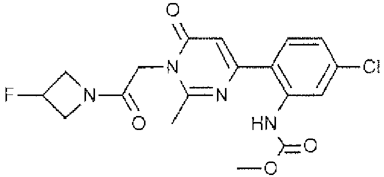 |
| I-0396 | 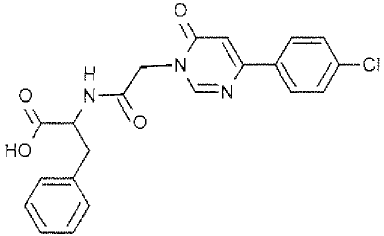 |
| I-0397 | 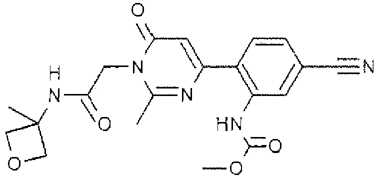 |
| I-0398 | 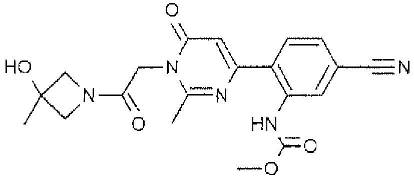 |
| I-0399 | 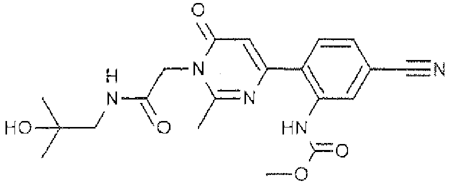 |
| I-0400 | 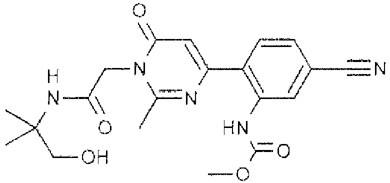 |

**[Table 70]**

| | |
|---|---|
| I-0401 | 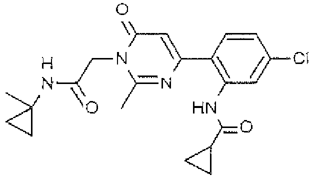 |
| I-0402 | 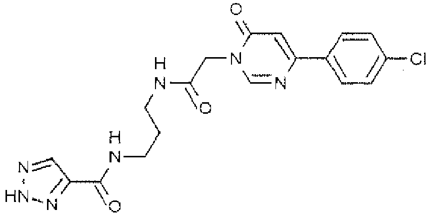 |
| I-0403 | 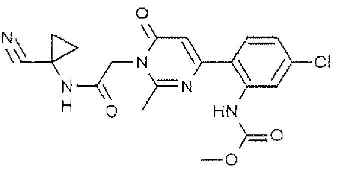 |
| I-0404 | 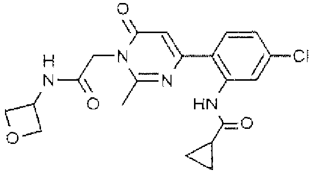 |
| I-0405 | 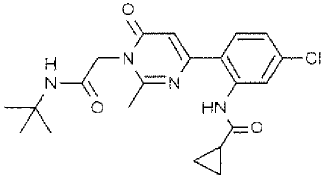 |
| I-0405 | 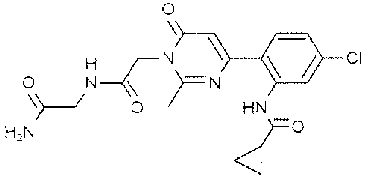 |

**[Table 71]**

| | |
|---|---|
| I-0407 | 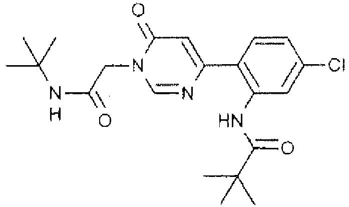 |
| I-0408 | 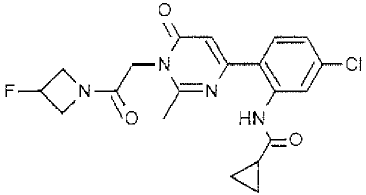 |
| I-0409 | 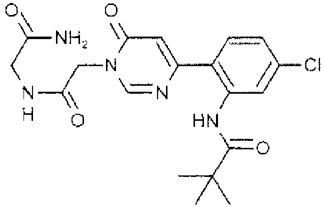 |
| I-0410 | 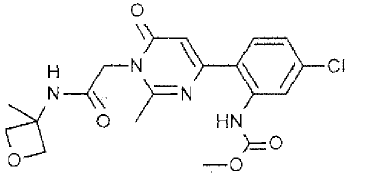 |
| I-0411 | 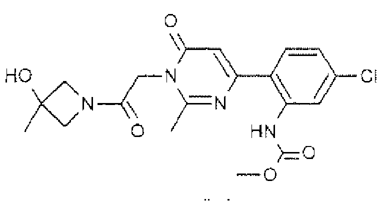 |
| I-0412 | 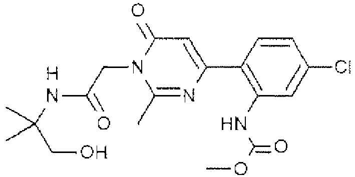 |

**[Table 72]**

| | |
|---|---|
| I-0413 | 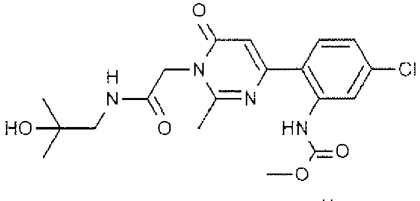 |
| I-0414 | 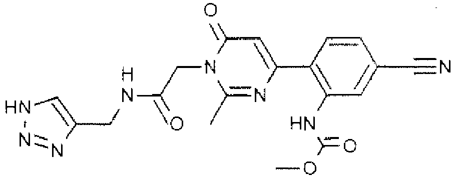 |
| I-0415 | 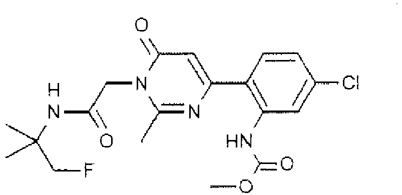 |
| I-0416 | 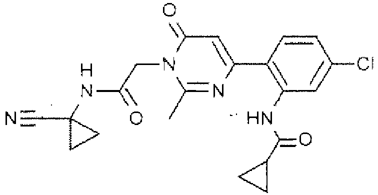 |
| I-0417 | 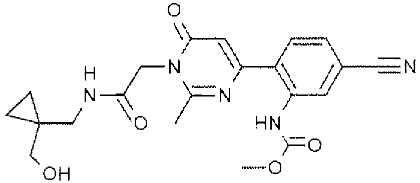 |
| I-0418 | 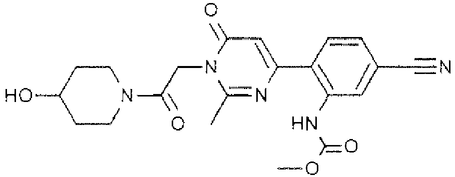 |

**[Table 73]**

| | |
|---|---|
| I-0419 | 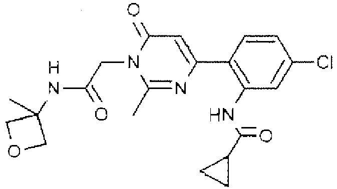 |
| I-0420 | 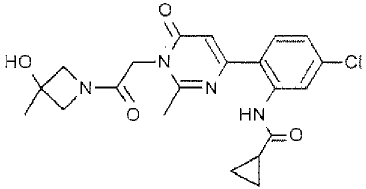 |
| I-0421 | 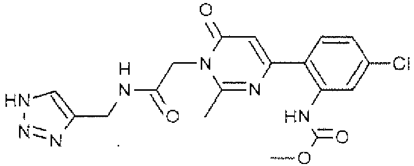 |
| I-0422 | 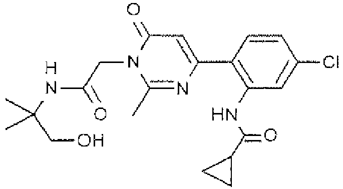 |
| I-0423 | 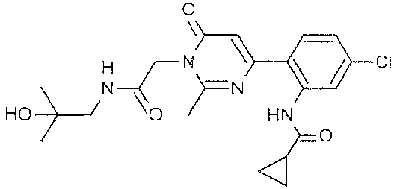 |
| I-0424 | 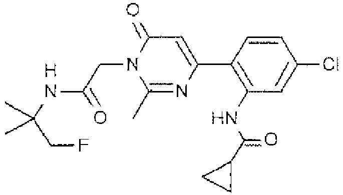 |

**[Table 74]**

| | |
|---|---|
| I-0425 | 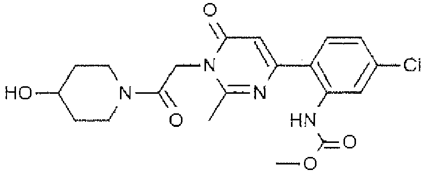 |
| I-0426 | 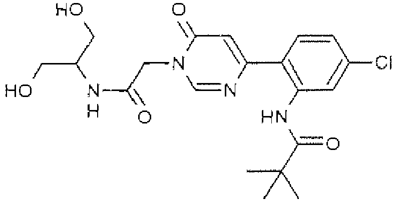 |
| I-0427 | 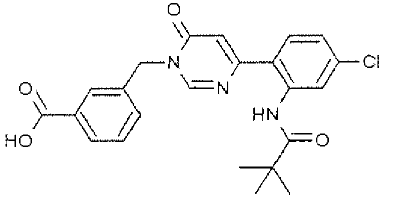 |
| I-0428 | 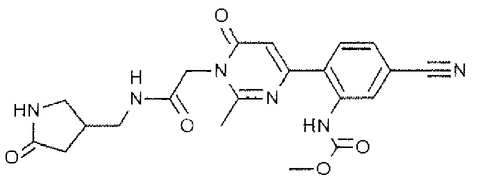 |
| I-0429 | 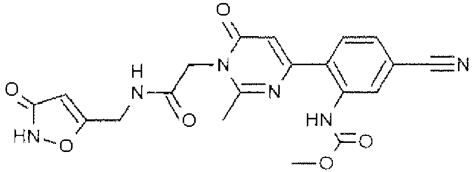 |
| I-0430 | 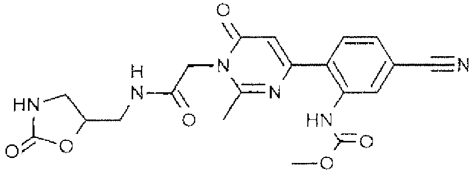 |

**[Table 75]**

| | |
|---|---|
| I-0431 | 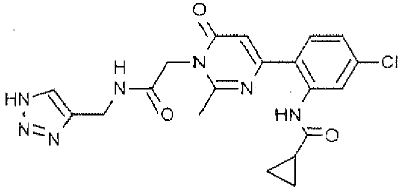 |
| I-0432 | 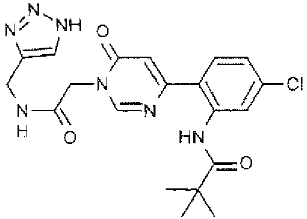 |
| I-0433 | 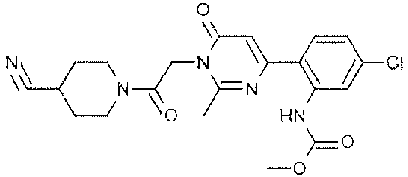 |
| I-0434 | 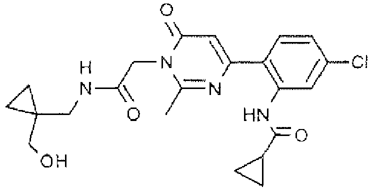 |
| I-0435 | 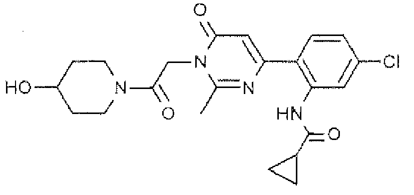 |
| I-0436 | 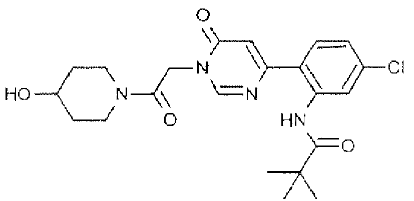 |

**[Table 76]**

| | |
|---|---|
| I-0437 | 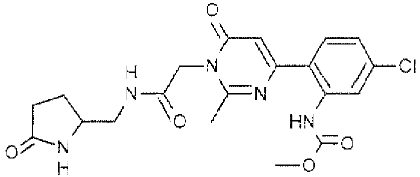 |
| I-0438 | 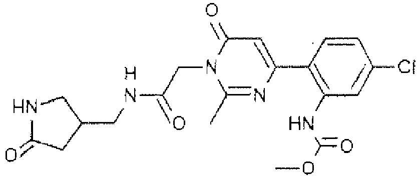 |
| I-0439 | 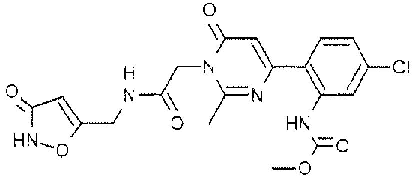 |
| I-0440 | 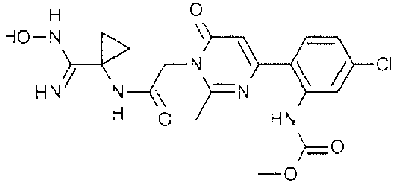 |
| I-0441 | 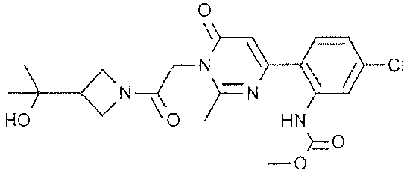 |
| I-0442 | 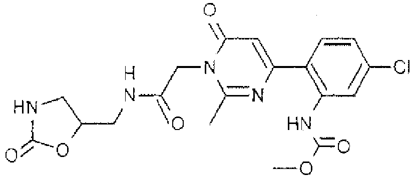 |

**[Table 77]**

| | |
|---|---|
| I-0443 | 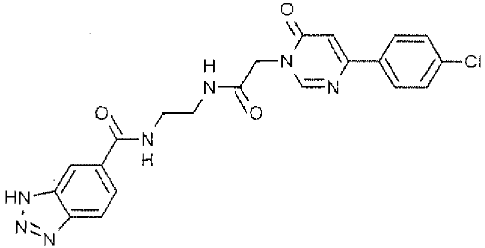 |
| I-0444 | 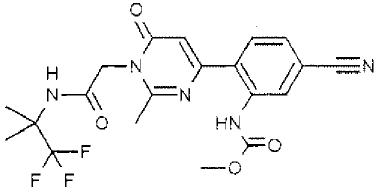 |
| I-0445 | 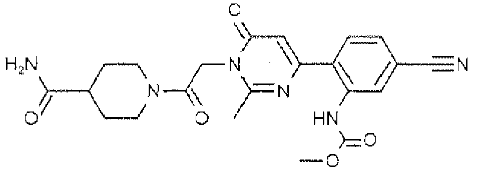 |
| I-0446 | 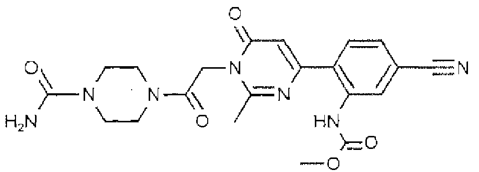 |
| I-0447 | 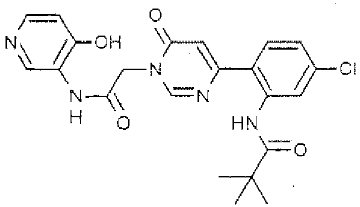 |
| I-0448 | 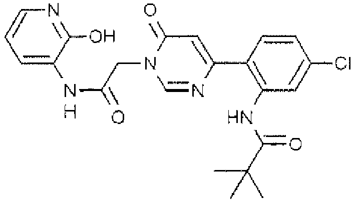 |

**[Table 78]**

| | |
|---|---|
| I-0449 | 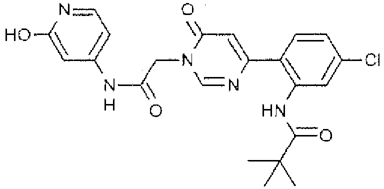 |
| I-0450 | 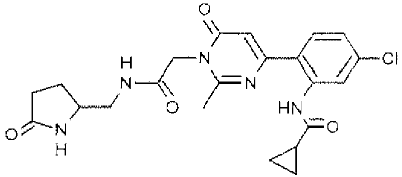 |
| I-0451 | 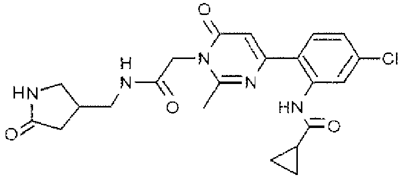 |
| I-0452 | 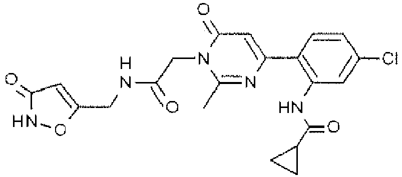 |
| I-0453 | 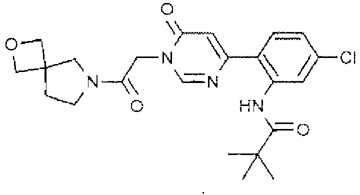 |
| I-0454 | 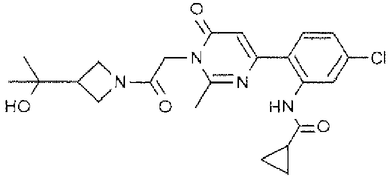 |

**[Table 79]**

| | |
|---|---|
| I-0455 | 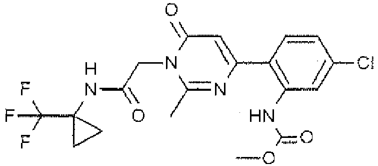 |
| I-0456 | 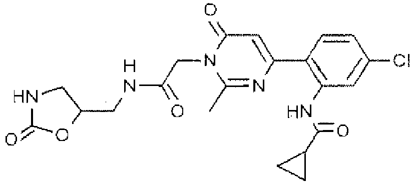 |
| I-0457 | 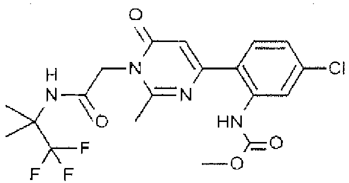 |
| I-0458 | 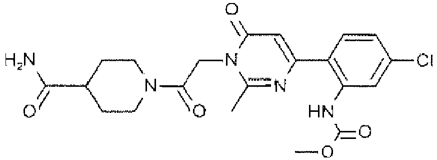 |
| I-0459 | 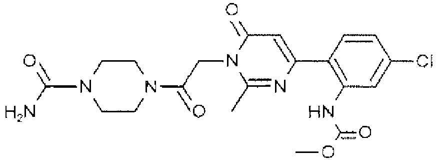 |
| I-0460 | 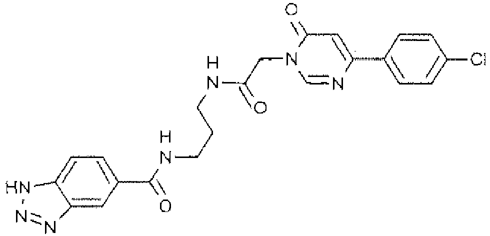 |

**[Table 80]**

| | |
|---|---|
| I-0461 | 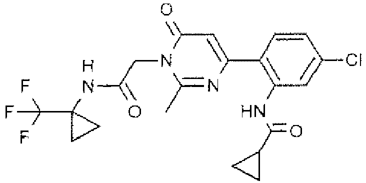 |
| I-0462 | 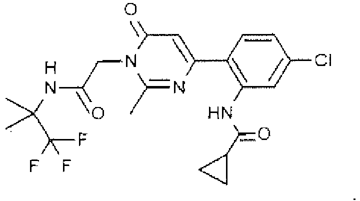 |
| I-0463 | 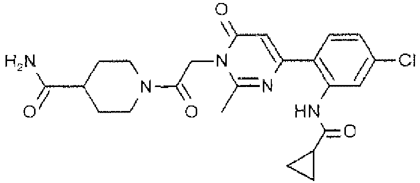 |
| I-0464 | 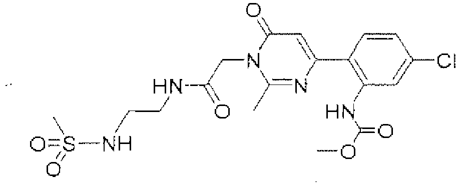 |
| I-0465 | 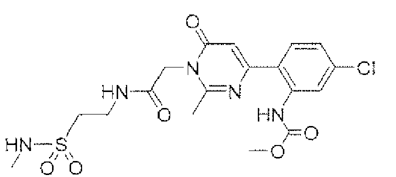 |
| I-0466 | 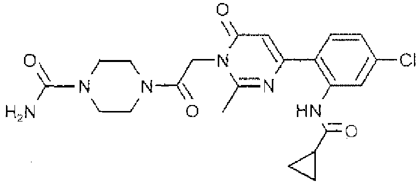 |

**[Table 81]**

| | |
|---|---|
| I-0467 | 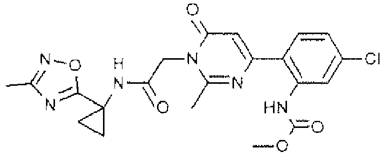 |
| I-0468 | 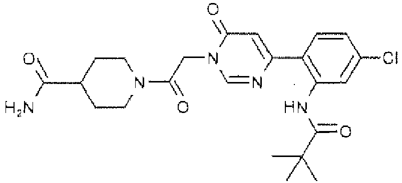 |
| I-0469 | 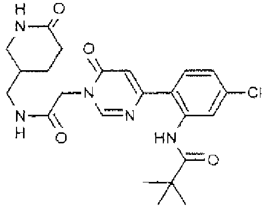 |
| I-0470 | 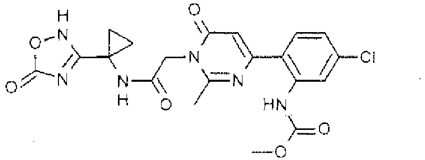 |
| I-0471 | 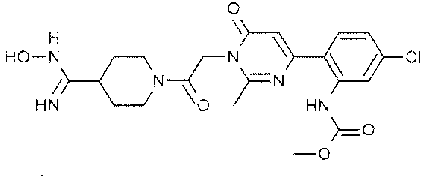 |
| I-0472 | 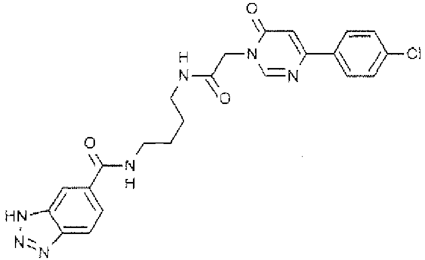 |

**[Table 82]**

| | |
|---|---|
| I-0473 | 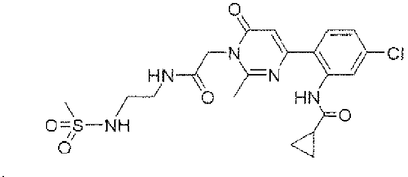 |
| I-0474 | 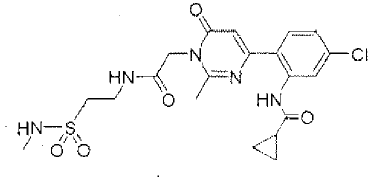 |
| I-0475 | 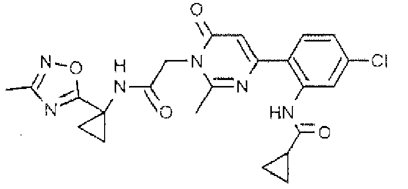 |
| I-0476 | 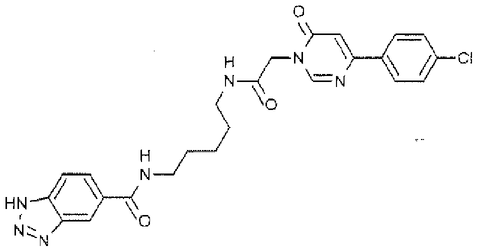 |
| I-0477 | 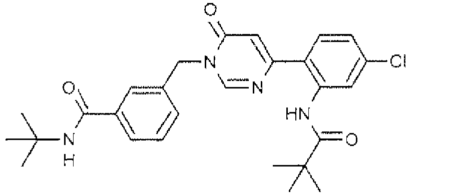 |
| I-0478 | 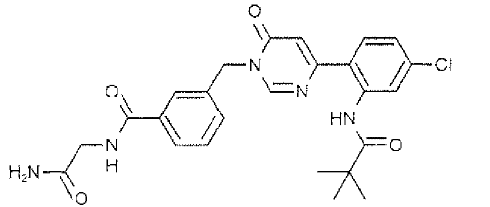 |

**[Table 83]**

| | |
|---|---|
| I-0479 | 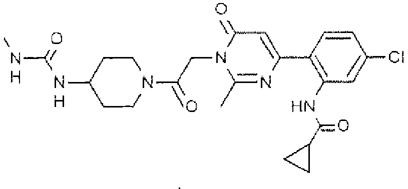 |
| I-0480 | 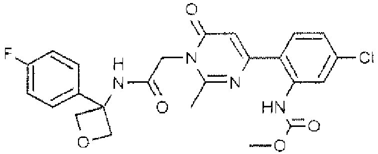 |
| I-0481 | 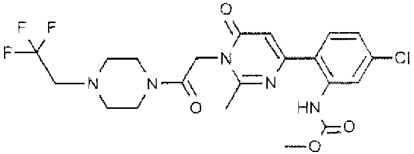 |
| I-0482 | 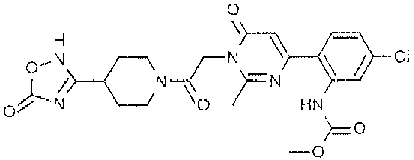 |
| I-0483 | 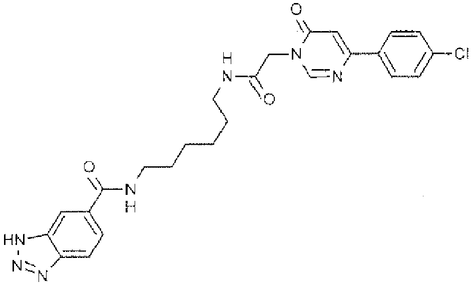 |
| I-0484 | 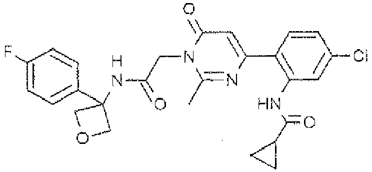 |

**[Table 84]**

| | |
|---|---|
| I-0485 | 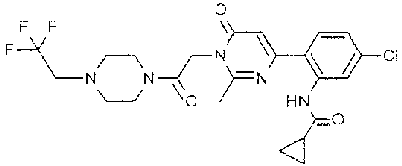 |
| I-0486 | 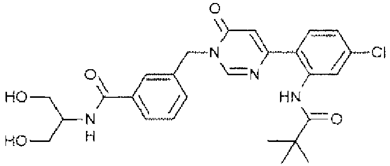 |
| I-0487 | 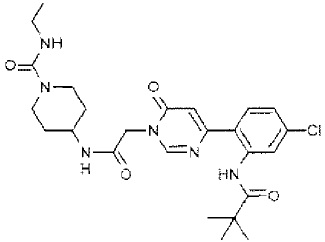 |
| I-0488 | 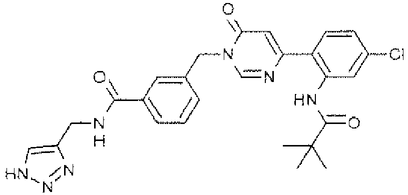 |
| I-0489 | 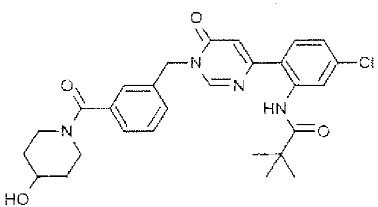 |
| I-0490 | 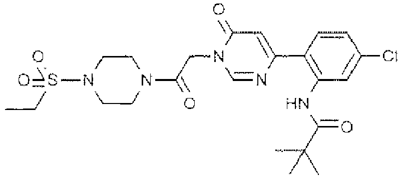 |

**[Table 85]**

| | |
|---|---|
| I-0491 | 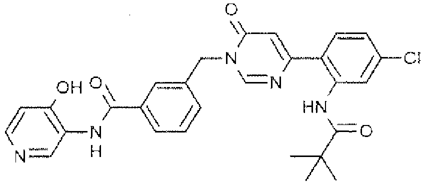 |
| I-0492 | 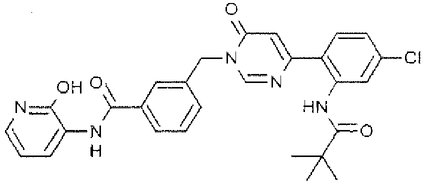 |
| I-0493 | 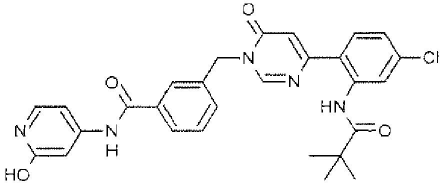 |
| I-0494 | 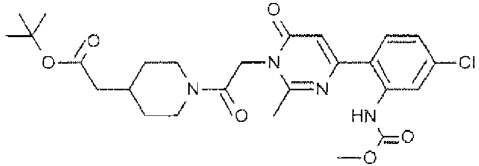 |
| I-0495 | 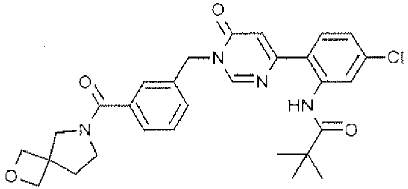 |
| I-0496 | 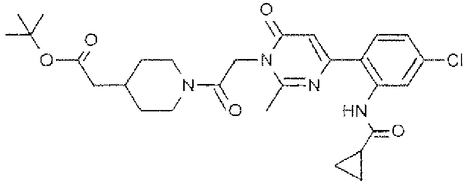 |

**[Table 86]**

| | |
|---|---|
| I-0497 | 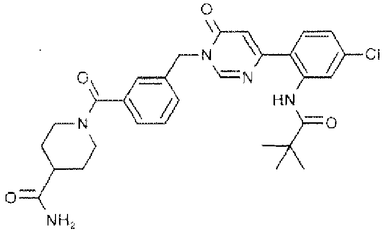 |
| I-0498 | 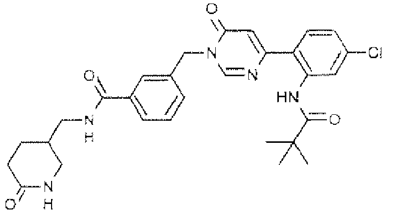 |
| I-0499 | 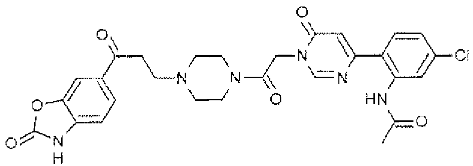 |
| I-0500 | 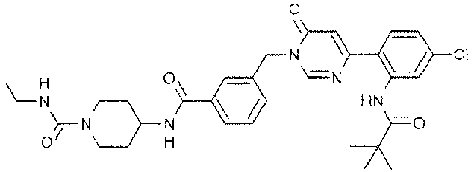 |
| I-0501 | 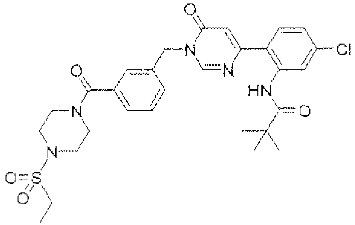 |
| I-0502 | 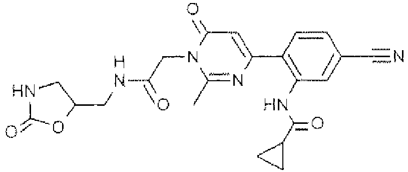 |

**[Table 87]**

| | |
|---|---|
| I-0503 | 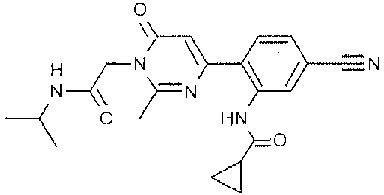 |
| I-0504 | 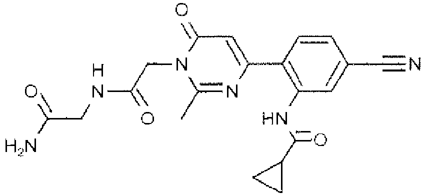 |
| I-0505 | 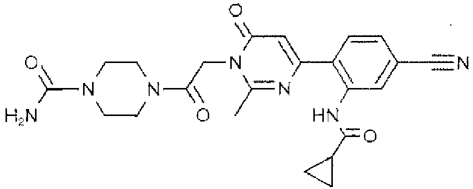 |
| I-0506 | 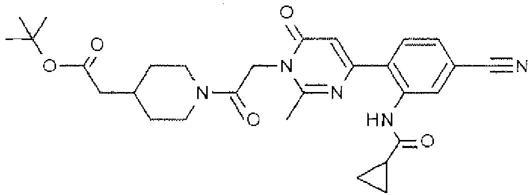 |
| I-0507 | 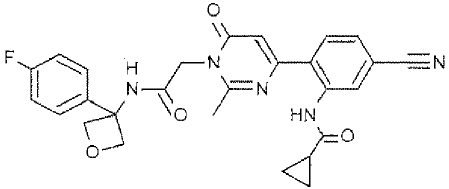 |
| I-0508 | 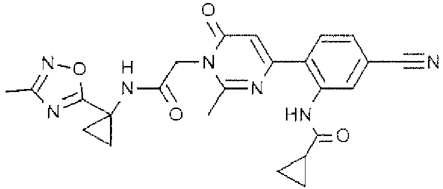 |

**[Table 88]**

| | |
|---|---|
| I-0509 | 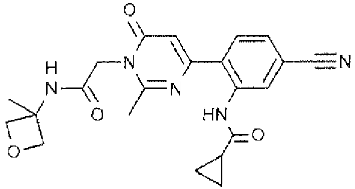 |
| I-0510 | 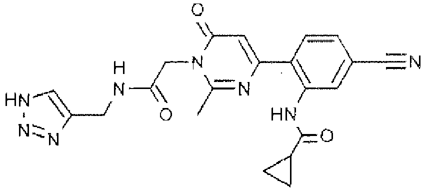 |
| I-0511 | 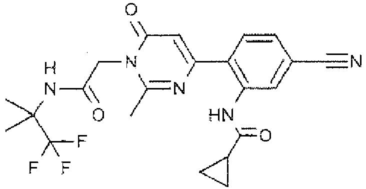 |
| I-0512 | 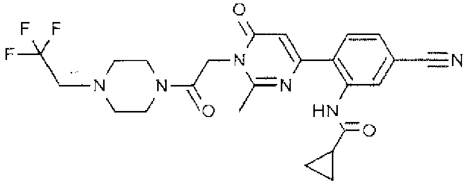 |
| I-0513 | 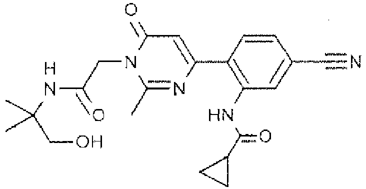 |
| I-0514 | 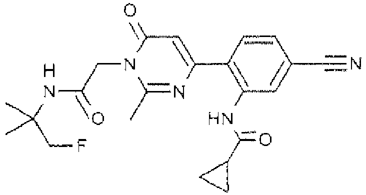 |

**[Table 89]**

| | |
|---|---|
| I-0515 | 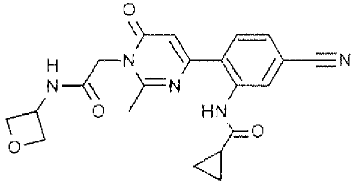 |
| I-0516 | 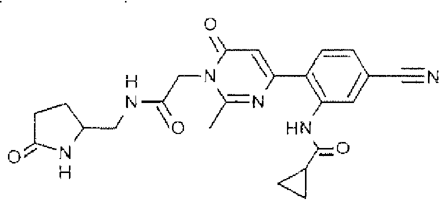 |
| I-0517 | 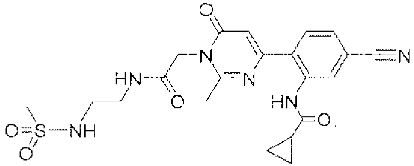 |
| I-0518 | 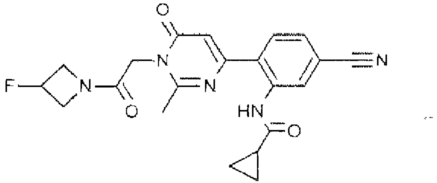 |
| I-0519 | 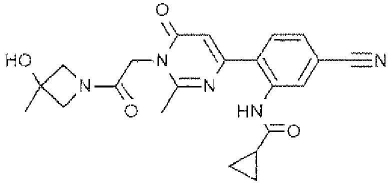 |
| I-0520 | 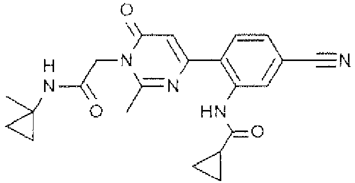 |

**[Table 90]**

| | |
|---|---|
| I-0521 | 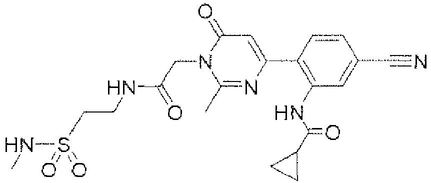 |
| I-0522 | 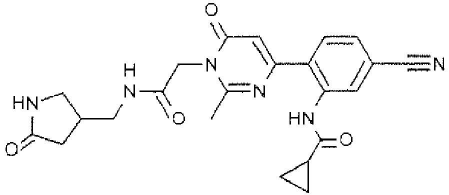 |
| I-0523 | 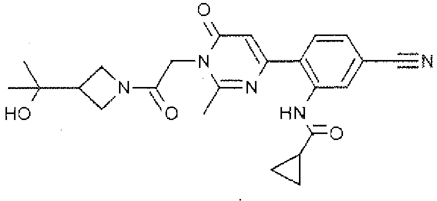 |
| I-0524 | 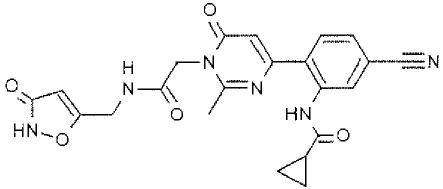 |
| I-0525 | 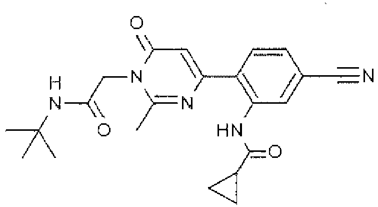 |
| I-0526 | 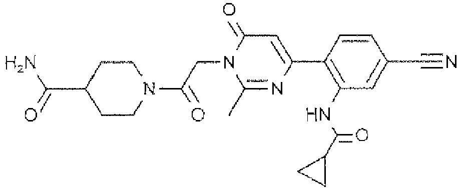 |

**[Table 91]**

| | |
|---|---|
| I-0527 | 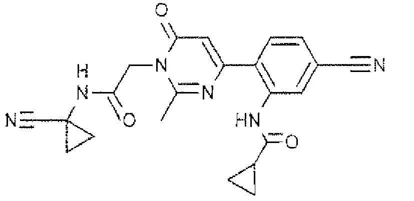 |
| I-0528 | 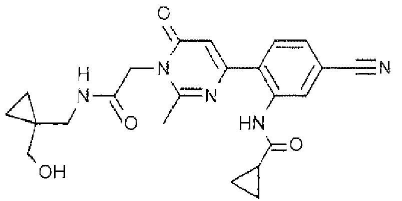 |
| I-0529 | 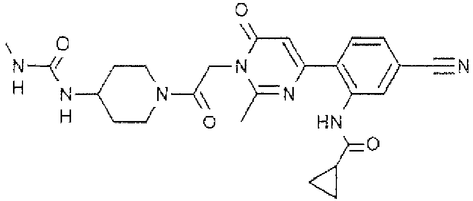 |
| I-0530 | 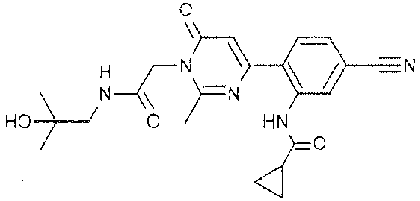 |
| I-0531 | 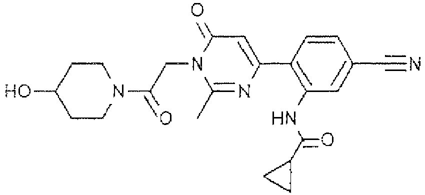 |

Physical constants of the compound are shown below. RT means retention time ( min), MS means [M + H]⁺.

**[Table 92]**

| Example No | LC/MS method | RT | MS | Example No | LC/MS method | RT | MS |
|---|---|---|---|---|---|---|---|
| I-0001 | B | 2.48 | 306 | 1-0041 | A | 2.33 | 469 |
| I-0002 | A | 1.98 | 411 | 1-0042 | A | 2.29 | 446 |
| I-0003 | A | 1.98 | 411 | 1-0043 | A | 2.28 | 448 |
| I-0004 | NMR | | | I-0044 | A | 2.17 | 420 |
| I-0005 | A | 2.17 | 291 | I-0045 | A | 2.35 | 460 |
| I-0006 | A | 2.35 | 305 | I-0046 | A | 2.65 | 522 |
| I-0007 | A | 2.66 | 367 | I-0047 | A | 2.59 | 522 |
| I-0008 | A | 2.18 | 411 | I-0048 | A | 1.86 | 461 |
| I-0009 | A | 2.56 | 411 | I-0049 | A | 2.41 | 446 |
| I-0010 | D | 2.09 | 460 | I-0050 | A | 2.40 | 434 |
| I-0011 | D | 2.33 | 516 | I-0051 | A | 2.30 | 420 |
| I-0012 | D | 1.67 | 460 | I-0052 | A | 2.04 | 422 |
| I-0013 | B | 2.23 | 516 | I-0053 | A | 2.57 | 522 |
| I-0014 | B | 2.62 | 607 | I-0054 | A | 2.46 | 469 |
| I-0015 | B | 2.34 | 517 | I-0055 | A | 2.01 | 449 |
| I-0016 | B | 2.33 | 531 | I-0056 | A | 2.24 | 420 |
| I-0017 | B | 2.60 | 607 | I-0057 | A | 2.36 | 446 |
| I-0018 | B | 2.30 | 517 | I-0058 | A | 2.14 | 462 |
| I-0019 | B | 2.21 | 516 | I-0059 | A | 2.12 | 503 |
| I-0020 | B | 2.33 | 532 | I-0060 | A | 2.23 | 484 |
| I-0021 | B | 2.76 | 473 | I-0061 | A | 2.46 | 482 |
| I-0022 | B | 2.15 | 417 | I-0062 | A | 2.57 | 474 |
| I-0023 | B | 2.59 | 348 | I-0063 | A | 2.35 | 460 |
| I-0024 | A | 2.62 | 482 | I-0064 | A | 2.21 | 434 |
| I-0025 | A | 2.29 | 421 | I-0065 | A | 2.12 | 392 |
| I-0026 | A | 2.54 | 376 | I-0066 | A | 2.23 | 432 |
| I-0027 | A | 2.13 | 378 | I-0067 | A | 2.23 | 448 |
| I-0028 | A | 2.26 | 390 | I-0068 | A | 2.23 | 432 |
| I-0029 | A | 221 | 392 | I-0069 | A | 2.16 | 418 |
| I-0030 | A | 2.16 | 392 | I-0070 | A | 2.35 | 434 |
| I-0031 | A | 2.06 | 405 | I-0071 | A | 2.21 | 432 |
| I-0032 | A | 1.79 | 405 | I-0072 | A | 2.45 | 486 |
| I-0033 | A | 2.31 | 418 | I-0073 | A | 2.28 | 432 |
| I-0034 | A | 2.73 | 424 | I-0074 | B | 2.91 | 425 |
| I-0035 | A | 2.14 | 434 | I-0075 | A | 2.55 | 486 |
| I-0036 | A | 2.86 | 439 | I-0076 | A | 2.78 | 480 |
| I-0037 | A | 2.09 | 446 | I-0077 | A | 2.67 | 441 |
| I-0038 | A | 2.20 | 456 | I-0078 | A | 2.17 | 443 |
| I-0039 | A | 2.20 | 406 | I-0079 | A | 2.38 | 401 |
| I-0040 | A | 2.14 | 406 | I-0080 | A | 2.29 | 387 |

**[Table 93]**

| Example No | LC/MS method | RT | MS | Example No | LC/MS method | RT | MS |
|---|---|---|---|---|---|---|---|
| I-0081 | A | 2.25 | 452 | I-0121 | A | 2.02 | 486 |
| I-0082 | A | 2.12 | 431 | I-0122 | A | 1.86 | 515 |
| I-0083 | A | 2.13 | 442 | I-0123 | A | 2.31 | 439 |
| I-0084 | A | 2.36 | 404 | I-0124 | A | 2.18 | 458 |
| I-0085 | A | 2.33 | 373 | I-0125 | A | 2.45 | 472 |
| I-0086 | A | 2.32 | 418 | I-0126 | A | 2.03 | 445 |
| I-0087 | A | 2.64 | 438 | I-0127 | A | 2.13 | 459 |
| I-0088 | A | 2.56 | 469 | I-0128 | A | 2.16 | 528 |
| I-0089 | A | 2.33 | 451 | I-0129 | A | 1.78 | 528 |
| I-0090 | A | 2.39 | 399 | I-0130 | A | 2.03 | 488 |
| I-0091 | A | 2.24 | 416 | I-0131 | A | 1.77 | 487 |
| I-0092 | A | 2.54 | 469 | I-0132 | A | 2.00 | 462 |
| I-0093 | A | 2.23 | 466 | I-0133 | A | 2.07 | 481 |
| I-0094 | A | 2.19 | 440 | I-0134 | A | 2.17 | 446 |
| I-0095 | A | 2.58 | 432 | I-0135 | A | 1.97 | 478 |
| I-0096 | A | 2.19 | 406 | I-0136 | A | 1.95 | 496 |
| I-0097 | A | 2.12 | 445 | I-0137 | A | 2.22 | 427 |
| I-0098 | A | 2.24 | 406 | I-0138 | A | 1.80 | 496 |
| I-0099 | A | 2.71 | 441 | I-0139 | A | 2.10 | 474 |
| I-0100 | A | 2.82 | 480 | I-0140 | A | 2.05 | 506 |
| I-0101 | A | 2.01 | 496 | I-0141 | A | 2.28 | 470 |
| I-0102 | A | 2.83 | 452 | I-0142 | A | 1.99 | 481 |
| I-0103 | A | 2.75 | 438 | I-0143 | A | 2.19 | 492 |
| I-0104 | A | 2.18 | 443 | I-0144 | A | 2.16 | 476 |
| I-0105 | B | 1.89 | 369 | I-0145 | A | 2.26 | 509 |
| I-0106 | B | 1.79 | 342 | I-0146 | A | 1.72 | 505 |
| I-0107 | B | 2.13 | 548 | I-0147 | A | 2.04 | 474 |
| I-0108 | B | 1.78 | 303 | I-0148 | A | 1.82 | 515 |
| I-0109 | B | 2.34 | 389 | I-0149 | A | 2.51 | 444 |
| I-0110 | B | 2.13 | 374 | I-0150 | A | 1.61 | 428 |
| I-0111 | B | 2.63 | 465 | I-0151 | A | 2.39 | 498 |
| I-0112 | B | 2.33 | 451 | I-0152 | A | 2.63 | 498 |
| I-0113 | A | 2.48 | 460 | I-0153 | A | 2.65 | 536 |
| I-0114 | A | 2.44 | 434 | I-0154 | A | 1.96 | 558 |
| I-0115 | A | 1.85 | 495 | I-0155 | A | 2.52 | 503 |
| I-0116 | A | 2.07 | 495 | I-0156 | A | 2.23 | 535 |
| I-0117 | A | 1.99 | 462 | I-0157 | A | 2.46 | 517 |
| I-0118 | A | 2.07 | 485 | I-0158 | A | 2.27 | 534 |
| I-0119 | A | 2.10 | 432 | I-0159 | A | 2.31 | 548 |
| I-0120 | A | 2.15 | 471 | I-0160 | A | 2.45 | 520 |

**[Table 94]**

| Example No | LC/MS method | RT | MS | Example No | LC/MS method | RT | MS |
|---|---|---|---|---|---|---|---|
| I-0161 | A | 2.50 | 522 | I-0201 | A | 2.63 | 458 |
| I-0162 | A | 2.26 | 538 | I-0202 | A | 2.35 | 412 |
| I-0163 | A | 2.29 | 508 | I-0203 | B | 2.05 | 401 |
| I-0164 | A | 2.47 | 489 | I-0204 | B | 1.88 | 406 |
| I-0165 | A | 2.16 | 524 | I-0205 | A | 2.11 | 456 |
| I-0166 | A | 2.65 | 548 | I-0206 | A | 2.79 | 431 |
| I-0167 | A | 2.57 | 570 | I-0207 | A | 2.20 | 425 |
| I-0168 | A | 2.19 | 507 | I-0208 | A | 1.85 | 412 |
| I-0169 | A | 2.36 | 542 | I-0209 | B | 2.04 | 413 |
| I-0170 | A | 2.35 | 547 | I-0210 | B | 2.02 | 441 |
| I-0171 | A | 2.23 | 562 | I-0211 | B | 1.92 | 460 |
| I-0172 | A | 2.25 | 494 | I-0212 | B | 1.94 | 432 |
| I-0173 | A | 2.17 | 524 | I-0213 | B | 1.93 | 408 |
| I-0174 | A | 2.34 | 548 | I-0214 | B | 1.92 | 420 |
| I-0175 | A | 2.42 | 534 | I-0215 | B | 1.91 | 448 |
| I-0176 | A | 2.18 | 557 | I-0216 | A | 2.75 | 417 |
| I-0177 | A | 2.35 | 556 | I-0217 | A | 2.09 | 480 |
| I-0178 | A | 2.56 | 570 | I-0218 | A | 2.38 | 457 |
| I-0179 | A | 2.17 | 547 | I-0219 | A | 2.34 | 488 |
| I-0180 | A | 2.40 | 464 | I-0220 | A | 2.21 | 528 |
| I-0181 | A | 2.48 | 478 | I-0221 | B | 1.93 | 401 |
| I-0182 | A | 2.24 | 521 | I-0222 | B | 1.81 | 408 |
| I-0183 | A | 2.35 | 535 | I-0223 | B | 1.82 | 420 |
| I-0184 | A | 2.20 | 535 | I-0224 | A | 2.35 | 468 |
| I-0185 | A | 2.45 | 520 | I-0225 | A | 2.33 | 473 |
| I-0186 | A | 2.29 | 557 | I-0226 | A | 2.22 | 455 |
| I-0187 | A | 2.38 | 522 | I-0227 | A | 1.94 | 430 |
| I-0188 | A | 2.19 | 554 | I-0228 | A | 2.30 | 457 |
| I-0189 | A | 2.46 | 546 | I-0229 | B | 1.85 | 401 |
| I-0190 | A | 2.62 | 546 | I-0230 | B | 1.74 | 408 |
| I-0191 | A | 2.19 | 521 | I-0231 | B | 1.75 | 420 |
| I-0192 | A | 2.19 | 557 | I-0232 | A | 2.31 | 477 |
| I-0193 | A | 2.25 | 557 | I-0233 | A | 2.08 | 474 |
| I-0194 | A | 2.27 | 550 | I-0234 | A | 2.23 | 475 |
| I-0195 | A | 2.37 | 546 | I-0235 | A | 1.97 | 448 |
| I-0196 | A | 2.20 | 561 | I-0236 | A | 2.05 | 412 |
| I-0197 | A | 2.16 | 538 | I-0237 | A | 1.99 | 448 |
| I-0198 | A | 2.60 | 584 | I-0238 | A | 1.78 | 442 |
| I-0199 | A | 2.62 | 548 | I-0239 | A | 1.20 | 469 |
| I-0200 | A | 2.67 | 482 | I-0240 | A | 2.53 | 454 |

**[Table 95]**

| Example No | LC/MS method | RT | MS |
|---|---|---|---|
| I-0241 | A | 2.18 | 498 |
| I-0242 | B | 2.02 | 441 |
| I-0243 | B | 1.66 | 432 |
| I-0244 | A | 1.91 | 378 |
| I-0245 | B | 2.00 | 392 |
| I-0246 | B | 1.21 | 321 |
| I-0247 | B | 1.33 | 336 |
| I-0248 | B | 1.46 | 400 |
| I-0249 | B | 1.32 | 386 |
| I-0250 | B | 1.58 | 439 |
| I-0251 | B | 1.90 | 422 |
| I-0252 | B | 1.64 | 439 |
| I-0253 | A | 2.22 | 424 |
| I-0254 | B | 2.37 | 423 |
| I-0255 | B | 2.18 | 409 |
| I-0256 | B | 1.17 | 414 |
| I-0257 | B | 1.77 | 440 |

**[Table 96]**

| Example No | LC/MS method | RT | MS | Example No | LC/MS method | RT | MS |
|---|---|---|---|---|---|---|---|
| I-0258 | E | 1.42 | 353 | I-0298 | B | 1.54 | 461 |
| I-0259 | B | 1.63 | 362 | I-0299 | B | 2.35 | 461 |
| I-0260 | B | 1.57 | 369 | I-0300 | E | 1.60 | 461 |
| I-0261 | B | 1.77 | 381 | I-0301 | E | 1.89 | 461 |
| I-0262 | B | 2.00 | 393 | I-0302 | E | 2.02 | 463 |
| I-0263 | B | 2.04 | 399 | I-0303 | A | 2.13 | 466 |
| I-0264 | E | 1.85 | 406 | I-0304 | B | 2.49 | 466 |
| I-0265 | B | 2.03 | 407 | I-0305 | A | 2.13 | 466 |
| I-0266 | B | 2.31 | 408 | I-0306 | A | 2.55 | 466 |
| I-0267 | B | 1.70 | 412 | I-0307 | A | 2.46 | 466 |
| I-0268 | B | 1.53 | 416 | I-0308 | B | 1.44 | 468 |
| I-0269 | B | 2.14 | 421 | I-0309 | B | 1.61 | 469 |
| I-0270 | B | 1.80 | 422 | I-0310 | B | 1.88 | 470 |
| I-0271 | B | 1.75 | 429 | I-0311 | A | 2.60 | 471 |
| I-0272 | B | 2.12 | 433 | I-0312 | A | 2.76 | 471 |
| I-0273 | B | 2.22 | 433 | I-0313 | A | 2.74 | 471 |
| I-0274 | B | 1.75 | 435 | I-0314 | A | 2.35 | 473 |
| I-0275 | B | 1.64 | 435 | I-0315 | B | 1.69 | 474 |
| I-0276 | B | 2.24 | 435 | I-0316 | B | 2.03 | 474 |
| I-0277 | E | 1.89 | 437 | I-0317 | B | 1.70 | 477 |
| I-0278 | B | 1.98 | 438 | I-0318 | A | 2.21 | 478 |
| I-0279 | B | 2.00 | 438 | I-0319 | A | 2.11 | 478 |
| I-0280 | A | 2.30 | 441 | I-0320 | A | 2.17 | 478 |
| I-0281 | B | 1.34 | 445 | I-0321 | A | 2.20 | 480 |
| I-0282 | B | 1.92 | 445 | I-0322 | B | 2.03 | 481 |
| I-0283 | E | 2.69 | 446 | I-0323 | A | 1.99 | 482 |
| I-0284 | B | 2.72 | 449 | I-0324 | B | 1.72 | 483 |
| I-0285 | B | 1.99 | 449 | I-0325 | B | 2.15 | 484 |
| I-0286 | B | 1.50 | 451 | I-0326 | A | 2.71 | 486 |
| I-0287 | B | 1.24 | 452 | I-0327 | A | 2.32 | 487 |
| I-0288 | A | 2.16 | 452 | I-0328 | B | 1.74 | 488 |
| I-0289 | A | 2.29 | 452 | I-0329 | E | 1.84 | 488 |
| I-0290 | B | 2.16 | 454 | I-0330 | A | 2.09 | 489 |
| I-0291 | A | 2.62 | 455 | I-0331 | A | 2.12 | 490 |
| I-0292 | A | 2.47 | 457 | I-0332 | A | 2.33 | 492 |
| I-0293 | A | 2.41 | 457 | I-0333 | A | 2.13 | 502 |
| I-0294 | A | 2.23 | 458 | I-0334 | B | 1.61 | 504 |
| I-0295 | B | 1.89 | 457 | I-0335 | A | 2.07 | 505 |
| I-0296 | B | 2.50 | 459 | I-0336 | A | 2.03 | 505 |
| I-0297 | A | 2.37 | 461 | I-0337 | A | 2.08 | 508 |

**[Table 97]**

| Example No | LC/MS method | RT | MS | Example No | LC/MS method | RT | MS | Example No | LC/MS methods | RT | MS |
|---|---|---|---|---|---|---|---|---|---|---|---|
| I-0338 | A | 214 | 508 | I-0387 | E | 2.23 | 532 | I-0436 | A | 1.83 | 447 |
| I-0339 | B | 2.00 | 512 | I-0388 | A | 2.12 | 321 | I-0437 | A | 1.50 | 448 |
| I-0340 | A | 2.02 | 515 | I-0389 | A | 1.52 | 384 | I-0438 | A | 1.47 | 448 |
| I-0341 | B | 2.68 | 516 | I-0390 | A | 1.83 | 393 | I-0439 | A | 1.65 | 448 |
| I-0342 | A | 2.11 | 519 | I-0391 | A | 1.78 | 403 | I-0440 | B | 1.21 | 449 |
| I-0343 | A | 2.04 | 519 | I-0392 | A | 1.84 | 405 | I-0441 | A | 1.65 | 449 |
| I-0344 | A | 2.44 | 520 | I-0393 | A | 1.58 | 407 | I-0442 | A | 1.52 | 450 |
| I-0345 | B | 2.18 | 520 | I-0394 | A | 1.46 | 407 | I-0443 | A | 1.36 | 452 |
| I-0346 | A | 1.81 | 521 | I-0395 | A | 1.74 | 409 | I-0444 | A | 1.93 | 452 |
| I-0347 | A | 1.92 | 525 | I-0396 | A | 1.81 | 412 | I-0445 | A | 1.23 | 453 |
| I-0348 | A | 2.17 | 525 | I-0397 | A | 1.35 | 412 | I-0446 | A | 1.21 | 454 |
| I-0349 | A | 2.28 | 526 | I-0398 | A | 1.27 | 412 | I-0447 | A | 1.75 | 456 |
| I-0350 | A | 2.15 | 533 | I-0399 | A | 1.31 | 414 | I-0448 | A | 1.97 | 456 |
| I-0351 | A | 2.16 | 533 | I-0400 | A | 1.39 | 414 | I-0449 | A | 1.80 | 456 |
| I-0352 | A | 2.33 | 534 | I-0401 | A | 1.80 | 415 | I-0450 | A | 1.48 | 458 |
| I-0353 | A | 2.30 | 535 | I-0402 | A | 1.36 | 416 | I-0451 | A | 1.44 | 458 |
| I-0354 | A | 2.19 | 536 | I-0403 | B | 1.74 | 416 | I-0452 | A | 1.61 | 458 |
| I-0355 | A | 2.14 | 540 | I-0404 | A | 1.55 | 417 | I-0453 | A | 1.89 | 459 |
| I-0356 | A | 2.14 | 548 | I-0405 | A | 2.01 | 417 | I-0454 | A | 1.62 | 459 |
| I-0357 | A | 1.87 | 549 | I-0406 | A | 1.41 | 418 | I-0455 | A | 2.07 | 459 |
| I-0358 | A | 2.12 | 551 | I-0407 | A | 2.30 | 419 | I-0456 | A | 1.49 | 460 |
| I-0359 | A | 2.32 | 555 | I-0408 | A | 1.70 | 419 | I-0457 | A | 2.22 | 461 |
| I-0360 | B | 1.97 | 559 | I-0409 | A | 1.68 | 420 | I-0458 | A | 1.54 | 462 |
| I-0361 | A | 1.99 | 560 | I-0410 | A | 1.67 | 421 | I-0459 | A | 1.52 | 463 |
| I-0362 | A | 2.18 | 560 | I-0411 | A | 1.57 | 421 | I-0460 | A | 1.44 | 466 |
| I-0363 | A | 2.19 | 562 | I-0412 | A | 1.70 | 423 | I-0461 | A | 2.01 | 469 |
| I-0364 | A | 2.22 | 562 | I-0413 | A | 1.62 | 423 | I-0462 | A | 2.17 | 471 |
| I-0365 | A | 1.80 | 562 | I-0414 | A | 1.23 | 423 | I-0463 | A | 1.51 | 472 |
| I-0366 | A | 238 | 569 | I-0415 | A | 2.03 | 425 | I-0464 | A | 1.60 | 472 |
| I-0367 | B | 2.13 | 571 | I-0416 | A | 1.75 | 426 | I-0465 | A | 1.64 | 472 |
| I-0368 | A | 2.47 | 580 | I-0417 | A | 1.34 | 426 | I-0466 | A | 1.49 | 473 |
| I-0369 | A | 2.57 | 582 | I-0418 | A | 1.27 | 426 | I-0467 | A | 1.82 | 473 |
| I-0370 | A | 2.35 | 583 | I-0419 | A | 1.63 | 431 | I-0468 | A | 1.78 | 474 |
| I-0371 | B | 1.82 | 364 | I-0420 | A | 1.54 | 431 | I-0469 | A | 1.73 | 474 |
| I-0372 | B | 1.75 | 398 | I-0421 | A | 1.53 | 432 | I-0470 | B | 1.67 | 475 |
| I-0373 | B | 1.79 | 410 | I-0422 | A | 1.66 | 433 | I-0471 | B | 1.22 | 477 |
| I-0374 | B | 1.86 | 412 | I-0423 | A | 1.58 | 433 | I-0472 | A | 1.45 | 480 |
| I-0375 | B | 2.08 | 419 | I-0424 | A | 1.98 | 435 | I-0473 | A | 1.57 | 482 |
| I-0376 | B | 1.93 | 442 | I-0425 | A | 1.58 | 435 | I-0474 | A | 1.61 | 482 |
| I-0377 | E | 1.54 | 443 | I-0426 | A | 1.66 | 437 | I-0475 | A | 1.78 | 483 |
| I-0378 | B | 1.76 | 459 | I-0427 | E | 2.09 | 439 | I-0476 | A | 1.52 | 494 |
| I-0379 | B | 2.03 | 468 | I-0428 | A | 118 | 439 | I-0477 | A | 2.58 | 495 |
| I-0380 | B | 1.34 | 475 | I-0429 | A | 1.33 | 439 | I-0478 | A | 1.92 | 496 |
| I-0381 | B | 1.30 | 482 | I-0430 | A | 1.20 | 441 | I-4479 | A | 1.55 | 501 |
| I-0382 | B | 2.30 | 486 | I-0431 | A | 1.50 | 442 | I-0480 | A | 2.07 | 501 |
| I-0383 | B | 1.57 | 491 | I-0432 | A | 1.76 | 444 | I-0481 | A | 2.08 | 502 |
| I-0384 | B | 1.37 | 491 | I-0433 | B | 1.80 | 444 | I-0482 | B | 1.71 | 503 |
| I-0385 | B | 1.61 | 492 | I-0434 | A | 1.61 | 445 | I-0483 | A | 1.58 | 508 |
| I-0386 | B | 1.85 | 501 | I-0435 | A | 1.54 | 445 | I-0484 | A | 2.03 | 511 |

**[Table 98]**

| Example No | LC/MS method | RT | MS | Example No | LC/MS method | RT | MS |
|---|---|---|---|---|---|---|---|
| I-0485 | A | 2.03 | 512 | I-0509 | A | 1.33 | 422 |
| I-0486 | A | 1.90 | 513 | I-0510 | A | 1.23 | 433 |
| I-0487 | A | 1.92 | 517 | I-0511 | A | 1.90 | 462 |
| I-0488 | A | 2.01 | 520 | I-0512 | A | 1.77 | 503 |
| I-0489 | A | 2.01 | 523 | I-0513 | A | 1.37 | 424 |
| I-0490 | A | 2.09 | 524 | I-0514 | A | 1.69 | 426 |
| I-0491 | A | 1.99 | 532 | I-0515 | A | 1.24 | 408 |
| I-0492 | A | 2.22 | 532 | I-0516 | A | 1.19 | 449 |
| I-0493 | A | 2.02 | 532 | I-0517 | A | 1.27 | 473 |
| I-0494 | A | 2.40 | 533 | I-0518 | A | 1.38 | 410 |
| I-0495 | A | 2.13 | 535 | I-0519 | A | 1.24 | 422 |
| I-0496 | A | 2.36 | 543 | I-0520 | A | 1.50 | 4.06 |
| I-0497 | A | 1.97 | 550 | I-0521 | A | 1.30 | 473 |
| I-0498 | A | 1.96 | 550 | I-0522 | A | 1.15 | 449 |
| I-0499 | B | 1.13 | 579 | I-0523 | A | 1.34 | 450 |
| I-0500 | A | 2.11 | 593 | I-0524 | A | 1.31 | 449 |
| I-0501 | A | 2.29 | 600 | I-0525 | A | 1.73 | 408 |
| I-0502 | A | 1.18 | 451 | I-0526 | A | 1.22 | 463 |
| I-0503 | A | 1.49 | 394 | I-0527 | A | 1.43 | 417 |
| I-0504 | A | 1.10 | 409 | I-0528 | A | 1.31 | 436 |
| I-0505 | A | 1.19 | 464 | I-0529 | A | 1.26 | 492 |
| I-0506 | A | 2.12 | 534 | I-0530 | A | 1.29 | 424 |
| I-0507 | A | 176 | 502 | I-0531 | A | 1.25 | 436 |
| I-0508 | A | 1.49 | 474 | | | | |

NMR data of Compound I-0004:
¹H-NMR (CDCl₃) 8 8.02 (m, 2H), 7.35 (m, 3H), 7.29 (d, J = 1.0 Hz, 2H), 7.27 (d , J = 0.7 Hz, 2H), 6.44 (s, 1H), 5.27 (s, 2H), 4.62 (t, J = 4.9 Hz, 1H), 3.41 (td, J = 6.9, 5.3 Hz, 2H), 1.45 (m, 2H), 1.19 (m, 2H), 1.04 (m, 2H), 0.82 (t, J = 7.3 Hz, 3H).

Similarly, the following compounds were synthesized.
Physical constants of the compound are shown below. RT means retention time (min), MS means [M + H]⁺.

**[Table 99]**

| Example No | Structure | LC/MS method | RT | MS |
|---|---|---|---|---|
| II-0001 | 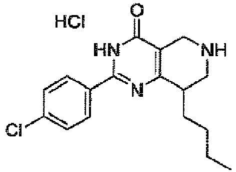 | B | 1.39 | 318 |
| II-0002 | 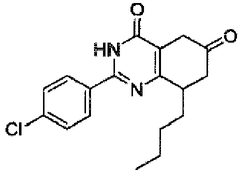 | C | 2.24 | 331 |
| II-0003 | 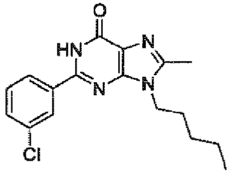 | A | 2.13 | 331 |
| II-0004 | 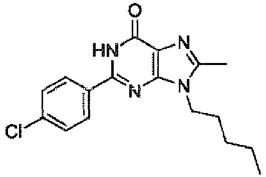 | A | 2.13 | 331 |
| II-0005 | 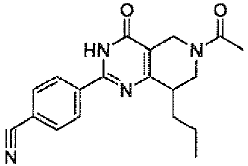 | B | 1.66 | 337 |

**[Table 100]**

| Example No | Structure | LC/MS method | RT | MS |
|---|---|---|---|---|
| II-0006 | 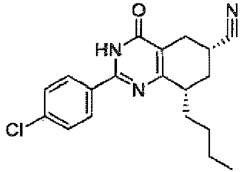 | C | 2.44 | 342 |
| II-0007 | 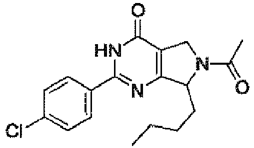 | C | 1.93 | 346 |
| II-0008 | 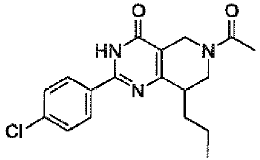 | B | 1.93 | 346 |
| II-0009 | 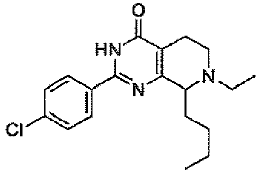 | B | 1.75 | 346 |
| II-0010 | 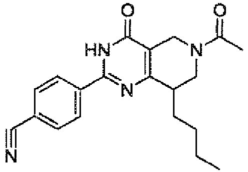 | B | 1.82 | 351 |

**[Table 101]**

| Example No | Structure | LC/MS method | RT | MS |
|---|---|---|---|---|
| II-0011 | 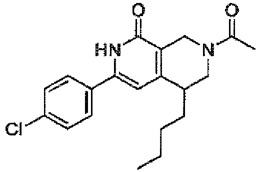 | C | 2.07 | 359 |
| II-0012 | 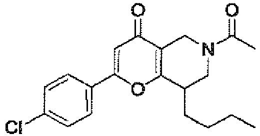 | C | 2.22 | 360 |
| II-0013 | 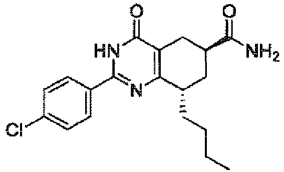 | C | 2.03 | 360 |
| II-0014 | 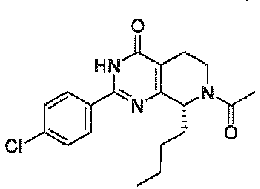 | B | 2.01 | 360 |
| II-0015 | 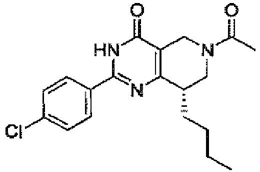 | B | 2.07 | 360 |

**[Table 102]**

| Example No | Structure | LC/MS method | RT | MS |
|---|---|---|---|---|
| II-0016 | 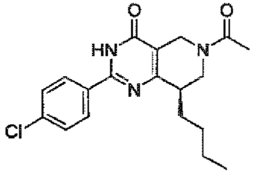 | B | 2.07 | 360 |
| II-0017 | 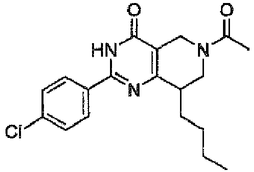 | B | 2.08 | 360 |
| II-0018 | 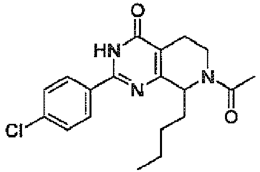 | A | 2.01 | 360 |
| II-0019 | 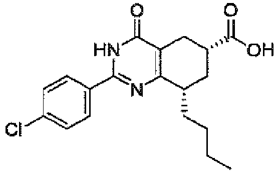 | C | 2.25 | 361 |
| II-0020 | 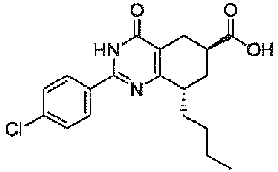 | C | 2.21 | 361 |

**[Table 103]**

| Example No | Structure | LC/MS method | RT | MS |
|---|---|---|---|---|
| II-0021 | 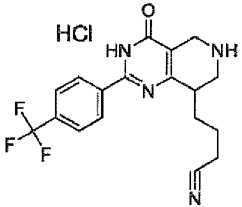 | B | 1.38 | 363 |
| II-0022 | 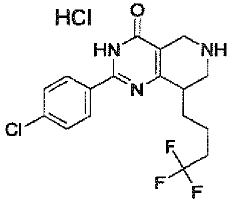 | B | 1.56 | 372 |
| II-0023 | 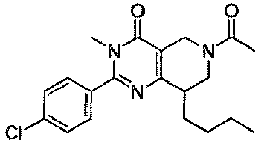 | C | 2.16 | 374 |
| II-0024 | 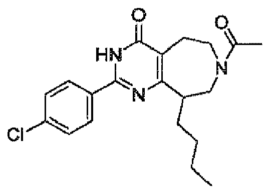 | C | 2.06 | 374 |
| II-0025 | 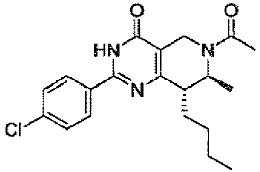 | B | 2.07 | 374 |

**[Table 104]**

| Example No | Structure | LC/MS method | RT | MS |
|---|---|---|---|---|
| II-0026 | 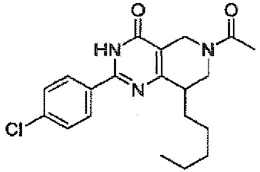 | B | 2.23 | 374 |
| II-0027 | 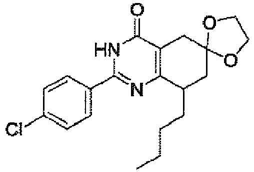 | C | 2.48 | 375 |
| II-0028 | 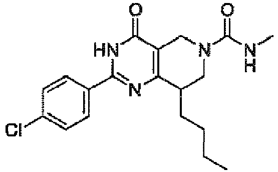 | B | 2.04 | 375 |
| II-0029 | 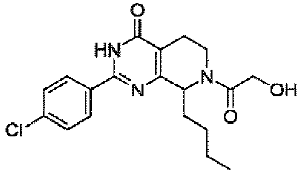 | A | 1.89 | 376 |
| II-0030 | 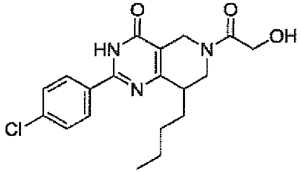 | A | 1.94 | 376 |

**[Table 105]**

| Example No | Structure | LC/MS method | RT | MS |
|---|---|---|---|---|
| II-0031 | 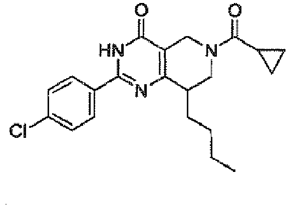 | B | 2.30 | 386 |
| II-0032 | 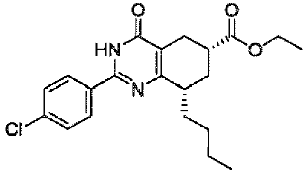 | C | 2.71 | 389 |
| II-0033 | 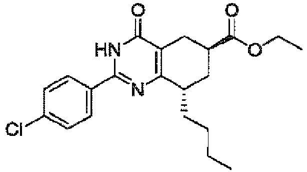 | C | 2.65 | 389 |
| II-0034 | 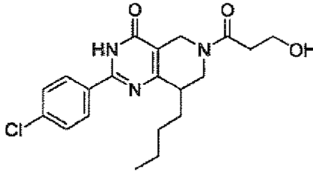 | A | 1.88 | 390 |
| II-0035 | 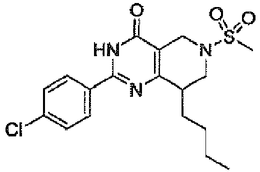 | B | 2.24 | 396 |

**[Table 106]**

| Example No | Structure | LC/MS method | RT | MS |
|---|---|---|---|---|
| II-0036 | 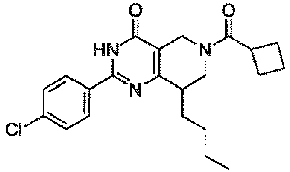 | A | 2.47 | 400 |
| II-0037 | 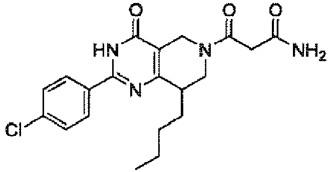 | A | 1.77 | 403 |
| II-0038 | 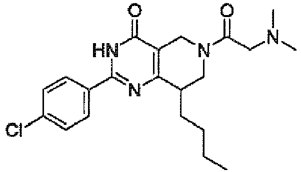 | A | 1.60 | 403 |
| II-0039 | 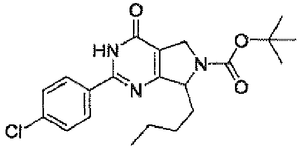 | C | 2.64 | 404 |
| II-0040 | 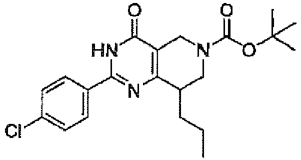 | A | 2.70 | 404 |

**[Table 107]**

| Example No | Structure | LC/MS method | RT | MS |
|---|---|---|---|---|
| II-0041 | 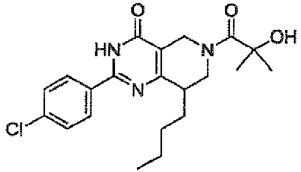 | A | 2.10 | 404 |
| II-0042 | 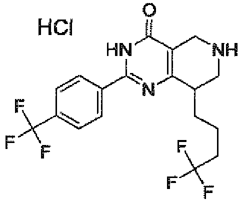 | B | 1.67 | 406 |
| II-0043 | 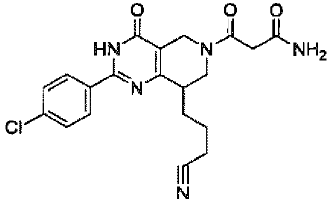 | B | 1.48 | 414 |
| II-0044 | 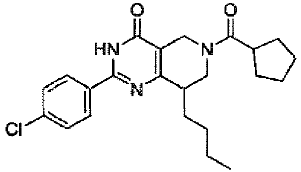 | A | 2.60 | 414 |
| II-0045 | 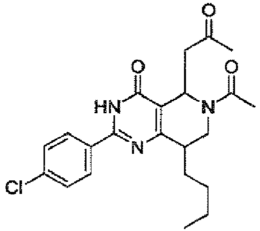 | B | 2.12 | 416 |
| | | | 2.21 | |

**[Table 108]**

| Example No | Structure | LC/MS method | RT | MS |
|---|---|---|---|---|
| II-0046 | 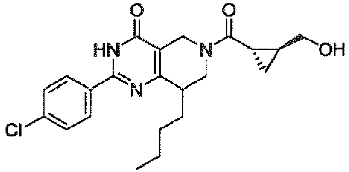 | A | 1.94 | 416 |
| II-0047 | 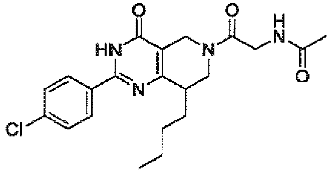 | A | 1.84 | 417 |
| II-0048 | 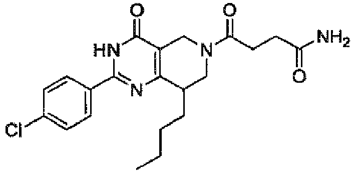 | A | 1.80 | 417 |
| II-0049 | 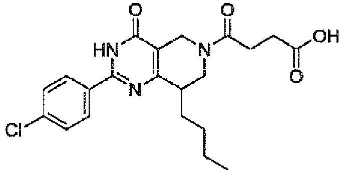 | B | 2.00 | 418 |
| II-0050 | 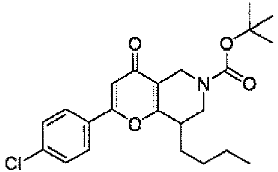 | C | 2.82 | 418 |

**[Table 109]**

| Example No | Structure | LC/MS method | RT | MS |
|---|---|---|---|---|
| II-0051 | 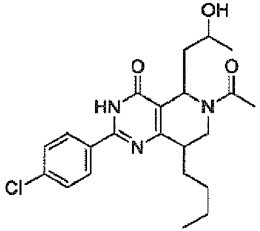 | B | 2.17 | 418 |
| II-0052 | 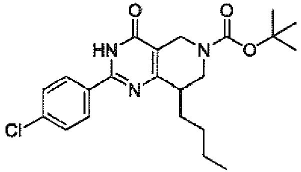 | B | 2.74 | 418 |
| II-0053 | 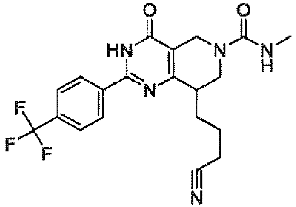 | B | 1.71 | 420 |
| II-0054 | 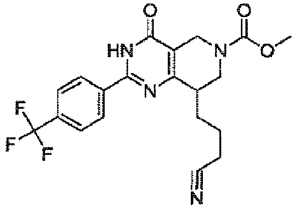 | B | 1.81 | 421 |
| II-0055 | 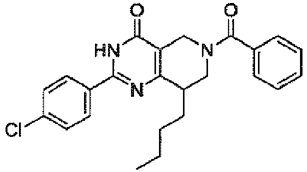 | A | 2.51 | 422 |

**[Table 110]**

| Example No | Structure | LC/MS method | RT | MS |
|---|---|---|---|---|
| II-0056 | 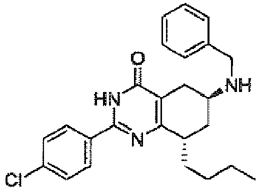 | C | 1.86 | 422 |
| II-0057 | 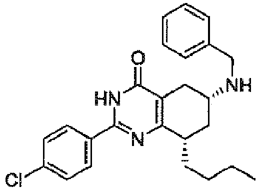 | C | 1.86 | 422 |
| II-0058 | 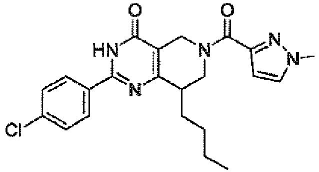 | A | 2.19 | 426 |
| II-0059 | 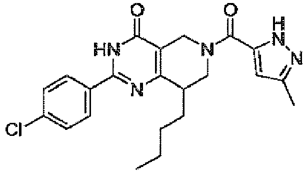 | A | 2.08 | 426 |
| II-0060 | 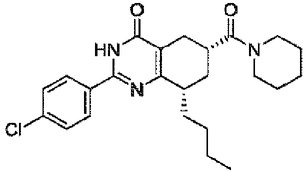 | C | 2.57 | 428 |

**[Table 111]**

| Example No | Structure | LC/MS method | RT | MS |
|---|---|---|---|---|
| II-0061 | 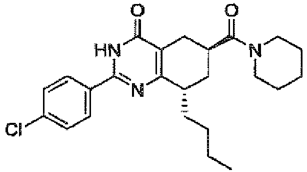 | C | 2.55 | 428 |
| II-0062 | 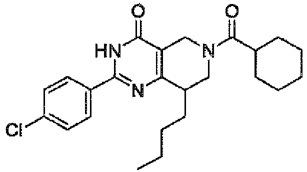 | A | 2.71 | 428 |
| II-0063 | 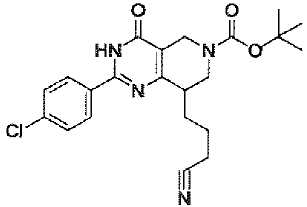 | B | 2.09 | 429 |
| II-0064 | 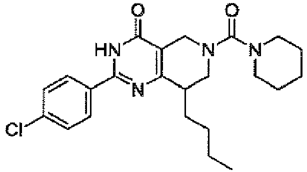 | B | 2.57 | 429 |
| II-0065 | 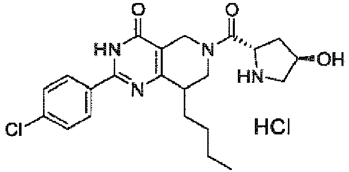 | B | 1.60 | 431 |

**[Table 112]**

| Example No | Structure | LC/MS method | RT | MS |
|---|---|---|---|---|
| II-0066 | 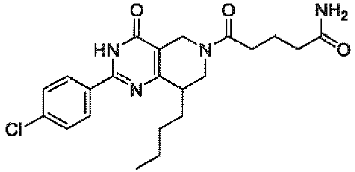 | A | 1.81 | 431 |
| II-0067 | 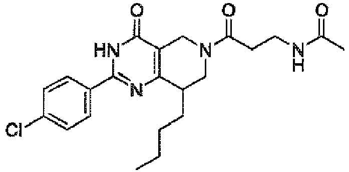 | A | 1.84 | 431 |
| II-0068 | 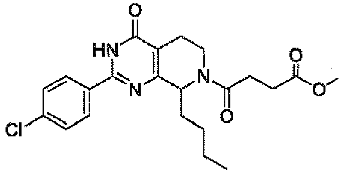 | A | 2.14 | 432 |
| II-0069 | 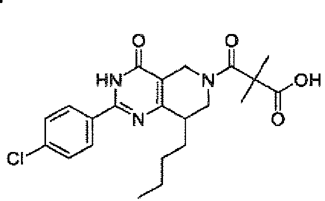 | A | 2.15 | 432 |
| II-0070 | 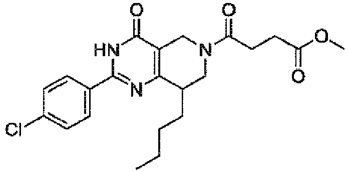 | A | 2.20 | 432 |

**[Table 113]**

| Example No | Structure | LC/MS method | RT | MS |
|---|---|---|---|---|
| II-0071 | 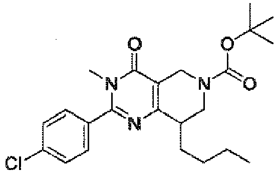 | C | 2.75 | 432 |
| II-0072 | 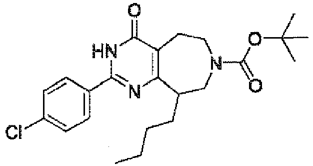 | C | 2.74 | 432 |
| II-0073 | 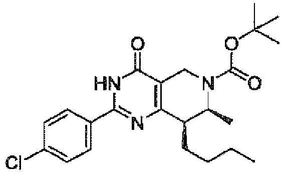 | C | 2.80 | 432 |
| II-0074 | 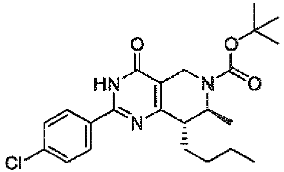 | C | 2.78 | 432 |
| II-0075 | 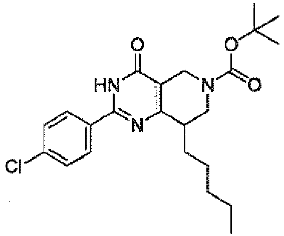 | B | 2.89 | 432 |

**[Table 114]**

| Example No | Structure | LC/MS method | RT | MS |
|---|---|---|---|---|
| II-0076 | 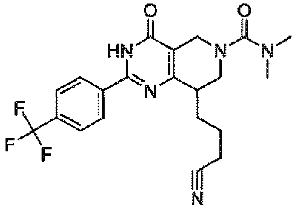 | B | 1.84 | 434 |
| II-0077 | 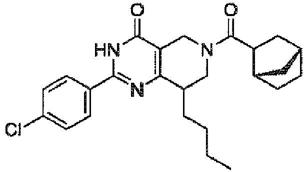 | A | 2.83 | 440 |
| II-0078 | 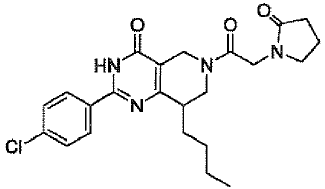 | A | 1.97 | 443 |
| II-0079 | 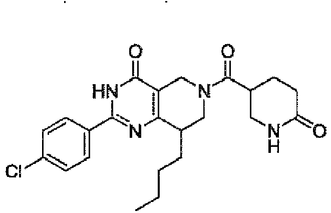 | A | 1.83 | 443 |
| II-0080 | 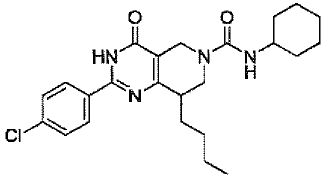 | B | 2.49 | 443 |

**[Table 115]**

| Example No | Structure | LC/MS method | RT | MS |
|---|---|---|---|---|
| II-0081 | 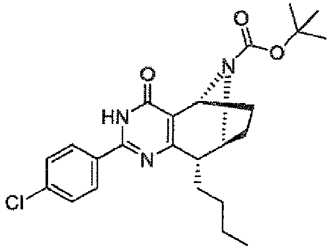 | B | 2.80 | 444 |
| II-0082 | 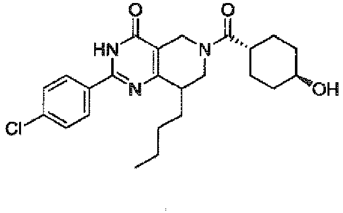 | A | 1.97 | 444 |
| II-0083 | 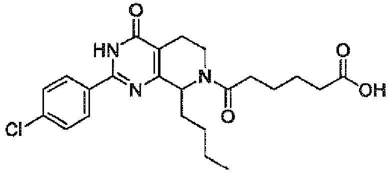 | A | 1.98 | 446 |
| II-0084 | 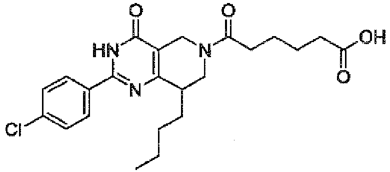 | A | 2.04 | 446 |
| II-0085 | 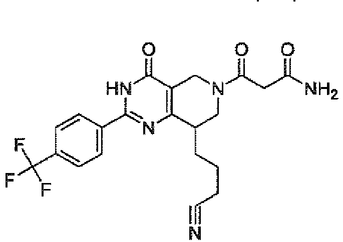 | B | 1.46 | 448 |

**[Table 116]**

| Example No | Structure | LC/MS method | RT | MS |
|---|---|---|---|---|
| II-0086 | 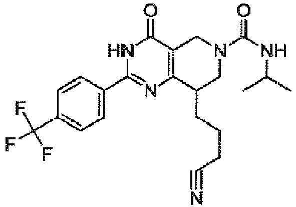 | B | 1.89 | 448 |
| II-0087 | 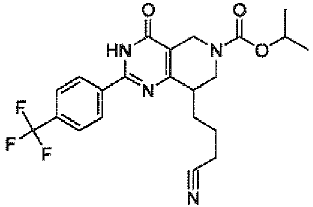 | B | 2.03 | 449 |
| II-0088 | 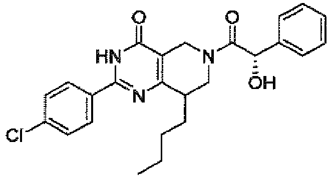 | A | 2.40 | 452 |
| II-0089 | 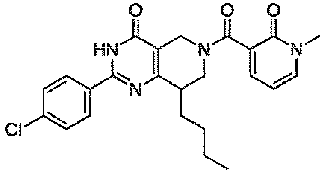 | A | 1.92 | 453 |
| II-0090 | 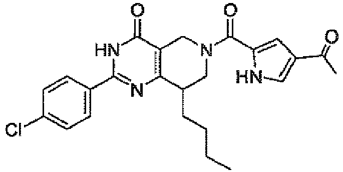 | A | 2.12 | 453 |

**[Table 117]**

| Example No | Structure | LC/MS method | RT | MS |
|---|---|---|---|---|
| II-0091 | 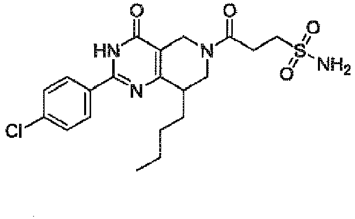 | A | 1.91 | 453 |
| II-0092 | 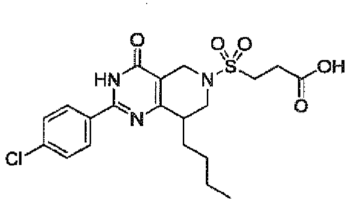 | B | 2.13 | 454 |
| II-0093 | 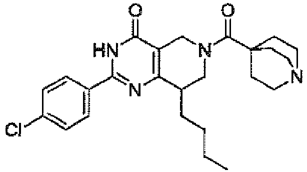 | A | 1.71 | 455 |
| II-0094 | 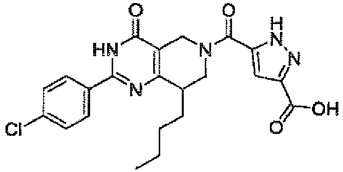 | A | 1.89 | 456 |
| II-0095 | 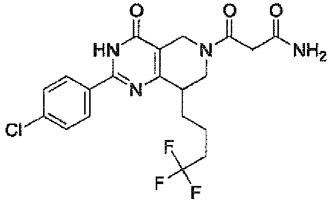 | B | 1.74 | 457 |

**[Table 118]**

| Example No | Structure | LC/MS method | RT | MS |
|---|---|---|---|---|
| II-0096 | 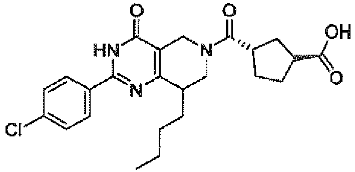 | A | 2.13 | 458 |
| II-0097 | 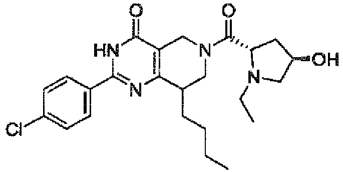 | B | 1.71 | 459 |
| II-0098 | 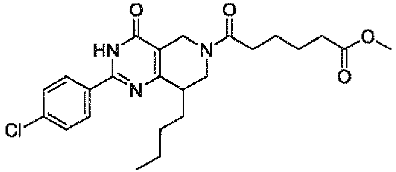 | A | 2.32 | 460 |
| II-0099 | 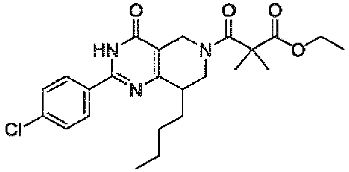 | A | 2.36 | 460 |
| II-0100 | 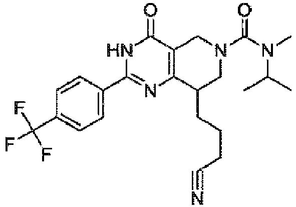 | B | 2.04 | 462 |

**[Table 119]**

| Example No | Structure | LC/MS method | RT | MS |
|---|---|---|---|---|
| II-0101 | 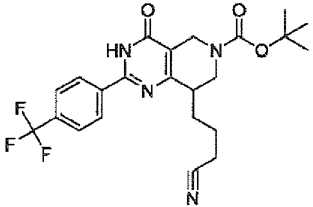 | B | 2.18 | 463 |
| II-0102 | 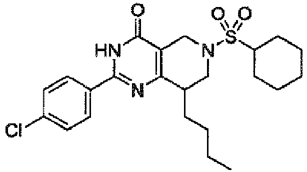 | B | 2.68 | 464 |
| II-0103 | 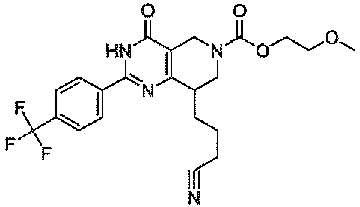 | B | 1.91 | 465 |
| II-0104 | 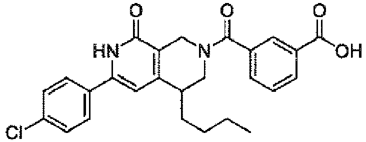 | C | 2.12 | 465 |
| II-0105 | 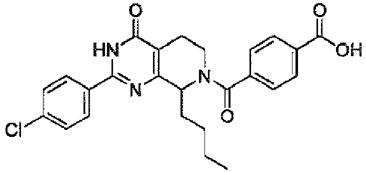 | A | 2.12 | 466 |

**[Table 120]**

| Example No | Structure | LC/MS method | RT | MS |
|---|---|---|---|---|
| II-0106 | 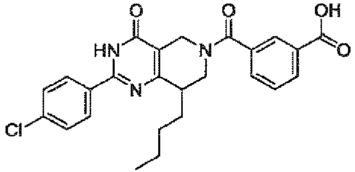 | A | 2.13 | 466 |
| II-0107 | 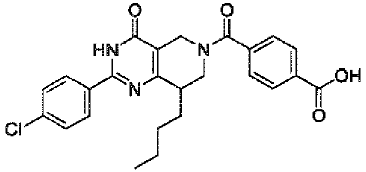 | A | 2.11 | 466 |
| II-0108 | 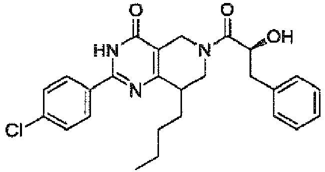 | A | 2.42 | 466 |
| II-0109 | 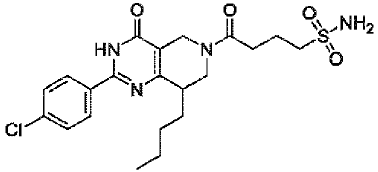 | A | 1.92 | 467 |
| II-0110 | 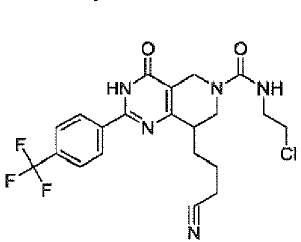 | B | 1.88 | 468 |

**[Table 121]**

| Example No | Structure | LC/MS method | RT | MS |
|---|---|---|---|---|
| II-0111 | 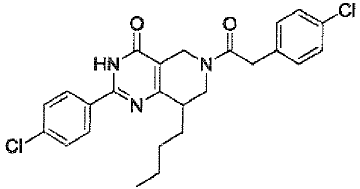 | A | 2.70 | 470 |
| II-0112 | 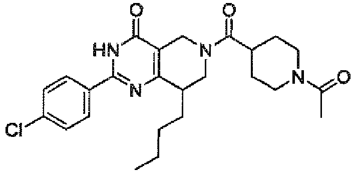 | A | 1.98 | 471 |
| II-0113 | 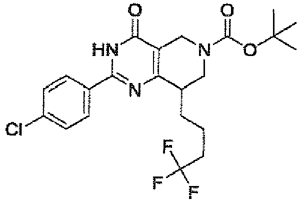 | B | 2.55 | 472 |
| II-0114 | 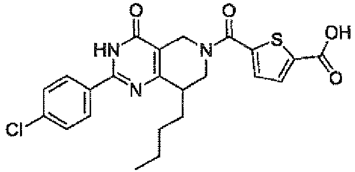 | A | 2.16 | 472 |
| II-0115 | 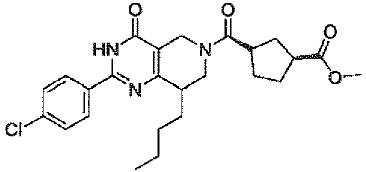 | A | 2.41 | 472 |

**[Table 122]**

| Example No | Structure | LC/MS method | RT | MS |
|---|---|---|---|---|
| II-0116 | 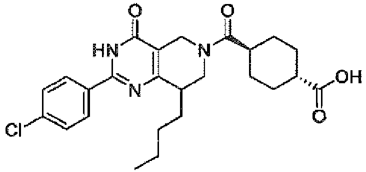 | A | 2.09 | 472 |
| II-0117 | 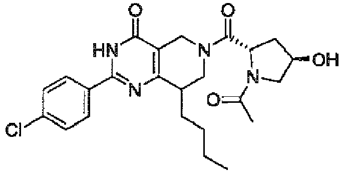 | B | 1.84 | 473 |
| II-0118 | 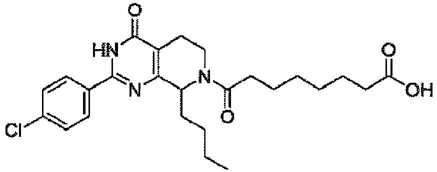 | A | 2.16 | 474 |
| II-0119 | 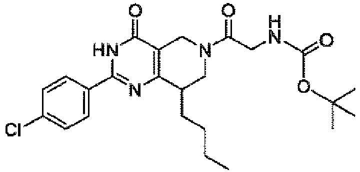 | A | 2.44 | 475 |
| II-0120 | 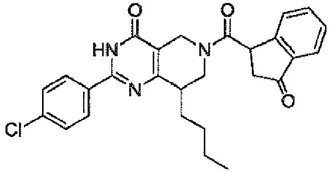 | A | 2.34 | 476 |

**[Table 123]**

| Example No | Structure | LC/MS method | RT | MS |
|---|---|---|---|---|
| II-0121 | 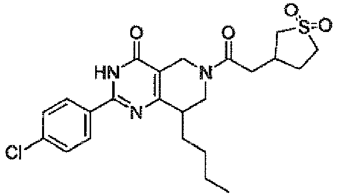 | A | 2.08 | 478 |
| II-0122 | 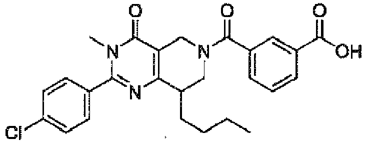 | C | 2.19 | 480 |
| II-0123 | 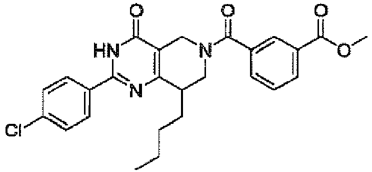 | A | 2.48 | 480 |
| II-0124 | 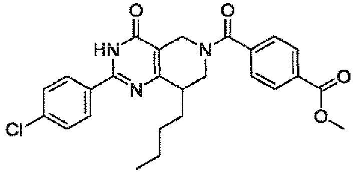 | A | 2.48 | 480 |
| II-0125 | 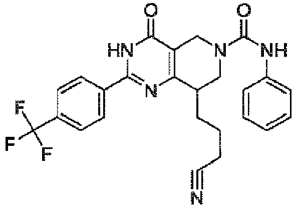 | B | 2.03 | 482 |

**[Table 124]**

| Example No | Structure | LC/MS method | RT | MS |
|---|---|---|---|---|
| II-0126 | 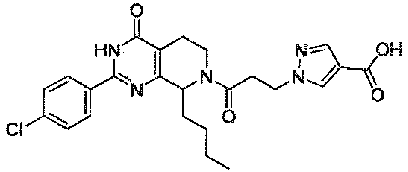 | A | 1.85 | 484 |
| II-0127 | 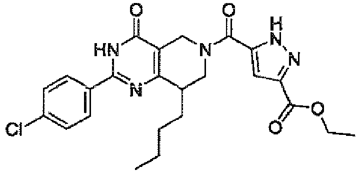 | A | 2.26 | 484 |
| II-0128 | 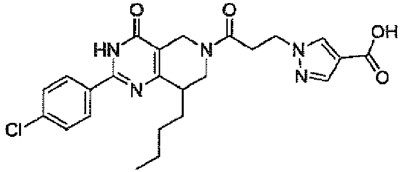 | A | 1.90 | 484 |
| II-0129 | 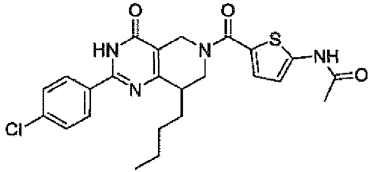 | A | 2.12 | 485 |
| II-0130 | 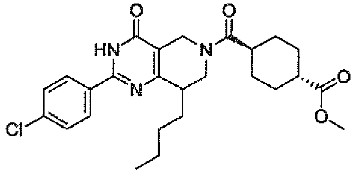 | A | 2.44 | 486 |

**[Table 125]**

| Example No | Structure | LC/MS method | RT | MS |
|---|---|---|---|---|
| II-0131 | 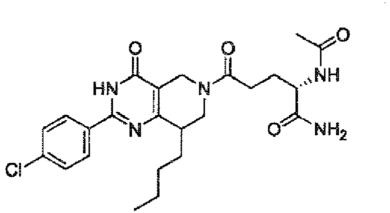 | A | 1.73 | 488 |
| II-0132 | 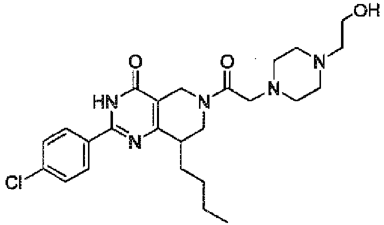 | A | 1.55 | 488 |
| II-0133 | 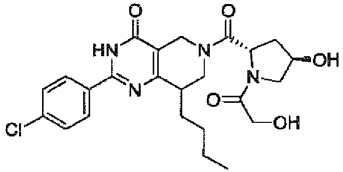 | A | 1.65 | 489 |
| II-0134 | 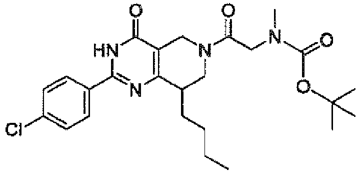 | A | 2.37 | 489 |
| II-0135 | 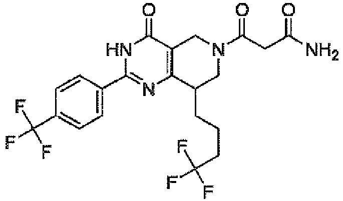 | B | 1.83 | 491 |

**[Table 126]**

| Example No | Structure | LC/MS method | RT | MS |
|---|---|---|---|---|
| II-0136 | 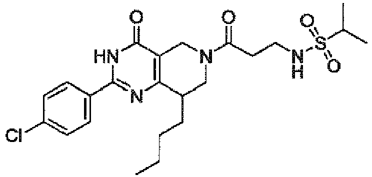 | A | 2.17 | 495 |
| II-0137 | 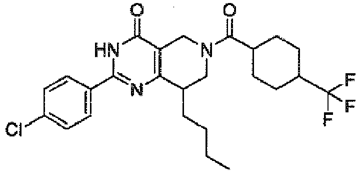 | A | 2.81 | 496 |
| II-0138 | 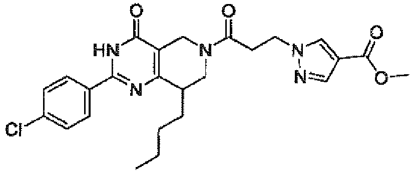 | A | 2.16 | 498 |
| II-0139 | 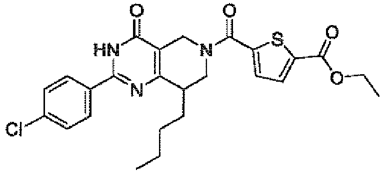 | A | 2.68 | 500 |
| II-0140 | 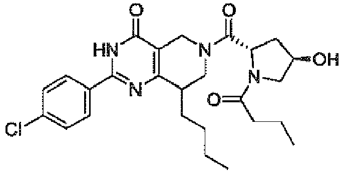 | A | 1.88 | 501 |

**[Table 127]**

| Example No | Structure | LC/MS method | RT | MS |
|---|---|---|---|---|
| II-0141 | 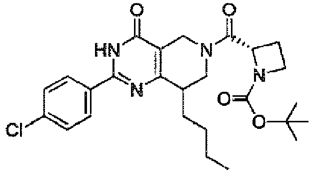 | A | 2.45 | 501 |
| II-0142 | 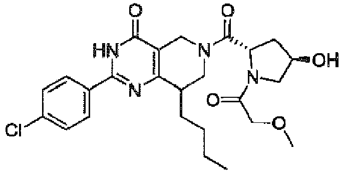 | A | 1.72 | 503 |
| II-0143 | 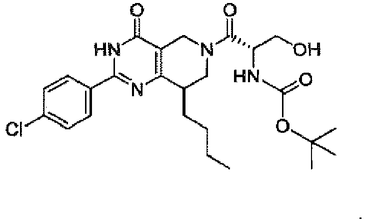 | A | 2.29 | 505 |
| II-0144 | 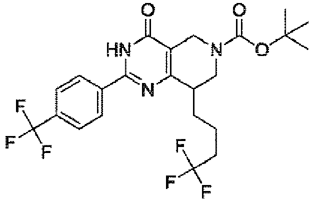 | B | 2.61 | 506 |
| II-0145 | 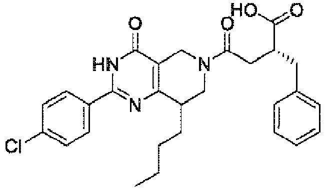 | A | 2.44 | 508 |

**[Table 128]**

| Example No | Structure | LC/MS method | RT | MS |
|---|---|---|---|---|
| II-0146 | 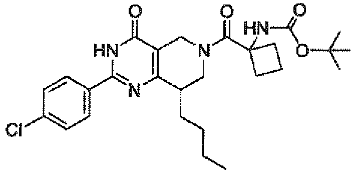 | A | 2.53 | 515 |
| II-0147 | 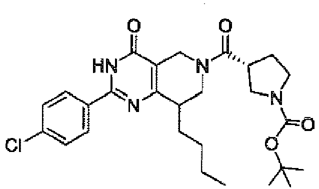 | A | 2.60 | 515 |
| II-0148 | 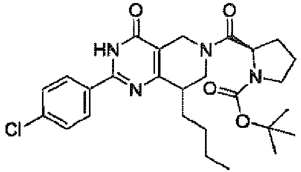 | B | 2.51 | 515 |
| II-0149 | 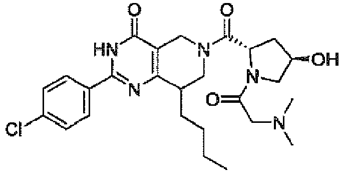 | A | 1.47 | 516 |
| II-0150 | 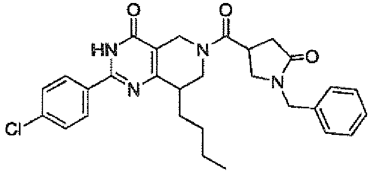 | A | 2.35 | 519 |

**[Table 129]**

| Example No | Structure | LC/MS method | RT | MS |
|---|---|---|---|---|
| II-0151 | 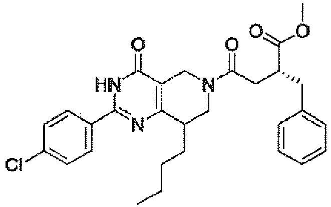 | A | 2.70 | 522 |
| II-0152 | 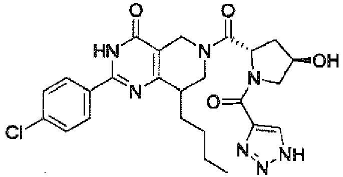 | A | 1.70 | 526 |
| II-0153 | 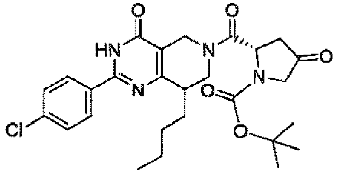 | A | 2.48 | 529 |
| II-0154 | 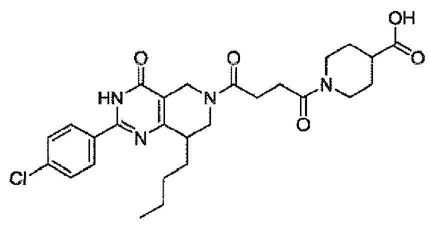 | A | 1.94 | 529 |
| II-0155 | 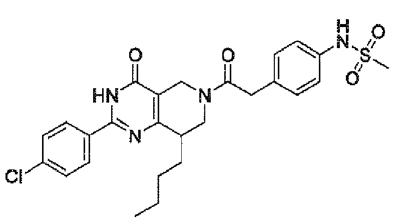 | A | 2.16 | 529 |

**[Table 130]**

| Example No | Structure | LC/MS method | RT | MS |
|---|---|---|---|---|
| II-0156 | 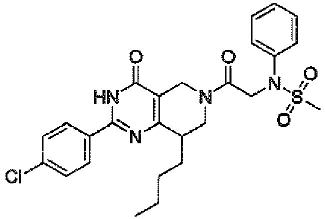 | A | 2.44 | 529 |
| II-0157 | 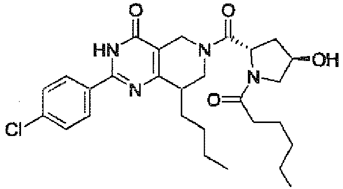 | A | 2.14 | 529 |
| II-0158 | 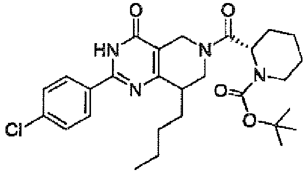 | A | 2.88 | 529 |
| II-0159 | 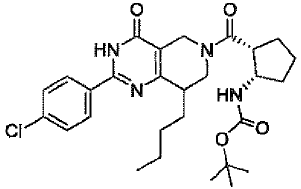 | A | 2.76 | 529 |
| II-0160 | 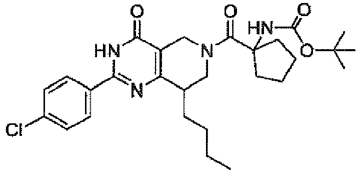 | A | 2.63 | 529 |

**[Table 131]**

| Example No | Structure | LC/MS method | RT | MS |
|---|---|---|---|---|
| II-0161 | 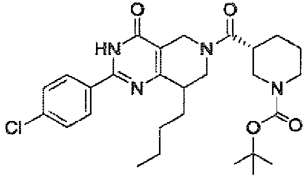 | A | 2.73 | 529 |
| II-0162 | 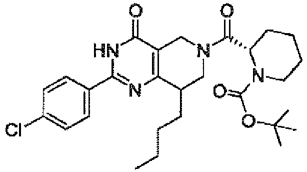 | A | 2.83 | 529 |
| II-0163 | 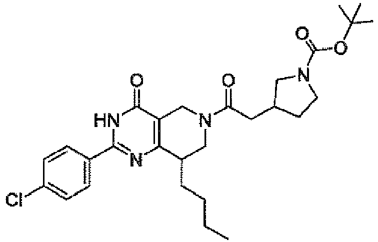 | A | 2.63 | 529 |
| II-0164 | 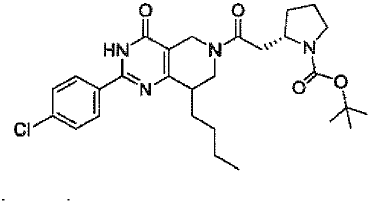 | A | 2.73 | 529 |
| II-0165 | 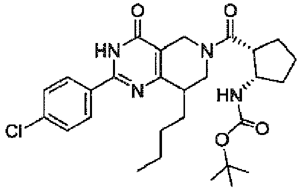 | A | 2.69 | 529 |

**[Table 132]**

| Example No | Structure | LC/MS method | RT | MS |
|---|---|---|---|---|
| II-0166 | 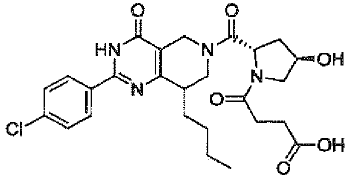 | B | 1.81 | 531 |
| II-0167 | 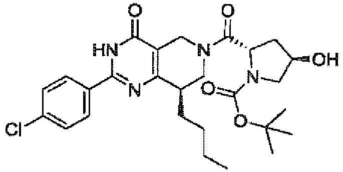 | B | 2.21 | 531 |
| II-0168 | 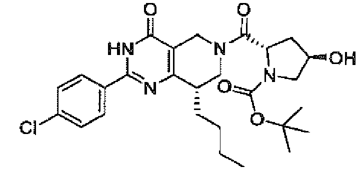 | B | 2.21 | 531 |
| II-0169 | 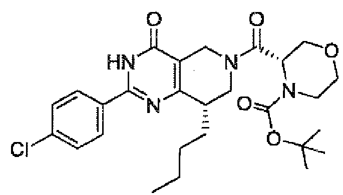 | A | 2.55 | 531 |
| II-0170 | 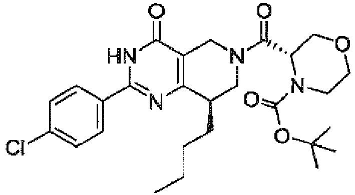 | A | 2.51 | 531 |

**[Table 133]**

| Example No | Structure | LC/MS method | RT | MS |
|---|---|---|---|---|
| II-0171 | 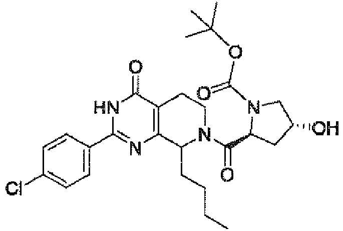 | A | 2.05 | 531 |
| II-0172 | 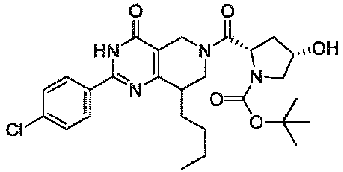 | B | 2.30 | 531 |
| II-0173 | 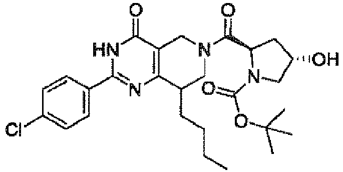 | B | 2.19 | 531 |
| II-0174 | 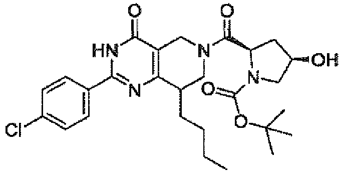 | B | 2.30 | 531 |
| II-0175 | 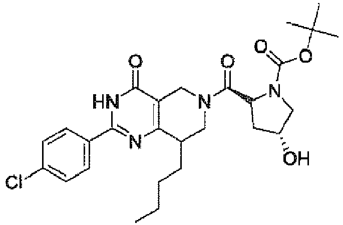 | A | 2.17 | 531 |

**[Table 134]**

| Example No | Structure | LC/MS method | RT | MS |
|---|---|---|---|---|
| II-0176 | 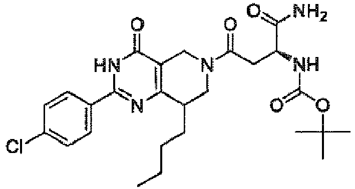 | A | 2.17 | 532 |
| II-0177 | 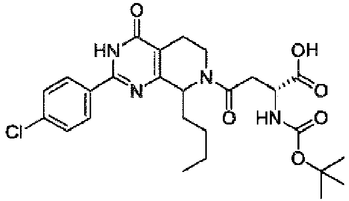 | B | 2.18 | 533 |
| II-0178 | 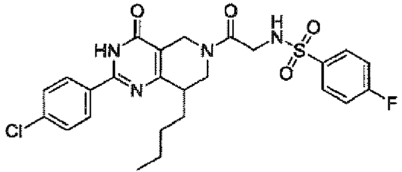 | A | 2.40 | 533 |
| II-0179 | 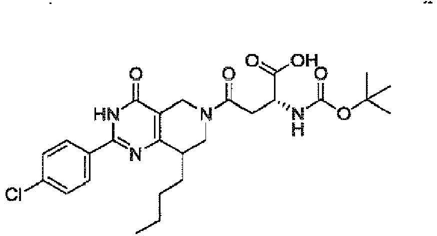 | A | 2.30 | 533 |
| II-0180 | 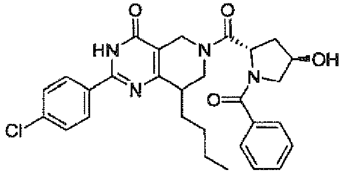 | A | 1.97 | 535 |

**[Table 135]**

| Example No | Structure | LC/MS method | RT | MS |
|---|---|---|---|---|
| II-0181 | 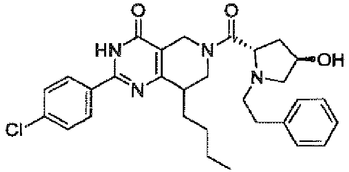 | B | 1.94 | 535 |
| II-0182 | 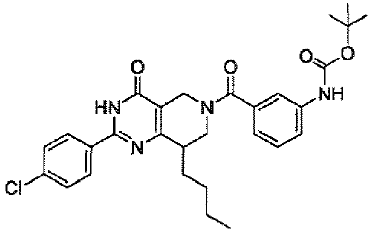 | A | 2.72 | 537 |
| II-0183 | 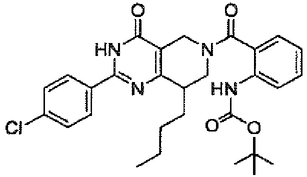 | A | 2.88 | 537 |
| II-0184 | 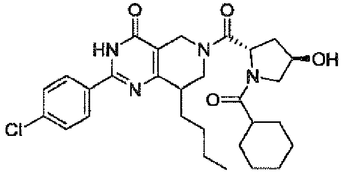 | A | 2.12 | 541 |
| II-0185 | 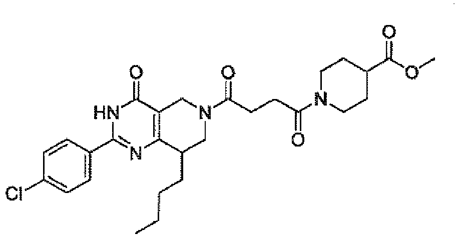 | A | 2.16 | 543 |

**[Table 136]**

| Example No | Structure | LC/MS method | RT | MS |
|---|---|---|---|---|
| II-0186 | 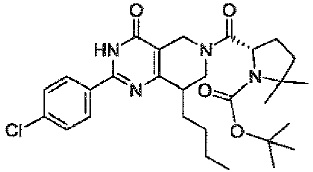 | A | 2.88 | 543 |
| II-0187 | 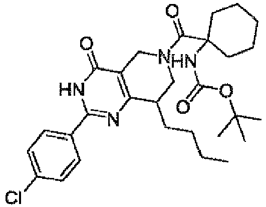 | A | 2.74 | 543 |
| II-0188 | 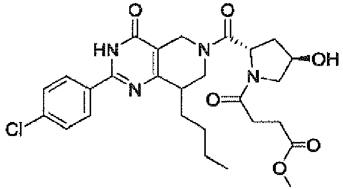 | A | 1.82 | 545 |
| II-0189 | 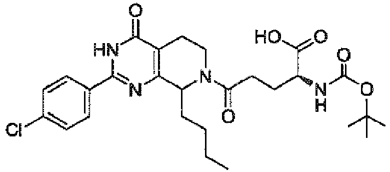 | A | 2.21 | 547 |
| II-0190 | 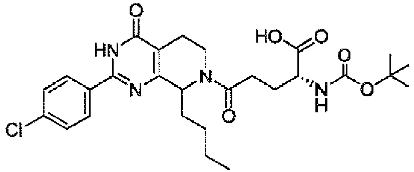 | A | 2.21 | 547 |

**[Table 137]**

| Example No | Structure | LC/MS method | RT | MS |
|---|---|---|---|---|
| II-0191 | 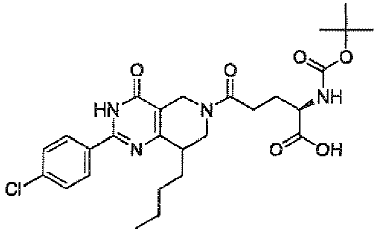 | A | 2.27 | 547 |
| II-0192 | 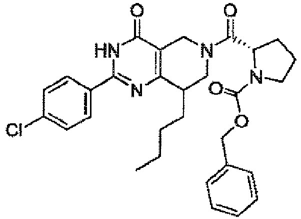 | A | 2.51 | 549 |
| II-0193 | 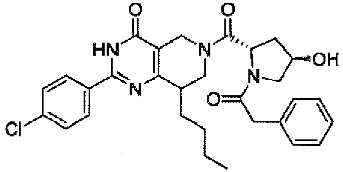 | B | 2.12 | 549 |
| | | | 2.15 | |
| II-0194 | 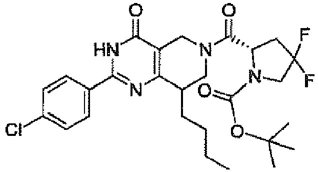 | A | 2.67 | 551 |
| II-0195 | 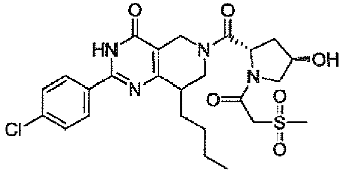 | A | 1.75 | 551 |

**[Table 138]**

| Example No | Structure | LC/MS method | RT | MS |
|---|---|---|---|---|
| II-0196 | 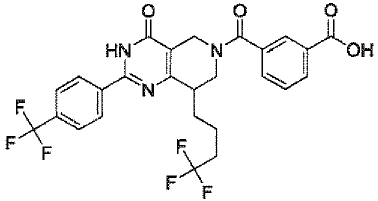 | B | 2.15 | 554 |
| II-0197 | 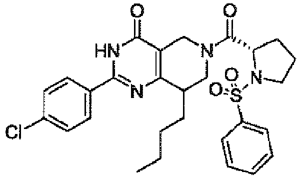 | A | 2.47 | 555 |
| II-0198 | 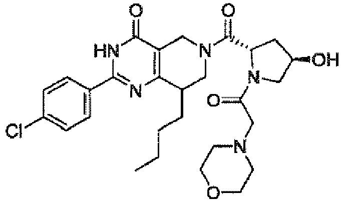 | A | 1.49 | 558 |
| II-0199 | 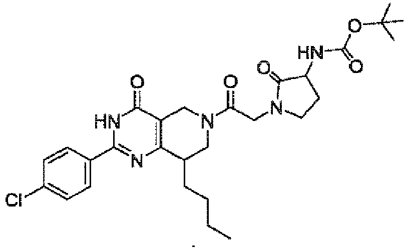 | A | 2.25 | 558 |
| II-0200 | 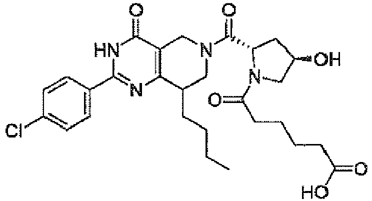 | A | 1.72 | 559 |

**[Table 139]**

| Example No | Structure | LC/MS method | RT | MS |
|---|---|---|---|---|
| II-0201 | 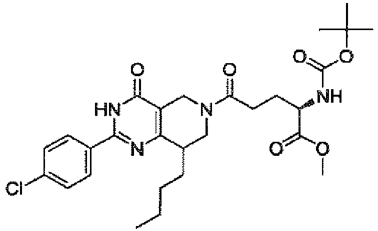 | A | 2.49 | 561 |
| II-0202 | 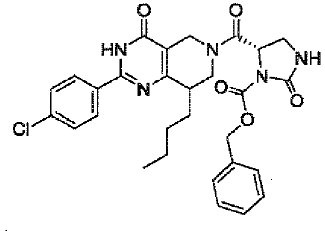 | A | 2.17 | 564 |
| II-0203 | 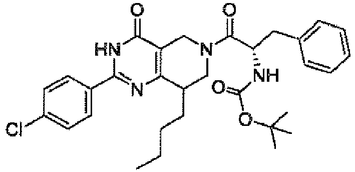 | A | 2.84 | 565 |
| II-0204 | 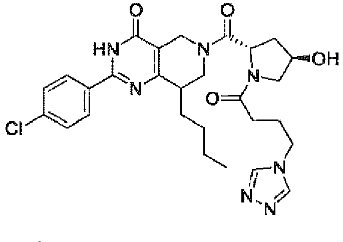 | A | 1.68 | 568 |
| II-0205 | 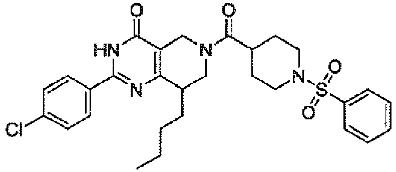 | A | 2.52 | 569 |

**[Table 140]**

| Example No | Structure | LC/MS method | RT | MS |
|---|---|---|---|---|
| II-0206 | 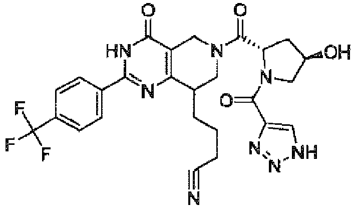 | B | 1.58 | 571 |
| II-0207 | 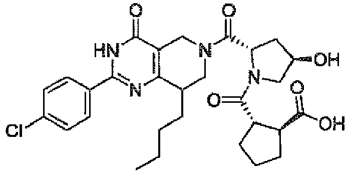 | A | 1.83 | 571 |
| II-0208 | 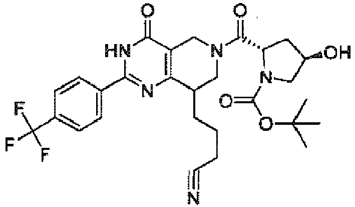 | B | 1.87 | 576 |
| II-0209 | 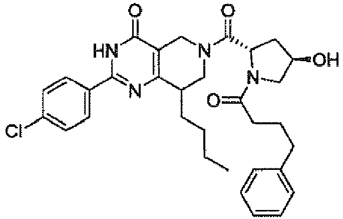 | A | 2.23 | 577 |
| II-0210 | 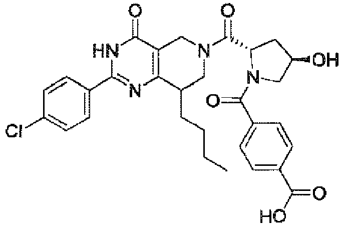 | A | 1.78 | 579 |

**[Table 141]**

| Example No | Structure | LC/MS method | RT | MS |
|---|---|---|---|---|
| II-0211 | 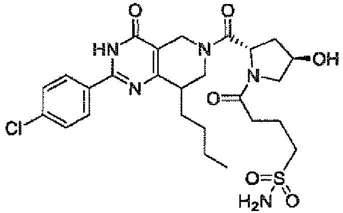 | A | 1.67 | 580 |
| II-0212 | 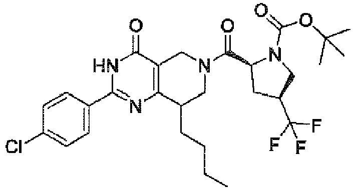 | A | 2.74 | 583 |
| II-0213 | 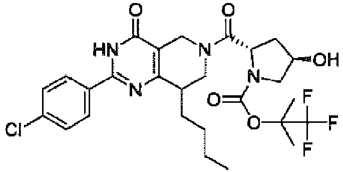 | B | 2.34 | 585 |
| II-0214 | 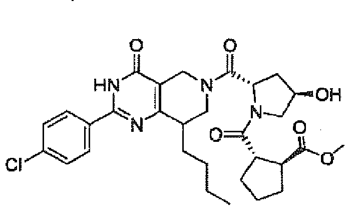 | A | 2.00, 2.04 | 585 |
| II-0215 | 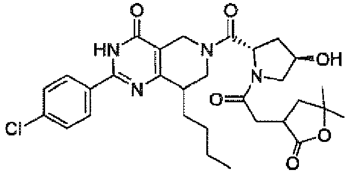 | A | 1.95 | 585 |

**[Table 142]**

| Example No | Structure | LC/MS method | RT | MS |
|---|---|---|---|---|
| II-0216 | 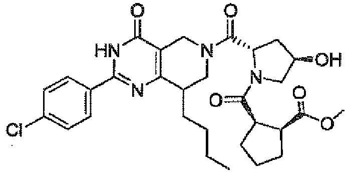 | A | 1.99 | 585 |
| II-0217 | 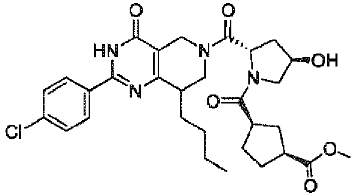 | A | 1.94 | 585 |
| II-0218 | 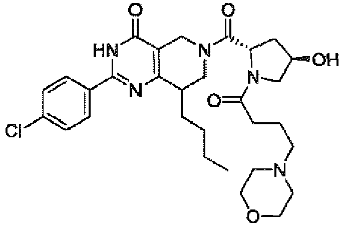 | A | 1.48 | 586 |
| II-0219 | 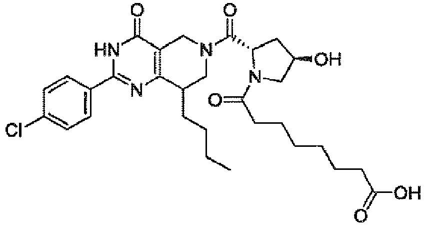 | A | 1.84 | 587 |
| II-0220 | 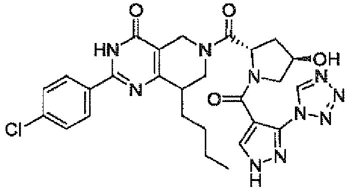 | A | 1.73 | 593 |

**[Table 143]**

| Example No | Structure | LC/MS method | RT | MS |
|---|---|---|---|---|
| II-0221 | 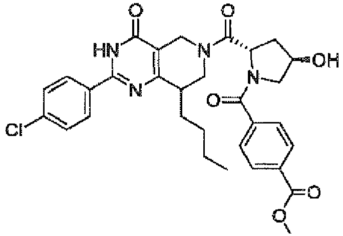 | A | 1.99 | 593 |
| II-0222 | 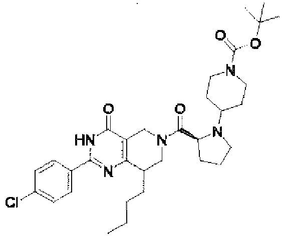 | A | 2.09 | 598 |
| II-0223 | 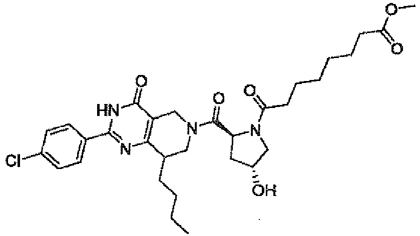 | A | 2.07 | 601 |
| II-0224 | 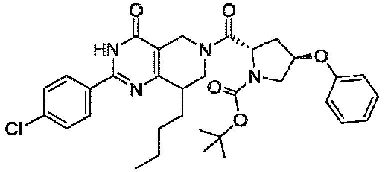 | A | 2.91 | 607 |
| II-0225 | 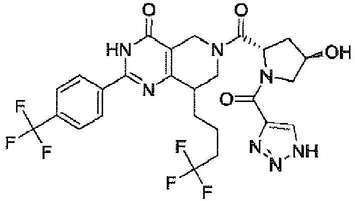 | B | 1.90 | 614 |

**[Table 144]**

| Example No | Structure | LC/MS method | RT | MS |
|---|---|---|---|---|
| II-0226 | 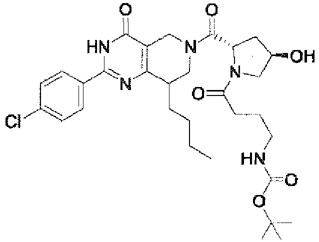 | A | 2.05 | 616 |
| II-0227 | 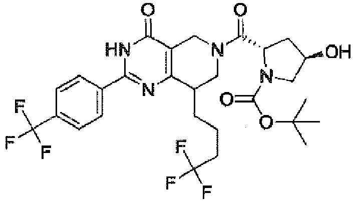 | B | 2.23 | 619 |
| II-0228 | 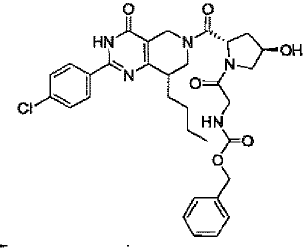 | A | 2.11 | 622 |
| II-0229 | 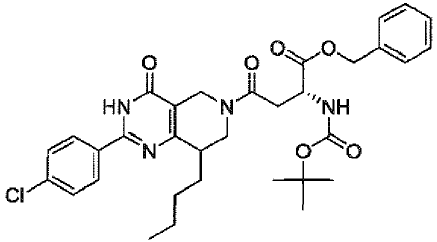 | A | 2.86 | 623 |
| II-0230 | 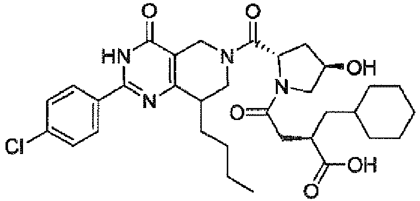 | A | 2.27 | 627 |

**[Table 145]**

| Example No | Structure | LC/MS method | RT | MS |
|---|---|---|---|---|
| II-0231 | 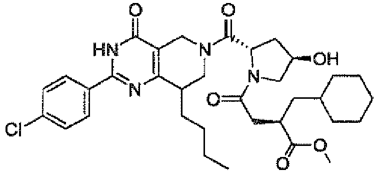 | A | 2.51 | 641 |
| II-0232 | 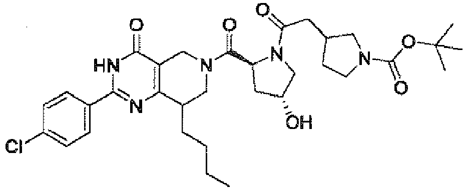 | A | 2.18 | 642 |
| II-0233 | 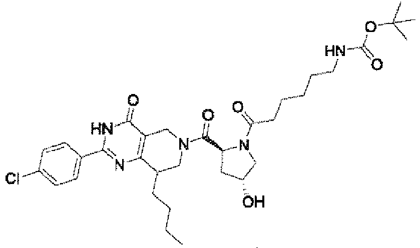 | A | 2.17 | 644 |
| II-0234 | 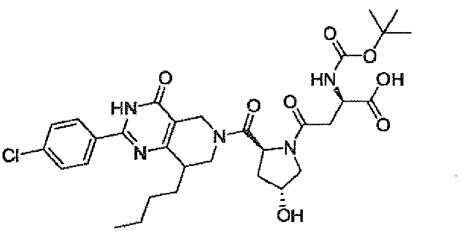 | A | 1.98 | 646 |
| II-0235 | 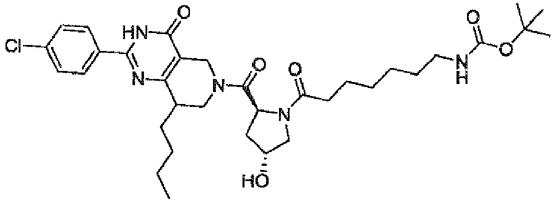 | A | 2.25 | 658 |

**[Table 146]**

| Example No | Structure | LC/MS method | RT | MS |
|---|---|---|---|---|
| II-0236 | 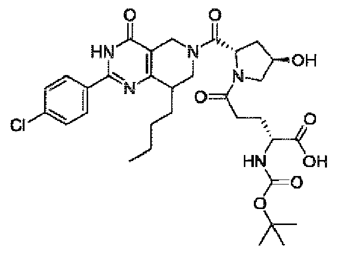 | A | 1.93 | 660 |
| II-0237 | 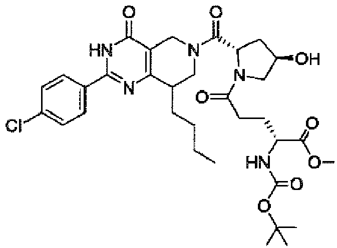 | A | 2.09 | 674 |
| II-0238 | 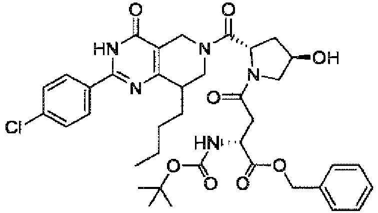 | A | 2.47 | 736 |

### Test Example 1 Evaluation of aoutotaxin inhibitor

Solution A containing 25mM Tris-HCl buffer (pH7.5), 100mM NaCl, 5mM MgCl2, and 0.1% BSA was prepared. Mouse autotaxin enzyme (purchased from R&D system) was diluted with Solution A, and 5µl of which was added to a solution of test compound in DMSO. Furthermore, 5µl of 0.5µM TG-mTMP in Solution A was added and allowed react at room temperature for 2 hours. 5µl of 150mM EDTA in Solution A was added to quench the reaction, and a fluorescent dye TokyoGreen, which was produced by the reaction, was detected. The fluorescence was detected using ViewLux (PerkinElmer, Inc.) with an excitation wavelength of 480nm and a fluorescence wavelength of 540nm.

The percent inhibition of the test compound was calculated by assuming the sample with no test compound as 0% inhibition and the test sample with no enzyme as 100% inhibition, and the percent inhibitions at different concentrations of the test compound were plotted to obtain a concentration-dependent curve. The IC50 value, which is the concentration of the test compound that resulted in 50% inhibition, was determined from the curve.

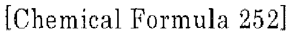

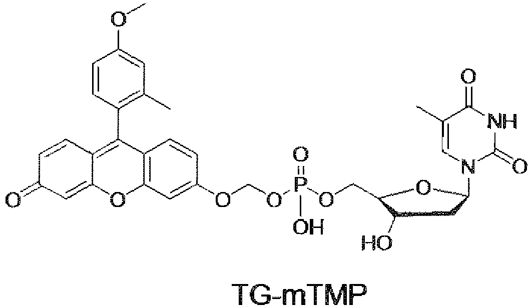

### Test Example 2 Evaluation of autotaxin inhibitor

Solution 13 containing 100mM Tris-HCl buffer (pH7.5), 150mM NaCl, 5mM MgCl2, and 0.05% Triton X-100 was prepared. Human autotaxin enzyme (purchased from R&D System) was diluted with Solution B, and 2.5µl of which was added to a solution of test compound in DMSO. Furthermore, 2.5µl of 200µM 18:0 Lyso PC (purchased from Avanti Polar Lipids) in Solution B was added and allowed to react at room temperature for 2 hours. After completion of the reaction, 15µl of the coline assay reagent (100mM Tris-HCl buffer (pH7.5), 5mM MgCl2, 77µg/ml coline oxidase, 10µg/ml peroxydase, 25µM 10-acetyl-3,7-dihydroxyphenoxazine, and excess autotaxin inhibitor) was added and allowed to react at room temperature for 20 minutes. The fluorescent dye Resorufin, which was produced by the reaction, was detected. The fluorescence was detected using ViewLux (PerkinElmer,Inc.) with a excitation wavelength of 531nm and a fluorescence wavelength of 598nm.

The percent inhibition of the test compound was calculated by assuming the sample with no test compound as 0% inhibition and the sample with no enzyme as 100% inhibition, and the percent inhibitions at different concentrations of the test compound were plotted to obtain a concentration-dependent curve. The IC50 value, which is the concentration of the test compound that resulted in 50% inhibition, was determined from the curve.

The results obtained by the test method described the above Test Example 3 are shown in the following tables.
A:IC50 < 10 nM, B:10 nM ≤ IC50 < 100 nM, C:100 nM ≤ IC50 < 1000 nM,
D:1000 nM ≤ IC50

**[Table 147]**

| Example No | Inhibitor activity | Example No | Inhibitor activity | Example No | Inhibitor activity |
|---|---|---|---|---|---|
| I-0001 | D | I-0041 | B | I-0081 | B |
| I-0002 | C | I-0042 | A | I-0082 | C |
| I-0003 | C | I-0043 | B | I-0083 | D |
| I-0004 | D | I-0044 | A | I-0084 | B |
| I-0005 | D | I-0045 | A | I-0085 | B |
| I-0006 | D | I-0046 | B | I-0086 | C |
| I-0007 | D | I-0047 | C | I-0087 | B |
| I-0008 | C | I-0048 | A | I-0088 | B |
| I-0009 | B | I-0049 | C | I-0089 | B |
| I-0010 | C | I-0050 | C | I-0090 | B |
| I-0011 | D | I-0051 | A | I-0091 | D |
| I-0012 | B | I-0052 | A | I-0092 | C |
| I-0013 | C | I-0053 | B | I-0093 | B |
| I-0014 | C | I-0054 | C | I-0094 | B |
| I-0015 | B | I-0055 | A | I-0095 | B |
| I-0016 | C | I-0056 | A | I-0096 | C |
| I-0017 | C | I-0057 | B | I-0097 | C |
| I-0018 | C | I-0058 | D | I-0098 | B |
| I-0019 | D | I-0059 | B | I-0099 | D |
| I-0020 | C | I-0060 | B | I-0100 | C |
| I-0021 | C | I-0061 | B | I-0101 | B |
| I-0022 | B | I-0062 | B | I-0102 | C |
| I-0023 | B | I-0063 | A | I-0103 | B |
| I-0024 | A | I-0064 | A | I-0104 | C |
| I-0025 | C | I-0065 | B | I-0105 | C |
| I-0026 | B | I-0066 | D | I-0106 | D |
| I-0027 | C | I-0067 | C | I-0107 | D |
| I-0028 | B | I-0068 | D | I-0108 | D |
| I-0029 | C | I-0069 | B | I-0109 | B |
| I-0030 | C | I-0070 | A | I-0110 | D |
| I-0031 | C | I-0071 | A | I-0111 | B |
| I-0032 | C | I-0072 | A | I-0112 | B |
| I-0033 | B | I-0073 | A | I-0113 | B |
| I-0034 | C | I-0074 | D | I-0114 | B |
| I-0035 | B | I-0075 | D | I-0115 | D |
| I-0036 | C | I-0076 | C | I-0116 | B |
| I-0037 | C | I-0077 | C | I-0117 | B |
| I-0038 | C | I-0078 | D | I-0118 | B |
| I-0039 | B | I-0079 | B | I-0119 | B |
| I-0040 | B | I-0080 | B | I-0120 | A |

**[Table 148]**

| Example No | Inhibitor activity | Example No | Inhibitor activity | Example No | Inhibitor activity |
|---|---|---|---|---|---|
| I-0121 | C | I-0161 | C | I-0201 | C |
| I-0122 | C | I-0162 | C | I-0202 | C |
| I-0123 | B | I-0163 | C | I-0203 | C |
| I-0124 | B | I-0164 | C | I-0204 | C |
| I-0125 | B | I-0165 | C | I-0205 | B |
| I-0126 | C | I-0166 | C | I-0206 | A |
| I-0127 | C | I-0167 | C | I-0207 | D |
| I-0128 | B | I-0168 | C | I-0208 | D |
| I-0129 | C | I-0169 | C | I-0209 | B |
| I-0130 | B | I-0170 | B | I-0210 | B |
| I-0131 | B | I-0171 | C | I-0211 | B |
| I-0132 | C | I-0172 | C | I-0212 | C |
| I-0133 | B | I-0173 | C | I-0213 | C |
| I-0134 | A | I-0174 | C | I-0214 | C |
| I-0135 | B | I-0175 | C | I-0215 | B |
| I-0136 | C | I-0176 | C | I-0216 | C |
| I-0137 | B | I-0177 | C | I-0217 | B |
| I-0138 | A | I-0178 | C | I-0218 | C |
| I-0139 | B | I-0179 | B | I-0219 | C |
| I-0140 | C | I-0180 | C | I-0220 | B |
| I-0141 | B | I-0181 | B | I-0221 | B |
| I-0142 | C | I-0182 | C | I-0222 | C |
| I-0143 | B | I-0183 | B | I-0223 | C |
| I-0144 | B | I-0184 | C | I-0224 | C |
| I-0145 | B | I-0185 | C | I-0225 | A |
| I-0146 | C | I-0186 | C | I-0226 | C |
| I-0147 | B | I-0187 | C | I-0227 | A |
| I-0148 | C | I-0188 | C | I-0228 | A |
| I-0149 | C | I-0189 | C | I-0229 | C |
| I-0150 | D | I-0190 | C | I-0230 | C |
| I-0151 | C | I-0191 | C | I-0231 | C |
| I-0152 | B | I-0192 | B | I-0232 | A |
| I-0153 | B | I-0193 | C | I-0233 | B |
| I-0154 | B | I-0194 | C | I-0234 | B |
| I-0155 | C | I-0195 | A | I-0235 | A |
| I-0156 | C | I-0196 | B | I-0236 | D |
| I-0157 | B | I-0197 | C | I-0237 | C |
| I-0158 | B | I-0198 | C | I-0238 | C |
| I-0159 | A | I-0199 | B | I-0239 | D |
| I-0160 | B | I-0200 | D | I-0240 | C |

**[Table 149]**

| Example No | Inhibitor activity |
|---|---|
| I-0241 | D |
| I-0242 | C |
| I-0243 | B |
| I-0244 | B |
| I-0245 | A |
| I-0246 | D |
| I-0247 | D |
| I-0248 | B |
| I-0249 | C |
| I-0250 | B |
| I-0251 | B |
| I-0252 | B |
| I-0253 | C |
| I-0254 | B |
| I-0255 | D |
| I-0256 | B |
| I-0257 | B |

**[Table 150]**

| Example No | Inhibitor Activity | Example No | Inhibitor Activity | Example No | Inhibitor Activity |
|---|---|---|---|---|---|
| I-0258 | D | I-0298 | C | I-0338 | B |
| I-0259 | D | I-0299 | A | I-0339 | B |
| I-0260 | D | I-0300 | B | I-0340 | C |
| I-0261 | C | I-0301 | B | I-0341 | C |
| I-0262 | C | I-0302 | B | I-0342 | B |
| I-0263 | A | I-0303 | B | I-0343 | C |
| I-0264 | D | I-0304 | B | I-0344 | B |
| I-0265 | B | I-0305 | B | I-0345 | B |
| I-0266 | A | I-0306 | B | I-0346 | B |
| I-0267 | A | I-0307 | C | I-0347 | B |
| I-0268 | D | I-0308 | B | I-0348 | B |
| I-0269 | B | I-0309 | B | I-0349 | B |
| I-0270 | B | I-0310 | B | I-0350 | B |
| I-0271 | A | I-0311 | C | I-0351 | C |
| I-0272 | B | I-0312 | C | I-0352 | B |
| I-0273 | B | I-0313 | C | I-0353 | B |
| I-0274 | B | I-0314 | B | I-0354 | B |
| I-0275 | B | I-0315 | D | I-0355 | C |
| I-0276 | B | I-0316 | B | I-0356 | A |
| I-0277 | B | I-0317 | B | I-0357 | C |
| I-0278 | B | I-0318 | B | I-0358 | B |
| I-0279 | A | I-0319 | C | I-0359 | B |
| I-0280 | B | I-0320 | C | I-0360 | C |
| I-0281 | C | I-0321 | B | I-0361 | B |
| I-0282 | A | I-0322 | B | I-0362 | B |
| I-0283 | C | I-0323 | B | I-0363 | B |
| I-0284 | B | I-0324 | B | I-0364 | C |
| I-0285 | B | I-0325 | B | I-0365 | C |
| I-0286 | B | I-0326 | B | I-0366 | C |
| I-0287 | B | I-0327 | B | I-0367 | B |
| I-0288 | B | I-0328 | B | I-0368 | B |
| I-0289 | C | I-0329 | B | I-0369 | C |
| I-0290 | A | I-0330 | B | I-0370 | C |
| I-0291 | B | I-0331 | A | I-0371 | D |
| I-0292 | B | I-0332 | B | I-0372 | B |
| I-0293 | B | I-0333 | B | I-0373 | B |
| I-0294 | B | I-0334 | B | | |
| I-0295 | B | I-0335 | B | | |
| I-0296 | C | I-0336 | B | | |
| I-0297 | B | I-0337 | B | | |

**[Table 151]**

| Example No | Inhibitor Activity | Example No | Inhibitor Activity | Example No | Inhibitor Activity |
|---|---|---|---|---|---|
| I-0374 | B | I-0424 | B | I-0474 | B |
| I-0375 | A | I-0425 | B | I-0475 | B |
| I-0376 | B | I-0426 | D | I-0476 | D |
| I-0377 | B | I-0427 | B | I-0477 | C |
| I-0378 | A | I-0428 | B | I-0478 | C |
| I-0379 | A | I-0429 | A | I-0479 | B |
| I-0380 | B | I-0430 | B | I-0480 | B |
| I-0381 | B | I-0431 | B | I-0481 | B |
| I-0382 | B | I-0432 | B | I-0482 | A |
| I-0383 | B | I-0433 | B | I-0483 | C |
| I-0384 | D | I-0434 | B | I-0484 | B |
| I-0385 | B | I-0435 | B | I-0485 | B |
| I-0386 | B | I-0436 | C | I-0486 | C |
| I-0387 | B | I-0437 | B | I-0487 | C |
| I-0388 | D | I-0438 | B | I-0488 | C |
| I-0389 | B | I-0439 | A | I-0489 | B |
| I-0390 | B | I-0440 | B | I-0490 | C |
| I-0391 | B | I-0441 | C | I-0491 | C |
| I-0392 | B | I-0442 | B | I-0492 | B |
| I-0393 | B | I-0443 | D | I-0493 | D |
| I-0394 | A | I-0444 | A | I-0494 | A |
| I-0395 | C | I-0445 | A | I-0495 | B |
| I-0396 | D | I-0446 | B | I-0496 | B |
| I-0397 | B | I-0447 | C | I-0497 | B |
| I-0398 | C | I-0448 | C | I-0498 | C |
| I-0399 | B | I-0449 | C | I-0499 | C |
| I-0400 | B | I-0450 | B | I-0500 | B |
| I-0401 | B | I-0451 | B | I-0501 | B |
| I-0402 | D | I-0452 | B | | |
| I-0403 | B | I-0453 | D | | |
| I-0404 | B | I-0454 | C | | |
| I-0405 | B | I-0455 | B | | |
| I-0406 | B | I-0456 | B | | |
| I-0407 | B | I-0457 | A | | |
| I-0408 | C | I-0458 | A | | |
| I-0409 | C | I-0459 | B | | |
| I-0410 | A | I-0460 | D | | |
| I-0411 | C | I-0461 | B | | |
| I-0412 | A | I-0462 | B | | |
| I-0413 | B | I-0463 | B | | |
| I-0414 | A | I-0464 | B | | |
| I-0415 | A | I-0465 | A | | |
| I-0416 | B | I-0466 | B | | |
| I-0417 | B | I-0467 | B | | |
| I-0418 | B | I-0468 | B | | |
| I-0419 | B | I-0469 | C | | |
| I-0420 | C | I-0470 | A | | |
| I-0421 | A | I-0471 | B | | |
| I-0422 | B | I-0472 | D | | |
| I-0423 | B | I-0473 | B | | |

**[Table 152]**

| Example No | Inhibitor Actibity | Example No | Inhibitor Actibity | Example No | Inhibitor Actibity |
|---|---|---|---|---|---|
| II-0001 | D | II-0041 | B | II-0081 | D |
| II-0002 | C | II-0042 | D | II-0082 | C |
| II-0003 | D | II-0043 | C | II-0083 | D |
| II-0004 | B | II-0044 | B | II-0084 | B |
| II-0005 | D | II-0045 | D | II-0085 | C |
| II-0006 | D | II-0048 | C | II-0086 | B |
| II-0007 | C | II-0047 | C | II-0087 | B |
| II-0008 | D | II-0048 | C | II-0088 | B |
| II-0009 | D | II-0049 | B | II-0089 | C |
| II-0010 | C | II-0050 | C | II-0090 | D |
| II-0011 | C | II-0051 | C | II-0091 | C |
| II-0012 | C | II-0052 | C | II-0092 | B |
| II-0013 | D | II-0053 | B | II-0093 | B |
| II-0014 | C | II-0054 | B | II-0094 | B |
| II-0015 | C | II-0055 | C | II-0095 | C |
| II-0016 | D | II-0056 | C | II-0096 | A |
| II-0017 | C | II-0057 | D | II-0097 | C |
| II-0018 | D | II-0058 | C | II-0098 | C |
| II-0019 | C | II-0059 | C | II-0099 | C |
| II-0020 | C | II-0060 | C | II-0100 | A |
| II-0021 | C | II-0061 | C | II-0101 | B |
| II-0022 | D | II-0062 | B | II-0102 | D |
| II-0023 | D | II-0063 | B | II-0103 | C |
| II-0024 | D | II-0064 | B | II-0104 | B |
| II-0025 | C | II-0065 | C | II-0105 | C |
| II-0026 | C | II-0066 | C | II-0106 | B |
| II-0027 | D | II-0067 | C | II-0107 | B |
| II-0028 | C | II-0068 | D | II-0108 | B |
| II-0029 | D | II-0069 | C | II-0109 | C |
| II-0030 | C | II-0070 | C | II-0110 | C |
| II-0031 | B | II-0071 | D | II-0111 | C |
| II-0032 | C | II-0072 | D | II-0112 | C |
| II-0033 | C | II-0073 | D | II-0113 | C |
| II-0034 | C | II-0074 | C | II-0114 | A |
| II-0035 | C | II-0075 | C | II-0115 | B |
| II-0036 | B | II-0076 | B | II-0116 | B |
| II-0037 | C | II-0077 | B | II-0117 | C |
| II-0038 | C | II-0078 | C | II-0118 | B |
| II-0039 | D | II-0079 | C | II-0119 | B |
| II-0040 | C | II-0080 | C | II-0120 | C |

**[Table 153]**

| Example No | Inhibitor Actibity | Example No | Inhibitor Actibity | Example No | Inhibitor Activity |
|---|---|---|---|---|---|
| II-0121 | C | II-0161 | C | II-0201 | B |
| II-0122 | C | II-0162 | D | II-0202 | B |
| II-0123 | C | II-0163 | C | II-0203 | D |
| II-0124 | C | II-0164 | C | II-0204 | C |
| II-0125 | B | II-0165 | C | II-0205 | D |
| II-0126 | D | II-0166 | B | II-0206 | B |
| II-0127 | D | II-0167 | C | II-0207 | B |
| II-0128 | C | II-0168 | A | II-0208 | B |
| II-0129 | C | II-0169 | B | II-0209 | C |
| II-0130 | D | II-0170 | D | II-0210 | C |
| II-0131 | C | II-0171 | D | II-0211 | C |
| II-0132 | C | II-0172 | B | II-0212 | B |
| II-0133 | C | II-0173 | B | II-0213 | B |
| II-0134 | B | II-0174 | A | II-0214 | C |
| II-0135 | C | II-0175 | B | II-0215 | C |
| II-0136 | C | II-0176 | C | II-0216 | C |
| II-0137 | B | II-0177 | D | II-0217 | C |
| II-0138 | C | II-0178 | C | II-0218 | D |
| II-0139 | D | II-0179 | A | II-0219 | C |
| II-0140 | C | II-0180 | B | II-0220 | C |
| II-0141 | A | II-0181 | C | II-0221 | C |
| II-0142 | C | II-0182 | D | II-0222 | C |
| II-0143 | B | II-0183 | C | II-0223 | C |
| II-0144 | C | II-0184 | B | II-0224 | C |
| II-0145 | A | II-0185 | C | II-0225 | B |
| II-0146 | B | II-0186 | B | II-0226 | C |
| II-0147 | C | II-0187 | B | II-0227 | B |
| II-0148 | B | II-0188 | D | II-0228 | D |
| II-0149 | D | II-0189 | D | II-0229 | D |
| II-0150 | B | II-0190 | D | II-0230 | B |
| II-0151 | C | II-0191 | A | II-0231 | C |
| II-0152 | B | II-0192 | C | II-0232 | C |
| II-0153 | B | II-0193 | C | II-0233 | D |
| II-0154 | B | II-0194 | B | II-0234 | B |
| II-0155 | C | II-0195 | C | II-0235 | C |
| II-0156 | C | II-0196 | B | II-0236 | B |
| II-0157 | C | II-0197 | C | II-0237 | C |
| II-0158 | C | II-0198 | D | II-0238 | D |
| II-0159 | B | II-0199 | C | | |
| II-0160 | B | II-0200 | C | | |

### Test Example 4: CYP Inhibition Test

Using commercially available pooled human hepatic microsome, the compound was tested to assess inhibitory effect on the typical substrate metabolism reactions of human main five CYP enzyme forms (CYP1A2, 2C9, 2C19, 2D6, 3A4), specifically, 7-ethoxyresorufin O-deothylation (CYP1A2), tolbutamide methyl-hydroxylation (CYP2C9), mephenyloin 4'-hydroxylation (CYP2C19), dextromethorphan O-demethylation (CYP2D6) terfenedine hydroxylation (CYP3A4).

The reaction conditions were as follows.
Substrates:
   0.5 µmol/L ethoxyresorufin (CYP1A2),
   100 µmol/L tolbutamide (CYP2C9),
   50 µmol/L S-mephenitoin (CYP2C19),
   5 µmol/L dextromethorphan (CYP2D6),
   1 µmol/L terfcanadine (CYP3A4);
Reaction Time: 15 minutes;
Reaction Temperature: 37°C;
Enzyme: pooled human hepatic microsome 0.2 mg protein/mL;
Concentration of Test Compound: 1, 5, 10, 20 µmol/L (four points).

A test sample, which contains the substrate, humen hepatic microsome and test compound at the amounts as described above in 50 mM Hepes buffer, was added to a 96-well plate. The cofoator NADPH was added to initiate metabolism reaction. After the incubation at 37°C for 15 minutes, a methanol/acetonitrile=1/1 (v/v) solution was added to stop the reaction. After centrifugation at 3000 rpm for 15 minutes, resotufin (CYP1A2 metabolize) in the supernatant was quantified by fluorescent multilabel counter. Tributamide hydroxide (CYP2C9 metabolite), mephenyloin 4' hydroxide (CYP2C19 metabolite), dextromethorphan (CYP2D6 metabolite) and terfenadine alcohol (CYP3A4 metabolite) were determined by LC/MS/MS.

Only DMSO, which was the solvent for the test compound, was added to the reaction system as the control (100%). For each concentration of the test compound, the remaining activity (%) was calculated, and the 1C50 was calculated by reverse presumption by a logistic model using the concentration and the inhibition rate.

### Test Example 5: Evaluation or Metabolism Stability

Metabolism stability evaluation by human hapatic microsomes: To a tris-HCl buffer (pH7.4) was added NADPH (final concentration 1mM, the case of oxi dative matabolism), human hepatic microsomes (final concentration 0.5mg protein /ml) and the test compound (final concentration 2µM), the mixture was reacted a t 37°C for 0minute and 30 minutes. In the case of glucuronidation, UDPGA (fin al concentration 5mM) was added instead of NADPH. After the reaction was sto pped by adding double volume of the reaction solution of acetonitrile / methanol = 1/1 (v/v), the compound into the centrifugal supernatant was measured by HP LC. The loss amount due to metabolic reactions was calculated by comparing be tween value of 0 minute and 30minute to confirm the metabolic stability of the compound of the present invention.

### Test Example 6: Powder solubility test

Appropriate quantity of the test compound was put in a suitable container and 200µL of JP-1 solution (water was added to 2.0g of sodium chloride and 7. 0mL of hydrochloric acid to reach 1000mL), JP-2 solution (500mL of water was added to 500 mL of phosphate buffer with a pH6.8), or 20mmol/L TCA (sodium t aurocholate) / JP-2 solution (water was added to 1.08g of TCA to reach 100mL) was independently added to each container. When total amount was dissolved a fter adding the test reagent, the test compound was added appropriately. The c ontainer was sealed and shaken at 37°C for 1 hour. The solution was filtered a nd 100µL of methanol was added to 100µL of each filtrate to dilute two-fold. T he dilution rate was changed as necessary. After checking that there is no bub ble and deposit, the container was sealed and shaken. The test compound was measured using HPLC by absolute calibration curve method.

### Test Example 7: DMSO method, Solubility test

The solubility of the compound o the present invention was determined un der 1% DMSO addition condition. A 10mmol/L solution of the compound was pr epared with DMSO, and 2µL of the solution of the compound of the present inve ntion was added, respectively, to 198µL of JP-1 solution or JP-2 solution. After leaving to stand at 25°C for 16 hours, the mixture was filtered under reduced pressure. The filtrate was ten-fold diluted with methanol/water=1/1(v/v) and the compound concentration in the filtrate was measured with LC/MS by the absolu te calibration method.
Compound II-0054 JP 1: >50µmol/L, JP2: >50µmol/L
Compound II-0076 JP 1: >50µmol/L, JP2: >50µmol/L
Compound II-0168 JP 1: 48.3µmol/L, JP2: >50µmol/L
Compound II-0175 JP 1: 45.7µmol/L, JP2: >50µmol/L
Compound II-0208 JP 1: 49.9µmol/L, JP2: >50µmol/L

### Test Example 8: BA test

### Material and method for experiments to evaluate oral absorption

(1) Experimental animals: mice or SD rats were used.
(2) Rearing condition: mice or SD rats were allowed free access to solid feed and sterilized tap water.
(3) Setting of dosage and grouping: Oral administration and intravenous adminis tration were performed with the predetermined dosage. Grouping was set as bel ow. (Dosage was changed per compound)
   Oral administration 1 to 30 mg/kg (n=2 to 3)
   Intravenous administration 0.5 to 10 mg/kg (n=2 to 3)
(4) Preparation of administration solutions: Oral administration was performed a s solution or suspension. Intravenous administration was performed after solubil ization.
(5) Routes of administration: Oral administration was performed mandatory into the stomach by oral sonde. Intravenous administration was performed from cau dal vein by syringes with needle.
(6) Evaluation items: Blood was collected serially and concentration of the compo und of the present invention was calculated from AUCs of the oral administratio n group and the intravenous administration group.
(7) Statistical analysis: About transition of concentration of the compound of the present invention in plasma, the area under the plasma concentration versus ti me curve (AUC) was calculated by non-linear least-squares method program, Win Nonlin (registered trademark), and bioavailability (BA) of the compound of the p resent invention was calculated from AUCs of the oral administration group and the intravenous administration group.

### Test Example 9: Metabolism Stability Test

Using commercially available pooled human hepatic microsomes, the compou nd of the present invention was reacted for a constant time, and a remaining ra te was calculated by comparing a reacted sample and an unreacted sample, ther eby, a degree of metabolism in liver was assessed.

A reaction was performed (oxidative reaction) at 37°C for 0 minute or 30 minutes in the presence of 1mmol/L NADPH in 0.2mL of a buffer (50mmol/L Tri s-HCl pH 7.4, 150mmol/L potassium chloride, 10mmol/L magnesium chloride) con taining 0.5mg protein/mL of human liver microsomes. After the reaction, 50µL o f the reaction solution was added to 100µL of a methanol/acetonitrile = 1/1(v/v), mixed an centrifuged at 3000 rpm for 15 minutes. The compound of the present invention in the supernatant was quantified by LC/MS/MS, and a remaining am ount of the compound o the present invention after the reaction was calculated, letting the compound amount at 0minute reaction time to be 100%. Indeed, hyd rolysis reaction was performed in the presence of 5mM UDP-glucuronic acid inst ead of NADPH, followed by similar operation.

### Test Example 10: CYP3A4 fluorescent MBI test

The CYP3A4 fluorescent MBI test is a test of investigating enhancement o f CYP3A4 inhibition of a compound of the present invention by a metabolism re action, and the test was performed using, as CYP3A4 enzyme expressed in Esche richia coli and employing, as an index, a reaction in which 7-benzyloxytrifluorom ethylcoumarin (7-BFC) is debenzylated by the CYP3A4 enzyme to produce a met abolite, 7-hydroxytrifluoromethylcouomarin (7-HFC) emitting fluorescent light.

The reaction conditions were as follows: substrate, 5.6µmol/L 7-BFC; pre-re action time, 0 or 30 minutes; reaction time, 15 minutes; reaction temperature, 2 5°C (room temperature); CYP3A4 content (expressed in Escherichia coli), pre-reac tion 62.5pmol/mL, at reaction 6.25pmol/mL (at 10-fold dilution); test drug concen tration of the compound of the present invention, 0.625, 1.25, 2.5, 5, 10, 20µmol/ L (6 points).

An enzyme in a K-Pi buffer (pH7.4) and a solution of the solution of the present invention as a pre-reaction solution were added to a 96-well plate at the above composition of the pre-reaction, a part of it was transferred to another 9 6-well plate so that it was 1/10 diluted with a substrate and a K-Pi buffer, NA DPH as a co-factor was added to initiate a reaction as an index (without preinc ubation) and, after a predetermined time of a reaction, acetonitrile/0.5 mol/L Tri s(trishydroxyaminomethane)=4/1(V/V) was added to stop the reaction. In additio n, NADPH was added to a remaining preincubation solution to initiate a preincu bation (with preincubation) and, after a predetermined time of a preincubation, a part was transferred to another plate so that it was 1/10 diluted with a subst rate and a K-Pi buffer to initiate a reaction as an index. After a predetermined time of a reaction, acetonitrile/0.5 mol/L Tris(trishydroxyaminomethane)=4/1(V/V) was added to stop the reaction. For the plate on which each index reaction ha d been performed, a fluorescent value of 7-HFC which is a metabolite was meas ured with a fluorescent plate reader. (Ex=420nm, Em=535nm)

Addition of only DMSO which is a solvent dissolving the compound of the present invention to a reaction system was adopted as a control (100%), remaini ng activity (%) was calculated at each concentration of the compound of the pres ent invention added as the solution, and IC₅₀ was calculated by reverse-presump tion by a logistic model using a concentration and an inhibition rate. When a d ifference between IC₅₀ value was 5µmol/L or more, this was defined as (+) and, when the difference was 3µmol/L or less, this was defined as (-).

### Test Example 11: Fluctuation Ames Test

Mutagenicity of compounds of the present invention was evaluated.

20 µL of freezing-stored rat typhoid bacillus (Salmonella typhimurium TA9 8 strain, TA100 strain) is inoculated on 10 mL of a liquid nutrient medium (2.5 % Oxoid nutrient broth No.2), and this was cultured before shaking at 37°C for 10 hours. 9 mL of a bacterial solution of the TA98 strain was centrifuged (200 0 × g, 10 minutes) to remove a culturing solution. The bacteria was suspended in 9 mL of a Micro F buffer (K₂HPO₄: 3.5 g/L, KH₂PO₄: 1 g/L, (NH₄)₂SO₄: 1 g/L, trisodium citrate dehydrate: 0.25 g/L, MgSO₄ · 7H₂O: 0.1 g/L), the suspension w as added to 110 mL of an Exposure medium (Micro F buffer containing Biotin: 8 µg/mL, histidine: 0.2 µg/mL, glucose: 8 mg/mL). The TA100 strain was added t o 120 mL of the Exposure medium relative to 3.16 mL of the bacterial solution to prepare a test bacterial solution. Each 12 µL of DMSO solution of a compou nd of the present invention (several stage dilution from maximum dose 50 mg/m L at 2 to 3 fold ratio), DMSO as a negative control, and 50 µg/mL of 4-nitroqui noline-1-oxide DMSO solution for the TA98 strain, 0.25 µg/mL of 2-(2-furyl)-3-(5-nitro-2-furyl)acrylamide DMSO solution for the TA100 strain under the non-meta bolism activating condition, 40 µg/mL of 2-aminoanthracene DMSO solution for t he TA98 strain, 20 µg/mL of 2-aminoanthracene DMSO solution for the TA100 st rain under the metabolism activating condition as a positive control, and 588 µL of the test bacterial solution (a mixed solution of 498 µl of the test bacterial so lution and 90 µL of S9 mix under the metabolism activating condition) were mix ed, and this was shaking-cultured at 37°C for 90 minutes. 460 µL of the bacter ial solution exposed to a compound of the present invention was mixed with 230 0 µL of an Indicator medium (Micro F buffer containing biotin: 8 µg/mL, histidi ne: 0.2 µg/mL, glucose: 8 mg/mL, Bromo Cresol Purple: 37.5 µg/mL), each 50 µl was dispensed into microplate 48 wells/dose, and this was subjected to stationar y culturing at 37°C for 3 days. Since a well containing a bacterium which has obtained the proliferation ability by mutation of an amino acid (histidine) synthe sizing enzyme gene turns from purple to yellow due to a pH change, the bacteri um proliferation well which has turned to yellow in 48 wells per dose is counted , and was assessed by comparing with a negative control group. (-) means that mutagenicity is negative and (+) is positive.

### Test Example 12: hERG Test

For the purpose of assessing risk of an electrocardiogram QT interval prol ongation of the compound of the present invention, effects of the compound of th e present invention on delayed rectifier K+ current (l_{Kr}), which plays an importa nt role in the ventricular repolarization process, was studied using HEK293 cells expressing human ether-a-go-go related gene (hERG) channel.

After a cell was retained at a membrane potential of -80mV by whole cell patch clamp method using an automated patch clamp system (PatchXpress 7000 A, AxonInstruments Inc.), I_{Kr} induced by depolarization pulse stimulation at +40 mV for 2 seconds further reporlarization pulse stimulation at -50mV for 2 secon ds, is recorded. After the generated current was stabilized, extracellular solutio n (NaCl: 135 mmol/L, KCl: 5.4 mmol/L, NaH₂ PO₄: 0.3mmol/L, CaCl₂ 2H₂O: 1.8m mol/L, MgCl₂ 6H₂O: 1mmol/L, Glucose: 10mmol/L, HEPES(4-(2-hydroxyethyl)-1-pip erazineethanesulfonic acid, 4-(2-hydroxyothyl)-1-piperazinethanesulfonic acid): 10 mmol/L, pH=7.4), in which the compound of the present invention had been diss olved at an objective concentration, was applied to the cell at room temperature for 10 minutes.

From the recording I_{Kr}, an absolute value of the tail peak current was measured based on the current value at the resting membrane potential using analysis software (DataXpress ver.1, Molecular Devices Corporation). Further, the % inhibition relative to the tail peak current before application of the compound of the present invention was calculated, and compared with the vehicle-applied group (0.1% dimethyl sulfoxide solution) to assess influence of the compound of the present invention on I_{Kr}.

### Formulation Examples

The following Fromulation Examples are only exemplified and not intended to limit the scope of the invention.

**Formulation Example 1: Tablets**

| | |
|---|---|
| Compound of formula (I) | 15mg |
| Starch | 15mg |
| Lactose | 15mg |
| Crystalline cellulose | 19mg |
| Polyvinyl alcohol | 3mg |
| Distilled water | 30ml |
| Calcium stearate | 3mg |

The above ingredients except calcium stearate are uniformly mixed and milled to granylate, and dried to obtain a suitable size of granules. Then, the granules are added with calcium stearate and compressed to form a tablet.

**Formulation Examples 2: Capsules**

| | |
|---|---|
| Compound of formula (I) | 10mg |
| Magnesium stearate | 10mg |
| Lactose | 80mg |

The above ingredients are mixed uniformly to obtain powders or fine granules, which are then filled in a capsule.

**Formulation Examples 3: Granules**

| | |
|---|---|
| Compound of formula (I) | 30g |
| Lactose | 265g |
| Magnesium stearate | 5g |

The above ingredients are mixed uniformaly and compressed. The compressed mixture is milled, granylated and sirved to obtain the desired size of granules.

### Industrial Applicability

The present invention is applicable in the pharmaceutical firld, for example, in the development and production of medicaments for the treatment of fibrotic diseases.

## Claims

1. A compound according to any one of formula (Ia) to (Ic) or (II), or its pharm aceutically acceptable salt: 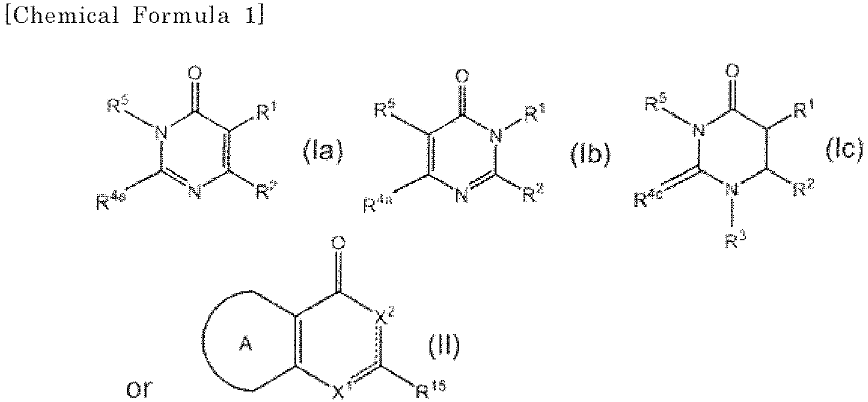 wherein
R¹ is hydrogen, halogen, hydroxy, formyl, substituted or unsubstituted alke nyl, substituted or unsubstituted alkynyl, substituted or unsubstituted non-aroma tic carbocyclyl, substituted or unsubstituted aromatic carbocyclyl", substituted or unsubstituted non-aromatic heterocyclyl, substituted or unsubstituted aromatic he terocyclyl, substituted or unsubstituted amino, substituted or unsubstituted alkyl oxy, substituted or unsubstituted alkenyloxy, substituted or unsubstituted alkyny loxy, substituted or unsubstituted non-aromatic carbocyclyloxy, substituted or uns ubstituted aromatic carbocyclyloxy, substituted or unsubstituted non-aromatic het erocyclyloxy, substituted or unsubstituted aromatic heterocyclyloxy, substituted or unsubstituted alkylthio, substituted or unsubstituted alkenylthio, substituted or unsubstituted alkynylthio, substituted or unsubstituted non-aromatic carbocyclylt hio, substituted or unsubstituted aromatic carbocyclylthio, substituted or unsubst ituted non-aromatic heterocyclylthio, substituted or unsubstituted aromatic hetero cyclylthio, substituted or unsubstituted alkylcarbonyl, substituted or unsubstitute d alkenylcarbonyl, substituted or unsubstituted alkynylcarbonyl, substituted or u nsubstituted non-aromatic carbocyclylcarbonyl, substituted or unsubstituted arom atic carbocyclylcarbonyl, substituted or unsubstituted non-aromatic heterocyclylcar bonyl, substituted or unsubstituted aromatic heterocyclylcarbonyl, substituted or unsubstituted alkenyloxycarbonyl, substituted or unsubstituted alkynyloxycarbonyl , substituted or unsubstituted non-aromatic carbocyclyloxycarbonyl, substituted or unsubstituted aromatic carbocyclyloxycarbonyl, substituted or unsubstituted non-aromatic heterocyclyloxycarbonyl, substituted or unsubstituted aromatic heterocycl yloxycarbonyl, substituted or unsubstituted carbamoyl, substituted or unsubstitut ed sulfamoyl, substituted or unsubstituted alkylsulfinyl, substituted or unsubstit uted alkenylsulfinyl, substituted or unsubstituted alkynylsulfinyl, substituted or unsubstituted non-aromatic carbocyclylsulfinyl, substituted or unsubstituted arom atic carbocyclylsulfinyl, substituted or unsubstituted non-aromatic heterocyclylsulf inyl, substituted or unsubstituted aromatic heterocyclylsulfinyl, substituted or un substituted alkylsulfonyl, substituted or unsubstituted alkenylsulfonyl, substitute d or unsubstituted alkynylsulfonyl, substituted or unsubstituted non-aromatic car bocyclylsulfonyl, substituted or unsubstituted aromatic carbocyclylsulfonyl, substit uted or unsubstituted non-aromatic heterocyclylsulfonyl or substituted or unsubst ituted aromatic heterocyclylsulfonyl;
R² is a group represented by formula: 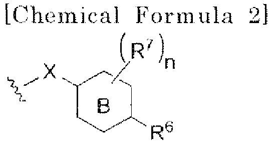
X is a single bond or O,
Ring B is a benzene ring, 6-membered aromatic heterocycle, non-aromatic carbocycle or 6-membered non-aromatic heterocycle,
R⁶ is halogen, hydroxy, cyano, formyl, carboxy, substituted or unsubstitute d alkyl, substituted or unsubstituted alkenyl, substituted or unsubstituted alkyny l, substituted or unsubstituted amino, substituted or unsubstituted alkyloxy, subs tituted or unsubstituted alkenyloxy, substituted or unsubstituted alkynyloxy, sub stituted or unsubstituted alkylthio, substituted or unsubstituted alkenylthio, subs tituted or unsubstituted alkynylthio, substituted or unsubstituted alkylcarbonyl, s ubstituted or unsubstituted alkenylcarbonyl, substituted or unsubstituted alkynyl, carbonyl, substituted or unsubstituted alkyloxycarbonyl, substituted or unsubstitu ted alkenyloxycarbonyl, substituted or unsubstituted alkynyloxycarbonyl, substitut ed or unsubstituted carbamoyl, substituted or unsubstituted sulfamoyl, substitute d or unsubstituted alkylsulfinyl, substituted or unsubstituted alkenylsulfinyl, sub stituted or unsubstituted alkynylsulfinyl, substituted or unsubstituted alkylsulfon yl, substituted or unsubstituted alkenylsulfonyl or substituted or unsubstituted a lkynylsulfonyl,
R⁷ are each independently, halogen, hydroxy, cyano, formyl, carboxy, substi tuted or unsubstituted alkyl, substituted or unsubstituted alkenyl, substituted or unsubstituted, alkynyl, substituted or unsubstituted amino, substituted or unsub stituted alkyloxy, substituted or unsubstituted alkenyloxy, substituted or unsubst ituted alkynyloxy, substituted or unsubstituted alkylthio, substituted or unsubstit uted alkenylthio, substituted or unsubstituted alkynylthio, substituted or unsubst ituted alkylcarbonyl , substituted or unsubstituted alkenylcarbonyl, substituted o r unsubstituted alkynylcarbanyl, substituted or unsubstituted alkyloxycarbonyl, s ubstituted or unsubstituted alkenyloxycarbonyl, substituted or unsubstituted alky nyloxycarbonyl, substituted or unsubstituted carbamoyl , substituted or unsubstit uted sulfamoyl, substituted or unsubstituted alkylsulfinyl, substituted or unsubst ituted alkenylsulfinyl, substituted or unsubstituted alkynylsulfinyl, substituted or unsubstituted alkylsulfonyl, substituted or unsubstituted alkenylsulfonyl or subs tituted or unsubstituted alkynylsulfonyl,
n is integer of 0 to 4,
R³ is substituted or unsubstituted alkyl, substituted or unsubstituted alken yl, substituted or unsubstituted alkynyl, substituted or unsubstituted aromatic ca rbocyclyl, substituted or unsubstituted aromatic heterocyclyl, substituted or unsu bstituted non-aromatic carbocyclyl, substituted or unsubstituted non-aromatic het erocyclyl substituted or unsubstituted alkylcarbonyl, substituted or unsubstituted alkenylcarbonyl, substituted or unsubstituted alkynylcarbonyl, substituted or un substituted aromatic carbocyclylcarbonyl, substituted or unsubstituted aromatic h eterocyclylcarbonyl, substituted or unsubstituted non-aromatic carbocyclylcarbonyl, substituted or unsubstituted non-aromatic heterocyclylcarbonyl, substituted or u unsubstituted alkylsulfonyl, substituted or unsubstituted alkenylsulfonyl, substitut ed or unsubstituted alkynylsulfonyl, substituted or unsubstituted aromatic carboc yclylsulfonyl, substituted or unsubstituted aromatic heterocyclylsulfonyl, substitut ed or unsubstituted non-aromatic carbocyclylsulfonyl or substituted or unsubstitu ted non-aromatic heterocyclylsulfonyl,
R^{4a} is halogen, cyano, formyl, carboxy, substituted or unsubstituted alkyl, substituted or unsubstituted alkenyl, substituted or unsubstituted alkynyl, substi tuted or unsubstituted non-aromatic carbocyclyl, substituted or unsubstituted aro matic carbocyclyl, substituted or unsubstituted non-aromatic heterocyclyl, substit uted or unsubstituted aromatic heterocyclyl, substituted or unsubstituted amino, substituted or unsubstituted alkyloxy, substituted or unsubstituted substituted al kenyloxy, substituted or unsubstituted alkynyloxy, substituted or unsubstituted n on-aromatic carbocyclyloxy, substituted or unsubstituted aromatic carbocyclyloxy, substituted or unsubstituted non-aromatic heterocyclyloxy, substituted or unsubst ituted aromatic heterocyclyloxy, substituted or unsubstituted alkylthio, substitute d or unsubstituted alkenylthio, substituted or unsubstituted alkynylthio, substitu ted or unsubstituted non-aromatic carbocyclyl, substituted or unsubstituted arom atic carbocyclylthio, substituted or unsubstituted non-aromatic heterocyclylthio, s ubstituted or unsubstituted aromatic heterocyclylthio, substituted or unsubstitute d alkylcarbonyl, substituted or unsubstituted alkenylcarbonyl, substituted or uns substituted alkynylcarbonyl, substituted or unsubstituted non-aromatic carbocyclylc arbonyl, substituted or unsubstituted aromatic carbocyclylcarbonyl, substituted or unsubstituted non-aromatic heterocyclylcarbonyl, substituted or unsubstituted, ar omatic heterocyclylcarbonyl, substituted or unsubstituted alkyloxycarbonyl, substi tuted or unsubstituted alkenyloxycarbonyl, substituted or unsubstituted, alkynylox ycarbonyl, substituted or unsubstituted non-aromatic carbocyclyloxycarbonyl, subs tituted or unsubstituted aromatic carbocyclyloxycarbonyl, substituted or unsubstit uted non-aromatic heterocyclyloxycarbonyl, substituted or unsubstituted aromatic heterocyclyoxycarbonyl, substituted or unsubstituted carbamoyl, substituted or un substituted sulfamoyl, a substituted or unsubstituted alkylsulfinyl, substituted or unsubstituted alkenylsulfinyl substituted, substituted or unsubstituted alkynylsu lfinyl, substituted or unsubstituted non-aromatic carbocyclylsulfinyl, substituted o r unsubstituted aromatic carbocyclylsulfinyl, substituted or unsubstituted non-aro matic heterocyclylsulfinyl, substituted or unsubstituted aromatic heterocyclylsulfi nyl, substituted or unsubstituted alkylsulfonyl, substituted or unsubstituted alke nylsulfonyl, substituted or unsubstituted alkynylsulfonyl, substituted or unsubstit uted non-aromatic carbocyclylsulfonyl, substituted or unsubstituted aromatic carb ocyclylsulfonyl, non-aromatic heterocyclylsulfonyl or substituted or unsubstituted aromatic heterocyclylsulfonyl,
R^{4c} is O or N (R⁹),
R⁹ is hydrogen, substituted or unsubstituted alkyl or substituted or unsubs tituted alkyloxy,
R⁵ is a group represented by formula: R¹¹-(C(R^{10a})(R^{10b}))m-
wherein
R^{10a} are each independently hydrogen, halogen or substituted or unsubstitu ted alkyl,
R^{10b} are each independently hydrogen, halogen or substituted or unsubstitu ted alkyl, or
R^{10a} and R^{10b} attached to the same carbon may be taken together to form substituted or unsubstituted carbocycle or substituted or unsubstituted heterocycl e,
m is integer of 1 to 6,
R¹¹ is hydrogen, carboxy, substituted or unsubstituted aromatic carbocyclyl, substituted or unsubstituted aromatic heterocyclyl, substituted or unsubstituted non-aromatic carbocyclyl, substituted or unsubstituted non aromatic heterocyclyl, substituted or unsubstituted aromatic carbocyclylcarbonyl, substituted or unsubsti tuted aromatic heterocyclylcarbonyl, substituted or unsubstituted non-aromatic ca rbocyclylcarbonyl, substituted or unsubstituted non-aromatic heterocyclylcarbonyl, substituted or unsubstituted alkyloxycarbonyl, substituted or unsubstituted alke nyloxycarbonyl, substituted or unsubstituted alkynyloxycarbonyl, substituted or u nsubstituted aromatic carbocyclyloxycarbonyl, substituted or unsubstituted aromat ic heterocyclyloxycarbonyl, substituted or unsubstituted non-aromatic carbocyclylo xycarbonyl, substituted or unsubstituted aromatic heterocyclyloxycarbonyl unsubst ituted, substituted or unsubstituted carbamoyl, substituted or unsubstituted sulfa moyl, or substituted or unsubstituted amino,
the one of the two broken lines represent the existence and the other repr esent the non-existence,
R¹⁵ is substituted or unsubstituted aromatic carbocyclyl, substituted or uns ubstituted aromatic heterocyclyl, substituted or unsubstituted non-aromatic carbo cyclyl or substituted or unsubstituted non-aromatic heterocyclyl,
the partial structure represented by formula: 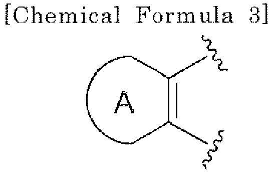 is 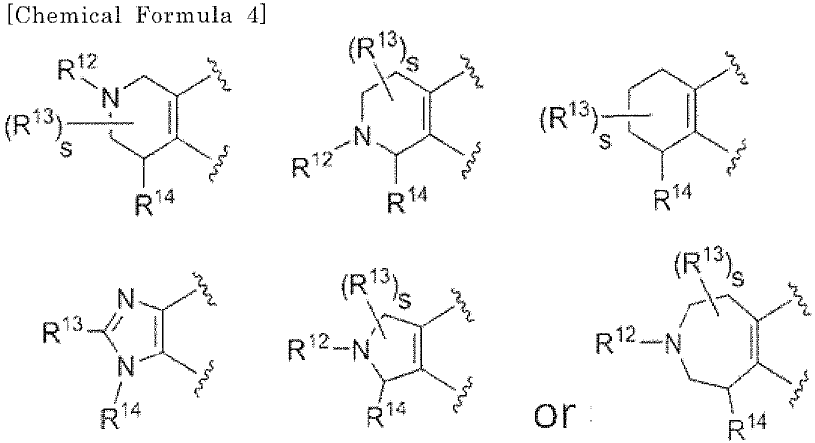
R¹² is hydrogen, substituted or unsubstituted alkyl, substituted or unsubsti tuted alkenyl, substituted or unsubstituted alkynyl, substituted or unsubstituted aromatic carbocyclyl, substituted or unsubstituted aromatic heterocyclyl, substitut ed or unsubstituted non-aromatic carbocyclyl, substituted or unsubstituted non-ar omatic heterocyclyl, substituted or unsubstituted alkylcarbonyl, substituted or un substituted alkenylcarbonyl, substituted or unsubstituted alkynylcarbonyl, substit uted or unsubstituted non-aromatic carbocyclylcarbonyl, substituted or unsubstitu ted aromatic carbocyclylcarbonyl, substituted or unsubstituted non-aromatic heter ocyclylcarbonyl, substituted or unsubstituted aromatic heterocyclylcarbonyl, substi tuted or unsubstituted alkyloxycarbonyl, substituted or unsubstituted alkenyloxyc arbonyl, substituted or unsubstituted alkynyloxycarbonyl, substituted or unsubstit uted non-aromatic carbocyclyloxycarbonyl, substituted or unsubstituted aromatic c arbocyclyloxycarbonyl, substituted or unsubstituted non-aromatic heterocyclyloxyca rbonyl, substituted or unsubstituted aromatic heterocyclyloxycarbonyl, substituted or unsubstituted carbamoyl, substituted or unsubstituted alkylsulfonyl, substitut ed or unsubstituted alkenylsulfonul, substituted or unsubstituted alkynylsulfonyl, substituted or unsubstituted non-aromatic carbocyclylsulfonyl, substituted or uns ubstituted aromatic carbocyclylsulfonyl, substituted or unsubstituted non-aromatic heterocyclylsulfonyl, substituted or unsubstituted aromatic heterocyclylsulfonyl o r substituted or unsubstituted sulfamoyl,
R¹³ is each independently hydroxy, halogen, cyano, substituted or unsubst uted alkyl, substituted or unsubstituted alkenyl or substituted or unsubstituted alkynyl,
two R¹³ attached to the same carbon atom may be taken together to form oxo, non-aromatic carbocycle or substituted or unsubstituted aromatic heterocycle or substituted or unsubstituted non-aromatic heterocycle, or
two R¹³ attached to the different carbon atom may be taken together to fo rm non-aromatic carbocycle or substituted or unsubstituted non-aromatic heterocy cle,
R¹⁴ is C1-C2 alkyl substituted with one or more substituents selected from Substituent group A consisting of substituted or unsubstituted alkyloxy, substit uted or unsubstituted alkenyloxy, substituted or unsubstituted alkynyloxy, substi tuted or unsubstituted alkylthio, substituted or unsubstituted alkenylthio, substit uted or unsubstituted substituted alkynylthio, substituted or unsubstituted non-a romatic carbocyclyloxy, substituted or unsubstituted aromatic carbocyclyloxy, subs tituted or unsubstituted non-aromatic heterocyclyloxy, substituted or unsubstitute d aromatic heterocyclyloxy, substituted or unsubstituted non-aromatic carbocyclylt hio, substituted or unsubstituted aromatic carbocyclylthio, substituted or unsubst ituted non-aromatic heterocyclylthio, substituted or unsubstituted aromatic hetero cyclylthio and substituted or unsubstituted amino,
substituted or unsubstituted C3-C10 alkyl, substituted or unsubstituted C 3-C10 alkenyl, substituted or unsubstituted C3-C10 alkynyl, substituted or unsub stituted non-aromatic carbocyclyl, substituted or unsubstituted aromatic carbocycl yl, substituted or unsubstituted non-aromatic heterocyclyl, substituted or unsubst ituted aromatic heterocyclyl, substituted or unsubstituted alkyloxy, substituted or unsubstituted alkenyloxy, substituted or unsubstituted alkynyloxy or substituted or unsubstituted amino,
s is integer of 0 to 4,
(b-1) when the partial structure represented by formula: 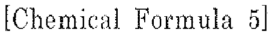 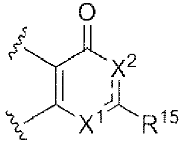 is a partial structure represented by formula: 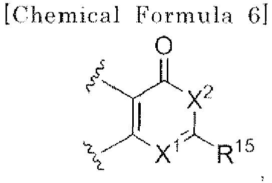
X¹ is N or C (R¹⁶),
R¹⁶ is hydrogen, halogen, substituted or unsubstituted alkyl, substituted or unsubstituted alkenyl, or substituted or unsubstituted alkynyl,
X² is N (R¹⁷) or C (R¹⁸) (R¹⁹),
R¹⁷ is hydrogen, substituted or unsubstituted alkyl, substituted or unsubsti tuted alkenyl or substituted or unsubstituted alkynyl
R¹⁸ and R¹⁹ are each independently hydrogen, hydroxy, halogen, cyano, sub stituted or unsubstituted alkyl, substituted or unsubstituted alkenyl, substituted or unsubstituted alkynyl, substituted or unsubstituted alkyloxy, substituted or u nsubstituted alkenyloxy, substituted or unsubstituted alkynyloxy or a substituted or unsubstituted amino,
(b-2) when the partial structure represented by formula: 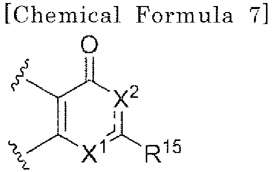 is a partial structure represented by formula: 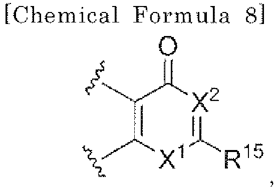
X¹ is O,
X² is N or C (R²⁰),
R²⁰ is hydrogen, hydroxy, halogen, cyano, substituted or unsubstituted alky 1, substituted or unsubstituted alkenyl, substituted or unsubstituted alkynyl, sub stituted or unsubstituted alkyloxy, substituted or unsubstituted alkenyloxy, subst ituted or substituted or unsubstituted alkynyloxy or a substituted or unsubstitut ed amino,
provided that the following compounds (i) to (v) are excluded.
(i) the compounds represented by formula(Ib), wherein (a) R¹ is hydrogen a nd R¹¹ is carboxy, unsubstituted methyloxycarbonyl or unsubstituted ethyloxycarb onyl, and the compounds wherein (b) R¹ is hydrogen, R^{4a} is unsubstituted methyl oxymethyl or unsubstituted methyl, and R¹¹ is a substituted or unsubstituted ph enyl,
(ii) the compounds represented by formula(Ic), wherein (a) R² is unsubstitu ted methylphenyl, and R³ is unsubstituted methylthiophenyl, and the compounds wherein (b) R³ is unsubstituted methyl, and R¹¹ is piperazinyl substituted with unsubstituted naphthyl or unsubstituted isoquinolinyl,
(iii) the compounds represented by (iii) (Ia), wherein R¹ is hydrogen or cy ano, R² is p- unsubstituted methyloxyphenyl, and R^{4a} is unsubstituted methyl,
(iv) the compounds represented by formula(II), wherein the partial structu re represented by formula: 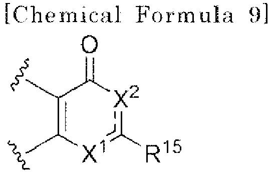 is a partial structure represented by formula: 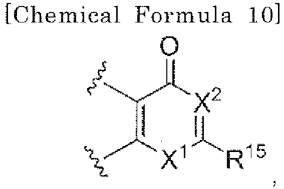
X¹ is N,
X² is N(R¹⁷),
R¹⁷ is hydrogen,
R¹⁵ is substituted or unsubstituted phenyl or substituted or unsubstituted pyridyl,
the partial structure represented by formula: 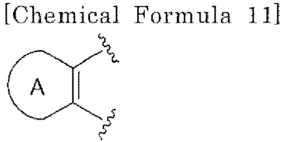 is a partial structure represented by formula: 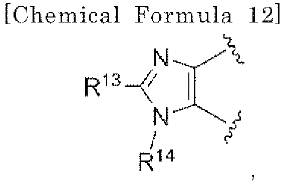
R¹³ is unsubstituted alkyl or substituted or unsubstituted phenylmethyl, a nd
R¹⁴ is unsubstituted propyl or unsubstituted phenylamino,
(v) the compounds represented as follows:

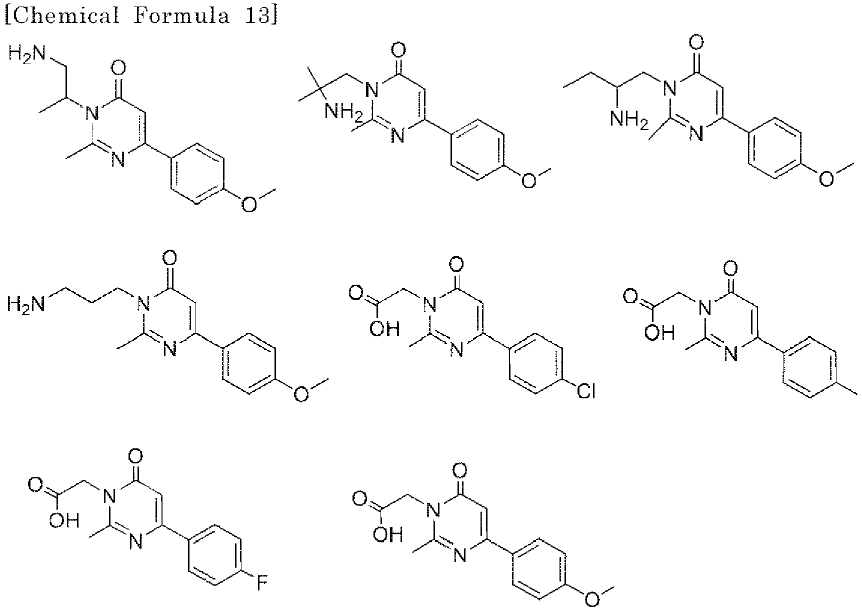

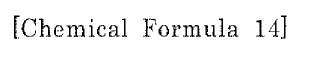

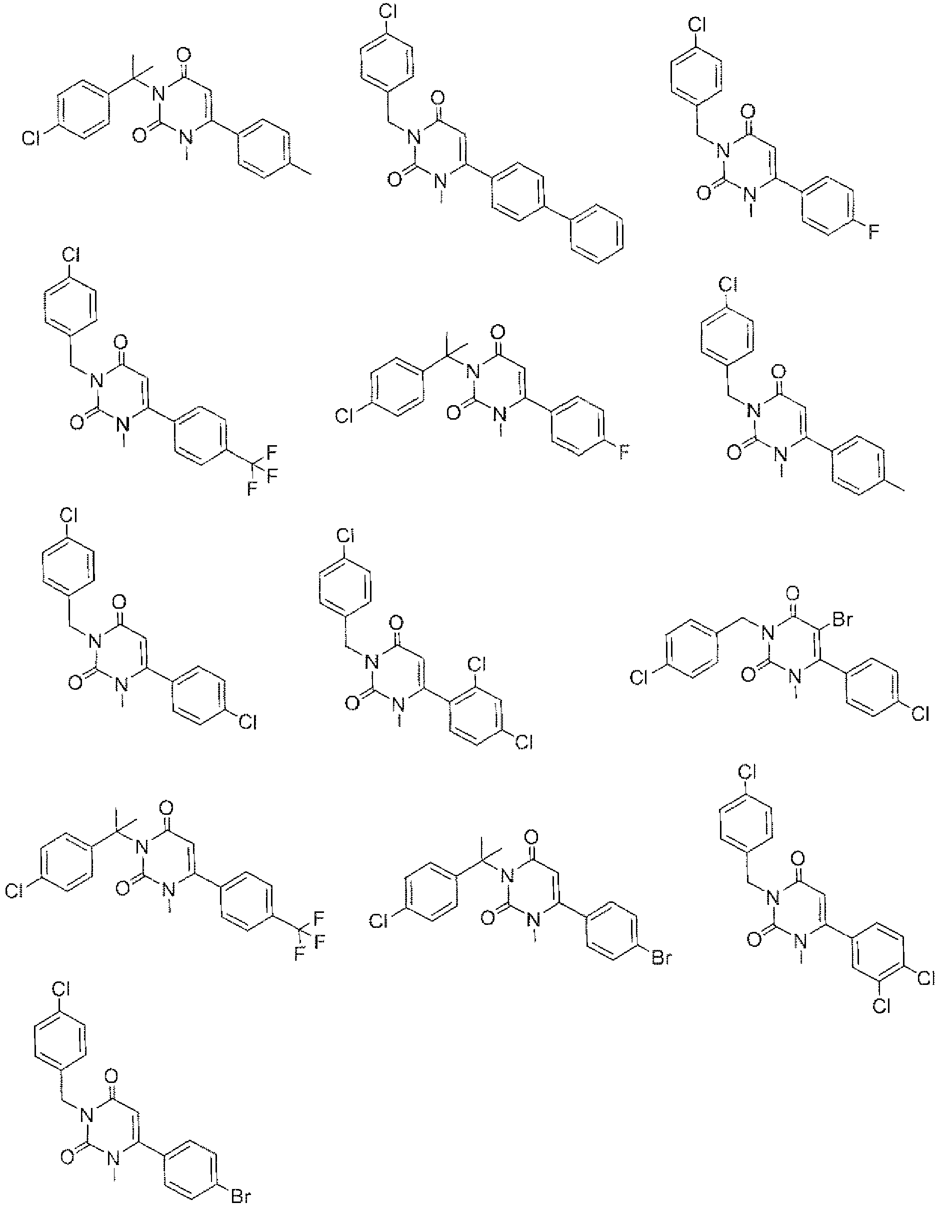

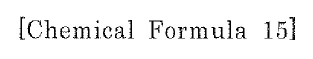

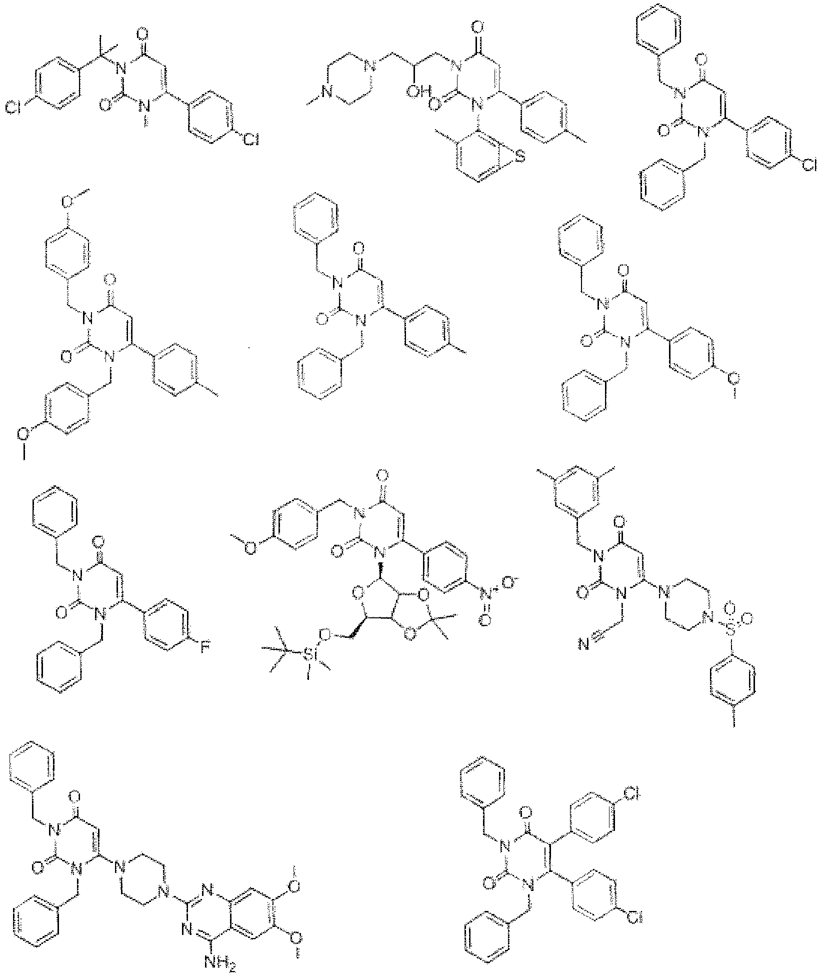

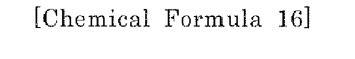

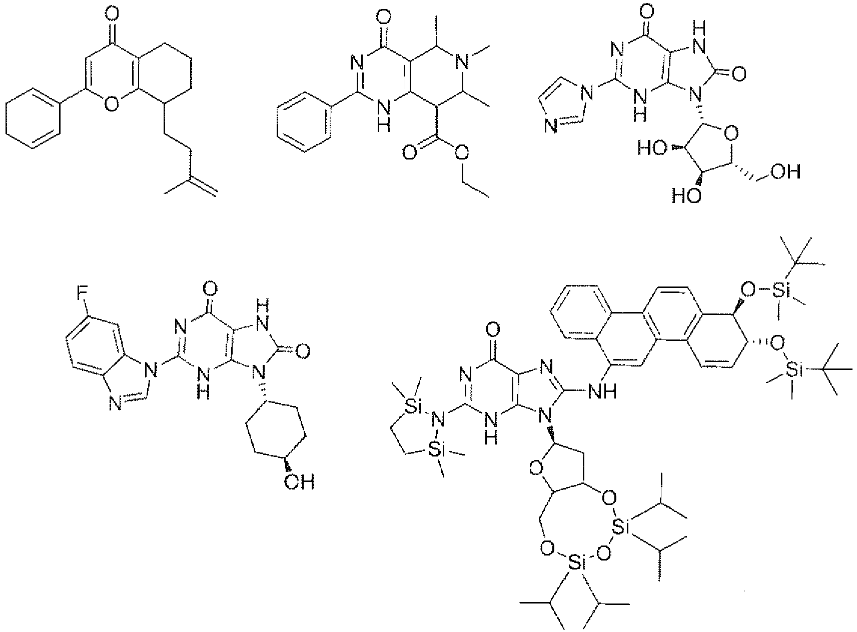

2. The compound according to claim 1, represented by formula (Ia), or its phar maceutically acceptable salt.

3. The compound according to claim 1, represented by formula (Ib), or its phar maceutically acceptable salt.

4. The compound according to claim 1, represented by formula (Ic), or its phar maceutically acceptable salt.

5. The compound according to claim 2, wherein R¹ is hydrogen or halogen, or it s pharmaceutically acceptable salt.

6. The compound according to claims 2 or 5, wherein R^{4a} is substituted or unsu bstituted alkyl, substituted or unsubstituted alkenyl, substituted or unsubstituted alkynyl, substituted or unsubstituted amino, substituted or unsubstituted alkylo xy, substituted or unsubstituted alkenyloxy, substituted or unsubstituted alkynyl oxy, substituted or unsubstituted alkylthio, substituted or unsubstituted alkenylt hio, or substituted or unsubstituted alkynylthio, or its pharmaceutically acceptab le salt.

7. The compound according to claim 6, wherein R^{4a} is alkyl optionally substitut ed with one or more substitutent(s) selected from the Substituent group α (cyano , halogen, substituted or unsubstituted alkyloxy, substituted or unsubstituted alk enyloxy, substituted or unsubstituted alkynyloxy, substituted or unsubstituted no n-aromatic carbocyclylalkyloxy, substituted or unsubstituted aromatic carbocyclyla lkyloxy, substituted or unsubstituted non-aromatic heterocyclylalkyloxy and subst ituted or unsubstituted aromatic heterocyclylalkyloxy), alkenyl optionally substitu ted with one or more substitutent(s) selected from the Substituent group α, alkyn yl optionally substituted with one or more substitutent(s) selected from the Subs tituent group α, alkylamino optionally substituted with one or more substituent( s) selected from the Substituent group α, alkenylamino optionally substituted wit h one or more substitutent(s) selected from the Substituent group α, alkynylamin o optionally substituted with one or more substitutent(s) selected from the Subst ituent group α, alkyloxy optionally substituted with one or more substitutent(s) s elected from the Substituent group α, alkenyloxy optionally substituted with one or more substitutent(s) selected from the Substituent group α, alkynyloxy optiona lly substituted with one or more substitutent(s) selected from the Substituent gr oup α, alkylthio optionally substituted with one or more substitutent(s) selected f rom the Substituent group α, alkynylthio optionally substituted with one or more substitutent(s) selected from the Substituent group α, alkynylthio optionally sub stituted with one or more substitutent(s) selected from the Substituent group α, or its pharmaceutically acceptable salt.

8. The compound according to any one of claims 2 to 7, wherein R¹¹ is carboxy, substituted or unsubstituted aromatic carbocyclyl, substituted or unsubstituted a romatic heterocyclyl, substituted or unsubstituted non-aromatic carbocyclyl, substi tuted or unsubstituted non-aromatic heterocyclyl, substituted or unsubstituted ar omatic carbocyclylcarbonyl, substituted or unsubstituted aromatic heterocyclylcarb onyl, substituted or unsubstituted non-aromatic carbocyclylcarbonyl, substituted o r unsubstituted non-aromatic heterocyclylcarbonyl, substituted or unsubstituted al kyloxycarbonyl, substituted or unsubstituted alkenyloxycarbonyl, substituted or u unsubstituted alkynyloxycarbonyl, substituted or unsubstituted aromatic carbocycly loxycarbonyl, substituted or unsubstituted aromatic heterocyclyloxycarbonyl, subst ituted or unsubstituted non-aromatic carbocyclyloxycarbonyl, substituted or unsub stituted non-aromatic heterocyclyloxycarbonyl, substituted or unsubstituted carba moyl or substituted or unsubstituted sulfamoyl, its pharmaceutically acceptable s alt.

9. The compound according to claim 8, wherein R¹¹ is substituted or unsubstitu ted aromatic carbocyclyl, substituted or unsubstituted aromatic heterocyclyl, subs tituted or unsubstituted non-aromatic carbocyclyl, or substituted or unsubstituted non-aromatic heterocyclyl, or its pharmaceutically acceptable salt.

10. The compound according to claim 9, wherein R¹¹ is aromatic carbocyclyl sub stituted with one or more substitutent(s) selected from the Substituent group β ( carboxy, substituted or unsubstituted alkylcarbonyl, substituted or unsubstituted alkenylcarbonyl, substituted or unsubstituted alkynylcarbonyl, substituted or uns ubstituted non-aromatic carbocyclylcarbonyl, substituted or unsubstituted aromatic c carbocyclycarbonyl, substituted or unsubstituted non-aromatic heterocyclylcarbo nyl, substituted or unsubstituted aromatic heterocyclylcarbonyl, substituted or un substituted alkyloxycarbonyl, substituted or unsubstituted alkenyloxycarbonyl, su bstituted or unsubstituted alkynyloxycaarbonyl, substituted or unsubstituted non-a romatic carbocyclyloxycarbonyl, substituted or unsubstituted aromatic carbocyclylo xycarbonyl, substituted or unsubstituted non-aromatic heterocyclyloxycarbonyl, su bstituted or unsubstituted aromatic heterocyclyloxycarbonyl, substituted or unsub stituted carbamoyl, substituted or unsubstituted sulfamoyl, a substituted or unsu bstituted alkylsulfonyl, substituted or unsubstituted alkenylsulfonyl, substituted o r unsubstituted alkynylsulfonyl, substituted or unsubstituted non-aromatic carboc yclylsulfonyl, and substituted or unsubstituted aromatic carbocyclylsulfonyl), aro matic heterocyclyl substituted with one or more substitutent(s) selected from the Substituent group β, non-aromatic carbocyclyl substituted with one or more sub stitutent(s) selected from the Substituent group β, or non-aromatic heterocyclyl s ubstituted with one or more substitutent(s) selected from the Substituent group β, or its pharmaceutically acceptable salt.

11. The compound according to claim 9, wherein R¹¹ is substituted or unsubst uted aromatic carbocyclylcarbonyl, substituted or unsubstituted aromatic heterocy clylcarbonyl, substituted or unsubstituted non-aromatic carbocyclylcarbonyl, substi tuted or unsubstituted non-aromatic heterocyclylcarbonyl, substituted or unsubsti tuted alkyloxycarbonyl, substituted or unsubstituted alkenyloxycarbonyl, substitut ed or unsubstituted alkynyloxycarbonyl, substituted or unsubstituted aromatic ca rbocyclyloxycarbonyl, substituted or unsubstituted aromatic heterocyclyloxycarbon yl, substituted or unsubstituted non-aromatic carbocyclyloxycarbonyl, substituted or unsubstituted non-aromatic heterocyclyloxycarbonyl, substituted or unsubstitut ed carbamoyl or substituted or unsubstituted sulfamoyl, or its pharmaceutically acceptable salt.

12. The compound according to claim 9, wherein R¹¹ is carbamoyl optionally sub stituted with one or more substitutent(s) selected from the Substituent group Y ( substituted or unsubstituted alkyl, substituted or unsubstituted alkenyl, substitut ed or unsubstituted alkynyl, substituted or unsubstituted aromatic carbocyclyl, s ubstituted or unsubstituted aromatic heterocyclyl, substituted or unsubstituted n on-aromatic carbocyclyl and substituted or unsubstituted non-aromatic heterocycly 1), or its pharmaceutically acceptable salt.

13. The compound according to claim 3, wherein R¹ is hydrogen, R^{4a} is substitu ted or unsubstituted C2-C10 alkyl, substituted or unsubstituted alkenyl or substi tuted or unsubstituted alkynyl, or its pharmaceutically acceptable salt.

14. The compound according to claim 5, wherein R¹ is hydrogen, R³ is substitut ed methyl, substituted or unsubstituted C2-C10 alkyl, substituted or unsubstitute d alkenyl or substituted or unsubstituted alkynyl, or its pharmaceutically accept able salt.

15. The compound according to claim 1, represented by formula (II), or its phar maceutically acceptable salt.

16. A pharmaceutical composition comprising the compound according to any on e of claims 1 to 15, or its pharmaceutically acceptable salt as an active ingredie nt.

17. The pharmaceutical composition according to claim 16, which is an autotaxi n inhibitor.

18. A method for the prevention or treatment of a disease related to autotaxin , which is **characterized by** administering the compound according to any one of claims 1 to 25, or its pharmaceutically acceptable salt.

19. The compound according to any one of claims 1 to 15, or its pharmaceutical ly acceptable salt for the prevention or treatment of a disease related to autotax in.

20. Use of the compound according to any one of claims 1 to 15, or its pharma ceutically acceptable salt for the manufacture of a medicament for the preventio n or treatment of a disease related to autotaxin.
